# EUROPEAN PATENT SPECIFICATION

(11) **EP 1 427 750 B1**
(45) Date of publication and mention of the grant of the patent: **08.12.2010**
(21) Application number: 02757486.2
(22) Date of filing: 30.08.2002
(51) Int. Cl.: C07K 1/00, C07K 14/00, C07K 17/00, C07H 21/04, C12N 15/00, C12N 15/09, C12N 15/63, C12N 15/70, C12N 15/74, C12N 5/00, C12N 5/02, C12N 15/62

(54) **MODIFIED TRANSFERRIN FUSION PROTEINS**
MODIFIZIERTE TRANSFERRIN-FUSIONSPROTEINE
PROTEINES DE FUSION DE TRANSFERINE MODIFIEES

(30) Priority: 30.08.2001 US 315745 P; 30.11.2001 US 334059 P
(43) Date of publication of application: 16.06.2004
(73) Proprietor: Biorexis Pharmaceutical Corporation, New York, NY 10017-5755 (US)
(72) Inventor: PRIOR, Christopher P., King of Prussia, PA 19406-2675 (US)
(74) Representative: Ford, Timothy James
(86) International application number: PCT/US2002/027637
(87) International publication number: WO 2003/020746

(56) References cited:
- US-A- 5 986 067
- US-A- 6 027 921
- HOEFKENS P ET AL: "Influence of transferrin glycans on receptor binding and iron-donation" GLYCOCONJUGATE JOURNAL, vol. 14, no. 2, 1997, pages 289-295, XP009057458 ISSN: 0282-0080
- NEWTON D.L. ET AL.: 'Antitransferrin receptor antibody-RNase fusion protein expressed in the mammary gland of transgenic mice' J. IMMUNOL. METH. vol. 231, 1999, pages 159 - 167, XP002934495
- PARK E. ET AL.: 'Production and characterization of fusion proteins containing transferrin and nerve growth factor' J. DRUG TAR. vol. 6, no. 1, 1998, pages 53 - 64, XP002960815
- PRINCE L.S. ET AL.: 'Efficient endocytosis of the cystic fibrosis transmembrane conductance regulator requires a tyrosine-based signal' J. BIOL. CHEM. vol. 274, no. 6, 05 February 1999, pages 3602 - 3609, XP002960816
- SHIN S.U. ET AL.: 'Transferrin-antibody fusion proteins are effective in brain targeting' PROC. NATL. ACAD. SCI. USA vol. 92, 1995, pages 2820 - 2824, XP001126925

### SEQUENCE LISTING

<110> Prior, Christopher P.
<120> Modified Transferrin Fusion Proteins
<130> 54710-5001-WO
<140>
   <141>
<150> US 60/315,745
   <151> 2001-08-30
<150> US 60/334,059
   <151> 2001-11-30
<160> 30
<170> PatentIn Ver. 2.1
<210> 1
   <211> 2318
   <212> DNA
   <213> Homo sapiens
<220>
   <221> CDS
   <222> (51)..(2147)
   <223> GenBank No. NM_001063, transferrin gene and protein
<220>
   <221> sig_peptide
   <222> (51)..(107)
<400> 1
   gcacagaagc gagtccgact gtgctcgctg ctcagcgccg cacccggaag atg agg     56
                                                          Met Arg
                                                            1
   ctc gcc gtg gga gcc ctg ctg gtc tgc gcc gtc ctg ggg ctg tgt ctg   104
   Leu Ala Val Gly Ala Leu Leu Val Cys Ala Val Leu Gly Leu Cys Leu
             5                  10                  15
   gct gtc cct gat aaa act gtg aga tgg tgt gca gtg tcg gag cat gag   152
   Ala Val Pro Asp Lys Thr Val Arg Trp Cys Ala Val Ser Glu His Glu
        20                  25                  30
   gcc act aag tgc cag agt ttc cgc gac cat atg aaa agc gtc att cca   200
   Ala Thr Lys Cys Gln Ser Phe Arg Asp His Met Lys Ser Val Ile Pro
    35                  40                  45                  50
   tcc gat ggt ccc agt gtt gct tgt gtg aag aaa gcc tcc tac ctt gat   248
   Ser Asp Gly Pro Ser Val Ala Cys Val Lys Lys Ala Ser Tyr Leu Asp
                    55                  60                  65
   tgc atc agg gcc att gcg gca aac gaa gcg gat gct gtg aca ctg gat   296
   Cys Ile Arg Ala Ile Ala Ala Asn Glu Ala Asp Ala Val Thr Leu Asp
                70                  75                  80
   gca ggt ttg gtg tat gat gct tac ctg gct ccc aat aac ctg aag cct   344
   Ala Gly Leu Val Tyr Asp Ala Tyr Leu Ala Pro Asn Asn Leu Lys Pro
           85                  90                  95
   gtg gtg gca gag ttc tat ggg tca aaa gag gat cca cag act ttc tat   392
   Val Val Ala Glu Phe Tyr Gly Ser Lys Glu Asp Pro Gln Thr Phe Tyr
       100                 105                 110
   tat gct gtt gct gtg gtg aag aag gat agt ggc ttc cag atg aac cag   440
   Tyr Ala Val Ala Val Val Lys Lys Asp Ser Gly Phe Gln Met Asn Gln
   115                 120                 125                 130
   ctt cga ggc aag aag tcc tgc cac acg ggt cta ggc agg tcc gct ggg   488
   Leu Arg Gly Lys Lys Ser Cys His Thr Gly Leu Gly Arg Ser Ala Gly
                   135                 140                 145
   tgg aac atc ccc ata ggc tta ctt tac tgt gac tta cct gag cca cgt   536
   Trp Asn Ile Pro Ile Gly Leu Leu Tyr Cys Asp Leu Pro Glu Pro Arg
               150                 155                 160
   aaa cct ctt gag aaa gca gtg gcc aat ttc ttc tcg ggc agc tgt gcc   584
   Lys Pro Leu Glu Lys Ala Val Ala Asn Phe Phe Ser Gly Ser Cys Ala
           165                 170                 175
   cct tgt gcg gat ggg acg gac ttc ccc cag ctg tgt caa ctg tgt cca   632
   Pro Cys Ala Asp Gly Thr Asp Phe Pro Gln Leu Cys Gln Leu Cys Pro
       180                 185                 190
   ggg tgt ggc tgc tcc acc ctt aac caa tac ttc ggc tac tcg gga gcc   680
   Gly Cys Gly Cys Ser Thr Leu Asn Gln Tyr Phe Gly Tyr Ser Gly Ala
   195                 200                 205                 210
   ttc aag tgt ctg aag gat ggt gct ggg gat gtg gcc ttt gtc aag cac   728
   Phe Lys Cys Leu Lys Asp Gly Ala Gly Asp Val Ala Phe Val Lys His
                   215                 220                 225
   tcg act ata ttt gag aac ttg gca aac aag gct gac agg gac cag tat   776
   Ser Thr Ile Phe Glu Asn Leu Ala Asn Lys Ala Asp Arg Asp Gln Tyr
               230                 235                 240
   gag ctg ctt tgc ctg gac aac acc cgg aag ccg gta gat gaa tac aag   824
   Glu Leu Leu Cys Leu Asp Asn Thr Arg Lys Pro Val Asp Glu Tyr Lys
           245                 250                 255
   gac tgc cac ttg gcc cag gtc cct tct cat acc gtc gtg gcc cga agt   872
   Asp Cys His Leu Ala Gln Val Pro Ser His Thr Val Val Ala Arg Ser
       260                 265                 270
   atg ggc ggc aag gag gac ttg atc tgg gag ctt ctc aac cag gcc cag   920
   Met Gly Gly Lys Glu Asp Leu Ile Trp Glu Leu Leu Asn Gln Ala Gln
   275                 280                 285                 290
   gaa cat ttt ggc aaa gac aaa tca aaa gaa ttc caa cta ttc agc tct   968
   Glu His Phe Gly Lys Asp Lys Ser Lys Glu Phe Gln Leu Phe Ser Ser
                   295                 300                 305
   cct cat ggg aag gac ctg ctg ttt aag gac tct gcc cac ggg ttt tta   1016
   Pro His Gly Lys Asp Leu Leu Phe Lys Asp Ser Ala His Gly Phe Leu
               310                 315                 320
   aaa gtc ccc ccc agg atg gat gcc aag atg tac ctg ggc tat gag tat   1064
   Lys Val Pro Pro Arg Met Asp Ala Lys Met Tyr Leu Gly Tyr Glu Tyr
           325                 330                 335
   gtc act gcc atc cgg aat cta cgg gaa ggc aca tgc cca gaa gcc cca    1112
   Val Thr Ala Ile Arg Asn Leu Arg Glu Gly Thr Cys Pro Glu Ala Pro
       340                 345                 350
   aca gat gaa tgc aag cct gtg aag tgg tgt gcg ctg agc cac cac gag    1160
   Thr Asp Glu Cys Lys Pro Val Lys Trp Cys Ala Leu Ser His His Glu
   355                 360                 365                 370
   agg ctc aag tgt gat gag tgg agt gtt aac agt gta ggg aaa ata gag    1208
   Arg Leu Lys Cys Asp Glu Trp Ser Val Asn Ser Val Gly Lys Ile Glu
                   375                 380                 385
   tgt gta tca gca gag acc acc gaa gac tgc atc gcc aag atc atg aat    1256
   Cys Val Ser Ala Glu Thr Thr Glu Asp Cys Ile Ala Lys Ile Met Asn
               390                 395                 400
   gga gaa gct gat gcc atg agc ttg gat gga ggg ttt gtc tac ata gcg    1304
   Gly Glu Ala Asp Ala Met Ser Leu Asp Gly Gly Phe Val Tyr Ile Ala
           405                 410                 415
   ggc aag tgt ggt ctg gtg cct gtc ttg gca gaa aac tac aat aag agc    1352
   Gly Lys Cys Gly Leu Val Pro Val Leu Ala Glu Asn Tyr Asn Lys Ser
       420                 425                 430
   gat aat tgt gag gat aca cca gag gca ggg tat ttt gct gta gca gtg    1400
   Asp Asn Cys Glu Asp Thr Pro Glu Ala Gly Tyr Phe Ala Val Ala Val
   435                 440                 445                 450
   gtg aag aaa tca gct tct gac ctc acc tgg gac aat ctg aaa ggc aag    1448
   Val Lys Lys Ser Ala Ser Asp Leu Thr Trp Asp Asn Leu Lys Gly Lys
                   455                 460                 465
   aag tcc tgc cat acg gca gtt ggc aga acc gct ggc tgg aac atc ccc    1496
   Lys Ser Cys His Thr Ala Val Gly Arg Thr Ala Gly Trp Asn Ile Pro
               470                 475                 480
   atg ggc ctg ctc tac aat aag atc aac cac tgc aga ttt gat gaa ttt    1544
   Met Gly Leu Leu Tyr Asn Lys Ile Asn His Cys Arg Phe Asp Glu Phe
           485                 490                 495
   ttc agt gaa ggt tgt gcc cct ggg tct aag aaa gac tcc agt ctc tgt    1592
   Phe Ser Glu Gly Cys Ala Pro Gly Ser Lys Lys Asp Ser Ser Leu Cys
       500                 505                 510
   aag ctg tgt atg ggc tca ggc cta aac ctg tgt gaa ccc aac aac aaa    1640
   Lys Leu Cys Met Gly Ser Gly Leu Asn Leu Cys Glu Pro Asn Asn Lys
   515                 520                 525                 530
   gag gga tac tac ggc tac aca ggc gct ttc agg tgt ctg gtt gag aag    1688
   Glu Gly Tyr Tyr Gly Tyr Thr Gly Ala Phe Arg Cys Leu Val Glu Lys
                   535                 540                 545
   gga gat gtg gcc ttt gtg aaa cac cag act gtc cca cag aac act ggg    1736
   Gly Asp Val Ala Phe Val Lys His Gln Thr Val Pro Gln Asn Thr Gly
               550                 555                 560
   gga aaa aac cct gat cca tgg gct aag aat ctg aat gaa aaa gac tat    1784
   Gly Lys Asn Pro Asp Pro Trp Ala Lys Asn Leu Asn Glu Lys Asp Tyr
           565                 570                 575
   gag ttg ctg tgc ctt gat ggt acc agg aaa cct gtg gag gag tat gcg    1832
   Glu Leu Leu Cys Leu Asp Gly Thr Arg Lys Pro Val Glu Glu Tyr Ala
       580                 585                 590
   aac tgc cac ctg gcc aga gcc ccg aat cac gct gtg gtc aca cgg aaa    1880
   Asn Cys His Leu Ala Arg Ala Pro Asn His Ala Val Val Thr Arg Lys
   595                 600                 605                 610
   gat aag gaa gct tgc gtc cac aag ata tta cgt caa cag cag cac cta    1928
   Asp Lys Glu Ala Cys Val His Lys Ile Leu Arg Gln Gln Gln His Leu
                   615                 620                 625
   ttt gga agc aac gta act gac tgc tcg ggc aac ttt tgt ttg ttc cgg    1976
   Phe Gly Ser Asn Val Thr Asp Cys Ser Gly Asn Phe Cys Leu Phe Arg
               630                 635                 640
   tcg gaa acc aag gac ctt ctg ttc aga gat gac aca gta tgt ttg gcc    2024
   Ser Glu Thr Lys Asp Leu Leu Phe Arg Asp Asp Thr Val Cys Leu Ala
           645                 650                 655
   aaa ctt cat gac aga aac aca tat gaa aaa tac tta gga gaa gaa tat    2072
   Lys Leu His Asp Arg Asn Thr Tyr Glu Lys Tyr Leu Gly Glu Glu Tyr
       660                 665                 670
   gtc aag gct gtt ggt aac ctg aga aaa tgc tcc acc tca tca ctc ctg    2120
   Val Lys Ala Val Gly Asn Leu Arg Lys Cys Ser Thr Ser Ser Leu Leu
   675                 680                 685                 690
   gaa gcc tgc act ttc cgt aga cct taa aatctcagag gtagggctgc          2167
   Glu Ala Cys Thr Phe Arg Arg Pro
                   695
   caccaaggtg aagatgggaa cgcagatgat ccatgagttt gccctggttt cactggccca 2227
   agtggtttgt gctaaccacg tctgtcttca cagctctgtg ttgccatgtg tgctgaacaa 2287
   aaaataaaaa ttattattga ttttatattt c                                2318
<210> 2
   <211> 698
   <212> PRT
   <213> Homo sapiens
<400> 2
   Met Arg Leu Ala Val Gly Ala Leu Leu Val Cys Ala Val Leu Gly Leu
    1               5                  10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
                   85                  90                  95
   Lys Pro Val Val Ala Glu Phe Tyr Gly Ser Lys Glu Asp Pro Gln Thr
               100                 105                 110
   Phe Tyr Tyr Ala Val Ala Val Val Lys Lys Asp Ser Gly Phe Gln Met
           115                 120                 125
   Asn Gln Leu Arg Gly Lys Lys Ser Cys His Thr Gly Leu Gly Arg Ser
       130                 135                 140
   Ala Gly Trp Asn Ile Pro Ile Gly Leu Leu Tyr Cys Asp Leu Pro Glu
   145                 150                 155                 160
   Pro Arg Lys Pro Leu Glu Lys Ala Val Ala Asn Phe Phe Ser Gly Ser
                   165                 170                 175
   Cys Ala Pro Cys Ala Asp Gly Thr Asp Phe Pro Gln Leu Cys Gln Leu
               180                 185                 190
   Cys Pro Gly Cys Gly Cys Ser Thr Leu Asn Gln Tyr Phe Gly Tyr Ser
           195                 200                 205
   Gly Ala Phe Lys Cys Leu Lys Asp Gly Ala Gly Asp Val Ala Phe Val
       210                 215                 220
   Lys His Ser Thr Ile Phe Glu Asn Leu Ala Asn Lys Ala Asp Arg Asp
   225                 230                 235                 240
   Gln Tyr Glu Leu Leu Cys Leu Asp Asn Thr Arg Lys Pro Val Asp Glu
                   245                 250                 255
   Tyr Lys Asp Cys His Leu Ala Gln Val Pro Ser His Thr Val Val Ala
               260                 265                 270
   Arg Ser Met Gly Gly Lys Glu Asp Leu Ile Trp Glu Leu Leu Asn Gln
           275                 280                 285
   Ala Gln Glu His Phe Gly Lys Asp Lys Ser Lys Glu Phe Gln Leu Phe
       290                 295                 300
   Ser Ser Pro His Gly Lys Asp Leu Leu Phe Lys Asp Ser Ala His Gly
   305                 310                 315                 320
   Phe Leu Lys Val Pro Pro Arg Met Asp Ala Lys Met Tyr Leu Gly Tyr
                   325                 330                 335
   Glu Tyr Val Thr Ala Ile Arg Asn Leu Arg Glu Gly Thr Cys Pro Glu
               340                 345                 350
   Ala Pro Thr Asp Glu Cys Lys Pro Val Lys Trp Cys Ala Leu Ser His
           355                 360                 365
   His Glu Arg Leu Lys Cys Asp Glu Trp Ser Val Asn Ser Val Gly Lys
       370                 375                 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Val
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
           595                 600                 605
   Arg Lys Asp Lys Glu Ala Cys Val His Lys Ile Leu Arg Gln Gln Gln
       610                 615                 620
   His Leu Phe Gly Ser Asn Val Thr Asp Cys Ser Gly Asn Phe Cys Leu
   625                 630                 635                 640
   Phe Arg Ser Glu Thr Lys Asp Leu Leu Phe Arg Asp Asp Thr Val Cys
                   645                 650                 655
   Leu Ala Lys Leu His Asp Arg Asn Thr Tyr Glu Lys Tyr Leu Gly Glu
               660                 665                 670
   Glu Tyr Val Lys Ala Val Gly Asn Leu Arg Lys Cys Ser Thr Ser Ser
           675                 680                 685
   Leu Leu Glu Ala Cys Thr Phe Arg Arg Pro
       690                 695
<210> 3
   <211> 679
   <212> PRT
   <213> Homo sapiens
<220>
   <223> Mature transferrin protein
<400> 3
   Val Pro Asp Lys Thr Val Arg Trp Cys Ala Val Ser Glu His Glu Ala
    1               5                  10                  15
   Thr Lys Cys Gln Ser Phe Arg Asp His Met Lys Ser Val Ile Pro Ser
               20                  25                  30
   Asp Gly Pro Ser Val Ala Cys Val Lys Lys Ala Ser Tyr Leu Asp Cys
           35                  40                  45
   Ile Arg Ala Ile Ala Ala Asn Glu Ala Asp Ala Val Thr Leu Asp Ala
       50                  55                  60
   Gly Leu Val Tyr Asp Ala Tyr Leu Ala Pro Asn Asn Leu Lys Pro Val
   65                  70                  75                  80
   Val Ala Glu Phe Tyr Gly Ser Lys Glu Asp Pro Gln Thr Phe Tyr Tyr
                   85                  90                  95
   Ala Val Ala Val Val Lys Lys Asp Ser Gly Phe Gln Met Asn Gln Leu
               100                 105                 110
   Arg Gly Lys Lys Ser Cys His Thr Gly Leu Gly Arg Ser Ala Gly Trp
           115                 120                 125
   Asn Ile Pro Ile Gly Leu Leu Tyr Cys Asp Leu Pro Glu Pro Arg Lys
       130                 135                 140
   Pro Leu Glu Lys Ala Val Ala Asn Phe Phe Ser Gly Ser Cys Ala Pro
   145                 150                 155                 160
   Cys Ala Asp Gly Thr Asp Phe Pro Gln Leu Cys Gln Leu Cys Pro Gly
                   165                 170                 175
   Cys Gly Cys Ser Thr Leu Asn Gln Tyr Phe Gly Tyr Ser Gly Ala Phe
               180                 185                 190
   Lys Cys Leu Lys Asp Gly Ala Gly Asp Val Ala Phe Val Lys His Ser
           195                 200                 205
   Thr Ile Phe Glu Asn Leu Ala Asn Lys Ala Asp Arg Asp Gln Tyr Glu
       210                 215                 220
   Leu Leu Cys Leu Asp Asn Thr Arg Lys Pro Val Asp Glu Tyr Lys Asp
   225                 230                 235                 240
   Cys His Leu Ala Gln Val Pro Ser His Thr Val Val Ala Arg Ser Met
                   245                 250                 255
   Gly Gly Lys Glu Asp Leu Ile Trp Glu Leu Leu Asn Gln Ala Gln Glu
               260                 265                 270
   His Phe Gly Lys Asp Lys Ser Lys Glu Phe Gln Leu Phe Ser Ser Pro
           275                 280                 285
   His Gly Lys Asp Leu Leu Phe Lys Asp Ser Ala His Gly Phe Leu Lys
       290                 295                 300
   Val Pro Pro Arg Met Asp Ala Lys Met Tyr Leu Gly Tyr Glu Tyr Val
   305                 310                 315                 320
   Thr Ala Ile Arg Asn Leu Arg Glu Gly Thr Cys Pro Glu Ala Pro Thr
                   325                 330                 335
   Asp Glu Cys Lys Pro Val Lys Trp Cys Ala Leu Ser His His Glu Arg
               340                 345                 350
   Leu Lys Cys Asp Glu Trp Ser Val Asn Ser Val Gly Lys Ile Glu Cys
           355                 360                 365
   Val Ser Ala Glu Thr Thr Glu Asp Cys Ile Ala Lys Ile Met Asn Gly
       370                 375                 380
   Glu Ala Asp Ala Met Ser Leu Asp Gly Gly Phe Val Tyr Ile Ala Gly
   385                 390                 395                 400
   Lys Cys Gly Leu Val Pro Val Leu Ala Glu Asn Tyr Asn Lys Ser Asp
                   405                 410                 415
   Asn Cys Glu Asp Thr Pro Glu Ala Gly Tyr Phe Ala Val Ala Val Val
               420                 425                 430
   Lys Lys Ser Ala Ser Asp Leu Thr Trp Asp Asn Leu Lys Gly Lys Lys
           435                 440                 445
   Ser Cys His Thr Ala Val Gly Arg Thr Ala Gly Trp Asn Ile Pro Met
       450                 455                 460
   Gly Leu Leu Tyr Asn Lys Ile Asn His Cys Arg Phe Asp Glu Phe Phe
   465                 470                 475                 480
   Ser Glu Gly Cys Ala Pro Gly Ser Lys Lys Asp Ser Ser Leu Cys Lys
                   485                 490                 495
   Leu Cys Met Gly Ser Gly Leu Asn Leu Cys Glu Pro Asn Asn Lys Glu
               500                 505                 510
   Gly Tyr Tyr Gly Tyr Thr Gly Ala Phe Arg Cys Leu Val Glu Lys Gly
   515 520 525
   Asp Val Ala Phe Val Lys His Gln Thr Val Pro Gln Asn Thr Gly Gly
   530 535 540
   Lys Asn Pro Asp Pro Trp Ala Lys Asn Leu Asn Glu Lys Asp Tyr Glu
   545 550 555 560
   Leu Leu Cys Leu Asp Gly Thr Arg Lys Pro Val Glu Glu Tyr Ala Asn
   565 570 575
   Cys His Leu Ala Arg Ala Pro Asn His Ala Val Val Thr Arg Lys Asp
   580 585 590
   Lys Glu Ala Cys Val His Lys Ile Leu Arg Gln Gln Gln His Leu Phe
   595 600 605
   Gly Ser Asn Val Thr Asp Cys Ser Gly Asn Phe Cys Leu Phe Arg Ser
   610 615 620
   Glu Thr Lys Asp Leu Leu Phe Arg Asp Asp Thr Val Cys Leu Ala Lys
   625 630 635 640
   Leu His Asp Arg Asn Thr Tyr Glu Lys Tyr Leu Gly Glu Glu Tyr Val
   645 650 655
   Lys Ala Val Gly Asn Leu Arg Lys Cys Ser Thr Ser Ser Leu Leu Glu
   660 665 670
   Ala Cys Thr Phe Arg Arg Pro
   675
<210> 4
   <211> 36
   <212> PRT
   <213> Human immunodeficiency virus
<220>
   <223> Antifusogenic peptide
<400> 4
   Phe Trp Asn Trp Leu Ser Ala Trp Lys Asp Leu Glu Leu Leu Glu Gln
   1 5 10 15
   Glu Asn Lys Glu Gln Gln Asn Gln Ser Glu Glu Ile Leu Ser His Ile
   20 25 30
   Leu Ser Thr Tyr
   35
<210> 5
   <211> 35
   <212> PRT
   <213> Human respiratory syncytial virus
<220>
   <223> Antifusogenic peptide
<400> 5
   Val Tyr Pro Ser Asp Glu Tyr Asp Ala Ser Ile Ser Gln Val Asn Glu
   1               5                   10                  15
   Glu Ile Asn Gln Ala Leu Ala Tyr Ile Arg Lys Ala Asp Glu Leu Leu
               20                  25                  30
   Glu Asn Val
           35
<210> 6
   <211> 51
   <212> PRT
   <213> Human respiratory syncytial virus
<220>
   <223> Antifusogenic peptide
<400> 6
   Ala Val Ser Lys Val Leu His Leu Glu Gly Glu Val Asn Lys Ile Lys
   1               5                   10                  15
   Ser Ala Leu Leu Ser Thr Asn Lys Ala Val Val Ser Leu Ser Asn Gly
               20                  25                  30
   Val Ser Val Leu Thr Ser Lys Val Leu Asp Leu Lys Asn Tyr Ile Asp
           35                  40                  45
   Lys Gln Leu
       50
<210> 7
   <211> 35
   <212> PRT
   <213> Human respiratory syncytial virus
<220>
   <223> Antifusogenic peptide
<400> 7
   Val Phe Pro Ser Asp Glu Phe Asp Ala Ser Ile Ser Gln Val Asn Glu
   1               5                   10                  15
   Lys Ile Asn Gln Ser Leu Ala Phe Ile Arg Glu Ser Asp Glu Leu Leu
               20                  25                  30
   His Asn Val
           35
<210> 8
   <211> 12
   <212> PRT
   <213> Homo sapiens
<220>
   <223> Lactoferrin splice variant sequence
<400> 8
   Glu Asp Cys Ile Ala Leu Lys Gly Glu Ala Asp Ala
   1 5 10
<210> 9
   <211> 27
   <212> DNA
   <213> Artificial Sequence
<220>
   <223> Description of Artificial Sequence: Oligonucleotide for mutagenesis
<400> 9
   gcagaaaact acgataagag cgataat 27
<210> 10
   <211> 27
   <212> DNA
   <213> Artificial Sequence
<220>
   <223> Description of Artificial Sequence: Oligonucleotide for mutagenesis
<400> 10
   ctatttggaa gcgacgtaac tgactgc 27
<210> 11
   <211> 20
   <212> PRT
   <213> Artificial Sequence
<220>
   <223> Description of Artificial Sequence: EPO mimetic peptide
<400> 11
   Gly Gly Thr Tyr Ser Cys His Phe Gly Pro Leu Thr Trp Val Cys Lys
   1 5 10 15
   Pro Gln Gly Gly
   20
<210> 12
   <211> 18
   <212> PRT
   <213> Artificial Sequence
<220>
   <223> Description of Artificial Sequence: EPO mimetic peptide
<400> 12
   Asp Arg Glu Gly Cys Arg Arg Gly Trp Val Gly Gln Cys Lys Ala Trp
    1               5                  10                  15
   Phe Asn
<210> 13
   <211> 108
   <212> DNA
   <213> Artificial Sequence
<220>
   <223> Description of Artificial Sequence: HIV antifusogenic sequences
<220>
   <221> CDS
   <222> (1)..(108)
<400> 13
   tac aca agc tta ata cac tcc tta att gaa gaa tcg caa aac cag caa    48
   Tyr Thr Ser Leu Ile His Ser Leu Ile Glu Glu Ser Gln Asn Gln Gln
    1               5                  10                  15
   gaa aag aat gaa caa gaa tta ttg gaa tta gat aaa tgg gca agt ttg    96
   Glu Lys Asn Glu Gln Glu Leu Leu Glu Leu Asp Lys Trp Ala Ser Leu
               20                  25                  30
   tgg aat tgg ttt                                                    108
   Trp Asn Trp Phe
           35
<210> 14
   <211> 36
   <212> PRT
<213> Artificial Sequence
<220>
   <223> Description of Artificial Sequence: HIV antifusogenic sequences
<400> 14
   Tyr Thr Ser Leu Ile His Ser Leu Ile Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 15
   <211> 124
   <212> DNA
   <213> Artificial Sequence
<220>
   <223> Description of Artificial Sequence: HIV antifusogenic sequences for fusion proteins
<220>
   <221> CDS
   <222> (1)..(123)
<400> 15
   cta gag aaa agg tac act agc tta ata cac tcc tta att gaa gaa tcg 48
   Leu Glu Lys Arg Tyr Thr Ser Leu Ile His Ser Leu Ile Glu Glu Ser
   1 5 10 15
   caa aac cag caa gaa aag aat gaa caa gaa tta ttg gaa tta gat aaa 96
   Gln Asn Gln Gln Glu Lys Asn Glu Gln Glu Leu Leu Glu Leu Asp Lys
   20 25 30
   tgg gca agt ttg tgg aat tgg ttt gta c 124
   Trp Ala Ser Leu Trp Asn Trp Phe Val
   35 40
<210> 16
   <211> 41
   <212> PRT
   <213> Artificial Sequence
<220>
   <223> Description of Artificial Sequence: HIV antifusogenic sequences for fusion proteins
<400> 16
   Leu Glu Lys Arg Tyr Thr Ser Leu Ile His Ser Leu Ile Glu Glu Ser
   1 5 10 15
   Gln Asn Gln Gln Glu Lys Asn Glu Gln Glu Leu Leu Glu Leu Asp Lys
   20 25 30
   Trp Ala Ser Leu Trp Asn Trp Phe Val
   35 40
<210> 17
   <211> 174
   <212> PRT
   <213> Homo sapiens
<220>
   <223> INGAP protein
<400> 17
   Met Met Leu Pro Met Thr Leu Cys Arg Met Ser Trp Met Leu Leu Ser
   1               5                   10                  15
   Cys Leu Met Phe Leu Ser Trp Val Glu Gly Glu Glu Ser Gln Lys Lys
               20                  25                  30
   Leu Pro Ser Ser Arg Ile Thr Cys Pro Gln Gly Ser Val Ala Tyr Gly
           35                  40                  45
   Ser Tyr Cys Tyr Ser Leu Ile Leu Ile Pro Gln Thr Trp Ser Asn Ala
       50                  55                  60
   Glu Leu Ser Cys Gln Met His Phe Ser Gly His Leu Ala Phe Leu Leu
   65                  70                  75                  80
   Ser Thr Gly Glu Ile Thr Phe Val Ser Ser Leu Val Lys Asn Ser Leu
                   85                  90                  95
   Thr Ala Tyr Gln Tyr Ile Trp Ile Gly Leu His Asp Pro Ser His Gly
               100                 105                 110
   Thr Leu Pro Asn Gly Gly Trp Lys Trp Ser Ser Ser Asn Val Leu Thr
           115                 120                 125
   Phe Tyr Asn Trp Glu Arg Asn Pro Ser Ile Ala Ala Asp Arg Gly Tyr
       130                 135                 140
   Cys Ala Val Leu Ser Gln Lys Ser Gly Phe Gln Lys Trp Arg Asp Phe
   145                 150                 155                 160
   Asn Cys Glu Asn Glu Leu Pro Tyr Ile Cys Lys Phe Lys Val
                   165                 170
<210> 18
   <211> 525
   <212> DNA
   <213> Artificial Sequence
<220>
   <223> Description of Artificial Sequence: INGAP sequences
<220>
   <221> CDS
   <222> (1)..(525)
<400> 18
   atg atg ttg cca atg act ttg tgt aga atg tct tgg atg ttg ttg tct    48
   Met Met Leu Pro Met Thr Leu Cys Arg Met Ser Trp Met Leu Leu Ser
    1               5                  10                  15
   tgt ttg atg ttt ttg tct tgg gtt gaa ggt gaa gaa tct caa aaa aaa    96
   Cys Leu Met Phe Leu Ser Trp Val Glu Gly Glu Glu Ser Gln Lys Lys
               20                  25                  30
   ttg cca tct tct aga att act tgt cca caa ggt tct gtt gct tat ggt    144
   Leu Pro Ser Ser Arg Ile Thr Cys Pro Gln Gly Ser Val Ala Tyr Gly
           35                  40                  45
   tct tat tgt tat tct ttg att ttg att cca caa act tgg tct aat gct    192
   Ser Tyr Cys Tyr Ser Leu Ile Leu Ile Pro Gln Thr Trp Ser Asn Ala
       50                  55                  60
   gaa ttg tct tgt caa atg cat ttt tct ggt cat ttg gct ttt ttg ttg    240
   Glu Leu Ser Cys Gln Met His Phe Ser Gly His Leu Ala Phe Leu Leu
   65                  70                  75                  80
   tct act ggt gaa att act ttt gtt tct tct ttg gtt aaa aat tct ttg    288
   Ser Thr Gly Glu Ile Thr Phe Val Ser Ser Leu Val Lys Asn Ser Leu
                   85                  90                  95
   act gct tat caa tat att tgg att ggt ttg cat gat cca tct cat ggt    336
   Thr Ala Tyr Gln Tyr Ile Trp Ile Gly Leu His Asp Pro Ser His Gly
               100                 105                 110
   act ttg cca aat ggt tct ggt tgg aaa tgg tct tct tct aat gtt ttg    384
   Thr Leu Pro Asn Gly Ser Gly Trp Lys Trp Ser Ser Ser Asn Val Leu
           115                 120                 125
   act ttt tat aat tgg gaa aga aat cca tct att gct gct gat aga ggt    432
   Thr Phe Tyr Asn Trp Glu Arg Asn Pro Ser Ile Ala Ala Asp Arg Gly
       130                 135                 140
   tat tgt gct gtt ttg tct caa aaa tct ggt ttt caa aaa tgg aga gat    480
   Tyr Cys Ala Val Leu Ser Gln Lys Ser Gly Phe Gln Lys Trp Arg Asp
   145                 150                 155                 160
   ttt aat tgt gaa aat gaa ttg cca tat att tgt aaa ttt aaa gtt        525
   Phe Asn Cys Glu Asn Glu Leu Pro Tyr Ile Cys Lys Phe Lys Val
                   165                 170                 175
<210> 19
   <211> 175
   <212> PRT
   <213> Artificial Sequence
<220>
   <223> Description of Artificial Sequence: INGAP sequences
<400> 19
   Met Met Leu Pro Met Thr Leu Cys Arg Met Ser Trp Met Leu Leu Ser
   1               5                   10                  15
   Cys Leu Met Phe Leu Ser Trp Val Glu Gly Glu Glu Ser Gln Lys Lys
               20                  25                  30
   Leu Pro Ser Ser Arg Ile Thr Cys Pro Gln Gly Ser Val Ala Tyr Gly
           35                  40                  45
   Ser Tyr Cys Tyr Ser Leu Ile Leu Ile Pro Gln Thr Trp Ser Asn Ala
       50                  55                  60
   Glu Leu Ser Cys Gln Met His Phe Ser Gly His Leu Ala Phe Leu Leu
   65                  70                  75                  80
   Ser Thr Gly Glu Ile Thr Phe Val Ser Ser Leu Val Lys Asn Ser Leu
                   85                  90                  95
   Thr Ala Tyr Gln Tyr Ile Trp Ile Gly Leu His Asp Pro Ser His Gly
               100                 105                 110
   Thr Leu Pro Asn Gly Ser Gly Trp Lys Trp Ser Ser Ser Asn Val Leu
           115                 120                 125
   Thr Phe Tyr Asn Trp Glu Arg Asn Pro Ser Ile Ala Ala Asp Arg Gly
       130                 135                 140
   Tyr Cys Ala Val Leu Ser Gln Lys Ser Gly Phe Gln Lys Trp Arg Asp
   145                 150                 155                 160
   Phe Asn Cys Glu Asn Glu Leu Pro Tyr Ile Cys Lys Phe Lys Val
                   165                 170                 175
<210> 20
   <211> 445
   <212> DNA
   <213> Artificial Sequence
<220>
   <223> Description of Artificial Sequence: INGAP sequences for fusion proteins
<220>
   <221> CDS
   <222> (1)..(444)
<400> 20
   cta gag aaa agg ttg cca tct tcc aga att act tgt cca caa ggt tct    48
   Leu Glu Lys Arg Leu Pro Ser Ser Arg Ile Thr Cys Pro Gln Gly Ser
     1               5                  10                  15
   gtt gct tat ggt tct tat tgt tat tct ttg att ttg att cca caa act    96
   Val Ala Tyr Gly Ser Tyr Cys Tyr Ser Leu Ile Leu Ile Pro Gln Thr
                20                  25                  30
   tgg tct aat gct gaa ttg tct tgt caa atg cat ttt tct ggt cat ttg   144
   Trp Ser Asn Ala Glu Leu Ser Cys Gln Met His Phe Ser Gly His Leu
            35                  40                  45
   gct ttt ttg ttg tct act ggt gaa att act ttt gtt tct tct ttg gtt   192
   Ala Phe Leu Leu Ser Thr Gly Glu Ile Thr Phe Val Ser Ser Leu Val
        50                  55                  60
   aaa aat tct ttg act gct tat caa tat att tgg att ggt ttg cat gat   240
   Lys Asn Ser Leu Thr Ala Tyr Gln Tyr Ile Trp Ile Gly Leu His Asp
    65                  70                  75                  80
   cca tct cat ggt act ttg cca aat ggt tct ggt tgg aaa tgg tct tct   288
   Pro Ser His Gly Thr Leu Pro Asn Gly Ser Gly Trp Lys Trp Ser Ser
                    85                  90                  95
   tct aat gtt ttg act ttt tac aat tgg gaa aga aat cca tct att gct   336
   Ser Asn Val Leu Thr Phe Tyr Asn Trp Glu Arg Asn Pro Ser Ile Ala
               100                 105                 110
   gct gat aga ggt tat tgt gct gtt ttg tct caa aaa tct ggt ttt caa   384
   Ala Asp Arg Gly Tyr Cys Ala Val Leu Ser Gln Lys Ser Gly Phe Gln
           115                 120                 125
   aaa tgg aga gat ttt aat tgt gaa aat gaa ttg cca tat att tgt aaa   432
   Lys Trp Arg Asp Phe Asn Cys Glu Asn Glu Leu Pro Tyr Ile Cys Lys
       130                 135                 140
   ttt aaa gtt gta c                                                 445
   Phe Lys Val Val
   145
<210> 21
   <211> 148
   <212> PRT
   <213> Artificial Sequence
<220>
   <223> Description of Artificial Sequence: INGAP sequences for fusion proteins
<400> 21
   Leu Glu Lys Arg Leu Pro Ser Ser Arg Ile Thr Cys Pro Gln Gly Ser
    1               5                  10                  15
   Val Ala Tyr Gly Ser Tyr Cys Tyr Ser Leu Ile Leu Ile Pro Gln Thr
               20                  25                  30
   Trp Ser Asn Ala Glu Leu Ser Cys Gln Met His Phe Ser Gly His Leu
           35                  40                  45
   Ala Phe Leu Leu Ser Thr Gly Glu Ile Thr Phe Val Ser Ser Leu Val
       50                  55                  60
   Lys Asn Ser Leu Thr Ala Tyr Gln Tyr Ile Trp Ile Gly Leu His Asp
   65                  70                  75                  80
   Pro Ser His Gly Thr Leu Pro Asn Gly Ser Gly Trp Lys Trp Ser Ser
                   85                  90                  95
   Ser Asn Val Leu Thr Phe Tyr Asn Trp Glu Arg Asn Pro Ser Ile Ala
               100                 105                 110
   Ala Asp Arg Gly Tyr Cys Ala Val Leu Ser Gln Lys Ser Gly Phe Gln
           115                 120                 125
   Lys Trp Arg Asp Phe Asn Cys Glu Asn Glu Leu Pro Tyr Ile Cys Lys
       130                 135                 140
   Phe Lys Val Val
   145
<210> 22
   <211> 441
   <212> DNA
   <213> Artificial Sequence
<220>
   <223> Description of Artificial Sequence: INGAP sequences for fusion proteins
<220>
   <221> CDS
   <222> (2)..(436)
<400> 22
   t cga cct ttg cca tct tcc aga att act tgt cca caa ggt tct gtt gct 49
     Arg Pro Leu Pro Ser Ser Arg Ile Thr Cys Pro Gln Gly Ser Val Ala
      1               5                  10                  15
   tat ggt tct tat tgt tat tct ttg att ttg att cca caa act tgg tct   97
   Tyr Gly Ser Tyr Cys Tyr Ser Leu Ile Leu Ile Pro Gln Thr Trp Ser
               20                  25                  30
   aat gct gaa ttg tct tgt caa atg cat ttt tct ggt cat ttg gct ttt   145
   Asn Ala Glu Leu Ser Cys Gln Met His Phe Ser Gly His Leu Ala Phe
               35                  40                  45
   ttg ttg tct act ggt gaa att act ttt gtt tct tct ttg gtt aaa aat    193
   Leu Leu Ser Thr Gly Glu Ile Thr Phe Val Ser Ser Leu Val Lys Asn
        50                  55                  60
   tct ttg act gct tat caa tat att tgg att ggt ttg cat gat cca tct    241
   Ser Leu Thr Ala Tyr Gln Tyr Ile Trp Ile Gly Leu His Asp Pro Ser
    65                  70                  75                  80
   cat ggt act ttg cca aat ggt tct ggt tgg aaa tgg tct tct tct aat    289
   His Gly Thr Leu Pro Asn Gly Ser Gly Trp Lys Trp Ser Ser Ser Asn
                    85                  90                  95
   gtt ttg act ttt tac aat tgg gaa aga aat cca tct att gct gct gat    337
   Val Leu Thr Phe Tyr Asn Trp Glu Arg Asn Pro Ser Ile Ala Ala Asp
               100                 105                 110
   aga ggt tat tgt gct gtt ttg tct caa aaa tct ggt ttt caa aaa tgg    385
   Arg Gly Tyr Cys Ala Val Leu Ser Gln Lys Ser Gly Phe Gln Lys Trp
           115                 120                 125
   aga gat ttt aat tgt gaa aat gaa ttg cca tat att tgt aaa ttt aaa    433
   Arg Asp Phe Asn Cys Glu Asn Glu Leu Pro Tyr Ile Cys Lys Phe Lys
       130                 135                 140
   gtt taata                                                          441
   Val
   145
<210> 23
   <211> 145
   <212> PRT
   <213> Artificial Sequence
<220>
   <223> Description of Artificial Sequence: INGAP sequences for fusion proteins
<400> 23
   Arg Pro Leu Pro Ser Ser Arg Ile Thr Cys Pro Gln Gly Ser Val Ala
    1               5                  10                  15
   Tyr Gly Ser Tyr Cys Tyr Ser Leu Ile Leu Ile Pro Gln Thr Trp Ser
               20                  25                  30
   Asn Ala Glu Leu Ser Cys Gln Met His Phe Ser Gly His Leu Ala Phe
           35                  40                  45
   Leu Leu Ser Thr Gly Glu Ile Thr Phe Val Ser Ser Leu Val Lys Asn
       50                  55                  60
   Ser Leu Thr Ala Tyr Gln Tyr Ile Trp Ile Gly Leu His Asp Pro Ser
   65                  70                  75                  80
   His Gly Thr Leu Pro Asn Gly Ser Gly Trp Lys Trp Ser Ser Ser Asn
                   85                  90                  95
   Val Leu Thr Phe Tyr Asn Trp Glu Arg Asn Pro Ser Ile Ala Ala Asp
               100                 105                 110
   Arg Gly Tyr Cys Ala Val Leu Ser Gln Lys Ser Gly Phe Gln Lys Trp
           115                 120                 125
   Arg Asp Phe Asn Cys Glu Asn Glu Leu Pro Tyr Ile Cys Lys Phe Lys
       130                 135                 140
   Val
   145
<210> 24
   <211> 60
   <212> DNA
   <213> Artificial Sequence
<220>
   <223> Description of Artificial Sequence: EPO mimetic sequences
<220>
   <221> CDS
   <222> (1)..(60)
<400> 24
   ggt ggt act tac tct tgt cat ttt ggt cca ttg act tgg gtt tgt aag    48
   Gly Gly Thr Tyr Ser Cys His Phe Gly Pro Leu Thr Trp Val Cys Lys
    1               5                  10                  15
   cca caa ggt ggt                                                     60
   Pro Gln Gly Gly
               20
<210> 25
   <211> 20
   <212> PRT
   <213> Artificial Sequence
<220>
   <223> Description of Artificial Sequence: EPO mimetic sequences
<400> 25
   Gly Gly Thr Tyr Ser Cys His Phe Gly Pro Leu Thr Trp Val Cys Lys
    1               5                  10                  15
   Pro Gln Gly Gly
               20
<210> 26
   <211> 47
   <212> PRT
   <213> Artificial Sequence
<220>
   <223> Description of Artificial Sequence: Transferrin peptide insertion region
<400> 26
   Asp Lys Ser Lys Glu Phe Gln Leu Phe Ser Ser Pro His Gly Lys Asp
    1               5                  10                  15
   Leu Leu Phe Lys Asp Ser Ala His Gly Phe Leu Lys Val Pro Pro Arg
               20                  25                  30
   Met Asp Ala Lys Met Tyr Leu Gly Tyr Glu Tyr Val Thr Ala Ile
           35                  40                  45
<210> 27
   <211> 49
   <212> PRT
   <213> Artificial Sequence
<220>
   <223> Description of Artificial Sequence: Transferrin peptide insertion region
<400> 27
   Asn Val Thr Asp Cys Ser Gly Asn Phe Cys Leu Phe Arg Ser Glu Thr
    1               5                  10                  15
   Lys Asp Leu Leu Phe Arg Asp Asp Thr Val Cys Leu Ala Lys Leu His
               20                  25                  30
   Asp Arg Asn Thr Tyr Glu Lys Tyr Leu Gly Glu Glu Tyr Val Lys Ala
           35                  40                  45
   Val
<210> 28
   <211> 140
   <212> DNA
   <213> Artificial Sequence
<220>
   <223> Description of Artificial Sequence: Transferrin DNA sequence for peptide insertion region
<400> 28
   agacaaatca aaagaatttc aactattcag ctctcctcat ggtggtactt actcttgtca 60
   ttttggtcca ttgacttggg tttgtaagcc acaaggtggt gggaaggacc tgctgtttaa 120
   ggactctgcc cacgggtttt                                              140
<210> 29
   <211> 210
   <212> DNA
   <213> Artificial Sequence
<220>
   <223> Description of Artificial Sequence: Transferrin DNA sequence for peptide insertion region
<400> 29
   cctatttgga agcaacgtaa ctgactgctc gggcaacttt tgtttgttcc ggtcggaagg 60
   tggtacttac tcttgtcatt ttggtccatt gacttgggtt tgtaagccac aaggtggtac 120
   caaggacctt ctgttcagag atgacacagt atgtttggcc aaacttcatg acagaaacac 180
   atatgaaaaa tacttaggag aagaatatgt                                   210
<210> 30
   <211> 23
   <212> PRT
   <213> Artificial Sequence
<220>
   <223> Description of Artificial Sequence: EPO mimetic peptide
<400> 30
   Gln Arg Val Glu Ile Leu Glu Gly Arg Thr Glu Cys Val Leu Ser Asn
   1               5                   10                  15
   Leu Arg Gly Arg Thr Arg Tyr
               20

## Description

### FIELD OF THE INVENTION

The present invention relates to therapeutic proteins or peptides with extended serum stability or serum half-life, particularly to therapeutic proteins or peptides fused to or inserted in a transferrin molecule modified to reduce or inhibit glycosylation, iron binding and/or transferrin receptor binding.

### BACKGROUND OF THE INVENTION

Therapeutic proteins or peptides in their native state or when recombinantly produced are typically labile molecules exhibiting short periods of serum stability or short serum half-lives. In addition, these molecules are often extremely labile when formulated, particularly when formulated in aqueous solutions for diagnostic and therapeutic purpose.

Few practical solutions exist to extend or promote the stability *in vivo* or *in vitro* of proteinaceous therapeutic molecules. Polyethylene glycol (PEG) is a substance that can attach to a protein, resulting in longer-acting, sustained activity of the protein. If the activity of a protein is prolonged by the attachment to PEG, the frequency that the protein needs to be administered is decreased. PEG attachment, however, often decreases or destroys the protein's therapeutic activity.

Therapeutic proteins or peptides have also been stabilized by fusion to a heterologous protein capable of extending the serum half-life of the therapeutic protein. For instance, therapeutic proteins fused to albumin and antibody fragments may exhibit extended serum half-live when compared to the therapeutic protein in the unfused state. See U.S. Patents 5,876,969 and 5,766,88.

Another serum protein, glycosylated human transferrin (Tf) has also been used to make fusions with therapeutic proteins to target delivery intracellularly or to carry heterologous agents across the blood-brain baler. These fusion proteins comprising glycosylated human Tf have been used to target nerve growth factor (NGF) or ciliary neurotrophic factor (CNTF) across the blood-brain barrier by fusing full-length Tf to the either agent. See U.S. Patents 5,672,683 and 5977,307. In these fusion proteins, the Tf portion of the molecule is glycosylated and binds to two atoms of iron, which is required for Tf binding to its receptor on a cell and, according to the inventors of these patents, to target delivery of the NGF or CNTF moiety across the blood-brain barrier. Transferrin fusion proteins have also been produced by inserting an HIV-1 protease sequence into surface exposed loops of glycosylated transferrin to investigate the ability to produce another form of Tf fusion for targeted delivery to the inside of a cell via the Tf receptor (Ali et al. (1999) J. Biol. Chem. 274(34):24066-24073).

Serum transferrin (Tf) is a monomeric glycoprotein with a molecular weight of 80,000 daltons that binds iron in the circulation and transports it to various tissues via the transferrin receptor (TfR) (Aisen et al. (1980) Ann. Rev. Biochem. 49: 357-393; MacGillivray et al. (1981) J. Biol. Chem. 258: 3543-3553, U.S. Patent 5,026,651). Tf is one of the most common serum molecules, comprising up to about 5-10% of total serum proteins. Carbohydrate deficient transferrin occurs in elevated levels in the blood of alcoholics and exhibits a longer half life (approximately 14-17 days) than that of glycosylated transferrin (approximately 7-10 days). See van Eijk et al. (1983) Clin. Chim. Acta 132:167-171, Stibler (1991) Clin. Chem. 37:2029-2037 (1991), Arndt (2001) Clin. Chem. 47(1):13-27 and Stibler et al. in "Carbohydrate-deficient consumption", Advances in the Biosciences, (Ed Nordmann et al.), Pergamon, 1988, Vol. 71, pages 353-357).

The structure of Tf has been well characterized and the mechanism of receptor binding, iron binding and release and carbonate ion binding have been eluciated (U.S. Patents 5,026,651, 5,986,067 and MacGillivray et al. (1983) J. Biol. Chem. 258(6):3543-3546).

Transferrin and antibodies that bind the transferrin receptor have also been used to deliver or carry toxic agents to tumor cells as cancer therapy (Baselga and Mendelsohn, 1994), and transferrin has been used as a non-viral gene therapy vector to vehicle to deliver DNA to cells (Frank *et al.,* 1994; Wagner *et al.,* 1992). The ability to deliver proteins to the central nervous system (CNS) using the transferrin receptor as the entry point has been demonstrated with several proteins and peptides including CD4 (Walus *et al.,* 1996), brain derived neurotrophic factor (Pardridge *et al.,* 1994), glial derived neurotrophic factor (Albeck *et al.*), a vasointestinal peptide analogue (Bickel *et al.,* 1993), a betaamyloid peptide (Saito *et al.,* 1995), and an antisense oligonucleotide (Pardridge *et al*., 1995).

Hoefkens et al ((1997) Glycoconj J. J. 14, 289-295) reports on the influence of the glycosylation state of transferrin on the receptor binding and iron-donation. US-A-6027921 discloses the preparation of a recombinantly expressed transferrin/LDLR fusion protein. Newton et al ((1999) J. Immunol. Meth. 231, 159-167) discloses an antitransferrin receptor antibody-RNase fusion protein expressed in the mammary gland of transgenic mice. Park et al. ((1998) J. Drug Tar. 6, 53-64) discloses a fusion protein comprising transferrin and nerve growth factor. Prince et al. ((1999) J .Biol. Chem.274, 3602-3609) discloses chimeric molecules consisting of the amino-terminal and carboxyl-terminal tail regions of CFTR, each fused to the transmembrane and extracellular domains of the transferrin receptor. Shin et al. ((1995) Proc. Natl. Acad. Sci USA 92, 2820-2824) discloses transferrin antibody fusion proteins effective in brain targeting. US-A- 5986067 discloses the provision of genetically modified transferrin. EP-0413622 discloses fusion proteins comprising albumin as a carrier and CD4 as the therapeutic protein. EP0314317 discloses conjugates and chimeric proteins comprising CD4 and a plasma protein, such as human transferrin, albumin and a lipoprotein.

Transferrin fusion proteins have not, however, been modified or engineered to extend the serum half-life of a therapeutic protein or peptide or to increase bioavailability by reducing or inhibiting glycosylation of the Tf moiety or to reduce or prevent iron and/or Tf receptor binding.

### SUMMARY OF INVENTION

As described in more detail below, the present invention includes modified Tf fusion proteins comprising at least one therapeutic protein, polypeptide or peptide entity, wherein the Tf portion is engineered to extend the serum half-life or bioavailaibilty of the molecule. The invention also includes pharmaceutical formulations and compositions comprising the fusion proteins, methods of extending the serum stability, serum half-life and bioavailability of a therapeutic protein by fusion to modified transferrin, nucleic acid molecules encoding the modified Tf fusion proteins, and the like. Another aspect of the present invention relates to modified Tf fusion proteins for use in a method of medical treatment.

In a preferred embodiment, the present invention provides a purified fusion protein comprising a transferrin (Tf) protein exhibiting reduced glycosylation fused to at least one therapeutic protein or peptide, wherein the serum half-life or serum stability of the therapeutic protein or peptide is increased over the serum half-life or serum stability of the therapeutic protein or peptide in an unfused state and wherein said Tf protein comprises at least one mutation that reduces or prevents glycosylation.

In one embodiment, the invention provides a method of increasing the serum half life or serum stability of a therapeutic protein or peptide, comprising expressing said therapeutic protein or peptide as a fusion protein with a transferrin (Tf) protein, wherein:
said Tf protein comprises at least one mutation that reduces or prevents glycosylation;
such that the transferrin (Tf) protein exhibits reduced glycosylation.

In another embodiment, the invention provides a purified fusion protein comprising a Tf protein comprising mutation in an N-linked N-X-S/T glycosylation site fused to at least one therapeutic protein or peptide, wherein the transferrin protein exhibits reduced glycosylation as compared to a fully glycosylated Tf protein and wherein the serum half-life of the therapeutic protein or peptide is increased over the serum half-life of the therapeutic protein or peptide in an unfused state.

### BRIEF DESCRIPTION OF THE DRAWING

Figure 1 shows an alignment of the N and C Domains of Human (Hu) transferrin (Tf) with similarities and identities highlighted.
Figure 2A-2B shows an alignment of transferrin sequences from different species. Light shading: Similarity; Dark shading: Identity.
Figure 3 shows the location of a number of Tf surface exposed insertion sites for therapeutic proteins, polypeptides or peptides.
Figure 4A-4B shows the VH and VL regions for a number of preferred anti-TNFα antibodies used to produce modified Tf fusion proteins.
Figure 5 shows pREX0010
Figure 6 shows pREX0011
Figure 7 shows pREX0012
Figure 8 shows pREX0013
Figure 9 shows pREX0014
Figure 10 shows pREX0015

### DETAILED DESCRIPTION

### General Description

It has been discovered that a therapeutic protein (*e.g*., a polypeptide, antibody, or peptide, or fragments and variants thereof) can be stabilized to extend the serum half-life and/or retain the therapeutic protein's activity for extended periods of time *in vivo* by genetically fusing or chemically conjugating the therapeutic protein, polypeptide or peptide to all or a portion of modified transferrin sufficient to extend its half life in serum. The modified transferrin fusion proteins include a transferrin protein or domain covalently linked to a therapeutic protein or peptide, wherein the transferrin portion is modified to contain one or more amino acid substitutions, insertions or deletions compared to a wild-type transferrin sequence. In one embodiment, Tf fusion proteins are engineered to reduce or prevent glycosylation within the Tf or a Tf domain. In other embodiments, the Tf protein or Tf domain(s) is modified to exhibit reduced or no binding to iron or carbonate ion, or to have a reduced affinity or not bind to a Tf receptor (TfR).

The present invention therefore includes transferrin fusion proteins, therapeutic compositions comprising the fusion proteins, and methods of treating, preventing, or ameliorating diseases or disorders by administering the fusion proteins. A transferrin fusion protein of the invention includes at least a fragment or variant of a therapeutic protein and at least a fragment or variant of modified transferrin, which are associated with one another, preferably by genetic fusion (*i.e*., the transferrin fusion protein is generated by translation of a nucleic acid in which a polynucleotide encoding all or a portion of a therapeutic protein is joined in-frame with a polynucleotide encoding all or a portion of modified transferrin) or chemical conjugation to one another. The therapeutic protein and transferrin protein, once part of the transferrin fusion protein, may be referred to as a "portion", "region" or "moiety" of the transferrin fusion protein (*e.g*., a "therapeutic protein portion' or a "transferrin protein portion").

In one embodiment, the invention provides a transferrin fusion protein comprising, or alternatively consisting of, a therapeutic protein and a modified serum transferrin protein. In other embodiments, the invention provides a transferrin fusion protein comprising, or alternatively consisting of, a biologically active and/or therapeutically active fragment of a therapeutic protein and a modified transferrin protein. In other embodiments, the invention provides a transferrin fusion protein comprising, or alternatively consisting of, a biologically active and/or therapeutically active variant of a therapeutic protein and modified transferrin protein. In further embodiments, the invention provides a transferrin fusion protein comprising a therapeutic protein, and a biologically active and/or therapeutically active fragment of modified transferrin. In another embodiment, the therapeutic protein portion of the transferrin fusion protein is the active form of the therapeutic protein.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

### Definitions

As used herein, the term "biological activity" refers to a function or set of activities performed by a therapeutic molecule, protein or peptide in a biological context (*i.e*., in an organism or an *in vitro* facsimile thereof). Biological activities may include but are not limited to the functions of the therapeutic molecule portion of the claimed fusion proteins, such as, but not limited to, the induction of extracellular matrix secretion from responsive cell lines, the induction of hormone secretion, the induction of chemotaxis, the induction of mitogenesis, the induction of differentiation, or the inhibition of cell division of responsive cells. A fusion protein or peptide of the invention is considered to be biologically active if it exhibits one or more biological activities of its therapeutic protein's native counterpart.

As used herein, an "amino acid corresponding to" or an "equivalent amino acid" in a transferrin sequence is identified by alignment to maximize the identity or similarity between a first transferrin sequence and at least a second transferrin sequence. The number used to identify an equivalent amino acid in a second transferrin sequence is based on the number used to identify the corresponding amino acid in the first transferrin sequence. In certain cases, these phrases may be used to describe the amino acid residues in human transferrin compared to certain residues in rabbit serum transferrin.

As used herein, the terms "fragment of a Tf protein" or "Tf protein," or "portion of a Tf protein" refer to an amino acid sequence comprising at least about 5%, 10%, 20%, 30%, 40%, 50%, 60%, 70%, 80%, 90%, 95%, 96%, 97%, 98%, 99% or 100% of a naturally occurring Tf protein or mutant thereof.

As used herein, the term "gene" refers to any segment of DNA associated with a biological function. Thus, genes include, but are not limited to, coding sequences and/or the regulatory sequences required for their expression. Genes can also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

As used herein, a "heterologous polynucleotide" or a "heterologous nucleic acid" or a "heterologous gene" or a "heterologous sequence" or an "exogenous DNA segment" refers to a polynucleotide, nucleic acid or DNA segment that originates from a source foreign to the particular host cell, or, if from the same source, is modified from its original form. A heterologous gene in a host cell includes a gene that is endogenous to the particular host cell, but has been modified. Thus, the terms refer to a DNA segment which is foreign or heterologous to the cell, or homologous to the cell but in a position within the host cell nucleic acid in which the element is not ordinarily found. As an example, a signal sequence native to a yeast cell but attached to a human Tf sequence is heterologous.

As used herein, an "isolated" nucleic acid sequence refers to a nucleic acid sequence which is essentially free of other nucleic acid sequences, *e.g*., at least about 20% pure, preferably at least about 40% pure, more preferably about 60% pure, even more preferably about 80% pure, most preferably about 90% pure, and even most preferably about 95% pure, as determined by agarose gel electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

As used herein, two or more DNA coding sequences are said to be "joined" or "fused" when, as a result of in-frame fusions between the DNA coding sequences, the DNA coding sequences are translated into a polypeptide fusion. The term "fusion" in reference to Tf fusions includes, but is not limited to, attachment of at least one therapeutic protein, polypeptide or peptide to the N -terminal end of Tf, attachment to the C-terminal end of Tf, and/or insertion between any two amino acids within Tf.

"Modified transferrin" as used herein refers to a transferrin molecule that exhibits at least one modification of its amino acid sequence, compared to wildtype transferrin.

"Modified transferrin fusion protein" as used herein refers to a protein formed by the fusion of at least one molecule of modified transferrin (or a fragment or variant thereof) to at least one molecule of a therapeutic protein (or fragment or variant thereof).

As used herein, the terms "nucleic acid" or "polynucleotide" refer to deoxyribonucleotides or ribonucleotides and polymers thereof in either single- or doublestranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g*. degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19:5081; Ohtsuka et al. (1985) J. Biol. Chem. 260:2605-2608; Cassol *et al.* (1992); Rossolini et al. (1994) Mol. Cell. Probes 8:91-98). The term nucleic acid is used interchangeably with gene, cDNA, and mRNA encoded by a gene.

As used herein, a DNA segment is referred to as "operably linked" when it is placed into a functional relationship with another DNA segment. For example, DNA for a signal sequence is operably linked to DNA encoding a fusion protein of the invention if it is expressed as a preprotein that participates in the secretion of the fusion protein; a promoter or enhancer is operably linked to a coding sequence if it stimulates the transcription of the sequence. Generally, DNA sequences that are operably linked are contiguous, and in the case of a signal sequence or fusion protein both contiguous and in reading phase. However, enhancers need not be contiguous with the coding sequences whose transcription they control. Linking, in this context, is accomplished by ligation at convenient restriction sites or at adapters or linkers inserted in lieu thereof.

As used herein, the term "promoter" refers to a region of DNA involved in binding RNA polymerase to initiate transcription.

As used herein, the term "recombinant" refers to a cell, tissue or organism that has undergone transformation with recombinant DNA.

As used herein, a targeting entity, protein, polypeptide or peptide refers to such molecules that binds specifically to a particular cell type [normal (*e.g*., lymphocytes) or abnormal *e.g*., (cancer cell)] and therefore may be used to target a Tf fusion protein or compound (drug, or cytotoxic agent) to that cell type specifically.

As used herein, "therapeutic protein" refers to proteins, polypeptides, antibodies, peptides or fragments or variants thereof, having one or more therapeutic and/or biological activities. Therapeutic proteins encompassed by the invention include but are not limited to proteins, polypeptides, peptides, antibodies, and biologics. The terms peptides, proteins, and polypeptides are used interchangeably herein. Additionally, the term "therapeutic protein" may refer to the endogenous or naturally occurring correlate of a therapeutic protein. By a polypeptide displaying a "therapeutic activity" or a protein that is "therapeutically active" is meant a polypeptide that possesses one or more known biological and/or therapeutic activities associated with a therapeutic protein such as one or more of the therapeutic proteins described herein or otherwise known in the art. As a nonlimiting example, a "therapeutic protein" is a protein that is useful to treat, prevent or ameliorate a disease, condition or disorder. Such a disease, condition or disorder may be in humans or in a non-human animal, *e.g*., veterinary use.

As used herein, the term "transformation" refers to the transfer of nucleic acid (*i.e*., a nucleotide polymer) into a cell. As used herein, the term "genetic transformation" refers to the transfer and incorporation of DNA, especially recombinant DNA, into a cell.

As used herein, the term "transformant" refers to a cell, tissue or organism that has undergone transformation.

As used herein, the term "transgene" refers to a nucleic acid that is inserted into an organism, host cell or vector in a manner that ensures its function.

As used herein, the term "transgenic" refers to cells, cell cultures, organisms, bacteria, fungi, animals, plants, and progeny of any of the preceding, which have received a foreign or modified gene and in particular a gene encoding a modified Tf fusion protein by one of the various methods of transformation, wherein the foreign or modified gene is from the same or different species than the species of the organism receiving the foreign or modified gene.

"Variants or variant" refers to a polynucleotide or nucleic acid differing from a reference nucleic acid or polypeptide, but retaining essential properties thereof. Generally, variants are overall closely similar, and, in many regions, identical to the reference nucleic acid or polypeptide. As used herein, "variant", refers to a therapeutic protein portion of a transferrin fusion protein of the invention, differing in sequence from a native therapeutic protein but retaining at least one functional and/or therapeutic property thereof as described elsewhere herein or otherwise known in the art.

As used herein, the term "vector" refers broadly to any plasmid, phagemid or virus encoding an exogenous nucleic acid. The term is also be construed to include non-plasmid, non-phagemid and non-viral compounds which facilitate the transfer of nucleic acid into virions or cells, such as, for example, polylysine compounds and the like. The vector may be a viral vector that is suitable as a delivery vehicle for delivery of the nucleic acid, or mutant thereof, to a cell, or the vector may be a non-viral vector which is suitable for the same purpose. Examples of viral and non-viral vectors for delivery of DNA to cells and tissues are well known in the art and are described, for example, in Ma et al. (1997, Proc. Natl. Acad. Sci. U.S.A. 94:12744-12746). Examples of viral vectors include, but are not limited to, a recombinant vaccinia virus, a recombinant adenovirus, a recombinant retrovirus, a recombinant adeno-associated virus, a recombinant avian pox virus, and the like (Cranage et al., 1986, EMBO J. 5:3057-3063; International Patent Application No. WO94/17810, published August 18, 1994; International Patent Application No. WO94/23744, published October 27, 1994). Examples of non-viral vectors include, but are not limited to, liposomes, polyamine derivatives of DNA, and the like.

As used herein, the term "wild type" refers to a polynucleotide or polypeptide sequence that is naturally occurring.

### Transferrin and Transferrin Modifications

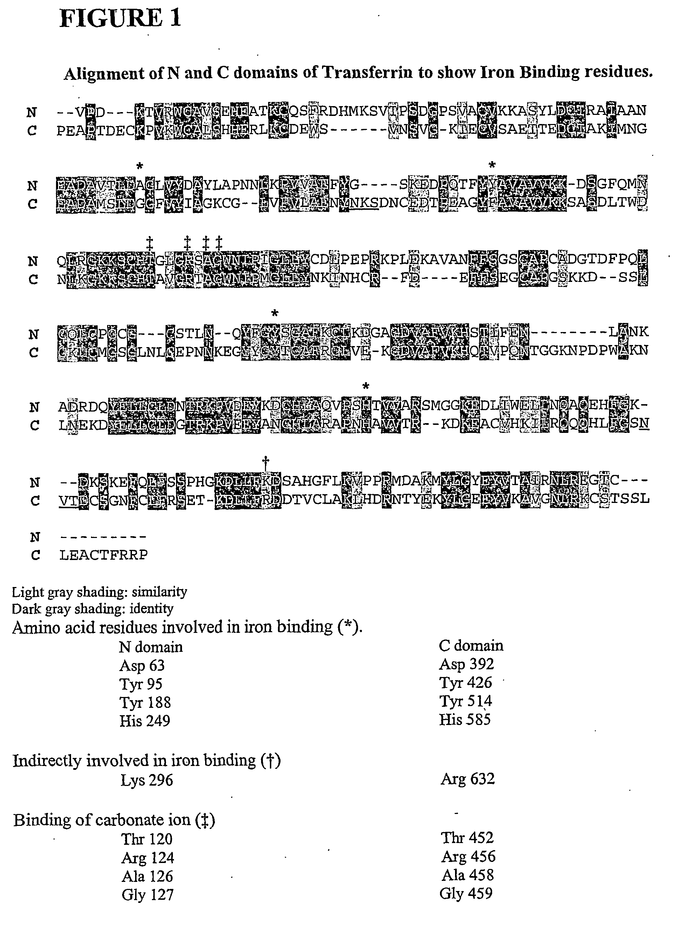

Any transferrin may be used to make modified Tf fusion proteins of the invention. Wild-type human Tf (Tf) is a 679 amino acid protein, of approximately 75kDa (not accounting for glycosylation), with two main domains, N (about 330 amino acids) and C (about 340 amino acids), which appear to originate from a gene duplication. See GenBank accession numbers NM001063, XM002793, M12530, XM039845, XM 039847 and S95936 (www.ncbi.nlm.nih.gov/), all of which are herein incorporated by reference in their entirety, as well as SEQ ID NOS 1, 2 and 3. The two domains have diverged over time but retain a large degree of identity/similarity (Fig. 1).

Each of the N and C domains is further divided into two subdomains, N1 and N2, C1 and C2. The function of Tf is to transport iron to the cells of the body. This process is mediated by the Tf receptor (TfR), which is expressed on all cells, particularly actively growing cells. TfR recognizes the iron bound form of Tf (two of which are bound per receptor), endocytosis then occurs whereby the TfR/Tf complex is transported to the endosome, at which point the localized drop in pH results in release of bound iron and the recycling of the TfR/Tf complex to the cell surface and release of Tf (known as apoTf in its un-iron bound form). Receptor binding is through the C domain of Tf. The two glycosylation sites in the C domain do not appear to be involved in receptor binding as unglycosylated iron bound Tf does bind the receptor.

Each Tf molecule can carry two iron atoms. These are complexed in the space between the N1 and N2, C1 and C2 sub domains resulting in a conformational change in the molecule. Tf crosses the blood brain barrier (BBB) via the Tf receptor.

In human transferrin, the iron binding sites comprise at least of amino acids Asp 63 (Asp 82 of SEQ ID NO: 2 which comprises the native Tf signal sequence); Asp 392 (Asp 411 of SEQ ID NO: 2); Tyr 95 (Tyr 114 of SEQ ID NO: 2); Tyr 426 (Tyr 445 of SEQ ID NO: 2); Tyr 188 (Tyr 207 of SEQ ID NO: 2); Tyr 514 or 517 (Tyr 533 or Tyr 536 SEQ ID NO:2); His 249 (His 268 of SEQ ID NO: 2); His 585 (His 604 of SEQ ID NO: 2), the hinge regions comprises of at least N domain amino acid residues 94-96, 245- 247 and/or 316-318 as well as C domain amino acid residues 425-427, 581-582 and/or 652-658., the carbonate binding sites comprise at least of amino acids Thr 120 (Thr 139 of SEQ ID NO: 2); Thr 452 (Thr 471 of SEQ ID NO: 2); Arg 124 (Arg 143 of SEQ ID NO: 2); Arg 456 (Arg 475 of SEQ ID NO: 2); Ala 126 (Ala 145 of SEQ ID NO: 2); Ala 458 (Ala 477 of SEQ ID NO: 2); Gly 127 (Gly 146 of SEQ ID NO: 2); Gly 459 (Gly 478 of SEQ ID NO: 2).

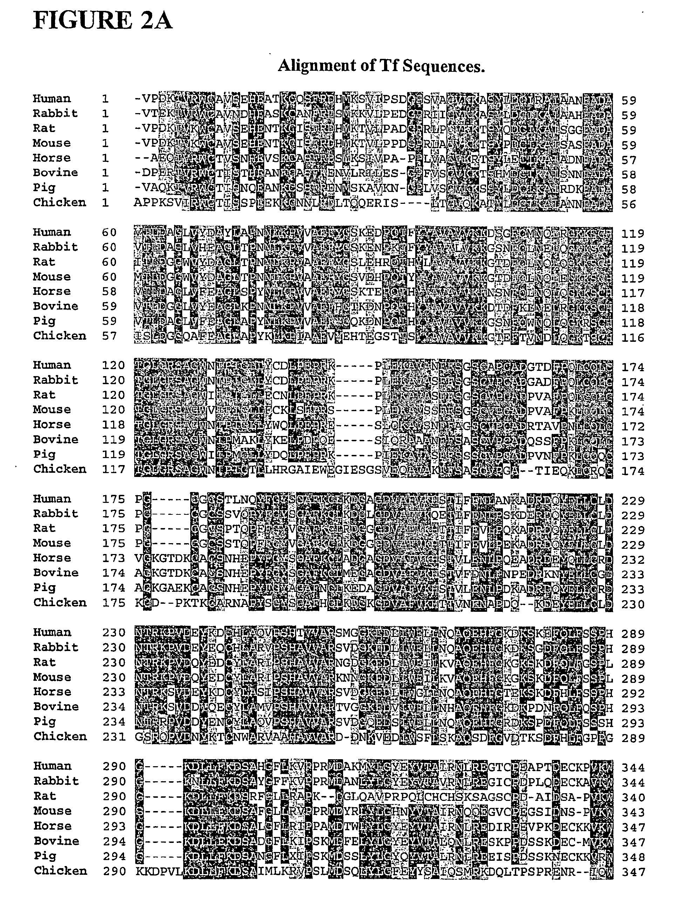

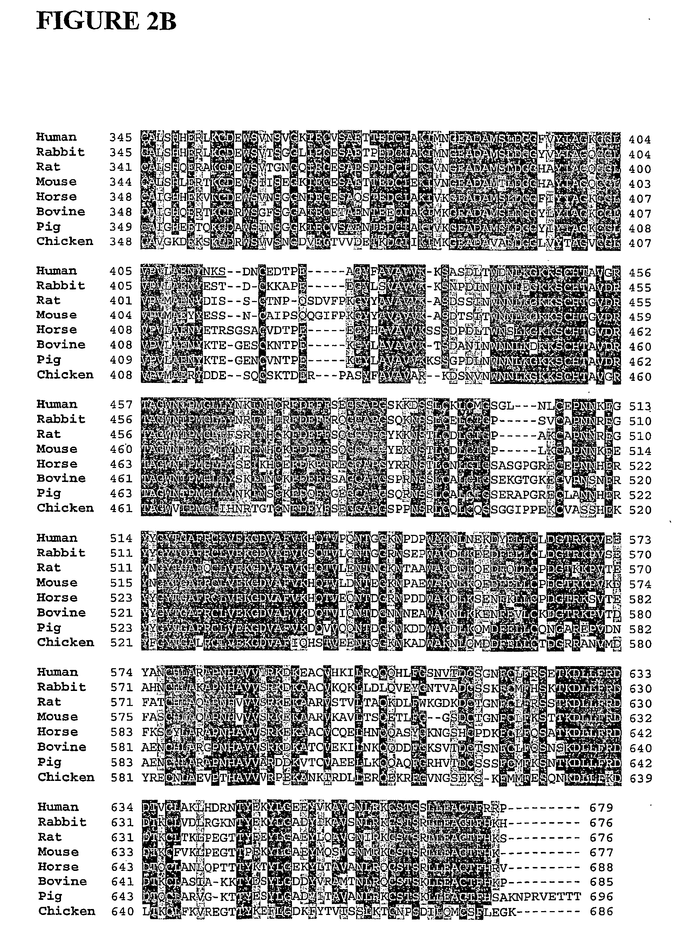

In one embodiment of the invention, the modified transferrin fusion protein includes a modified human transferrin, although any animal Tf molecule may be used to produce the fusion proteins of the invention, including human Tf variants, cow, pig, sheep, dog, rabbit, rat, mouse, hamster, echnida, platypus, chicken, frog, hornworm, monkey, as well as other bovine, canine and avian species (see Figure 2 for a representative set of Tf sequences). All of these Tf sequences are readily available in GenBank and other public databases. The human Tf nucleotide sequence is available (see SEQ ID NOS 1, 2 and 3 and the accession numbers described above and available at www.ncbi.nlin.nih.gov/) and can be used to make genetic fusions between Tf or a domain of Tf and the therapeutic molecule of choice. Fusions may also be made from related molecules such as lacto transferrin (lactoferrin) GenBank Acc: NM_002343).

Lactoferrin (Lf), a natural defense iron-binding protein, has been found to possess antibacterial, antimycotic, antiviral, antineoplastic and anti-inflammatory activity. The protein is present in exocrine secretions that are commonly exposed to normal flora: milk, tears, nasal exudate, saliva, bronchial mucus, gastrointestinal fluids, cervico-vaginal mucus and seminal fluid. Additionally, Lf is a major constituent of the secondary specific granules of circulating polymorphonuclear neutrophils (PMNs). The apoprotein is released on degranulation of the PMNs in septic areas. A principal function of Lf is that of scavenging free iron in fluids and inflamed areas so as to suppress free radical-mediated damage and decrease the availability of the metal to invading microbial and neoplastic cells. In a study that examined the turnover rate of ¹²⁵I Lf in adults, it was shown that LF is rapidly taken up by the liver and spleen, and the radioactivity persisted for several weeks in the liver and spleen (Bennett et al. (1979), Clin. Sci. (Lond.) 57: 453-460).

In another embodiment, the transferrin portion of the transferrin fusion protein of the invention includes a transferrin splice variant. In one example, a transferrin splice variant can be a splice variant of human transferrin. In one specific embodiment, the human transferrin splice variant can be that of Genbank Accession AAA61140. In another embodiment, the transferrin portion of the transferrin fusion protein of the invention includes a lactoferrin splice variant. In one example, a human serum lactoferrin splice variant can be a novel splice variant of a neutrophil lactoferrin. In one specific embodiment, the neutrophil lactoferrin splice variant can be that of Genbank Accession AAA59479. In another specific embodiment, the neutrophil lactoferrin splice variant can comprise the following amino acid sequence EDCIALKGEADA (SEQ ID NO: 8), which includes the novel region of splice-variance.

Modified Tf fusions may be made with any Tf protein, fragment, domain, or engineered domain. For instance, fusion proteins may be produced using the full-length Tf sequence, with or without the native Tf signal sequence. Tf fusion proteins may also be made using a single Tf domain, such as an individual N or C domain. In some embodiments, the use of a single N domain is advantageous as the Tf glycosylation sites reside in the C domain and the N domain, on its own, does not bind iron or the Tf receptor. In other embodiments, fusions of a therapeutic protein to a single C domain may be produced, wherein the C domain is altered to reduce, inhibit or prevent glycosylation, iron binding and/or Tf receptor binding.

In some embodiments, the Tf or Tf portion will be of sufficient length to increase the serum, *in vitro* solution stability or bioavailability of the therapeutic protein compared to the serum stability (half-life), *in vitro* stability or bioavailability of the therapeutic protein in an unfused state. Such an increase in stability, serum half-life or bioavailability may be about a 30%, 50%, 70%, 80%, 90% or more increase over the unfused therapeutic protein. In some cases, the modified transferrin fusion proteins exhibit a serum half-life of about 10-20 or more days, about 12-18 days or about 14-17 days.

When the C domain of Tf is part of the fusion protein, the two N-linked glycosylation sites, amino acid residues corresponding to N413 and N611 of SEQ ID NO:3 may be mutated for expression in a yeast system to prevent glycosylation or hypermannosylationn and extend the serum half-life of the fusion protein and/or therapeutic protein ( to produce asialo-, or in some instances, monosialo-Tf or disialo-Tf). In addition to Tf amino acids corresponding to N413 and N611, mutations may be to the adjacent residues within the N-X-S/T glycosylation site to prevent or substantially reduce glycosylation. See U.S. Patent 5,986,067 of Funk et al*.* It has also been reported that the N domain of Tf expressed in *Pichia pastoris* becomes O-linked glycosylated with a single hexose at S32 which also may be mutated or modified to prevent such glycosylation.

Accordingly, in one embodiment of the invention, the transferrin fusion protein includes a modified transferrin molecule wherein the transferrin exhibits reduced glycosylation, including but not limited to asialo- monosialo- and disialo- forms of Tf. In another embodiment, the transferrin portion of the transferrin fusion protein includes a recombinant transferrin mutant that is mutated to prevent glycosylation. In another embodiment, the transferrin portion of the transferrin fusion protein includes a recombinant transferrin mutant that is fully glycosylated. In a further embodiment, the transferrin portion of the transferrin fusion protein includes a recombinant human serum transferrin mutant that is mutated to prevent glycosylation, wherein at least one of Asn413 and Asn611 of SEQ ID NO:3 are mutated to an amino acid which does not allow glycosylation. In another embodiment, the transferrin portion of the transferrin fusion protein includes a recombinant human serum transferrin mutant that is mutated to prevent or substantially reduce glycosylation, wherein mutations may be to the adjacent residues within the N-X-S/T glycosylation site

As discussed below in more detail, modified Tf fusion proteins of the invention may also be engineered to not bind iron and/or not bind the Tf receptor. In other embodiments of the invention, the iron binding is retained and the iron binding ability of Tf may be used in two ways, one to deliver a therapeutic protein or peptide(s) to the inside of a cell and/or across the BBB. These embodiments that bind iron and/or the Tf receptor will often be engineered to reduce or prevent glycosylation to extend the serum half-life of the therapeutic protein. The N domain alone will not bind to TfR when loaded with iron, and the iron bound C domain will bind TfR but not with the same affinity as the whole molecule.

In another embodiment, the transferrin portion of the transferrin fusion protein includes a recombinant transferrin mutant having a mutation wherein the mutant does not retain the ability to bind metal. In an alternate embodiment, the transferrin portion of the transferrin fusion protein includes a recombinant transferrin mutant having a mutation wherein the mutant has a weaker binding avidity for metal than wild-type serum transferrin. In an alternate embodiment, the transferrin portion of the transferrin fusion protein includes a recombinant transferrin mutant having a mutation wherein the mutant has a stronger binding avidity for metal than wild-type serum transferrin.

In another embodiment, the transferrin portion of the transferrin fusion protein includes a recombinant transferrin mutant having a mutation wherein the mutant does not retain the ability to bind to the transferrin receptor. In an alternate embodiment, the transferrin portion of the transferrin fusion protein includes a recombinant transferrin mutant having a mutation wherein the mutant has a weaker binding avidity for the transferrin receptor than wild-type serum transferrin. In an alternate embodiment, the transferrin portion of the transferrin fusion protein includes a recombinant transferrin mutant having a mutation wherein the mutant has a stronger binding avidity for the transferrin receptor than wild-type serum transferrin.

In another embodiment, the transferrin portion of the transferrin fusion protein includes a recombinant transferrin mutant having a mutation wherein the mutant does not retain the ability to bind to carbonate. In an alternate embodiment, the transferrin portion of the transferrin fusion protein includes a recombinant transferrin mutant having a mutation wherein the mutant has a weaker binding avidity for carbonate than wild-type serum transferrin. In an alternate embodiment, the transferrin portion of the transferrin fusion protein includes a recombinant transferrin mutant having a mutation wherein the mutant has a stronger binding avidity for carbonate than wild-type serum transferrin.

In another embodiment, the transferrin portion of the transferrin fusion protein includes a recombinant human serum transferrin mutant having a mutation in at least one amino acid residue selected from the group consisting of Asp63, Gly65, Tyr95, Tyr188, His249, Asp392, Tyr426, Tyr514, Tyr517 and His585 of SEQ ID NO:3, wherein the mutant retains the ability to bind metal. In an alternate embodiment, a recombinant human serum transferrin mutant having a mutation in at least one amino acid residue selected from the group consisting of Asp63, Gly65, Tyr95, Tyr188, His249, Asp392, Tyr426, Tyr514, Tyr517 and His585 of SEQ ID NO:3, wherein the mutant has a reduced ability to bind metal. In another embodiment, a recombinant human serum transferrin mutant having a mutation in at least one amino acid residue selected from the group consisting of Asp63, Gly65, Tyr95, Tyr188, His249, Asp392, Tyr426, Tyr517 and His585 of SEQ ID NO:3, wherein the mutant does not retain the ability to bind metal.

In another embodiment, the transferrin portion of the transferrin fusion protein includes a recombinant Human serum transferrin mutant having a mutation at Lys206 or His207 of SEQ ID NO:3, wherein the mutant has a stronger binding avidity for metal than wild-type human serum transferrin (see U.S. Patent 5,986,067). In an alternate embodiment, the transferrin portion of the transferrin fusion protein includes a recombinant human serum transferrin mutant having a mutation at Lys206 or His207 of SEQ ID NO:3, wherein the mutant has a weaker binding avidity for metal than wild-type human serum transferrin. In a further embodiment, the transferrin portion of the transferrin fusion protein includes a recombinant human serum transferrin mutant having a mutation at Lys206 or His207 of SEQ ID NO:3, wherein the mutant does not bind metal.

Any available technique may be used to make the fusion proteins of the invention, including but not limited to molecular techniques commonly available, for instance, those disclosed in Sambrook et al. Molecular Cloning: A Laboratory Manual, 2nd Ed., Cold Spring Harbor Laboratory Press, 1989. When carrying out nucleotide substitutions using techniques for accomplishing site-specific mutagenesis that are well known in the art, the encoded amino acid changes are preferably of a minor nature, that is, conservative amino acid substitutions, although other, non-conservative, substitutions are contemplated as well, particularly when producing a modified transferrin portion of a Tf fusion protein, *e.g*., a modified Tf fusion protein exhibiting reduced glycosylation, reduced iron binding and the like. Specifically contemplated are amino acid substitutions, small deletions or insertions, typically of one to about 30 amino acids; insertions between transferrin domains; small amino- or carboxyl-terminal extensions, such as an amino-terminal methionine residue, or small linker peptides of less than 50, 40, 30, 20 or 10 residues between transferrin domains or linking a transferrin protein and a therapeutic protein or peptide; or a small extension that facilitates purification, such as a poly-histidine tract, an antigenic epitope or a binding domain.

Examples of conservative amino acid substitutions are substitutions made within the same group such as within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine).

Non-conservative substitutions encompass substitutions of amino acids in one group by amino acids in another group. For example, a non-conservative substitution would include the substitution of a polar amino acid for a hydrophobic amino acid. For a general description of nucleotide substitution, see *e.g.* Ford et al. (1991), Prot. Exp. Pur. 2: 95-107. Non-conservative substitutions, deletions and insertions are particularly useful to produce TF fusion proteins of the invention that exhibit no or reduced binding of iron, no or reduced binding of the fusion protein to the Tf receptor and/or no or reduced glycosylation.

In the polypeptide and proteins of the invention, the following system is followed for designating amino acids in accordance with the following conventional list:

**TABLE OF AMINO ACIDS**

| **AMINO ACID** | **ONE-LETTER SYMBOL** | **THREE-LETTER SYMBOL** |
|---|---|---|
| Alanine | A | Ala |
| Arginine | R | Arg |
| Asparagine | N | Asn |
| Aspartic Acid | D | Asp |
| Cysteine | C | Cys |
| Glutamine | Q | Gln |
| Glutamic Acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Iron binding and/or receptor binding may be reduced or disrupted by mutation, including deletion, substitution or insertion into, amino acid residues corresponding to one or more of Tf N domain residues Asp63, Tyr95, Tyr188, His249 and/or C domain residues Asp 392, Tyr 426, Tyr 514 and/or His 585. Iron binding may also be affected by mutation to amino acids Lys206, Hys207 or Arg632. Carbonate binding may be reduced or disrupted by mutation, including deletion, substitution or insertion into, amino acid residues corresponding to one or more of Tf N domain residues Thr120, Arg124, Ala126, Gly 127 and/or C domain residues Thr 452, Arg 456, Ala 458 and/or Gly 459. A reduction or disruption of carbonate binding may adversely affect iron and/or receptor binding.

Binding to the Tf receptor may be reduced or disrupted by mutation, including deletion, substitution or insertion into, amino acid residues corresponding to one or more of Tf N domain residues described above for iron binding.

As discussed above, glycosylation may be reduced or prevented by mutation, including deletion, substitution or insertion into, amino acid residues corresponding to one or more of Tf C domain residues around the N-X-S/T sites corresponding to C domain residues N413 and/or N611 (See U.S. Patent No. 5,986,067). For instance, the N413 and/or N611 may be mutated to Glu residues.

In instances where the Tf fusion proteins of the invention are not modified to prevent glycosylation, iron binding, carbonate binding and/or receptor binding, glycosylation, iron and/or carbonate ions may be stripped from or cleaved off of the fusion protein. For instance, available de-glycosylases may be used to cleave glycosylation residues from the fusion protein, in particular the sugar residues attached to the Tf portion, yeast deficient in glycosylation enzymes may be used to prevent glycosylation and/or recombinant cells may be grown in the presence of an agent that prevents glycosylation, *e.g*., tunicamycin.

Additional mutations may be made with Tf to alter the three dimensional structure of TF, such as modifications to the hinge region to prevent Tf folding needed for iron biding and Tf receptor recognition. For instance, mutations may be made in or around N domain amino acid residues 94-96, 245-247 and/or 316-318 as well as C domain amino acid residues 425-427, 581-582 and/or 652-658. In addition, mutations may be made in to or around the flanking regions of these sites to alter Tf structure and function.

In one aspect of the invention, the transferrin fusion protein can function as a carrier protein to extend the half life or bioavailability of the therapeutic protein as well as in some instances, delivering the therapeutic protein inside a cell and/or across the blood brain barrier. In an alternate embodiment, the transferrin fusion protein includes a modified transferrin molecule wherein the transferrin does not retain the ability to cross the blood brain barrier.

In another embodiment, the transferrin fusion protein includes a modified transferrin molecule wherein the transferrin molecule retains the ability to bind to the transferrin receptor and transport the therapeutic peptide inside cells. In an alternate embodiment, the transferrin fusion protein includes a modified transferrin molecule wherein the transferrin molecule does not retain the ability to bind to the transferrin receptor and transport the therapeutic peptide inside cells.

In further embodiments, the transferrin fusion protein includes a modified transferrin molecule wherein the transferrin molecule retains the ability to bind to the transferrin receptor and transport the therapeutic peptide inside cells, but does not retain the ability to cross the blood brain barrier. In an alternate embodiment, the transferrin fusion protein includes a modified transferrin molecule wherein the transferrin molecule retains the ability to cross the blood brain barrier, but does not retain the ability to bind to the transferrin receptor and transport the therapeutic peptide inside cells.

### Modified Transferrin Fusion Proteins

The fusion of proteins of the invention may contain one or more copies of the therapeutic protein attached to the N-terminus and/or the C-terminus of the Tf protein. In some embodiments, the therapeutic protein is attached to both the N- and C-terminus of the Tf protein and the fusion protein may contain one or more equivalents of the therapeutic protein on either or both ends of Tf. In other embodiments, the therapeutic protein or polypeptide is inserted into known domains of the Tf protein, for instance, into one or more of the loops of Tf (see Ali et al. (1999) J. Biolog. Chem. 274(34):24066-24073). In other embodiments, the therapeutic protein or therapeutic peptide is inserted between the N and C domains of Tf.

Generally, the transferrin fusion protein of the inventions of the invention may have one modified transferrin-derived region and one therapeutic protein-derived region. Multiple regions of each protein, however, may be used to make a transferrin fusion protein of the invention. Similarly, more than one therapeutic protein may be used to make a transferrin fusion protein of the invention of the invention, thereby producing a multi-functional modified Tf fusion protein.

In one embodiment, the transferrin fusion protein of the invention contains a therapeutic protein or portion thereof fused to a transferrin molecule or portion thereof. In another embodiment, the transferrin fusion protein of the inventions contains a therapeutic protein fused to the N terminus of a transferrin molecule. In an alternate embodiment, the transferrin fusion protein of the invention contains a therapeutic protein fused to the C terminus of a transferrin molecule. In a further embodiment, the transferrin fusion protein of the invention contains a transferrin molecule fused to the N terminus of a therapeutic protein. In an alternate embodiment, the transferrin fusion protein of the invention contains a transferrin molecule fused to the C terminus of a therapeutic protein.

In further embodiments, the modified transferrin molecule contains the N terminus of a transferrin molecule fused to what would be the N terminus of a therapeutic peptide. In an alternate embodiment, the modified transferrin molecule contains the N terminus of a transferrin molecule fused to the C terminus of a therapeutic peptide. In a further alternate embodiment, the modified transferrin molecule contains the C terminus of a transferrin molecule fused to what would be the C terminus of a therapeutic peptide. In an alternate embodiment, the modified transferrin molecule contains the C terminus of a transferrin molecule fused to the N terminus of a therapeutic peptide.

In other embodiments, the transferrin fusion protein of the inventions contains a therapeutic protein fused to both the N-terminus and the C-terminus of modified transferrin. In another embodiment, the therapeutic proteins fused at the N- and C- tennini are the same therapeutic proteins. In an alternate embodiment, the therapeutic proteins fused at the N- and C- tennini are different therapeutic proteins. In another alternate embodiment, the therapeutic proteins fused to the N- and C- termini are different therapeutic proteins which may be used to treat or prevent the same disease, disorder, or condition. In another embodiment, the therapeutic proteins fused at the N- and C- termini are different therapeutic proteins which may be used to treat or prevent diseases or disorders which are known in the art to commonly occur in patients simultaneously.

In addition to modified transferrin fusion protein of the inventions in which the modified transferrin portion is fused to the N terminal and/or C-terminal of the therapeutic protein portion, transferrin fusion protein of the inventions of the invention may also be produced by inserting the therapeutic protein or peptide of interest (*e.g*., a therapeutic protein or peptide as disclosed herein, or, for instance, a single chain antibody that binds a therapeutic protein or a fragment or variant thereof) into an internal region of the modified transferrin. Internal regions of modified transferrin include, but are not limited to, the iron binding sites, the hinge regions, the bicarbonate binding sites, or the receptor binding domain.

Within the protein sequence of the modified transferrin molecule a number of loops or turns exist, which are stabilized by disulfide bonds. These loops are useful for the insertion, or internal fusion, of therapeutically active peptides, particularly those requiring a secondary structure to be functional, or therapeutic proteins, to essentially generate a modified transferrin molecule with specific biological activity.

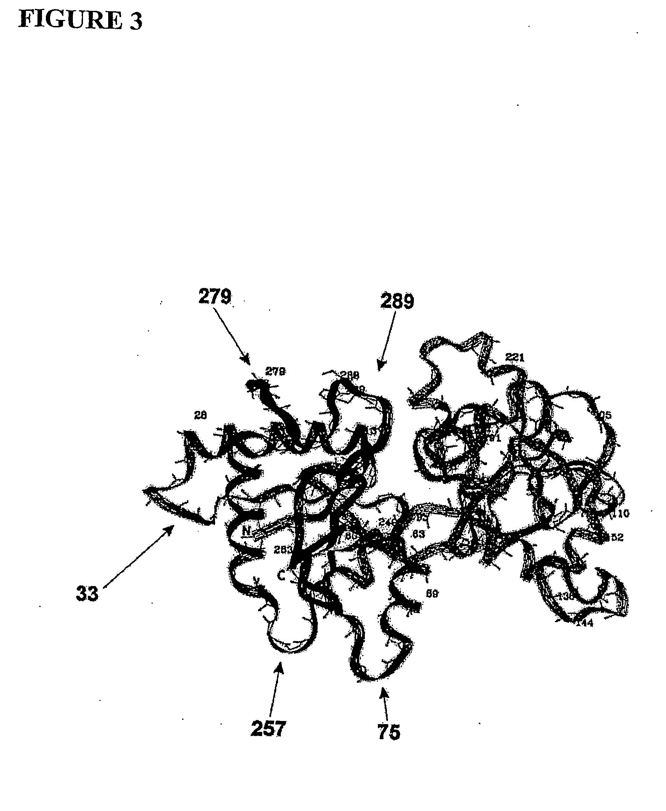

When therapeutic proteins or peptides are inserted into or replace at least one loop of a Tf molecule, insertions may be made within any of the surface exposed loop regions, in addition to other areas of Tf. For instance, insertions may be made within the loops comprising TF amino acids 32-33, 74-75, 256-257, 279-280 and 288-289 (Ali *et al*., *supra*) (See Figure 3). As previously described, insertions may also be made within other regions of Tf such as the sites for iron and bicarbonate binding, hinge regions, and the receptor binding domain as described in more detail below. The loops in the Tf protein sequence that are amenable to modification/replacement for the insertion of proteins or peptides may also be used for the development of a screenable library of random peptide inserts. Any procedures may be used to produce nucleic acid inserts for the generation of peptide libraries, including available phage and bacterial display systems, prior to cloning into a Tf domain and/or fusion to the ends of Tf.

The N-terminus of Tf is free and points away from the body of the molecule. Fusions of proteins or peptides on the N-terminus may therefore be a preferred embodiment. Such fusions may include a linker region, such as but not limited to a poly-glycine stretch, to separate the therapeutic protein or peptide from Tf. Attention to the junction between the leader sequence, the choice of leader sequence, and the structure of the mRNA by codon manipulation/optimization (no major stem loops to inhibit ribosome progress) will increase secretion and can be readily accomplished using standard recombinant protein techniques.

The C-terminus of Tf appears to be more buried and secured by a disulfide bond 6 amino acids from the C-terminus. In human Tf, the C-terminal amino acid is a proline which, depending on the way that it is orientated, will either point a fusion away or into the body of the molecule. A linker or spacer moiety at the C-terminus may be used in some embodiments of the invention.

In yet other embodiments, small molecule therapeutics may be complexed with iron and loaded on a modified Tf protein fusion for delivery to the inside of cells and across the BBB. The addition of a targeting peptide or, for example, a SCA will target the payload to a particular cell type, *e.g*., a cancer cell.

### Nucleic Acids

Nucleic acid molecules are also provided by the present invention. These encode a modified Tf fusion protein comprising a transferrin protein or a portion of a transferrin protein covalently linked or joined to a therapeutic protein. As discussed in more detail below, any therapeutic protein may be used. The fusion protein may further comprise a linker region, for instance a linker less than about 50, 40, 30, 20, or 10 amino acid residues. The linker can be covalently linked to and between the transferrin protein or portion thereof and the therapeutic protein. Nucleic acid molecules of the invention may be purified or not.

Host cells and vectors for replicating the nucleic acid molecules and for expressing the encoded fusion proteins are also provided. Any vectors or host cells may be used, whether prokaryotic or eukaryotic, but eukaryotic expression systems, in particular yeast expression systems, may be preferred. Many vectors and host cells are known in the art for such purposes. It is well within the skill of the art to select an appropriate set for the desired application.

DNA sequences encoding transferrin, portions of transferrin and therapeutic proteins of interest may be cloned from a variety of genomic or cDNA libraries known in the art. The techniques for isolating such DNA sequences using probe-based methods are conventional techniques and are well known to those skilled in the art. Probes for isolating such DNA sequences may be based on published DNA or protein sequences (see, for example, Baldwin, G.S. (1993) Comparison of Transferrin Sequences from Different Species. Comp. Biochem. Physiol. 106B/1:203-21S and all references cited therein). Alternatively, the polymerase chain reaction (PCR) method disclosed by Mullis et al. (U.S. Pat. No. 4,683,195) and Mullis (U.S. Pat. No. 4,683,202), may be used. The choice of library and selection of probes for the isolation of such DNA sequences is within the level of ordinary skill in the art.

As known in the art "similarity" between two polynucleotides or polypeptides is determined by comparing the nucleotide or amino acid sequence and its conserved nucleotide or amino acid substitutes of one polynucleotide or polypeptide to the sequence of a second polynucleotide or polypeptide. Also known in the art is "identify" which means the degree of sequence relatedness between two polypeptide or two polynucleotide sequences as determined by the identity of the mach between two strings of such sequences. Both identity and similarity can be readily calculated (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

While there exist a number of methods to measure identity and similarity between two polynucleotide or polypeptide sequences, the terms "identity" and "similarity" are well known to skilled artisans (Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., SIAM J. Applied Math., 48: 1073 (1988). Methods commonly employed to determine identity or similarity between two sequences include, but are not limited to those disclosed in Guide to Huge Computers, Martin J. Bishop, ed., Academic Press, San Diego, 1994, and Carillo, H., and Lipman, D., SIAM J. Applied Math. 48:1073 (1988).

Preferred methods to determine identity are designed to give the largest match between the two sequences tested. Methods to determine identity and similarity are codified in computer programs. Preferred computer program methods to determine identity and similarity between two sequences include, but are not limited to, GCG program package (Devereux, et al, Nucleic Acids Research 12(1):387 (1984)), BLASTP, BLASTN, PASTY (Atschul, et al., J. Molec. Biol. 215:403 (1990)). The degree of similarity or identity referred to above is determined as the degree of identity between the two sequences indicating a derivation of the first sequence from the second. The degree of identity between two nucleic acid sequences may be determined by means of computer programs known in the art such as GAP provided in the GCG program package (Needleman and Wunsch (1970) Journal of Molecular Biology 45:443-453). For purposes of determining the degree of identity between two nucleic acid sequences for the present invention, GAP is used with the following settings: GAP creation penalty of 5.0 and GAP extension penalty of 0.3.

### Codon Optimization

The degeneracy of the genetic code permits variations of the nucleotide sequence of a transferrin protein and/or therapeutic protein of interest, while still producing a polypeptide having the identical amino acid sequence as the polypeptide encoded by the native DNA sequence. The procedure, known as "codon optimization" (described in U.S. Patent 5,547,871) provides one with a means of designing such an altered DNA sequence. The design of codon optimized genes should take into account a variety of factors, including the frequency of codon usage in an organism, nearest neighbor frequencies, RNA stability, the potential for secondary structure formation, the route of synthesis and the intended future DNA manipulations of that gene. In particular, available methods may be used to alter the codons encoding a given fusion protein with those most readily recognized by yeast when yeast expression systems are used.

The degeneracy of the genetic code permits the same amino acid sequence to be encoded and translated in many different ways. For example, leucine, serine and arginine are each encoded by six different codons, while valine, proline, threonine, alanine and glycine are each encoded by four different codons. However, the frequency of use of such synonymous codons varies from genome to genome among eukaryotes and prokaryotes. For example, synonymous codon-choice patterns among mammals are very similar, while evolutionarily distant organisms such as yeast (*S. cerevisiae*), bacteria (such as *E. coli*) and insects (such as *D. melanogaster*) reveal a clearly different pattern of genomic codon use frequencies (Grantham, R., et al., Nucl. Acids Res., 8, 49-62 (1980); Grantham, R., et al., Nucl. Acids Res., 9, 43-74 (1981); Maroyama, T., et al., Nucl. Acids Res., 14, 151-197 (1986); Aota, S., et al., Nucl. Acids Res., 16, 315-402 (1988); Wada, K., et al., Nucl. Acids Res., 19 Supp., 1981-1985 (1991); Kurland, C. G., FEBS Letters, 285, 165-169 (1991)). These differences in codon-choice patterns appear to contribute to the overall expression levels of individual genes by modulating peptide elongation rates. (Kurland, C. G., FEBS Letters, 285, 165-169 (1991); Pedersen, S., EMBO J., 3, 2895-2898 (1984); Sorensen, M. A., J. Mol. Biol., 207, 365-377 (1989); Randall, L. L., et al., Eur. J. Biochem., 107, 375-379 (1980); Curran, J. F., and Yarus, M., J. Mol. Biol., 209, 65-77 (1989); Varenne, S., et al., J. Mol, Biol., 180, 549-576 (1984), Varenne, S., et al., J. Mol, Biol., 180, 549-576 (1984); Garel, J.-P., J. Theor. Biol., 43, 211-225 (1974); Ikemura, T., J. Mol. Biol., 146, 1-21 (1981); Ikemura, T., J. Mol. Biol., 151, 389-409 (1981)).

The preferred codon usage frequencies for a synthetic gene should reflect the codon usages of nuclear genes derived from the exact (or as closely related as possible) genome of the cell/organism that is intended to be used for recombinant protein expression, particularly that of yeast species. As discussed above, in one preferred embodiment the human Tf sequence is codon optimized, before or after modification as herein described for yeast expression as may be the therapeutic protein nucleotide sequence(s).

### Vectors

Expression units for use in the present invention will generally comprise the following elements, operably linked in a 5' to 3' orientation: a transcriptional promoter, a secretory signal sequence, a DNA sequence encoding a modified Tf fusion protein comprising transferrin protein or a portion of a transferrin protein joined to a DNA sequence encoding a therapeutic protein or peptide of interest and a transcriptional terminator. As discussed above, any arrangement of the therapeutic protein or peptide fused to or within the Tf portion may be used in the vectors of the invention. The selection of suitable promoters, signal sequences and terminators will be determined by the selected host cell and will be evident to one skilled in the art and are discussed more specifically below.

Suitable yeast vectors for use in the present invention are described in U.S. Patent 6,291,212 and include YRp7 (Struhl et al., Proc. Natl. Acad. Sci. USA 76: 1035-1039, 1978), YEp13 (Broach et al., Gene 8: 121-133, 1979), pJDB249 and pJDB219 (Beggs, Nature 275:104-108, 1978), pPPC0005, pSeCHSA, pScNHSA, pC4 and derivatives thereof. Useful yeast plasmid vectors also include pRS403-406, pRS413-416 and the *Pichia* vectors available from Stratagene Cloning Systems, La Jolla, CA 92037, USA. Plasmids pRS403, pRS404, pRS405 and pRS406 are Yeast Integrating plasmids (YIps) and incorporate the yeast selectable markers HIS3, 7RPI, LEU2 and URA3. PlasmidspRS413∼41.6 are Yeast Centromere plasmids (Ycps).

Such vectors will generally include a selectable marker, which may be one of any number of genes that exhibit a dominant phenotype for which a phenotypic assay exists to enable transformants to be selected. Preferred selectable markers are those that complement host cell auxotrophy, provide antibiotic resistance or enable a cell to utilize specific carbon sources, and include LEU2 (Broach *et al.* ibid.), URA3 (Botstein et al., Gene 8: 17, 1979), HIS3(Struhl *et al.,* ibid.) or POT1 (Kawasaki and Bell, EP 171,142). Other suitable selectable markers include the CAT gene, which confers chloramphenicol resistance on yeast cells. Preferred promoters for use in yeast include promoters from yeast glycolytic genes (Hitzeman et al., J Biol. Chem. 225: 12073-12080, 1980; Alber and Kawasaki, J. Mol. Appl. Genet. 1: 419-434, 1982; Kawasaki, U.S. Pat. No. 4,599,311) or alcohol dehydrogenase genes (Young et al., in Genetic Engineering of Microorganisms for Chemicals, Hollaender et al., (eds.), p. 355, Plenum, N.Y., 1982; Ammerer, Meth. Enzymol. 101: 192-201, 1983). In this regard, particularly preferred promoters are the TPI1 promoter (Kawasaki, U.S. Pat. No. 4,599,311) and the ADH2-4.sup.C [see U.S. Patent 6,291,212] promoter (Russell et al., Nature 304: 652-654, 1983). The expression units may also include a transcriptional terminator. A preferred transcriptional terminator is the TPI1 terminator (Alber and Kawasaki, ibid.).

In addition to yeast, modified fusion proteins of the present invention can be expressed in filamentous fungi, for example, strains of the fungi *Aspergillus.* Examples of useful promoters include those derived from *Aspergillus nidulans* glycolytic genes, such as the ADH3 promoter (McKnight et al., EMBO J. 4: 2093-2099, 1985) and the tpiA promoter. An example of a suitable terminator is the ADH3 terminator (McKnight *et al*., ibid.). The expression units utilizing such components may be cloned into vectors that are capable of insertion into the chromosomal DNA of *Aspergillus,* for example.

Mammalian expression vectors for use in carrying out the present invention will include a promoter capable of directing the transcription of the modified Tf fusion protein. Preferred promoters include viral promoters and cellular promoters. Preferred viral promoters include the major late promoter from adenovirus 2 (Kaufman and Sharp, Mol. Cell. Biol. 2: 1304-13199, 1982) and the SV40 promoter (Subramani et al., Mol. Cell. Biol. 1: 854-864, 1981). Preferred cellular promoters include the mouse metallothionein 1 promoter (Palmiter et al., Science 222: 809-814, 1983) and a mouse V.sub..kappa. [see U.S. Patent 6,291,212] promoter (Grant et al., Nuc. Acids Res. 15: 5496, 1987). A particularly preferred promoter is a mouse V.sub.H [see U.S. Patent 6,291,212] promoter (Loh *et al*., ibid.). Such expression vectors may also contain a set of RNA splice sites located downstream from the promoter and upstream from the DNA sequence encoding the transferrin fusion protein. Preferred RNA splice sites may be obtained from adenovirus and/or immunoglobulin genes.

Also contained in the expression vectors is a polyadenylation signal located downstream of the coding sequence of interest. Polyadenylation signals include the early or late polyadenylation signals from SV40 (Kaufman and Sharp, ibid.), the polyadenylation signal from the adenovirus 5 ElB region and the human growth hormone gene terminator (DeNoto et al., Nuc. Acids Res. 9: 3719-3730, 1981). A particularly preferred polyadenylation signal is the V.sub.H [see U.S. Patent 6,291,212] gene terminator (Loh *et al*., ibid.). The expression vectors may include a noncoding viral leader sequence, such as the adenovirus 2 tripartite leader, located between the promoter and the RNA splice sites. Preferred vectors may also include enhancer sequences, such as the SV40 enhancer and the mouse .mu. [see U.S. Patent 6,291,212] enhancer (Gillies, Cell 33: 717-728, 1983). Expression vectors may also include sequences encoding the adenovirus VA RNAs.

### Transformation

Techniques for transforming fungi are well known in the literature, and have been described, for instance, by Beggs (ibid.), Hinnen et al. (Proc. Natl. Acad. Sci. USA 75: 1929-1933, 1978), Yelton et al., (Proc. Natl. Acad. Sci. USA 81: 1740-1747, 1984), and Russell (Nature 301: 167-169, 1983). The genotype of the host cell will generally contain a genetic defect that is complemented by the selectable marker present on the expression vector. Choice of a particular host and selectable marker is well within the level of ordinary skill in the art.

Cloned DNA sequences comprising modified Tf fusion proteins of the invention may be introduced into cultured mammalian cells by, for example, calcium phosphate-mediated transfection (Wigler et al., Cell 14: 725, 1978; Corsaro and Pearson, Somatic Cell Genetics 7: 603, 1981; Graham and Van der Eb, Virology 52: 456, 1973.) Other techniques for introducing cloned DNA sequences into mammalian cells, such as electroporation (Neumann et al., EMBO J. 1: 841-845, 1982), or lipofection may also be used. In order to identify cells that have integrated the cloned DNA, a selectable marker is generally introduced into the cells along with the gene or cDNA of interest. Preferred selectable markers for use in cultured mammalian cells include genes that confer resistance to drugs, such as neomycin, hygromycin, and methotrexate. The selectable marker may be an amplifiable selectable marker. A preferred amplifiable selectable marker is the DHFR gene. A particularly preferred amplifiable marker is the DHFR.sup.r [see U.S. Patent 6,291,212] cDNA (Simonsen and Levinson, Proc. Natl. Adac. Sci. USA 80: 2495-2499, 1983). Selectable markers are reviewed by Thilly (Mammalian Cell Technology, Butterworth Publishers, Stoneham, Mass.) and the choice of selectable markers is well within the level of ordinary skill in the art.

### Host Cells

The present invention also includes a cell, preferably a yeast cell transformed to express a modified transferrin fusion protein of the invention. In addition to the transformed host cells themselves, the present invention also includes a culture of those cells, preferably a monoclonal (clonally homogeneous) culture, or a culture derived from a monoclonal culture, in a nutrient medium. If the polypeptide is secreted, the medium will contain the polypeptide, with the cells, or without the cells if they have been filtered or centrifuged away.

Host cells for use in practicing the present invention include eukaryotic cells, and in some cases prokaryotic cells, capable of being transformed or transfected with exogenous DNA and grown in culture, such as cultured mammalian, insect, fungal, plant and bacterial cells.

Fungal cells, including species of yeast (*e.g., Saccharomyces* spp., *Schizosaccharomyces* spp., *Pichia* spp.) may be used as host cells within the present invention. Exemplary genera of yeast contemplated to be useful in the practice, of the present invention as hosts for expressing the, transferrin fusion protein of the inventions are *Pichia* (formerly classified as *Hansenula*), *Saccharomyces, Kluyveromyces, Aspergillus, Candida, Torulopsis, Torulaspora, Schizosaccharomyces, Citeromyces, Pachysolen, Zygosaecharomyces, Debaromyces, Trichoderma, Cephalosporium, Humicola, Mucor, Neurospora, Yarrowia, Metschunikowia, Rhodosporidium, Leucosporidium, Botryoascus, Sporidiobolus, Endomycopyis,* and the like. Examples of *Saccharomyces* spp. are *S. cerevisiae, S. italicus and S. rouxii.* Examples of *Kluyveromyces* spp. are *K. ftagilis, K. lactis and K. marxianus.* A suitable *Tórulasppra* species is *T. delbrueckii.* Examples of *Pichia* (*Hansenula*) spp. are *P. angusta* (formerly *H. polymorpha*), *P. anomala* (formerly *H. anomala*) and *P. pastoris.*

Particularly useful host cells to produce the Tf fusion proteins of the invention are the methanoltrophic *Pichia pastoris* (Steinlein et al. (1995) Protein Express. Purif. 6:619-624*). Piclaia pastoris* has been developed to be an outstanding host for the production of foreign proteins since its alcohol oxidase promoter was isolated and cloned; its transformation was first reported in 1985. *P. pastoris* can utilize methanol as a carbon source in the absence of glucose. The *P. pastoris* expression system can use the methanol-induced alcohol oxidase (AOX1) promoter, which controls the gene that codes for the expression of alcohol oxidase, the enzyme which catalyzes the first step in the metabolism of methanol. This promoter has been characterized and incorporated into a series of *P. pastoris* expression vectors. Since the proteins produced in *P. pastoris* are typically folded correctly and secreted into the medium, the fermentation of genetically engineered *P. pastoris* provides an excellent alternative to E. coli expression systems. A number of proteins have been produced using this system, including tetanus toxin fragment, Bordatella pertussis pertactin, human serum albumin and lysozyme.

The transformation of *F. oxysporum* may, for instance, be carried out as described by Malardier et al. (1989) Gene 78:147-156.

Strains of the yeast *Saccharomyces cerevisiae* are another preferred host. In a preferred embodiment, a yeast cell, or more specifically, a *Saccharomyces cerevisiae* host cell that contains a genetic deficiency in a gene required for asparagine-linked glycosylation of glycoproteins is used. *S. cerevisiae* host cells having such defects may be prepared using standard techniques of mutation and selection, although many available yeast strains have been modified to prevent or reduce glycosylation or hypermannosylation. Ballou et al. (J. Biol. Chem. 255: 5986-5991, 1980) have described the isolation of mannoprotein biosynthesis mutants that are defective in genes which affect asparagine-linked glycosylation.

To optimize production of the heterologous proteins, it is also preferred that the host strain carries a mutation, such as the *S. cerevisiae* pep4 mutation (Jones, Genetics 85: 23-33, 1977), which results in reduced proteolytic activity. Host strains containing mutations in other protease encoding regions are particularly useful to produce large quantities of the Tf fusion proteins of the invention.

Host cells containing DNA constructs of the present invention are grown in an appropriate growth medium. As used herein, the term "appropriate growth medium" means a medium containing nutrients required for the growth of cells. Nutrients required for cell growth may include a carbon source, a nitrogen source, essential amino acids, vitamins, minerals and growth factors. The growth medium will generally select for cells containing the DNA construct by, for example, drug selection or deficiency in an essential nutrient which are complemented by the selectable marker on the DNA construct or cotransfected with the DNA construct. Yeast cells, for example, are preferably grown in a chemically defined medium, comprising a non-amino acid nitrogen source, inorganic salts, vitamins and essential amino acid supplements. The pH of the medium is preferably maintained at a pH greater than 2 and less than 8, preferably at pH 6.5. Methods for maintaining a stable pH include buffering and constant pH control, preferably through the addition of sodium hydroxide. Preferred buffering agents include succinic acid and Bis-Tris (Sigma Chemical Co., St. Louis, Mo.). Yeast cells having a defect in a gene required for asparagine-linked glycosylation are preferably grown in a medium containing an osmotic stabilizer. A preferred osmotic stabilizer is sorbitol supplemented into the medium at a concentration between 0.1 M and 1.5 M., preferably at 0.5 M or 1.0 M.

Cultured mammalian cells are generally grown in commercially available serum-containing or serum-free media. Selection of a medium appropriate for the particular cell line used is within the level of ordinary skill in the art. Transfected mammalian cells are allowed to grow for a period of time, typically 1-2 days, to begin expressing the DNA sequence(s) of interest. Drug selection is then applied to select for growth of cells that are expressing the selectable marker in a stable fashion. For cells that have been transfected with an amplifiable selectable marker the drug concentration may be increased in a stepwise manner to select for increased copy number of the cloned sequences, thereby increasing expression levels.

Baculovirus/insect cell expression systems may also be used to produce the modified Tf fusion proteins of the invention. The BacPAK™ Baculovirus Expression System (BD Biosciences (Clontech) expresses recombinant proteins at high levels in insect host cells. The target gene is inserted into a transfer vector, which is cotransfected into insect host cells with the linearized BacPAK6 viral DNA. The BacPAK6 DNA is missing an essential portion of the baculovirus genome. When the DNA recombines with the vector, the essential element is restored and the target gene is transferred to the baculovirus genome. Following recombination, a few viral plaques are picked and purified, and the recombinant phenotype is verified. The newly isolated recombinant virus can then be amplified and used to infect insect cell cultures to produce large amounts of the desired protein.

### Secretory Signal Sequences

The terms "secretory signal sequence" or "signal sequence" or "secretion leader sequence" are used interchangeably and are described, for example in U.S. Pat. 6,291,212 and U.S. Pat 5,547,871, both of which are herein incorporated by reference in their entirety. Secretory signal sequences or signal sequences or secretion leader sequences encode secretory peptides. A secretory peptide is an amino acid sequence that acts to direct the secretion of a mature polypeptide or protein from a cell. Secretory peptides are generally characterized by a core of hydrophobic amino acids and are typically (but not exclusively) found at the amino termini of newly synthesized proteins. Very often the secretory peptide is cleaved from the mature protein during secretion. Secretory peptides may contain processing sites that allow cleavage of the signal peptide from the mature protein as it passes through the secretory pathway. Processing sites may be encoded within the signal peptide or may be added to the signal peptide by, for example, *in vitro* mutagenesis.

Secretory peptides may be used to direct the secretion of modified Tf fusion proteins of the invention. One such secretary peptide that may be used in combination with other secretory peptides is the third domain of the yeast Barrier protein. Secretory signal sequences or signal sequences or secretion leader sequences are required for a complex series of post-translational processing steps which result in secretion of a protein. If an intact signal sequence is present, the protein being expressed enters the lumen of the rough endoplasmic reticulum and is then transported through the Golgi apparatus to secretory vesicles and is finally transported out of the cell. Generally, the signal sequence immediately follows the initiation codon and encodes a signal peptide at the amino-terminal end of the protein to be secreted. In most cases, the signal sequence is cleaved off by a specific protease, called a signal peptidase. Preferred signal sequences improve the processing and export efficiency of recombinant protein expression using viral, mammalian or yeast expression vectors. In some cases, the native Tf signal sequence may be used to express and secrete fusion proteins of the invention.

### Linkers

The Tf moiety and therapeutic protein moiety(s) of the modified transferrin fusion proteins of the invention can be fused directly or using a linker peptide of various lengths to provide greater physical separation and allow more spatial mobility between the fused proteins and thus maximize the accessibility of the therapeutic protein portion, for instance, for binding to its cognate receptor. The linker peptide may consist of amino acids that are flexible or more rigid. For example, a linker such as but not limited to a poly-glycine stretch. The linker can be less than about 50, 40, 30, 20, or 10 amino acid residues. The linker can be covalently linked to and between the transferrin protein or portion thereof and the therapeutic protein.

### Detection of Tf Fusion Proteins

Assays for detection of biologically active modified transferrin-therapeutic protein fusions may include Western transfer, protein blot or colony filter as well as activity based assays that detect the fused therapeutic protein. A Western transfer filter may be prepared using the method described by Towbin et al. (Proc. Natl. Acad. Sci. USA 76: 4350-4354, 1979). Briefly, samples are electrophoresed in a sodium dodecylsulfate polyacrylamide gel. The proteins in the gel are electrophoretically transferred to nitrocellulose paper. Protein blot filters may be prepared by filtering supernatant samples or concentrates through nitrocellulose filters using, for example, a Minifold (Schleicher & Schuell, Keene, N.H.). Colony filters may be prepared by growing colonies on a nitrocellulose filter that has been laid across an appropriate growth medium. In this method, a solid medium is preferred. The cells are allowed to grow on the filters for at least 12 hours. The cells are removed from the filters by washing with an appropriate buffer that does not remove the proteins bound to the filters. A preferred buffer comprises 25 mM Tris-base, 19 mM glycine, pH 8.3, 20% methanol.

Fusion proteins of the invention may also be detected by assaying for the activity of the therapeutic protein moiety. Such assays are readily available, including but not limited to, those assays described in Table 1. Specifically, transferrin fusion proteins of the invention may be assayed for functional activity (*e.g*., biological activity or therapeutic activity) using the assay referenced in the "Exemplary Activity Assay" column of Table 1. Additionally, one of skill in the art may routinely assay fragments of a therapeutic protein corresponding to a therapeutic protein portion of a fusion protein of the invention, for activity using assays referenced in its corresponding row of Table 1. Further, one of skill in the art may routinely assay fragments of a modified transferrin protein for activity using assays known in the art.

For example, in one embodiment where one is assaying for the ability of a transferrin fusion protein of the invention to bind or compete with a therapeutic protein for binding to an anti-therapeutic polypeptide antibody and/or anti-transferrin antibody, various immunoassays known in the art can be, used, including but not limited to, competitive and non-competitive assay systems using techniques such as radioimmunoassays, ELISA (enzyme linked immunosorbent assay), sandwich immunoassays, immunoradiometric assays, gel diffusion precipitation reactions, immunodiffusion assays, *in situ* immunoassays (using colloidal gold, enzyme or radioisotope labels, for example), western blots, precipitation reactions, agglutination assays (*e.g*., gel agglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are know in the art for detecting binding in an immunoassay and are within the scope of the present invention.

In a further embodiment, where a binding partner (*e.g*., a receptor or a ligand) of a therapeutic protein is identified, binding to that binding partner by a transferrin fusion protein containing that therapeutic protein as the therapeutic protein portion of the fusion can be assayed, *e.g*., by means well-known in the art, such as, for example, reducing and non-reducing gel chromatography, protein affinity chromatography, and affinity blotting. Other methods will be known to the skilled artisan and are within the scope of the invention.

### Isolation/Purification of Modified Transferrin Fusion Proteins

Secreted, biologically active, modified transferrin fusion proteins may be isolated from the medium of host cells grown under conditions that allow the secretion of the biologically active fusion proteins. The cell material is removed from the culture medium, and the biologically active fusion proteins are isolated using isolation techniques known in the art. Suitable isolation techniques include precipitation and fractionation by a variety of chromatographic methods, including gel filtration, ion exchange chromatography and affinity chromatography.

A particularly preferred purification method is affinity chromatography on an iron binding or metal chelating column or an immunoaffinity chromatography using an antibody directed against the transferrin or therapeutic protein or peptide portion of the polypeptide fusion. The antibody is preferably immobilized or attached to a solid support or substrate. A particularly preferred substrate is CNBr-activated Sepharose (Pharmacia LKB Technologies, Inc., Piscataway, N.J.). By this method, the medium is combined with the antibody/substrate under conditions that will allow binding to occur. The complex may be washed to remove unbound material, and the transferrin fusion protein is released or eluted through the use of conditions unfavorable to complex formation. Particularly useful methods of elution include changes in pH, wherein the immobilized antibody has a high affinity for the ligand at a first pH and a reduced affinity at a second (higher or lower) pH; changes in concentration of certain chaotropic agents; or through the use of detergents.

### Labeled Modified Transferrin Fusion Proteins

Transferrin fusion proteins of the present invention may also be labeled with a radioisotope or other imaging agent and used for *in vivo* diagnostic purposes. Preferred radioisotope imaging agents include iodine-125 and technetium-99, with technetium-99 being particularly preferred. Methods for producing protein-isotope conjugates are well known in the art, and are described by, for example, Eckelman et al. (U.S. Pat. No. 4,652,440), Parker et al. (WO 87/05030) and Wilber et al. (EP 203,764). Alternatively, the transferrin fusion proteins may be bound to spin label enhancers and used for magnetic resonance (MR) imaging. Suitable spin label enhancers include stable, sterically hindered, free radical compounds such as nitroxides. Methods for labeling ligands for MR imaging are disclosed by, for example, Coffman et al. (U.S. Pat. No. 4,656,026). For administration, the labeled transferrin fusion proteins are combined with a pharmaceutically acceptable carrier or diluent, such as sterile saline or sterile water. Administration is preferably by bolus injection, preferably intravenously.

### Production of Fusion Proteins

The present invention further provides methods for producing a modified fusion protein of the invention using nucleic acid molecules herein described. In general terms, the production of a recombinant form of a protein typically involves the following steps.

A nucleic acid molecule is first obtained that encodes a transferrin fusion protein of the invention. The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be accomplished in a variety of ways. For example, the construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier and are otherwise known to persons skilled in the art. Suitable restriction sites can, if not normally available, be added to the ends of the coding sequence so as to provide an excisable gene to insert into these vectors. A skilled artisan can readily adapt any host/expression system known in the art for use with the nucleic acid molecules of the invention to produce a desired recombinant protein.

As discussed above, any expression system may be used, including yeast, bacterial, animal, plant, eukaryotic and prokaryotic systems. In some embodiments, yeast, mammalian cell culture and transgenic animal or plant production systems are preferred. In other embodiments, yeast systems that have been modified to reduce native yeast glycosylation, hyper-glycosylation or proteolytic activity may be used.

### Therapeutic Molecules

Any therapeutic molecule may be used as the fusion partner to Tf according to the methods and compositions of the present invention. As used herein, a therapeutic molecule is typically a protein or peptide capable of exerting a beneficial biological effect *in vitro* or *in vivo* and includes proteins or peptides that exert a beneficial effect in relation to normal homeostasis, physiology or a disease state. Therapeutic molecules do not include, fusion partners commonly used as markers or protein purification aids, such as galactosidases (see for example, U.S. Patent 5, 986, 067 and Aldred et al. (1984) Biochem. Biophys. Res. Commun. 122: 960-965). For instance, a beneficial effect as related to a disease state includes any effect that is advantageous to the treated subject, including disease prevention, disease stabilization, the lessening or alleviation of disease symptoms or a modulation, alleviation or cure of the underlying defect to produce an effect beneficial to the treated subject.

A modified transferrin fusion protein of the invention includes at least a fragment or variant of a therapeutic protein and at least a fragment or variant of modified serum transferrin, which are associated with one another, preferably by genetic fusion or chemical conjugation.

In one embodiment, the transferrin fusion protein includes a modified transferrin molecule linked to a neuropharmaceutical agent. In another embodiment, the modified transferrin fusion protein includes transferrin at the carboxyl terminus linked to a neuropharmaceutical agent at the amino terminus. In an alternate embodiment, the modified transferrin fusion protein includes transferrin at the amino terminus linked to a neuropharmaceutical agent at the carboxy terminus. In specific embodiments, the neuropharmaceutical agent is either nerve growth factor or ciliary neurotrophic factor.

In further embodiments, a modified transferrin fusion protein of the invention may contain at least a fragment or variant of a therapeutic protein, and/or at least a fragment or variant of an antibody. In a further embodiment, the transferrin fusion proteins can contain peptide fragments or peptide variants of proteins or antibodies wherein the variant or fragment retains at least one biological or therapeutic activity. The transferrin fusion proteins can contain therapeutic proteins that can be peptide fragments or peptide variants at least about 4, at least 5, at least 6, at least 7, at least 8, at least 9, at least 10, at least 11, at least 12, at least 13, at least 14, at least 15, at least 20, at least 25, at least 30, at least 35, or at least about 40, at least about 50, at least about 55, at least about 60 or at least about 70 or more amino acids in length fused to the N and/or C termini, inserted within, or inserted into a loop of a modified transferrin.

In another embodiment, the modified transferrin fusion molecules contain a therapeutic protein portion that can be fragments of a therapeutic protein that include the full length protein as well as polypeptides having one or more residues deleted from the amino terminus of the amino acid sequence.

In another embodiment, the modified transferrin fusion molecules contain a therapeutic protein portion that can be fragments of a therapeutic protein that include the full length protein as well as polypeptides having one or more residues deleted from the carboxy terminus of the amino acid sequence.

In another embodiment, the modified transferrin fusion molecules contain a therapeutic protein portion that can have one or more amino acids deleted from both the amino and the carboxy termini.

In another embodiment, the modified transferrin fusion molecules contain a therapeutic protein portion that is at least about 80%, 85%, 90%, 95%, 96%, 97%, 98% or 99% identical to a reference therapeutic protein set forth herein, or fragments thereof In further embodiments, the transferrin fusion molecules contain a therapeutic protein portion that is at least about 80%, 85%, 90%, 95%, 96%, 97%, 98% or 99% identical to reference polypeptides having the amino acid sequence ofN- and C-terminal deletions as described above.

In another embodiment, the modified transferrin fusion molecules contain the therapeutic protein portion that is at least about 80%, 85%, 90%, 95%, 96%, 97%, 98%, 99% or 100%, identical to, for example, the native or wild-type amino acid sequence of a therapeutic protein. Fragments, of these polypeptides are also provided.

The therapeutic proteins corresponding to a therapeutic protein portion of a modified transferrin fusion protein of the invention, such as cell surface and secretory proteins, can be modified by the attachment, of one or more oligosaccharide groups. The modification referred to as glycosylation, can significantly affect the physical properties of proteins and can be important in protein stability, secretion, and localization. Glycosylation occurs at specific locations along the polypeptide backbone. There are usually two major types of glycosylation: glycosylation characterized by O-linked oligosaccharides, which are attached to serine or threonine residues; and glycosylation characterized by N-linked oligosaccharides, which are attached to asparagine residues in an Asn-X-Ser/Thr sequence, where X can be an amino add except proline. Variables such as protein structure and cell type influence the number and nature of the carbohydrate units within the chains at different glycosylation sites. Glycosylation isomers are also common at the same site within a given cell type. For example, several types of human interferon are glycosylated.

Therapeutic proteins corresponding to a therapeutic protein portion of a transferrin fusion protein of the invention, as well as analogs and variants thereof, may be modified so that glycosylation at one or more sites is altered as a result of manipulation(s) of their nucleic acid sequence by the host cell in which they are expressed, or due to other conditions of their expression. For example, glycosylation isomers may be produced by abolishing or introducing glycosylation sites, *e.g.*, by substitution or deletion of amino acid residues, such as substitution of glutamine for asparagine, or unglycosylated recombinant proteins may be produced by expressing the proteins in host cells that will not glycosylate them, *e.g.* in glycosylation-deficient yeast. These approaches are known in the art.

Therapeutic proteins and their nucleic acid sequences are well known in the art and available in public databases such as Chemical Abstracts Services Databases (*e.g*., the CAS Registry), GenBank, and GenSeq.

In other embodiments, the transferrin fusion proteins of the invention are capable of a therapeutic activity and/or biologic activity, corresponding to the therapeutic activity and/or biologic activity of the therapeutic protein listed in the corresponding row of Table 1 and elsewhere in this application. (See, *e.g*., the "Biological Activity" and "Therapeutic Protein X" columns of Table 1.) In further embodiments, the therapeutically active protein portions of the transferrin fusion proteins of the invention are fragments or variants of the reference sequences cited herein.

The present invention is further directed to modified Tf fusion proteins comprising fragments of the therapeutic proteins herein described. Even if deletion of one or more amino acids from the N-terminus of a protein results in modification or loss of one or more biological functions of the therapeutic protein portion, other therapeutic activities and/or functional activities (*e.g*., biological activities, ability to multimerize, ability to bind a ligand) may still be retained. For example, the ability of polypeptides with N-terminal deletions to induce and/or bind to antibodies which recognize the complete or mature forms of the polypeptides generally will be retained with less than the majority of the residues of the complete polypeptide removed from the N-terminus. Whether a particular polypeptide lacking N-terminal residues of a complete polypeptide retains such immunologic activities can be assayed by routine methods described herein and otherwise known in the art. It is not unlikely that a mutant with a large number of deleted N-terminal amino acid residues may retain some biological or immunogenic activities. In fact, peptides composed of as few as six amino acid residues may often evoke an immune response.

Also as mentioned above, even if deletion of one or more amino acids from the N-terminus or C-terminus of a therapeutic protein results in modification or loss of one or more biological functions of the protein, other functional activities (*e.g.,* biological activities, ability to multimerize, ability to bind a ligand) and/or therapeutic activities may still be retained. For example the ability of polypeptides with C-teminal deletions to induce and/or bind to antibodies which recognize the complete or mature forms of the polypeptide generally will be retained when less than the majority of the residues of the complete or mature polypeptide are removed from the C-terminus. Whether a particular polypeptide lacking the N-terminal and/or, C-terminal residues of a reference polypeptide retains therapeutic activity can readily be determined by routine methods described herein and/or otherwise known in the art.

Peptide fragments of the therapeutic proteins can be fragments comprising, or alternatively, consisting of, an amino acid sequence that displays a therapeutic activity and/or functional activity (*e.g*. biological activity) of the polypeptide sequence of the therapeutic protein of which the amino acid sequence is a fragment.

Other polypeptide fragments are biologically active fragments. Biologically active fragments are those exhibiting activity similar, but not necessarily identical, to an activity of a therapeutic protein used in the present invention. The biological activity of the fragments may include an improved desired activity, or a decreased undesirable activity.

Generally, variants of proteins are overall very similar, and, in many regions, identical to the amino acid sequence of the therapeutic protein corresponding to a therapeutic protein portion of a transferrin fusion protein of the invention. Nucleic acids encoding these variants are also encompassed by the invention.

Further therapeutic polypeptides that may be used in the invention are polypeptides encoded by polynucleotides which hybridize to the complement of a nucleic acid molecule encoding an amino acid sequence of a therapeutic protein under stringent hybridization conditions which are known to those of skill in the art. (see, for example, Ausubel, F.M. et al., eds., 1989 Current protocol in Molecular Biology, Green Publishing Associates, Inc., and John Wiley & Sons Inc., New. York). Polynucleotides encoding these polypeptides are also encompassed by the invention.

By a polypeptide-having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino- or carboxy-terminal positions of the reference amino acid sequence or anywhere between those terminal positions, interspersed either individually among residues in the reference sequence, or in one or more contiguous groups within the reference sequence.

As a practical matter, whether any particular polypeptide is at least about 80%, 85%, 90%,95%, 96%, 97%, 98% or 99% identical to, for instance, the amino acid sequence of a transferrin fusion protein of the invention or a fragment thereof (such, as the therapeutic protein portion of the transferrin fusion protein or the transferrin portion of the transferrin fusion protein), can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brufiag-et al. (Comp. App. Biosci 245- (1990)).

The polynucleotide variants of the invention may contain alterations in the coding regions, non-coding regions, or both. Polynucleotide variants containing alterations which produce silent substitutions, additions, or deletions, but do not alter the properties or activities of the encoded polypeptide may be used to produce modified Tf fusion proteins. Nucleotide variants produced by silent substitutions due to the degeneracy of the genetic code can be utilized. Moreover, polypeptide variants in which less than about 50, less than 40, less than 30, less than 20, less than 10, or 5-50, 5-25, 5-10, 1-5, or 1-2 amino acids are substituted, deleted, or added in any combination can also be utilized. Polynucleotide variants can be produced for a variety of reasons, *e.g*., to optimize codon expression for a particular host (change codons in the human mRNA to those preferred by a host, such as, yeast or *E. coli* as described above).

In other embodiments, the therapeutic protein moiety has conservative substitutions compared to the wild-type sequence. By "conservative substitutions" is intended swaps within groups such as replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly. Guidance concerning how to make phenotypically silent amino acid substitutions is provided, for example, in Bowie et al., "Deciphering the Message in Protein Sequences: Tolerance to Amino Acid Substitutions," Science 247:1306-1310 (1990). In specific embodiments, the polypeptides of the invention comprise, or alternatively, consist of, fragments or variants of the amino acid sequence of a therapeutic protein described herein and/or serum transferrin, and/ modified transferrin protein of the invention, wherein the fragments or variants have 1-5, 5-10, 5-25, 5-50, 10-50 or 50-150 amino acid residue additions, substitutions, and/or deletions when compared to the reference amino acid sequence. In further embodiments, the amino acid substitutions are conservative. Nucleic acids encoding these polypeptides are also encompassed by the invention.

The modified fusion proteins of the present invention can be composed of aminoacids joined to each other by peptide bonds or modified peptide bonds and may contain amino acids other than the 20 gene-encoded amino acids. The polypeptides may be modified by either natural processes, such as post-translational processing, or by chemical modification techniques which are well known in the art. Such modifications are well described in basic texts and in more detailed monographs, as well as in a voluminous research literature.

Modifications can occur anywhere in a polypeptide, including the peptide backbone, the amino acid side-chains and the amino or carboxy termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, glycosylation, GPI anchor formation, hydroxylation, iodination, methylation, myristylation, oxidation, pegylation, proteolytic processing, phosphorylation, prenylation, racemization, sulfation, transfer-RNA mediated addition of amino acids to proteins such as arginylaltion, and ubiquitination. (See, for instance, PROTEINS - STRUCTURE AND MOLECULAR PROPERTIES, 2nd Ed., T. E. Creighton, W. H. Freeman and Company, New York(1993); POST-TRANSLATIONAL COVALENT MODIFICATION OF PROTEINS, B. C. Johnson, Ed., Academic Press, New' York, pgs. 1-12 (1983); Seifter et al. (1990) Meth. Enzymol. 182:626-646; Rattan et al., Ann. N.Y. Acad. Sci. 663:48-62.

Therapeutic molecules that may be fused to or inserted into Tf include, but are not limited to, hormones, matrix proteins, immunosuppressants, bronchodilators, cardiovascular agents, enzymes, CNS agents, neurotransmitters, receptor proteins or peptides, growth hormones, growth factors, antiviral peptides, fusogenic inhibitor peptides, cytokine, lymphokines, monokines, interleukins, colony stimulating factors, differentiation factors, angiogenic factors, receptor ligands, cancer-associated proteins, antineoplastics, viral peptides, antibiotic peptides, blood proteins, antagonist proteins, transcription factors, anti-angiogenic factors, antagonist proteins or peptides, receptor antagonists, antibodies, single chain antibodies and cell adhesion molecules. Different therapeutic molecules may be combined into a single fusion protein to produce a bi or multi-functional therapeutic molecule. Different molecules may also be used in combination to produce a fusion protein with a therapeutic entity and a targeting entity.

Cytokines are soluble proteins released by cells of the immune system, which act nonenzymatically through specific receptors to regulate immune responses. Cytokines resemble hormones in that they act at low concentrations bound with high affinity to a specific receptor. The term "cytokine" is used herein to describe naturally occurring or recombinant proteins, analogs thereof, and fragments thereof which elicit a specific biological response in a cell which has a receptor for that cytokine. Cytokines preferably include interleukins such as interleukin-2 (IL-2) (GenBank Acc. No. S77834), IL-3 (GenBank Acc. No. M14743), IL-4 (GenBank Acc. No. M23442), IL-5 (GenBank Acc. No. J03478), IL-6 (GenBank Acc. No. M14584), IL-7 (GenBank Acc. No. NM_000880), IL-10 (GenBank Acc. No. NM_000572), IL-12 (GenBank Acc. No.AF180562 and GenBank Acc. No. AF180563), IL-13 (GenBank Acc. No. U10307), IL-14 (GenBank Acc. No. XM_170924), IL-15 (GenBank Acc. No. X91233), IL-16 (GenBank Acc. No. NM_004513), IL-17 (GenBank Acc. No. NM_002190) and IL-18 (GenBank Acc. No. NM_001562), hematopoietic factors such as granulocyte-macrophage colony stimulating factor (GM-CSF) (GenBank Acc. No. X03021), granulocyte colony stimulating factor (G-CSF) (GenBank Acc. No. X03656), platelet activating factor (GenBank Acc. No. NM_000437) and erythropoeitin (GenBank Acc. No. X02158), tumor necrosis factors (TNF) such as TNFα (GenBank Acc. No. X02910), lymphokines such as lymphotoxin- a (GenBank Acc. No. X02911), lymphotoxin-β (GenBank Acc. No. L11016), leukoregulin, macrophage migration inhibitory factor (GenBank Acc. No. M25639), and neuroleukin (GenBank Acc. No. K03515), regulators of metabolic processes such as leptin (GenBank Acc. No. U43415), interferons such as interferon α (IFNα) (GenBank Acc. No. M54886), IFNβ (GenBank Acc. No. V00534), IFNγ (GenBank Acc. No. J00219), IFNo (GenBank Acc. No. NM_002177), thrombospondin 1 (THBS1) (GenBank Acc. No. NM_003246), THBS2 (GenBank Acc. No. L12350), THBS3 (GenBank Acc. No. L38969), THBS4 (GenBank Acc. No. NM_003248), and chemokines. Preferably, the modified transferrin-cytokine fusion protein of the present invention displays cytokine biological activity.

The term "hormone" is used herein to describe any one of a number of biologically active substances that are produced by certain cells or tissues and that cause specific biological changes or activities to occur in another cell or tissue located elsewhere in the body. Hormones preferably include proinsulin (GenBank Acc. No. V00565), insulin (GenBank Acc. No. NM_000207), growth hormone 1 (GenBank Acc. No. V00520), growth hormone 2 (GenBank Acc. No. F006060), growth hormone release factor (GenBank Acc. No. NM_021081), insulin-like growth factor I (GenBank Acc. No. M27544), insulin-like growth factor II (GenBank Acc. No. NM_000612), insulin-like growth factor binding protein 1 (IGFBP-1) (GenBank Acc. No. M59316), IGFBP-2 (GenBank Acc. No. X16302), IGFBP-3 (GenBank Acc. No. NM_000598), IGFBP-4 (GenBank Acc. No. Y12508), IGFBP-5 (GenBank Acc. No. M65062), IGFBP-6 (GenBank Acc. No. NM_002178), IGFBP-7 (GenBank Acc. No. NM_001553), chorionic gonadotropin β chain (GenBank Acc. No. NM_033142), chorionic gonadotropin α chain (GenBank Acc. No. NM_000735), luteinizing hormone β (GenBank Acc. No. X00264), follicle-stimulating hormone β (GenBank Acc. No. NM_000510), thyroid-stimulating hormone β (GenBank Acc. No. NM_000549), prolactin (GenBank Acc. No. NM_000948), pro-opiomelanocortin (GenBank Acc. No. VO1510), corticotropin (ACTH), β-lipotropin, α-melanocyte stimulating hormone (α-MSH), γ-lipotropin, β -MSH, β -endorphin, and corticotropin-like intermediate lobe peptide (CLIP).

The term "growth factor" is used herein to describe any protein or peptide that binds to a receptor to stimulate cell proliferation. Growth factors preferably include platelet-derived growth factor-α (PDGF-α) (GenBank Acc. No. X03795), PDGF-β (GenBank Acc. No. X02811), steroid hormones, epidermal growth factor (EGF) (GenBank Acc. No. NM_001963), fibroblast growth factors such as fibroblast growth factor 1 (FGF1) (GenBank Acc. No. NM_000800), FGF2 (GenBank Acc. No. NM_002006), FGF3 (GenBank Acc. No. NM_005247), FGF4 (GenBank Acc. No. NM_002007), FGF5 (GenBank Acc. No. M37825), FGF6 (GenBank Acc. No. X57075), FGF7 (GenBank Acc. No. NM_002009), FGF8 (GenBank Acc. No. AH006649), FGF9 (GenBank Acc. No. NM_002010), FGF10 (GenBank Acc. No. AB002097), FGF11 (GenBank Acc. No. NM_004112), FGF12 (GenBank Acc. No. NM_021032), FGF13 (GenBank Acc. No. NM_004114), FGF14 (GenBank Acc. No. NM_004115), FGF16 (GenBank Acc. No. AB009391), FGF17 (GenBank Acc. No. NM_003867), FGF18 (GenBank Acc. No. AF075292), FGF19 (GenBank Acc. No. NM_005117), FGF20 (GenBank Acc. No. NM_019851), FGF21 (GenBank Acc. No. NM_019113), FGF22 (GenBank Acc. No. NM_020637), and FGF23 (GenBank Acc. No. NM_020638), angiogenin (GenBank Acc. No. M11567), brain-derived neurotrophic factor (GenBank Acc. No. M61176), ciliary neurotrophic growth factor (GenBank Acc. No. X60542), transforming growth factor-α (TGF-α) (GenBank Acc. No. X70340), TGF-β (GenBank Acc. No. X02812), nerve growth factor-α (NGF-α) (GenBank Acc. No. NM_010915), NGF-β (GenBank Acc. No. X52599), tissue inhibitor of metalloproteinase 1 (TIMP1) (GenBank Acc. No. NM_003254), TIMP2 (GenBank Acc. No. NM_003255), TIMP3 (GenBank Acc. No. U02571), TIMP4 (GenBank Acc. No. U76456) and macrophage stimulating 1 (GenBank Acc. No. L11924).

The term "matrix protein" is used herein to describe proteins or peptides that are normally found in the extracellular matrix. These proteins may be functionally important for strength, filtration, or adhesion. Matrix proteins preferably include collagens such as collagen I (GenBank Acc. No. Z74615), collagen II (GenBank Acc. No. X16711), collagen III (GenBank Acc. No. X14420), collagen IV (GenBank Acc. No. NM_001845), collagen V (GenBank Acc. No. NM_000393), collagen VI (GenBank Acc. No. NM_058175), collagen VII (GenBank Acc. No. L02870), collagen VIII (GenBank Acc. No. NM_001850), collagen IX (GenBank Acc. No. X54412), collagen X (GenBank Acc. No. X60382), collagen XI (GenBank Acc. No. J04177), and collagen XII (GenBank Acc. No. U73778), laminin proteins such as LAMA2 (GenBank Acc. No. NM_000426), LAMA3 (GenBank Acc. No. L34155), LAMA4 (GenBank Acc. No. NM_002290), LAMB (GenBank Acc. No. NM_002291), LAMB3 (GenBank Acc. No. L25541), LAMC1 (GenBank Acc. No. NM_002293), nidogen (GenBank Acc. No. NM_002508), α-tectorin (GenBank Acc. No. NM_005422), β-tectorin (GenBank Acc. No. NM_058222), and fibronectin (GenBank Acc. No. X02761).

The term "blood proteins" are traditionally defined as those sourced from plasma, many now commonly produced by recombinant means, and include, but are not limited to native serum proteins, derivatives, fragments and mutants or variants thereof, blood clotting factors, derivatives, mutants, variants and fragments (including factors VII, VIII, IX, X), protease inhibitors (antithrombin 3, alpha-1 antitrypsin), urokinase-type plasminogen activator, immunoglobulins, von Willebrand factor and von Willebrand mutants, fibronectin, fibrinogen, thrombin and hemoglobin.

The term "enzyme" is used herein to describe any protein or proteinaceous substance which catalyzes a specific reaction without itself being permanently altered or destroyed. Enzymes preferably include coagulation factors such as F2 (GenBank Acc. No. XM_170688), F7 (GenBank Acc. No. XM_027508), F8 (GenBank Acc. No. XM_013124), F9 (GenBank Acc. No. NM_000133), F10 (GenBank Acc. No. AF503510) and others, matrix metalloproteinases such as matrix metalloproteinase I (GenBank Acc. No. MMP1) (GenBank Acc. No. NM_002421), MMP2 (GenBank Acc. No. NM_004530), MMP3 (GenBank Acc. No. NM_002422),MMP7 (GenBank Acc. No. NM_002423), MMP8 (GenBank Acc. No. NM_002424), MMP9 (GenBank Acc. No. NM_004994), MMP10 (GenBank Acc. No. NM_002425), MMP12 (GenBank Acc. No. NM_002426), MMP13 (GenBank Acc. No. X75308), MMP20 (GenBank Acc. No. NM_004771), adenosine deaminase (GenBank Acc. No. NM_000022), mitogen activated protein kinases such as MAPK3 (GenBank Acc. No. XM_055766), MAP2K2 (GenBank Acc. No. NM_030662), MAP2K1 (GenBank Acc. No. NM_002755), MAP2K4 (GenBank Acc. No. N_003010), MAP2K7 (AF013588), and MAPK12 (NM_002969), kinases such as JNKK1 (GenBank Acc. No. U17743), JNKK2 (GenBank Acc. No. AF014401), JAK1 (M64174), JAK2 (NM_004972), and JAK3 (N_000215), and phosphatases such as PPM1A (GenBank Acc. No. NM_021003) and PPM1D (GenBank Acc. No. NM_003620).

The term "transcription factors" is used herein to describe any protein or peptide involved in the transcription of protein-coding genes. Transcription factors may include Sp1, Sp2 (GenBank Acc. No. NM_003110), Sp3 (GenBank Acc. No. AY070137), Sp4 (GenBank Acc. No. NM_003112) NFYB (GenBank Acc. No. NM_006166), Hap2 (GenBank Acc. No. M59079), GATA-1 (GenBank Acc. No. NM_002049), GATA-2 (GenBank Acc. No. NM_002050), GATA-3 (GenBank Acc. No. X55122), GATA-4 (GenBank Acc. No. L34357), GATA-5, GATA-6 (GenBank Acc. No. NM_005257), FOG2 (NM_012082), Eryf1 (GenBank Acc. No. X17254), TRPS1 (GenBank Acc. No. NM_014112), NF-E2 (GenBank Acc. No. NM_006163), NF-E3, NF-E4, TFCP2 (GenBank Acc. No. NM_005653), Oct-1 (GenBank Acc. No. X13403), homeobox proteins such as HOXB2 (GenBank Acc. No. NM_002145), HOX2H (GenBank Acc. No. X16665), hairless homolog (GenBank Acc. No. NM_005144), mothers against decapentaplegic proteins such as MADH1 (GenBank Acc. No. NM_005900), MADH2 (GenBank Ace. No. NM_005901), MADH3 (GenBank Acc. No. NM_005902), MADH4 (GenBank Acc. No. NM_005359), MADH5 (GenBank Acc. No. AF009678), MADH6 (GenBank Acc. No. NM_005585), MADH7 (GenBank Acc. No. NM_005904), MADH9 (GenBank Acc. No. NM_005905), and signal transducer and activator of transcription proteins such as STAT1 (GenBank Acc. No. XM_010893), STAT2 (GenBank Acc. No. NM_005419), STAT3 (GenBank Acc. No. AJ012463), STAT4 (GenBank Acc. No. NM_003151), STAT5 (GenBank Acc. No. L41142), and STAT6 (GenBank Acc. No. NM_003153).

In yet another embodiment of the invention, the therapeutic molecule is a non-human or non-mammalian protein. For example, HIV gp120, HIV Tat, surface proteins of other viruses such as hepatitis, herpes, influenza, adenovirus and RSV, other HIV components, parasitic surface proteins such as malarial antigens, and bacterial surface proteins are preferred. These non-human proteins may be used, for example, as antigens, or because they have useful activities. For example, the therapeutic molecule may be streptokinase, staphylokinase, urokinase, or other proteins with useful enzymatic activities.

In an alternative embodiment, the therapeutic molecule is a ligand-binding protein with biological activity. Such ligand-binding proteins may, for example, (1) block receptor-ligand interactions at the cell surface; or (2) neutralize the biological activity of a molecule in the fluid phase of the blood, thereby preventing it from reaching its cellular target. In some embodiments, the modified transferrin fusion proteins include a modified transferrin molecule fused to a ligand-binding domain of a receptor selected from the group consisting of, but not limited to, a low density lipoprotein (LDL) receptor, an acetylated LDL receptor, a tumor necrosis factor α receptor, a transforming growth factor β receptor, a cytokine receptor, an immunoglobulin Fc receptor, a hormone receptor, a glucose receptor, a glycolipid receptor, and a glycosaminoglycan receptor. In other embodiments, ligand-binding proteins include CD2 (M14362), CD3G (NM_000073), CD3D (NM_000732), CD3E (NM_000733), CD3Z (J04132), CD28 (NM_006139), CD4 (GenBank Acc. No. NM_000616), CD1A (GenBank Acc. No. M28825), CD1B (GenBank Acc. No. NM_001764), CD1C (GenBank Acc. No. NM_001765), CD1D (GenBank Acc. No. NM_001766), CD80 (GenBank Acc. No. NM_005191), GNB3 (GenBank Acc. No. AF501884), CTLA-4 (GenBank Acc. No. NM_005214), intercellular adhesion molecules such as ICAM-1 (NM_000201), ICAM-2 (NM_000873), and ICAM-3 (NM_002162), tumor necrosis factor receptors such as TNFRSF1A (GenBank Acc. No. X55313), TNFR1SFB (GenBank Acc. No. NM_001066), TNFRSF9 (GenBank Acc. No. NM_001561), TNFRSF10B (GenBank Acc. No. NM_003842), TNFRSF11B (GenBank Acc. No. NM_002546), and TNFRSF13B (GenBank Acc. No. No_006573), and interleukin receptors such as IL2RA (GenBank Acc. No. NM_000417), IL2RG (GenBank Acc. No. NM_000206), IL4R (GenBank Acc. No. AF421855), IL7R (GenBank Acc. No. NM_002185), IL9R (GenBank Acc. No. XM_015989), and IL13R (GenBank Acc. No. X95302). Preferably, the Tf-ligand-binding protein fusion of the present invention displays the biological activity of the ligand-binding protein.

The term "cancer-associated proteins" is used herein to describe proteins or polypeptides whose expression is associated with cancer or the maintenance of controlled cell growth, such as proteins encoded by tumor suppressor genes or oncogenes. Cancer-associated proteins may be p16 (GenBank Acc. No. AH005371), p53 (GenBank Acc. No. NM_000546), p63 (GenBank Acc. No. NM_003722), p73 (GenBank Acc. No. NM_005427), BRCA1 (GenBank Acc. No. U14680), BRCA2 (GenBank Ace. No. NM_000059), CTBP interacting protein (GenBank Acc. No. U72066), DMBT1 (GenBank Acc. No. NM_004406), HRAS (GenBank Acc. No.NM_005343), NCYM (GenBank Acc. No. NM_006316), FGR (GenBank Acc. No. NM_005248), myb (GenBank Acc. No. AF104863), rafl (GenBank Acc. No. NM_002880), erbB2 (GenBank Acc. No. NM_004448), VAV (GenBank Acc. No. X16316), c-fos (V GenBank Acc. No. 01512), c-fes (GenBank Acc. No. X52192), c-jun (GenBank Acc. No. NM_002228), MAS1 (GenBank Acc. No. M13150), pim-1 (GenBank Acc. No. M16750), TIF1 (GenBank Acc. No. NM_003852), c-fms (GenBank Acc. No. X03663), EGFR (GenBank Acc. No. NM_005228), erbA (GenBank Acc. No. X04707), c-src tyrosine kinase (GenBank. Acc. No. XM_044659), c-abl (GenBank Acc. No. M14752), N-ras (GenBank Acc. No. X02751), K-ras (GenBank Acc. No. M54968), jun-B (GenBank Acc. No. M29039), c-myc (GenBank Acc. No. AH001511), RB1 (GenBank Acc. No. M28419), DCC (GenBank Acc. No. X76132), APC (GenBank Acc. No. NM_000038), NF1 (GenBank Acc. No. M89914), NF2 (GenBank Acc. No. Y18000), and bcl-2 (GenBank Acc. No. M13994).

"Fusogenic inhibitor peptides" is used herein to describe peptides that show antiviral activity, anti-membrane fusion capability, and/or an ability to modulate intracellular processes, for instance, those involving coiled-coil peptide structures. Antiviral activity includes, but is not limited to, the inhibition of HIV-1, HIV-2, RSV, SIV, EBV. Measles virus, influenza virus, or CMV transmission to uninfected cells. Additionally, the antifusogenic capability, antiviral activity or intracellular modulatory activity of the peptides merely requires the presence of the peptides and specifically does not require the stimulation of a host immune response directed against such peptides. Antifusogenic refers to a peptide's ability to inhibit or reduce the level of membrane fusion events between two or more moieties relative to the level of membrane fusion which occurs between said moieties in the absence of the peptide. The moieties may be, for example, cell membranes or viral structures, such as viral envelopes or pili. The term "antiviral peptide", as used herein, refers to the peptide's ability to inhibit viral infection of cells or some viral activity required for productive viral infection and/or viral pathogenesis, via, for example, cell-cell fusion or free virus infection. Such infection may involve membrane fusion, as occurs in the case of enveloped viruses, or some other fusion event involving a viral structure and a cellular structure. Fusogenic inhibitor peptides and antiviral peptides often have amino acid sequences that are derived from greater than one viral protein (*e.g*., an HIV-1, HIV-2, RSV, and SIV-derived polypeptide).

Examples of fusogenic inhibitor peptides and antiviral peptides can be found in WO 94/2820, WO 96/19495, WO 96/40191, WO 01/64013 and US patents 6,333,395, 6,258,782, 6,228,983, 6,133,418, 6,093,794, 6,068,973, 6,060,065, 6,054,265, 6,020,459, 6,017,536, 6,013,263, 5,464,933, 5,346,989, 5,603,933, 5,656,480, 5,759,517, 6,245,737; 6,326,004, and 6,348,568; all of which are herein incorporated by reference. In a preferred embodiment, antifusogenic peptides are selected from the group consisting of HIV T-20 (FWNWLSAWKDLELLEQENKEQQNQSEEILSHILSTY, SEQ ID NO: 4), HIV T-1249, RSV T786 (VYPSDEYDASISQVNEEINQALAYIRKADELLENV, SEQ ID NO: 5), RSV T1584 (AVSKVLHLEGEVNKIKSALLSTNKAVVSLSNGVSVLTSKVLDLKNYIDKQL, SEQ ID NO: 6) and RSV T112 (VFPSDEFDASISQVNEKINQSLAFIRESDELLHNV, SEQ ID NO: 7).

Examples of other types of peptides, include fragments of therapeutic proteins as described herein, in particular, fragments of human proteins that retain at least one activity of the parent molecule. Peptides that may be used to produce modified TF fusion proteins of the invention also include mimetic peptides and peptides that exhibit a biological activity of a therapeutic protein but differ in sequence or three-dimensional structure from a full-length therapeutic protein. As a non-limited example, peptides include erythropoeitin mimetic peptides disclosed by Johnson et al. (2000) Nephrol. Dial. Transplant 15(9): 1274-7, Kuai et al. (2000) J. Pept. Res. 56(2):59-62, Barbone et al. (1999) Nephrol. Dial. Transplant. 14 Supp 2:80-4, Middleton et al. (1999) J. Biol. Chem. 274(20):14163-9, Johnson et al. (1998) Biochemistry 37(11):3699-710, Johnson et al. (1997) Chem. Biol. 12:939-50, Wrighton et al. (1997) Nat. Biotechnol. 15(12):1261-5, Livnah et al. (1996) Science 273:464-71, and Wrighton et al.,(1996) Science 273:458-64.

Therapeutic molecules also include allergenic proteins and digested fragments thereof. These include pollen allergens from ragweed, rye, June grass, orchard grass, sweet vernal grass, red top grass, timothy grass, yellow dock, wheat, corn, sagebrush, blue grass, California annual grass, pigweed, Bermuda grass, Russian thistle, mountain cedar, oak, box elder, sycamore, maple, elm, etc., dust and mites, bee venom, food allergens, animal dander, and other insect venoms.

Other therapeutic molecules include microbial vaccines which include viral, bacterial and protozoal vaccines and their various components such as surface antigens. These include vaccines which contain glycoproteins, proteins or peptides derived from these proteins. Such vaccines are prepared from *Staphylococcus aureus, Streptococcus pyogenes, Streptococcus pneumonia, Neisseria meningitidis, Neisseria gonorrhoeae, Salmonellae species, Shigellae species, Escherichia coli, Klebsiellae species, Proteus species, Vibrio cholera, Campylobacter pylori, Pseudomonas aeraginosa, Haemophilus influenzae, Bordetella pertussis, Mycobacterium tuberculosis, Legionella pneumophila, Treponema pallidum,* chlamydia, tetanus toxoid, diphtheria toxoid, influenza viruses, adenoviruses, paramyxoviruses (mumps, measles), rubella viruses, polio viruses, hepatitis viruses, herpes viruses, rabies virus, HIV-1, HIV-2, RSV and papilloma viruses.

Preferred fusion molecules may contain anti-HIV viral peptides, anti-RSV peptides, human growth hormone, α and/or β interferons, erythropoietin (EPO), EPO like peptides, granulocyte-colony stimulating factor (GCSF), granulocyte-macrophage colony-stimulating factor (GMCSF), insulin, insulin-like growth factor (IGF), thrombopoeitin, peptides corresponding to the CDR of an antibody, Islet Neogenesis Associated Protein (INGAP), calcitonin, angiostatin, endostatin, interleukin-2, growth hormone releasing factor, human parathyroid hormone, anti-tumor necrosis factor (TNF) peptides, interleukin-1 (IL-1) receptor and/or single chain antibodies.

Fusion proteins of the invention may also be prepared to include peptides or polypeptides derived from peptide libraries to screen for molecules with new or novel functions. Such peptide libraries may include those commercially or publicly available, *e.g.,* American Peptide Co. Inc., Cell Sciences Inc., Invitrogen Corporation, Phoenix Pharmaceutical Inc., United States Biological, as well as those produced by available technologies, *e.g.*, bacteriophage and bacterial display libraries made using standard procedures.

In yet other embodiments of the invention, Tf fusion proteins may be prepared by using therapeutic protein moieties as known in the art and exemplified by the peptides and proteins currently approved by the Food and Drug Administitration at (www.fda.gov/cber/efoi/approve.htm) as well as PCT Patent Publication Nos. WO 01/79258, WO 01/77137, WO 01/79442, WO 01/79443, WO 01/79444 and WO 01/79480.

Table 1 (adapted from PCT International Publication No. WO 01/79444) provides a non-exhaustive list of therapeutic proteins that correspond to a therapeutic protein portion of a modified transferrin fusion protein of the invention. The "Therapeutic Protein X" column discloses therapeutic protein molecules followed by parentheses containing scientific and brand names that comprise or alternatively consist of that therapeutic protein molecule or a fragment or variant thereof. "Therapeutic protein X" as used herein may refer either to an individual therapeutic protein molecule (as defined by the amino acid sequence obtainable from the CAS and Genbank accession numbers), or to the entire group of therapeutic proteins associated with a given therapeutic protein molecule disclosed in this column. The 'Exemplary Identifier' column provides Chemical Abstracts Services (CAS) Registry Numbers (published by the American Chemical Society) and/or Genbank Accession Numbers (*e.g*., Locus ID, NP - XXXXX (Reference Sequence Protein), and XP-XXXXX (Model Protein) identifiers available through the national, Center for Biotechnology Information (NCBI) webpage at www.ncbi.nlm.nih.gov) that correspond to entries in the CAS Registry or Genbank database which contain an amino acid sequence of the protein molecule or of a fragment or variant of the therapeutic protein molecule. In addition GenSeq Accession numbers and/or journal publication citations are given to identify the exemplary amino acid sequence for some polypeptides

The summary pages associated with each of these CAS and Genbank and GenSeq Accession Numbers as well as the cited journal publications are available (*e.g.,* PubMed ID number (PMID)). The PCT/Patcnt Reference column provides U. S. Patent number, or PCT International Publication Numbers corresponding to patents and/or published patent- applications that describe the therapeutic protein molecule all of which are herein incorporated by reference in their entirety. The Biological Activity column describes biological activities associated with the therapeutic protein molecule. The Exemplary Activity Assay column provides references that describe assays which may be used to test the therapeutic and/or biological activity of a therapeutic protein or a transferrin fusion protein of the invention comprising a therapeutic protein X portion. These references are also herein incorporated by reference in their entirety. "The Preferred Indication Y" column describes disease, disorders, and/or conditions that may be treated, prevented, diagnosed, or ameliorated by therapeutic protein X or a transferrin fusion protein of the invention comprising a therapeutic protein X portion.

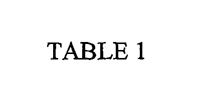

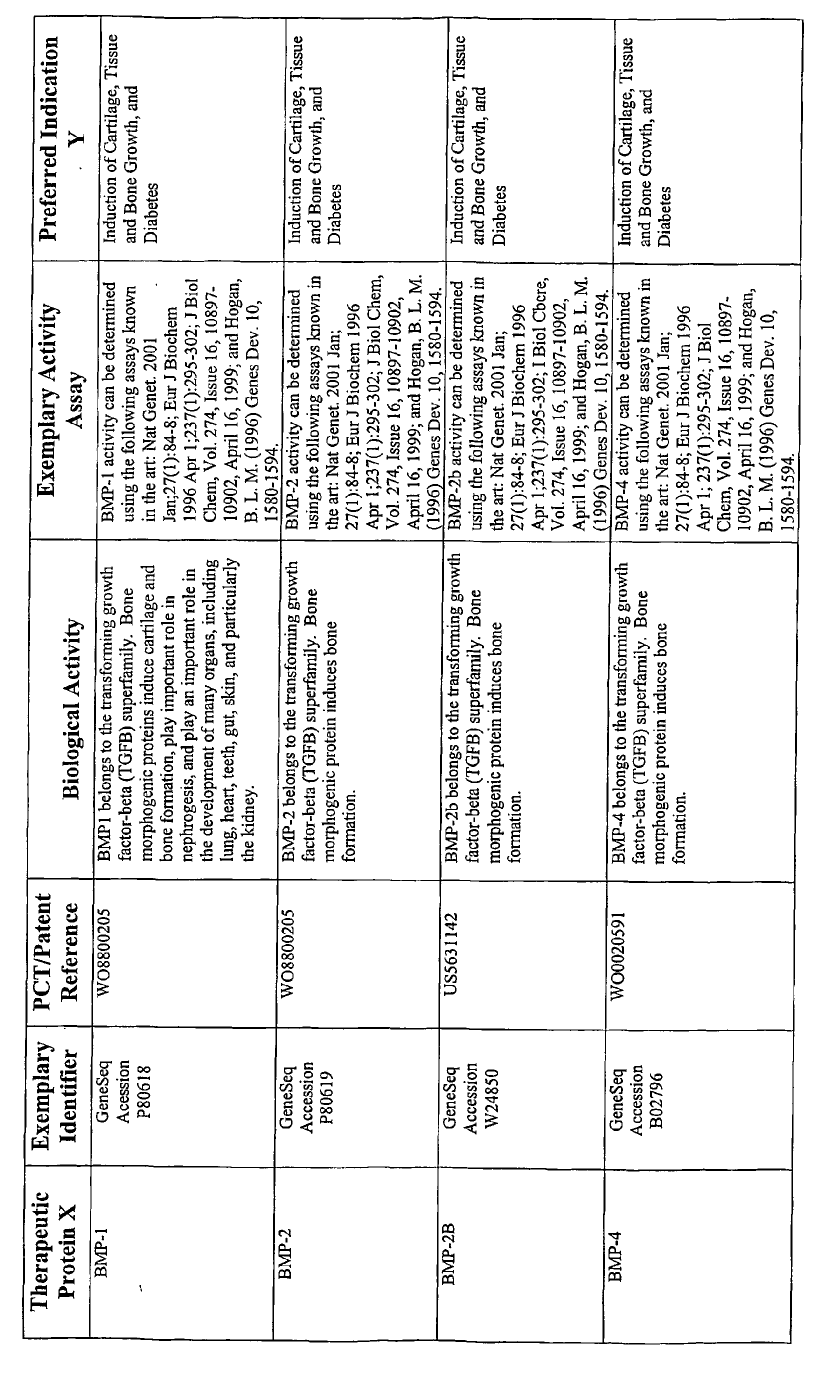

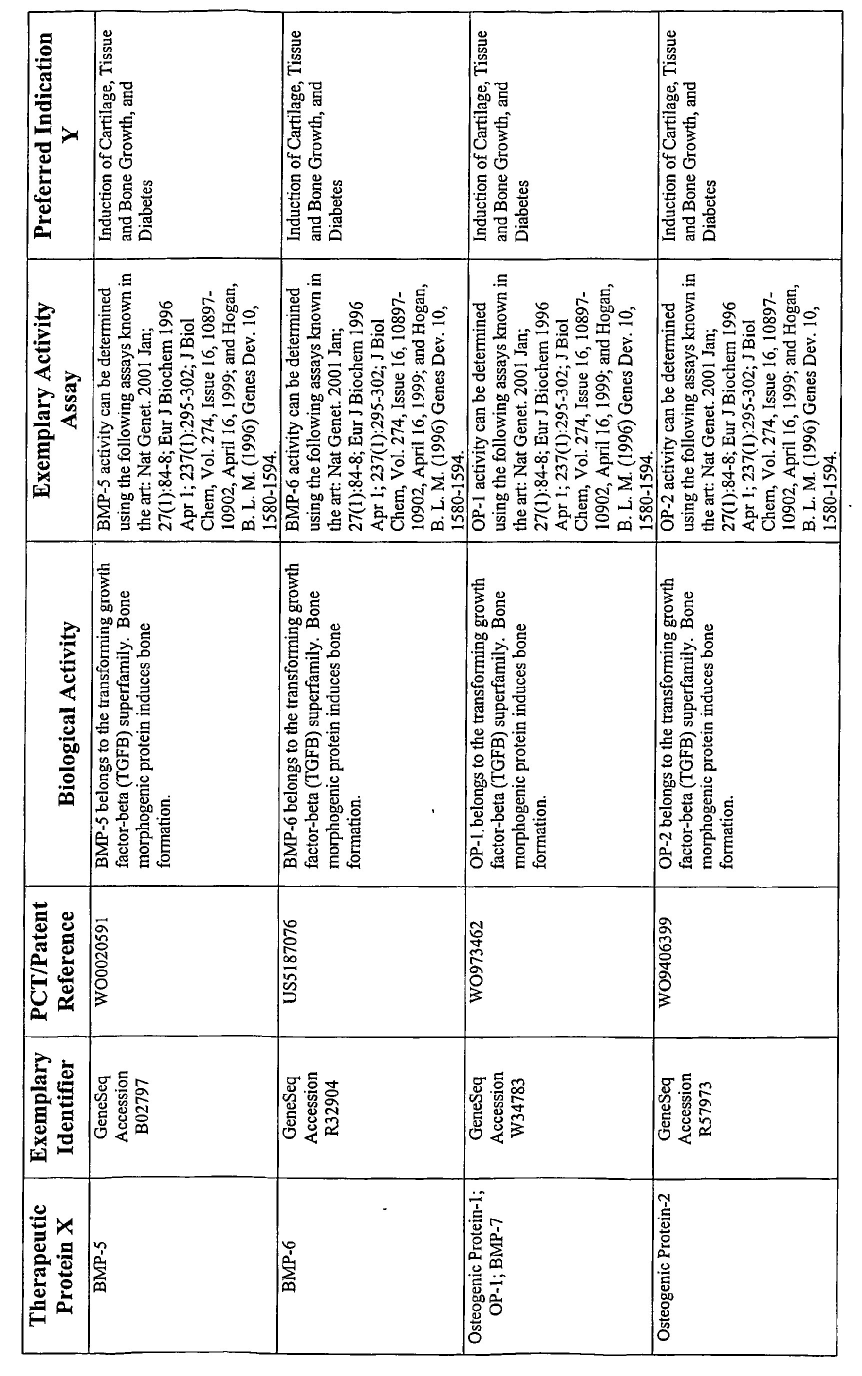

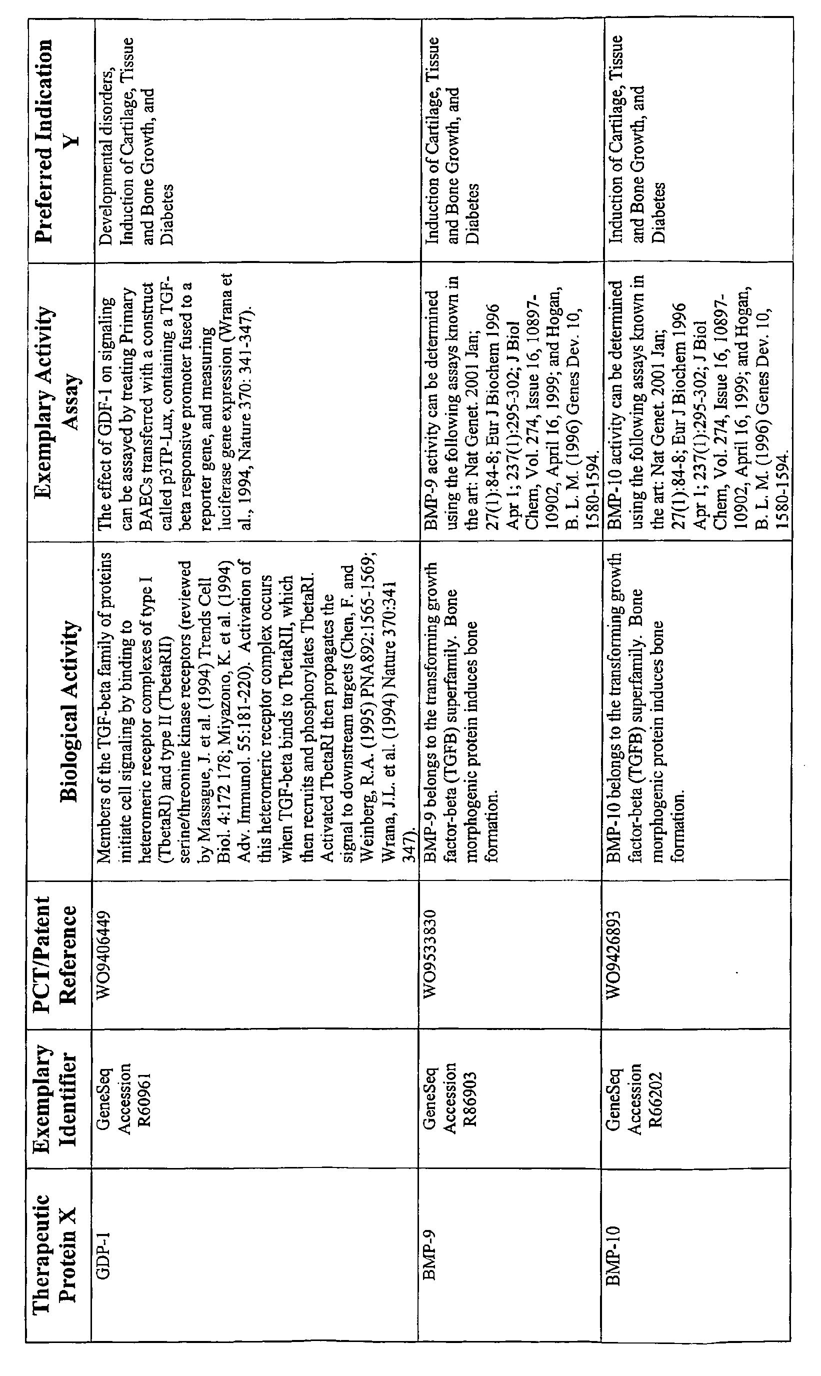

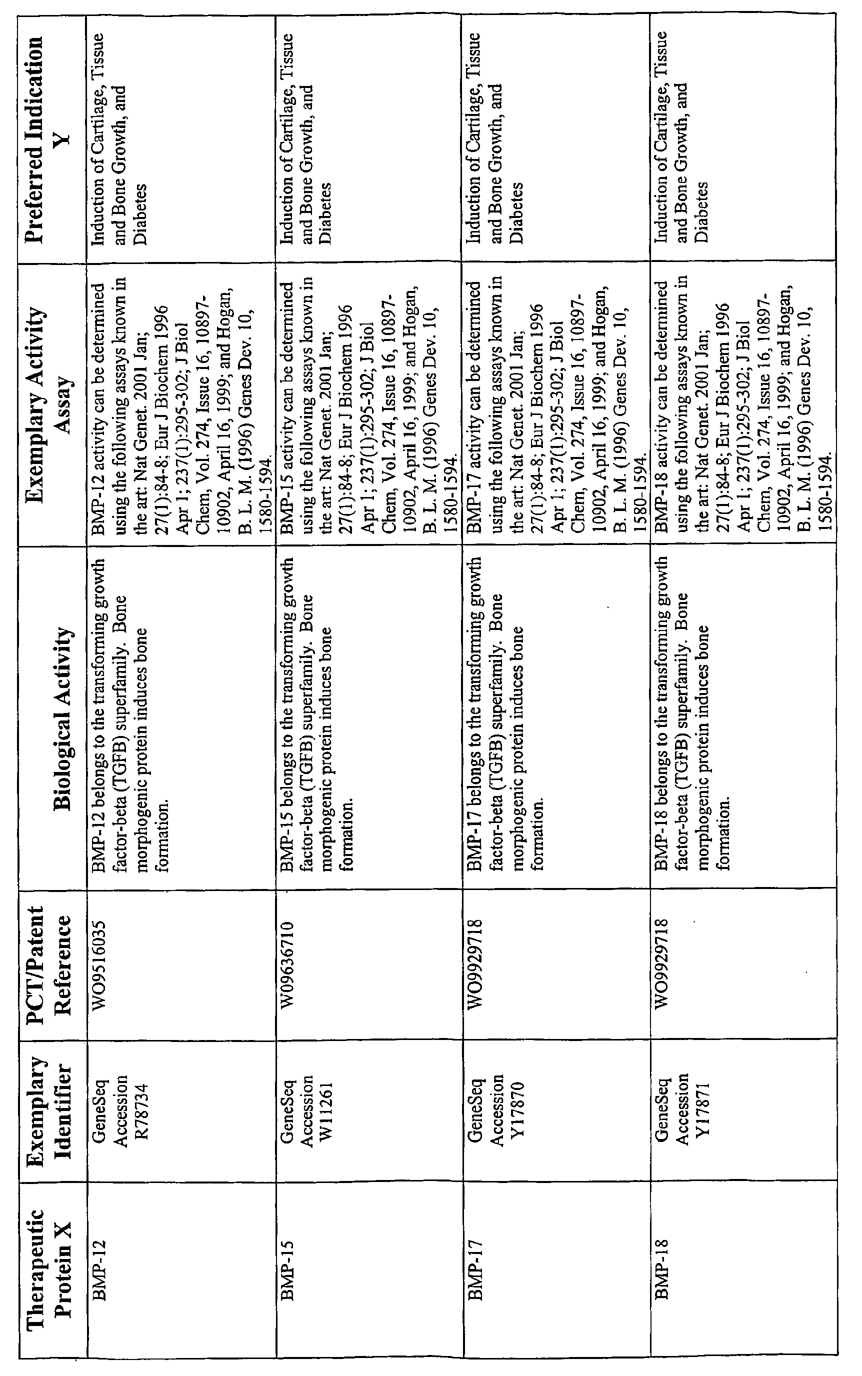

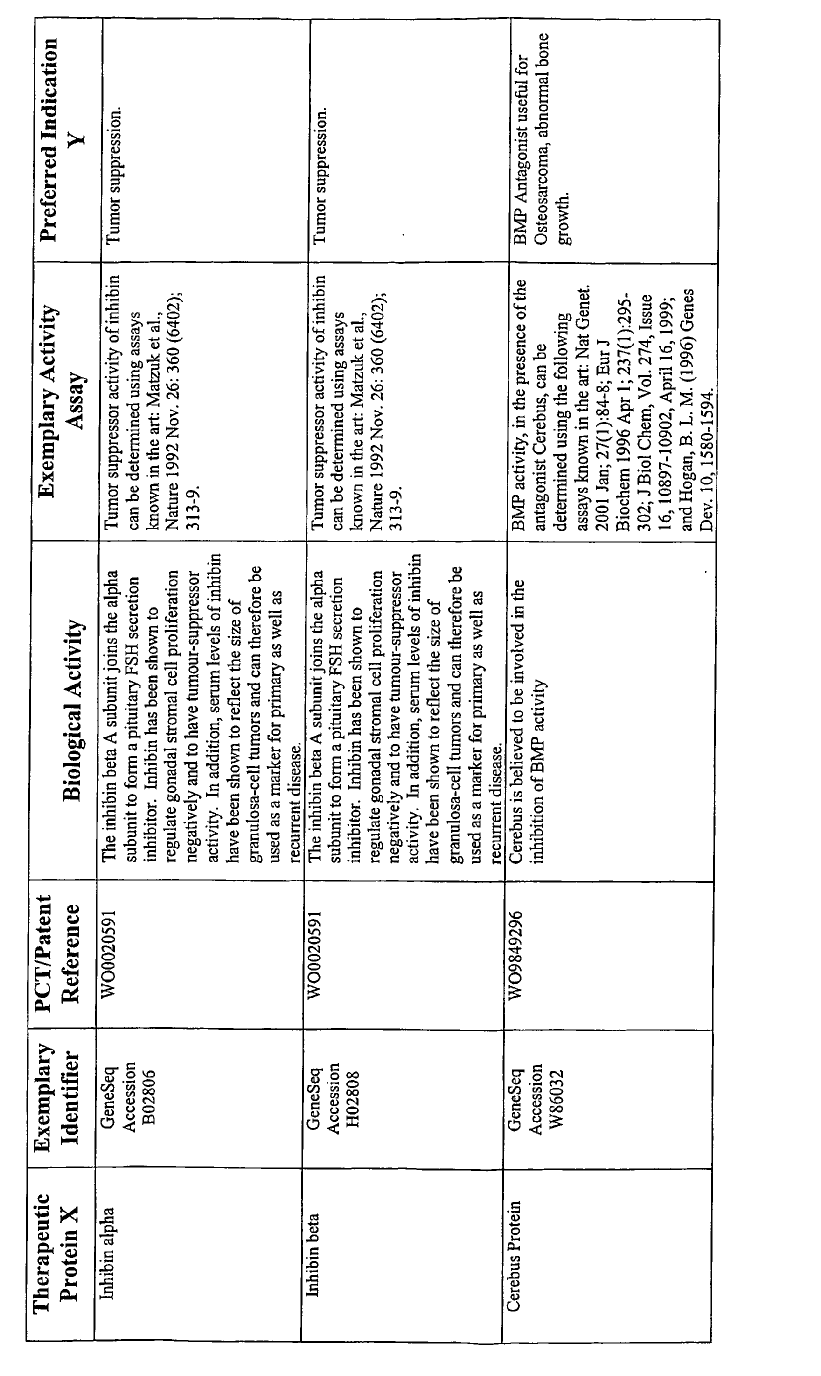

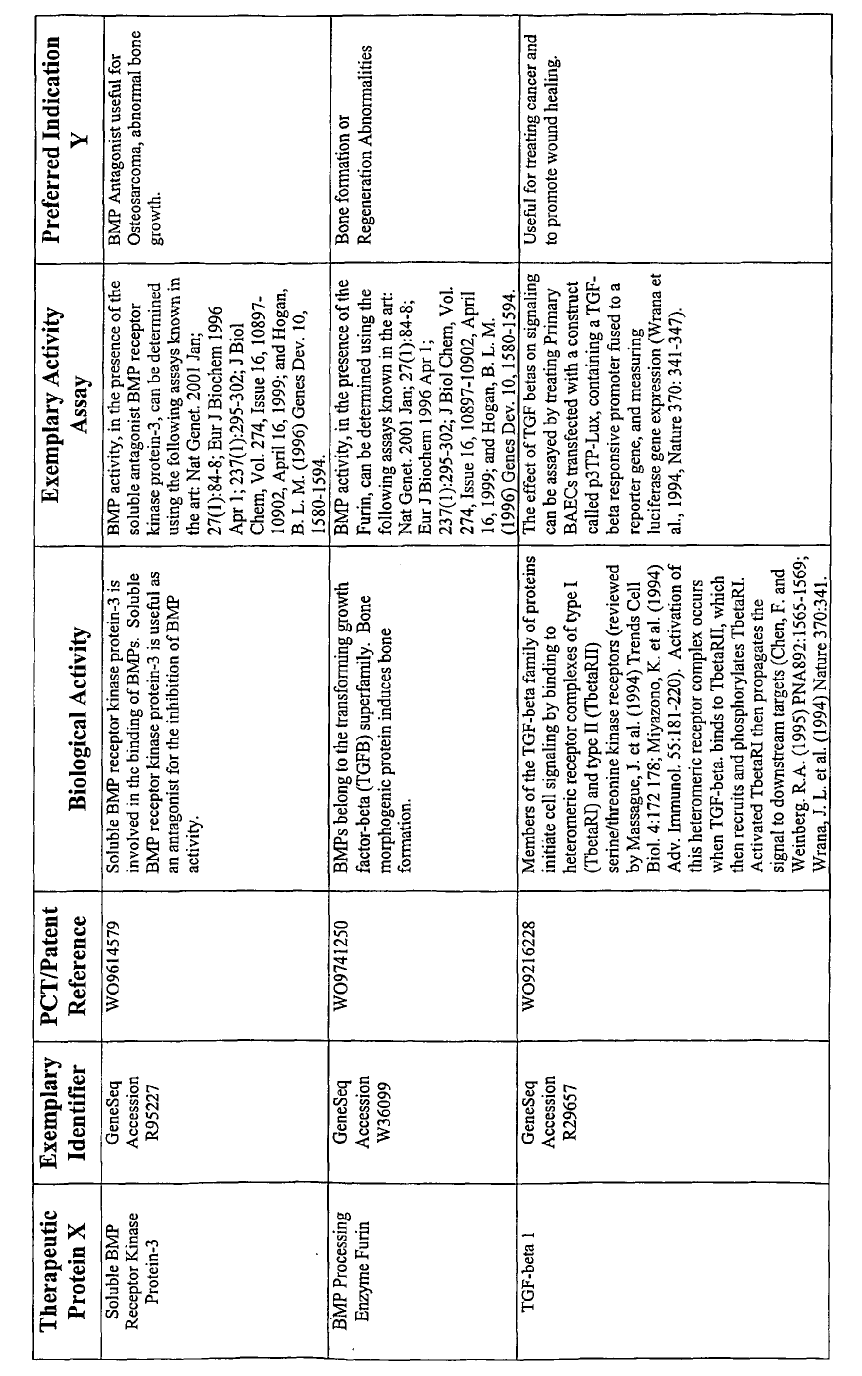

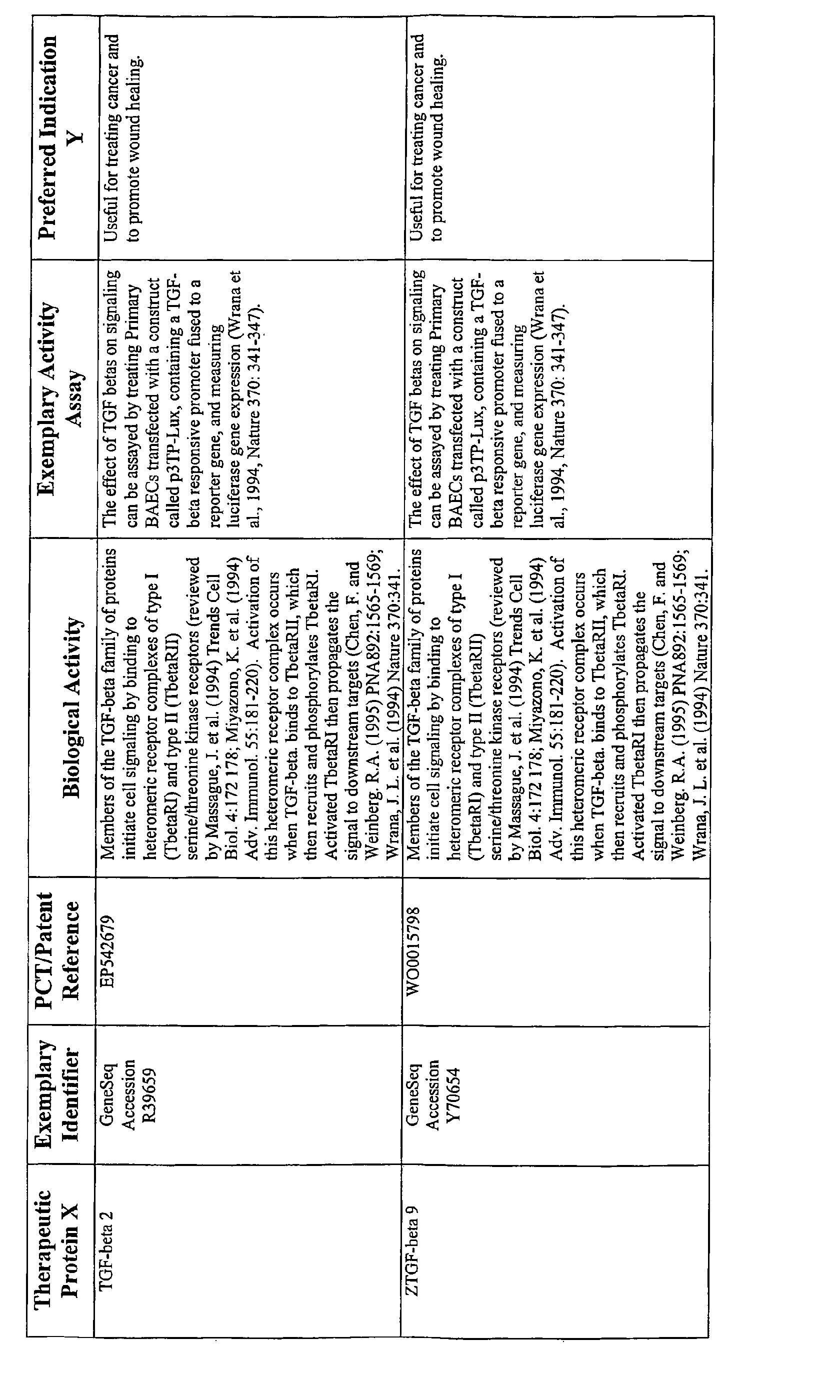

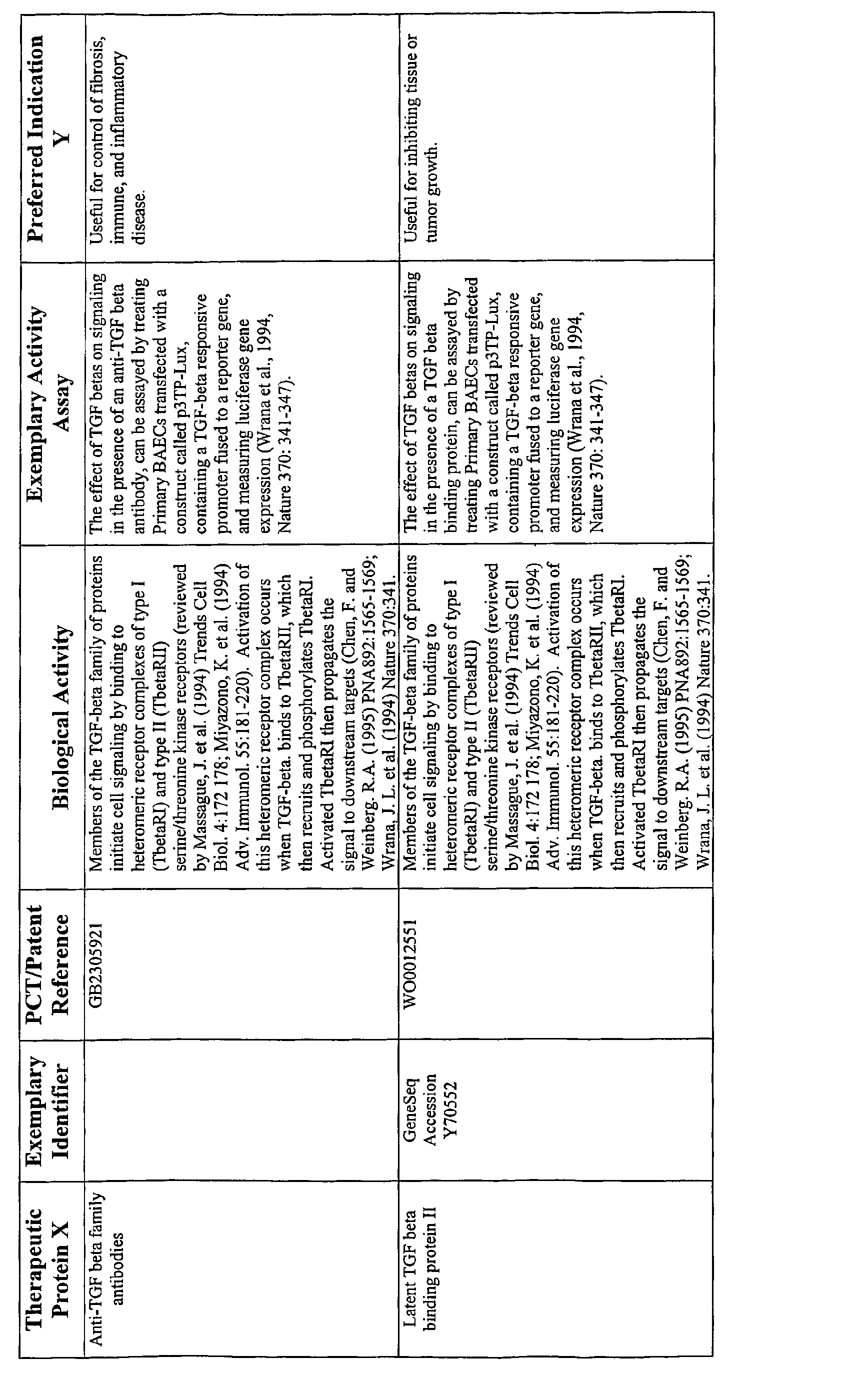

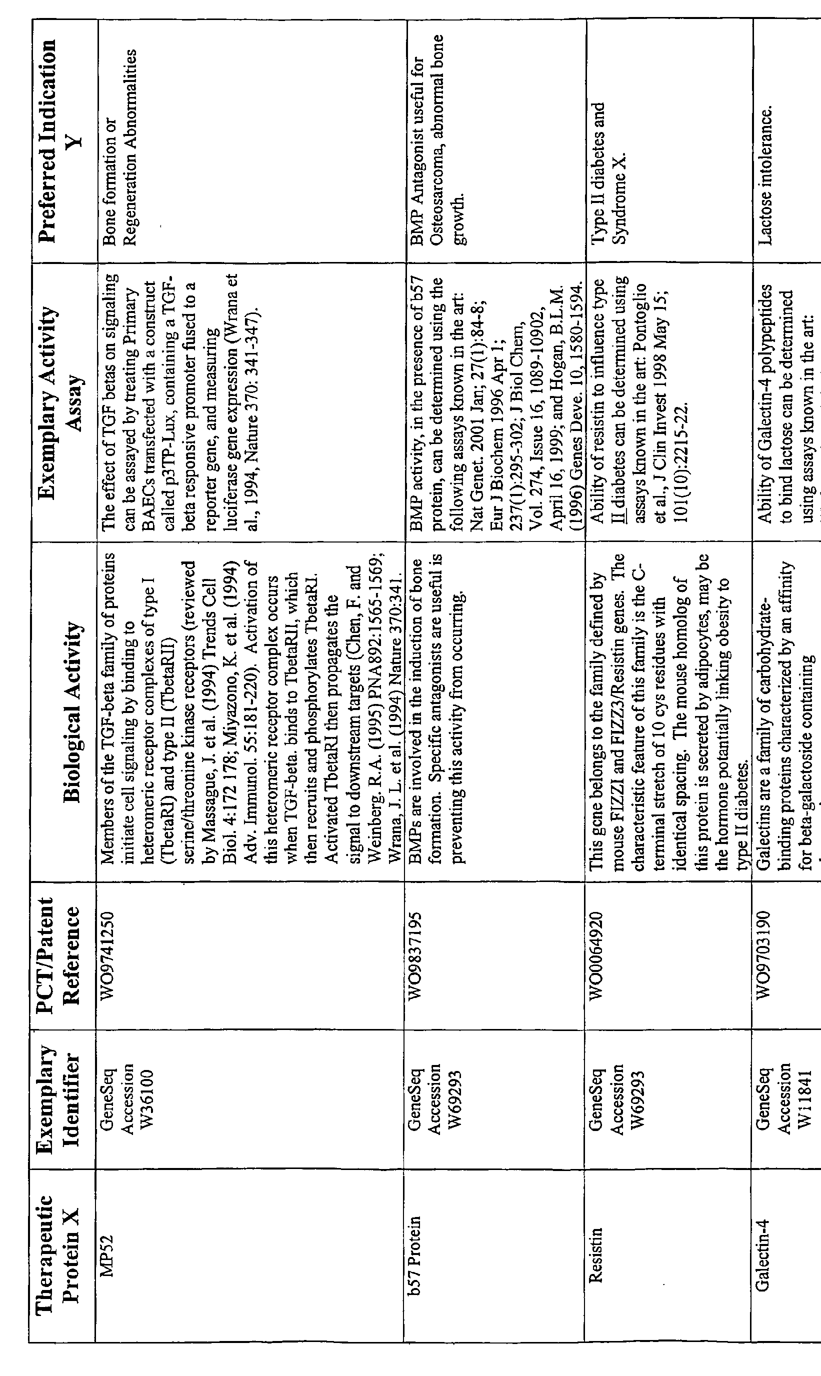

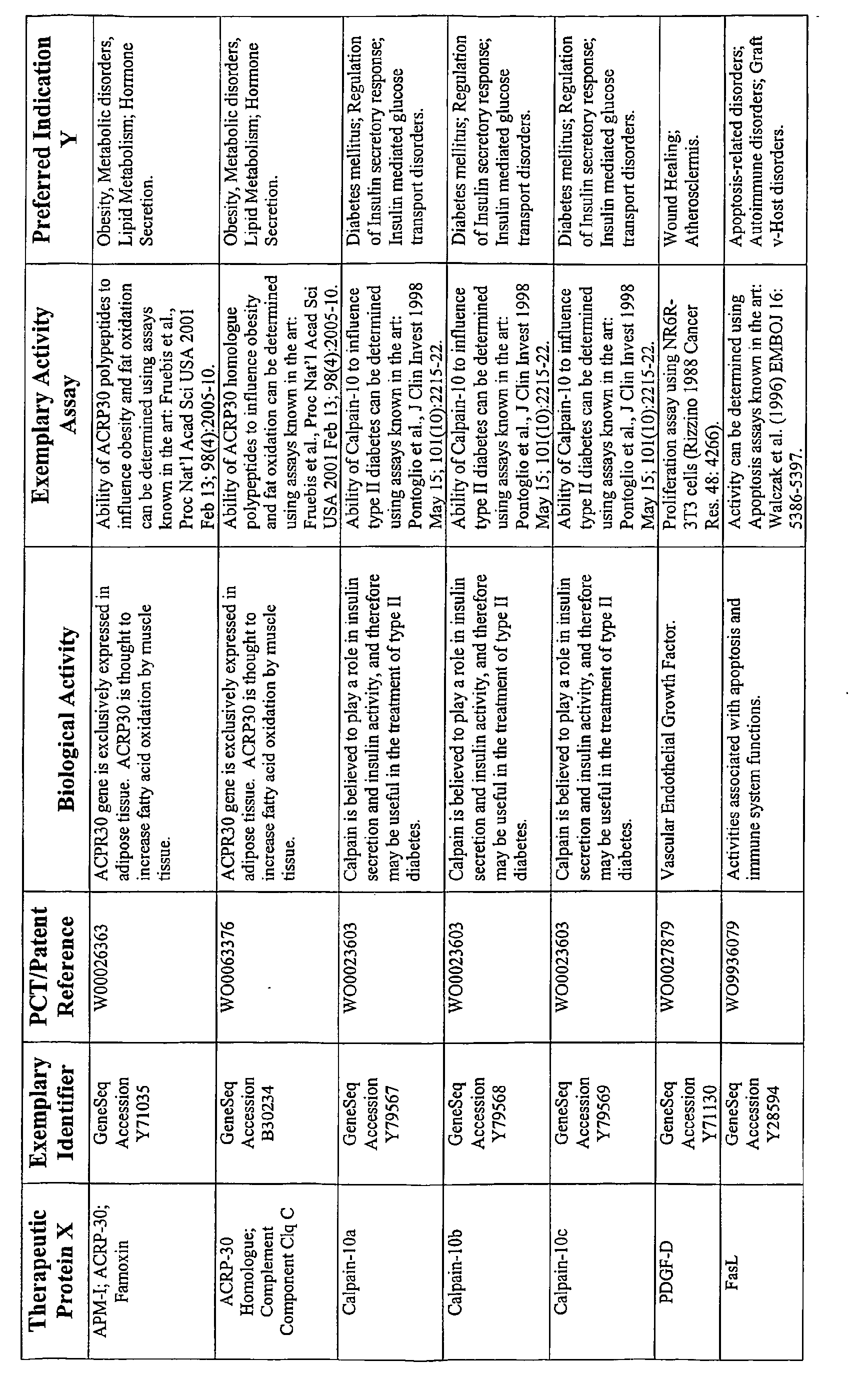

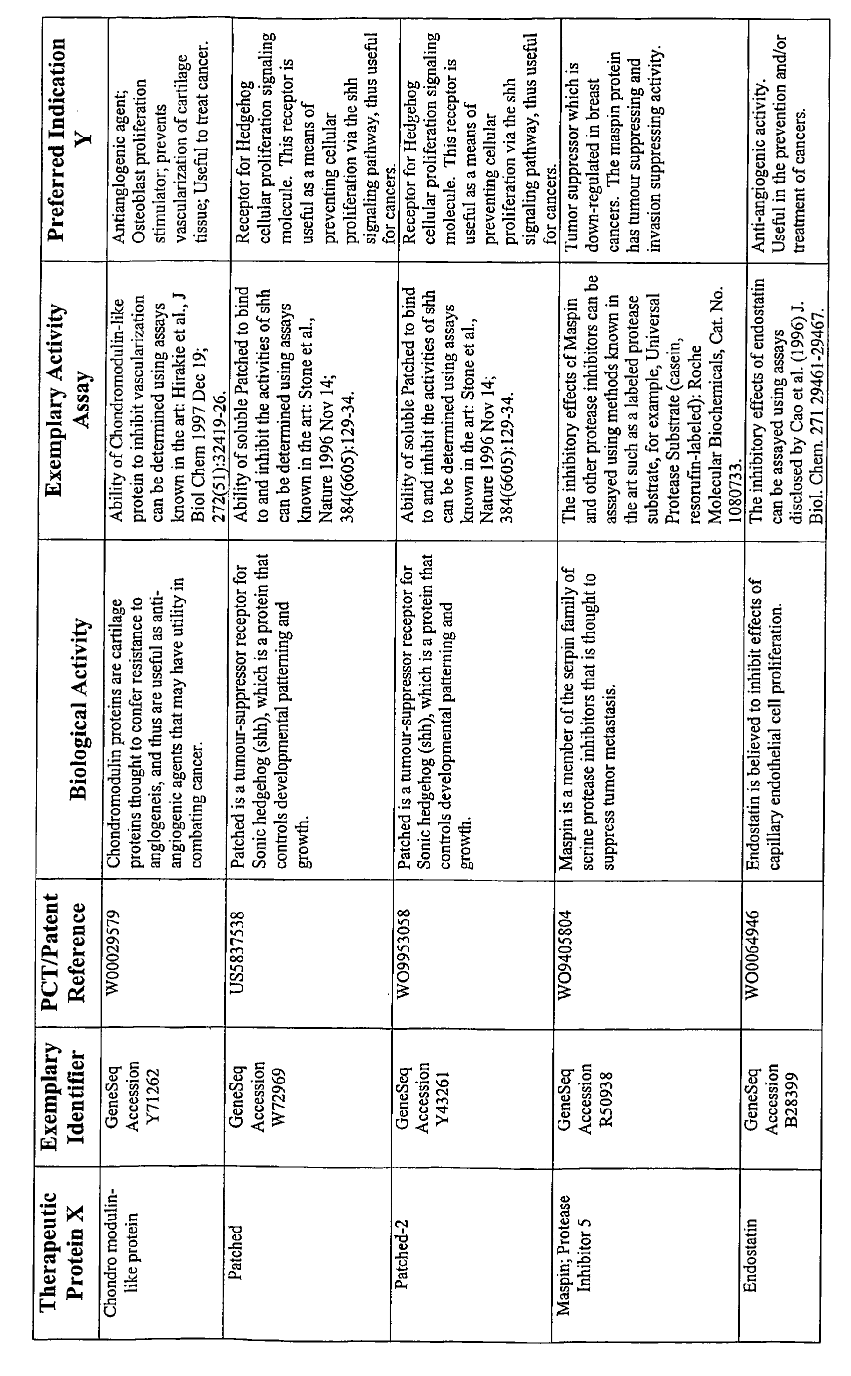

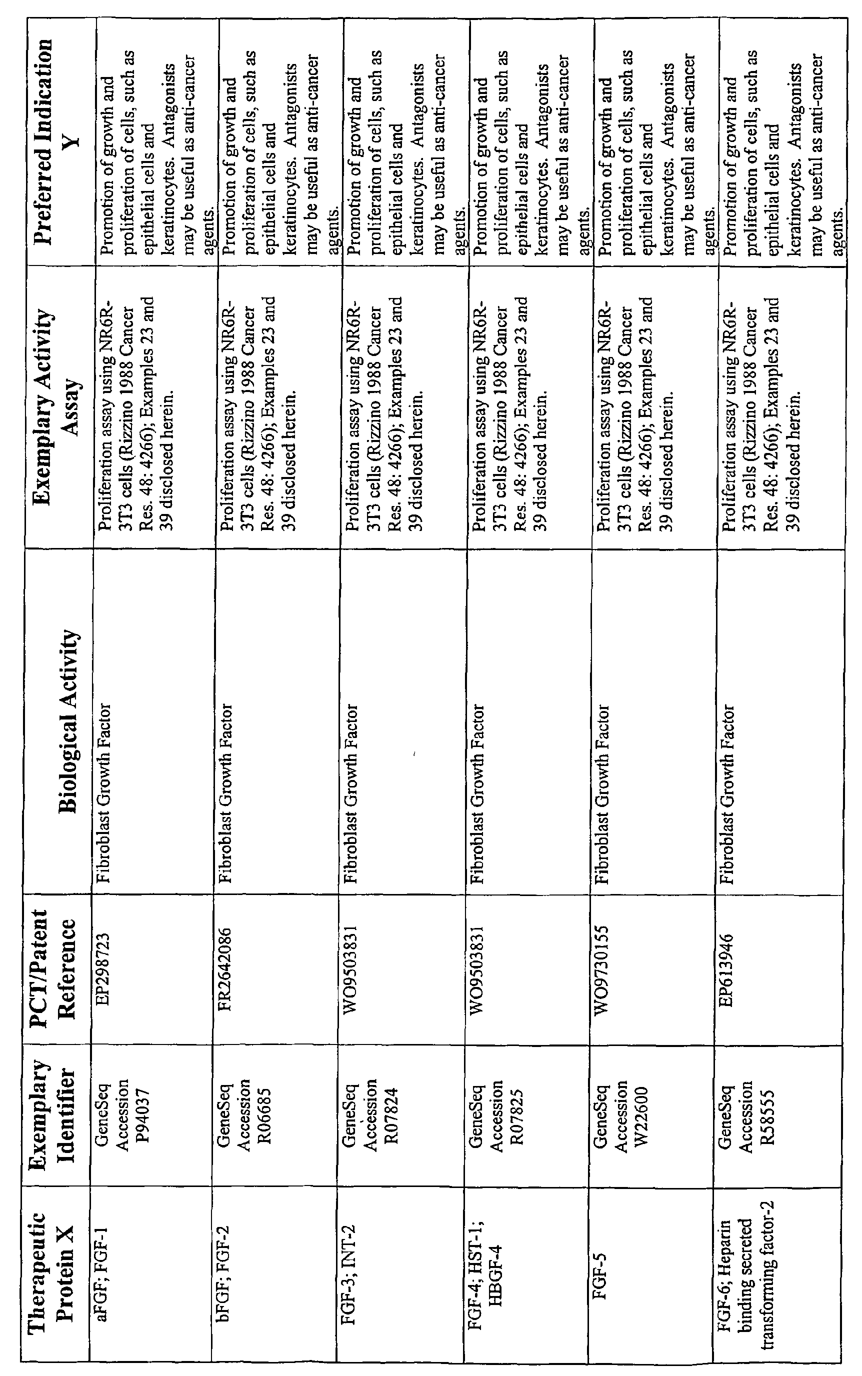

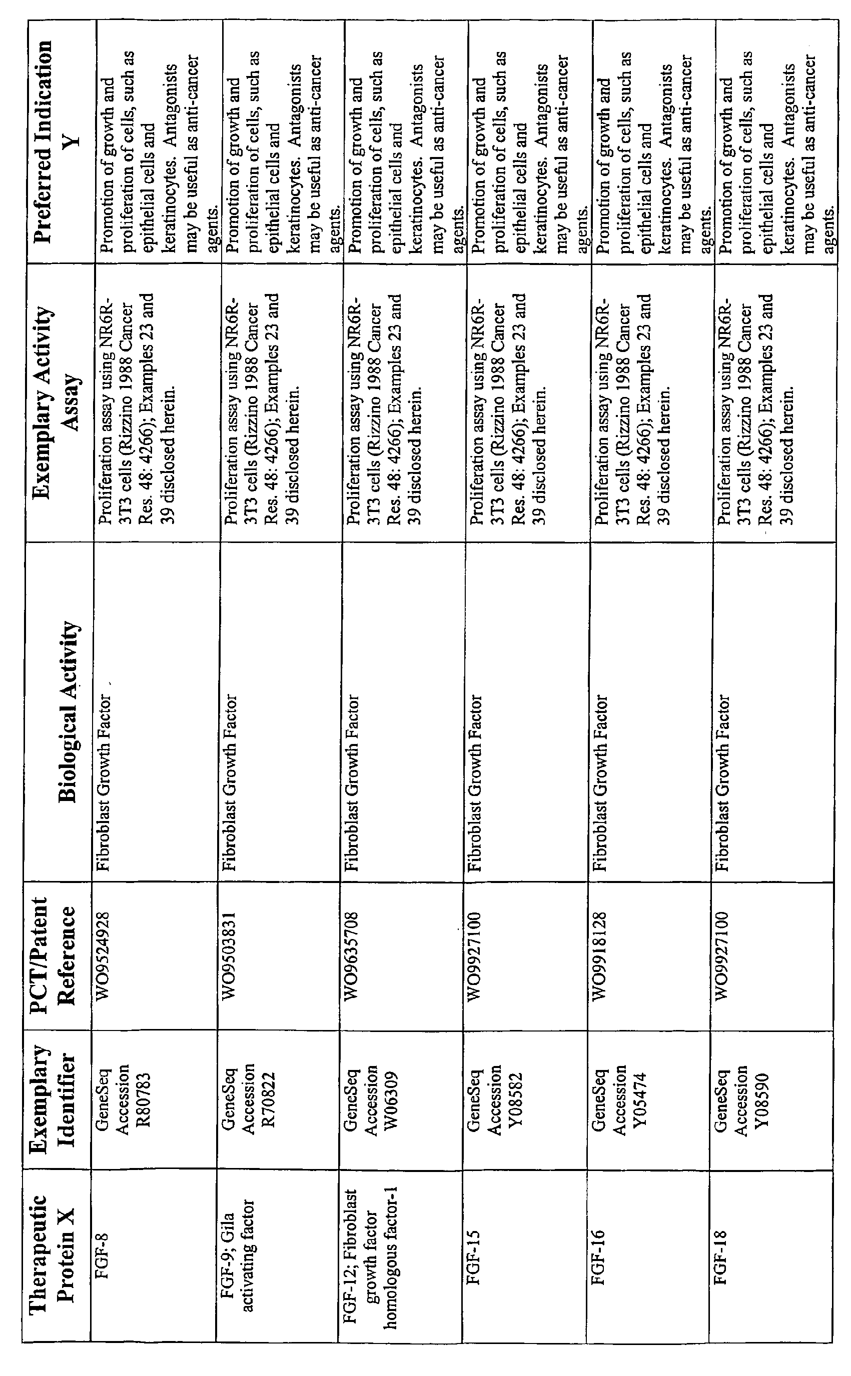

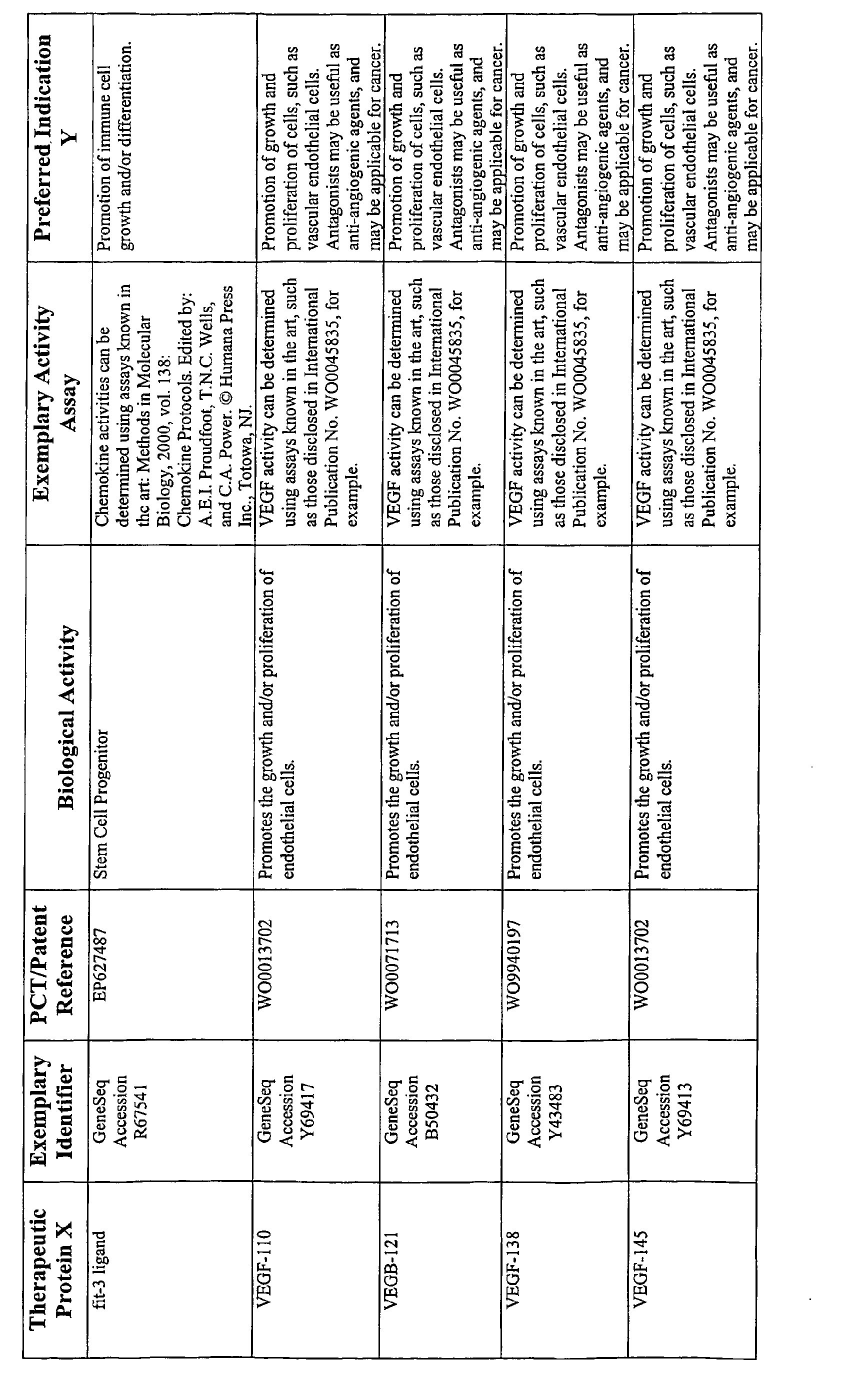

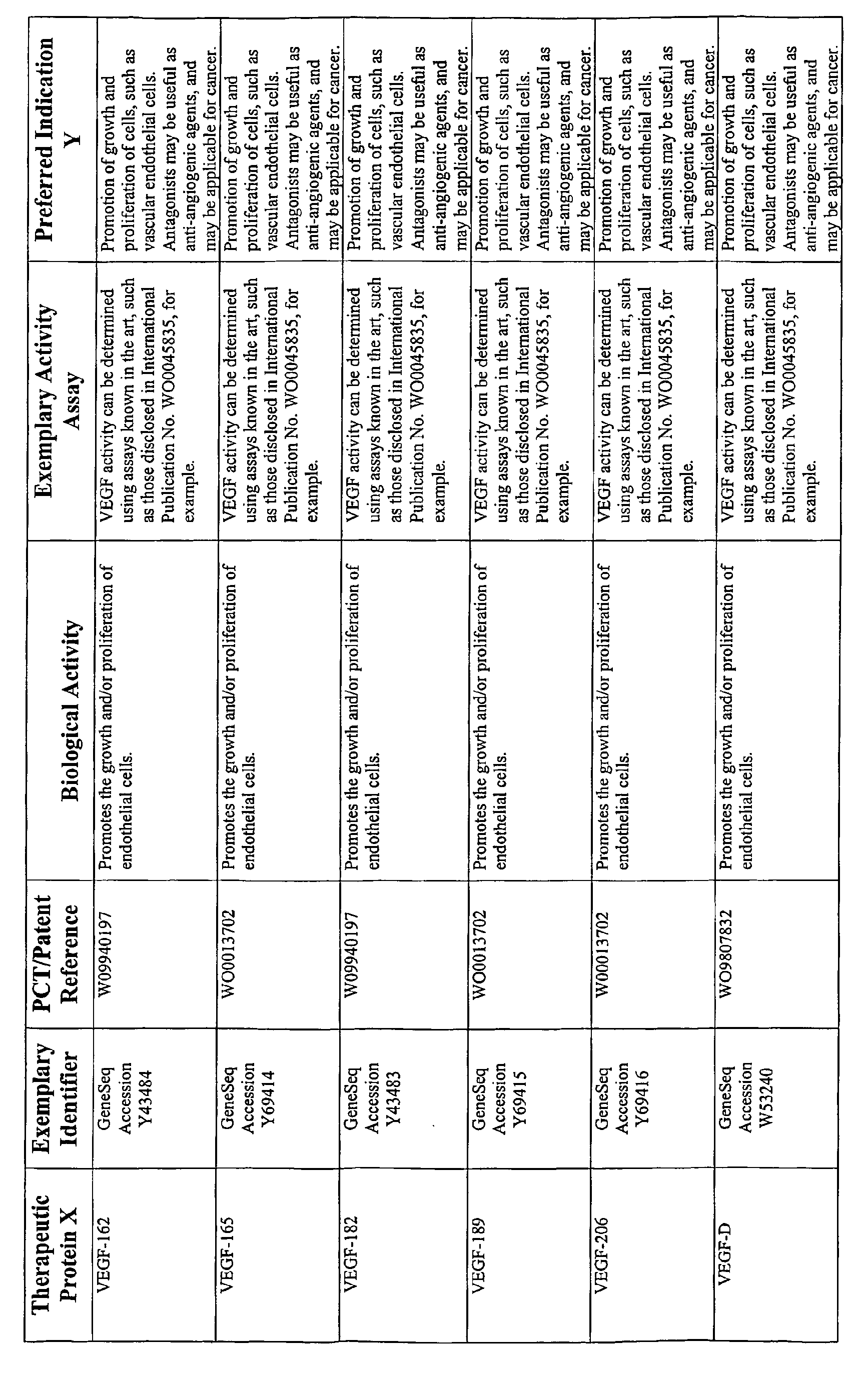

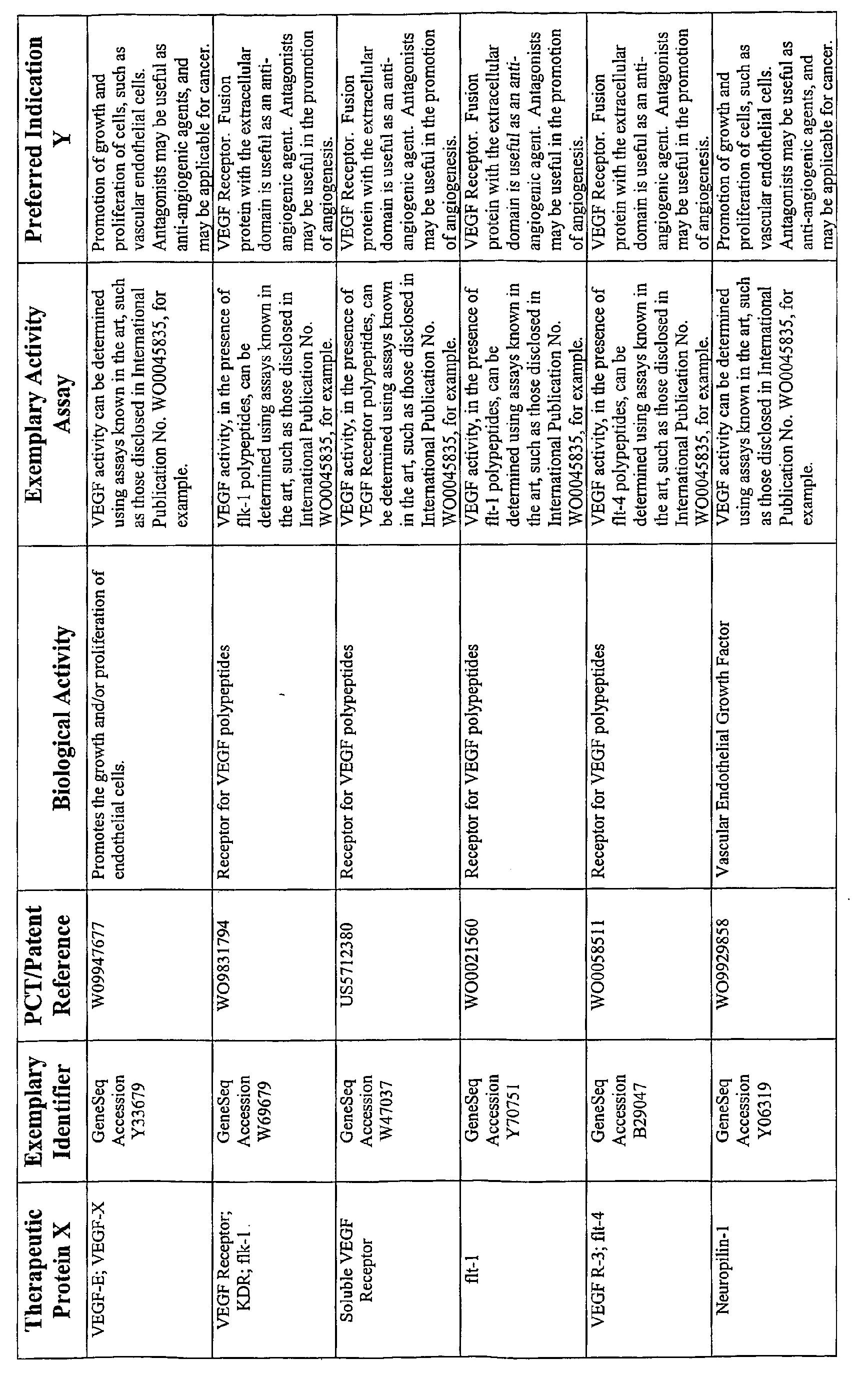

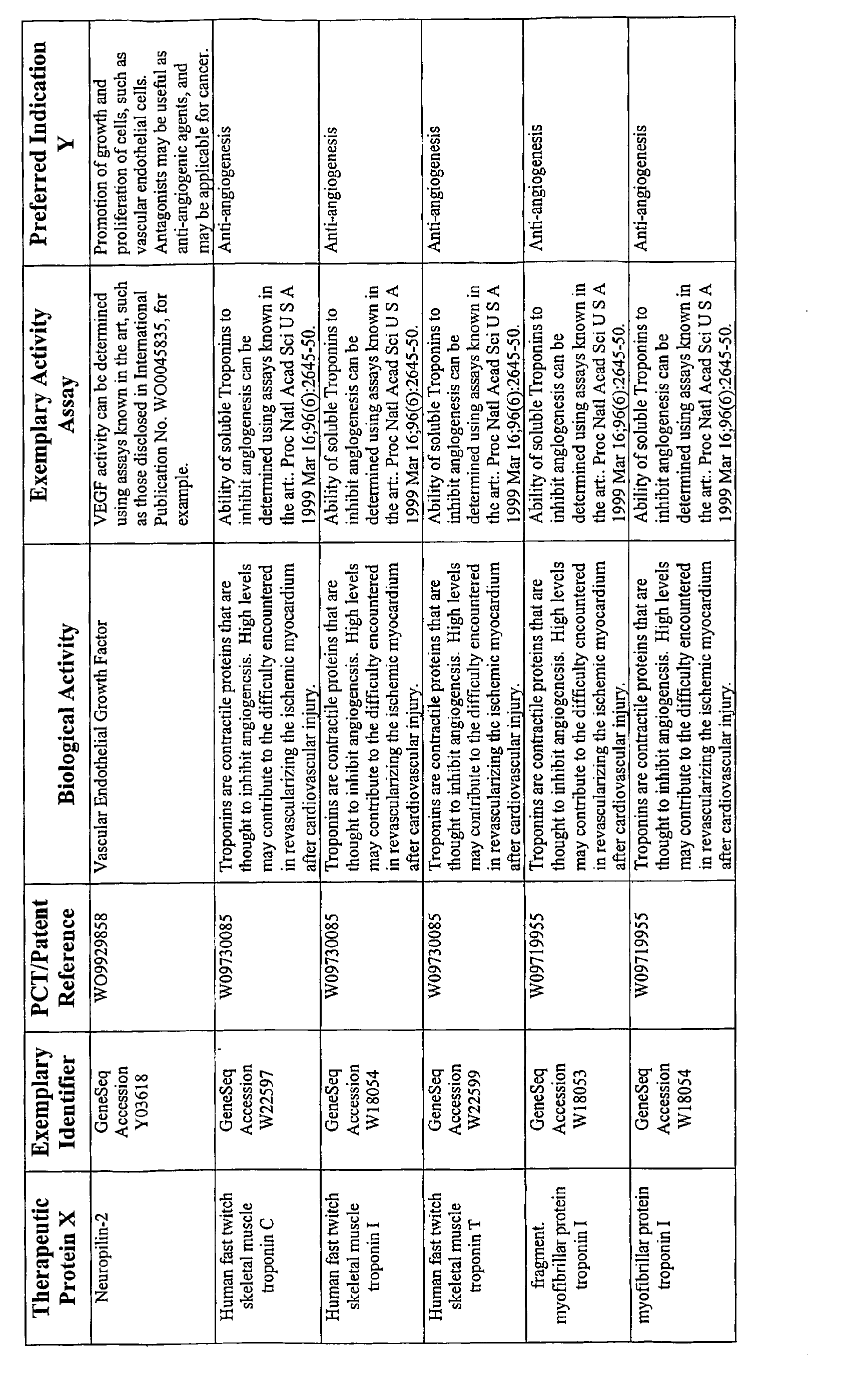

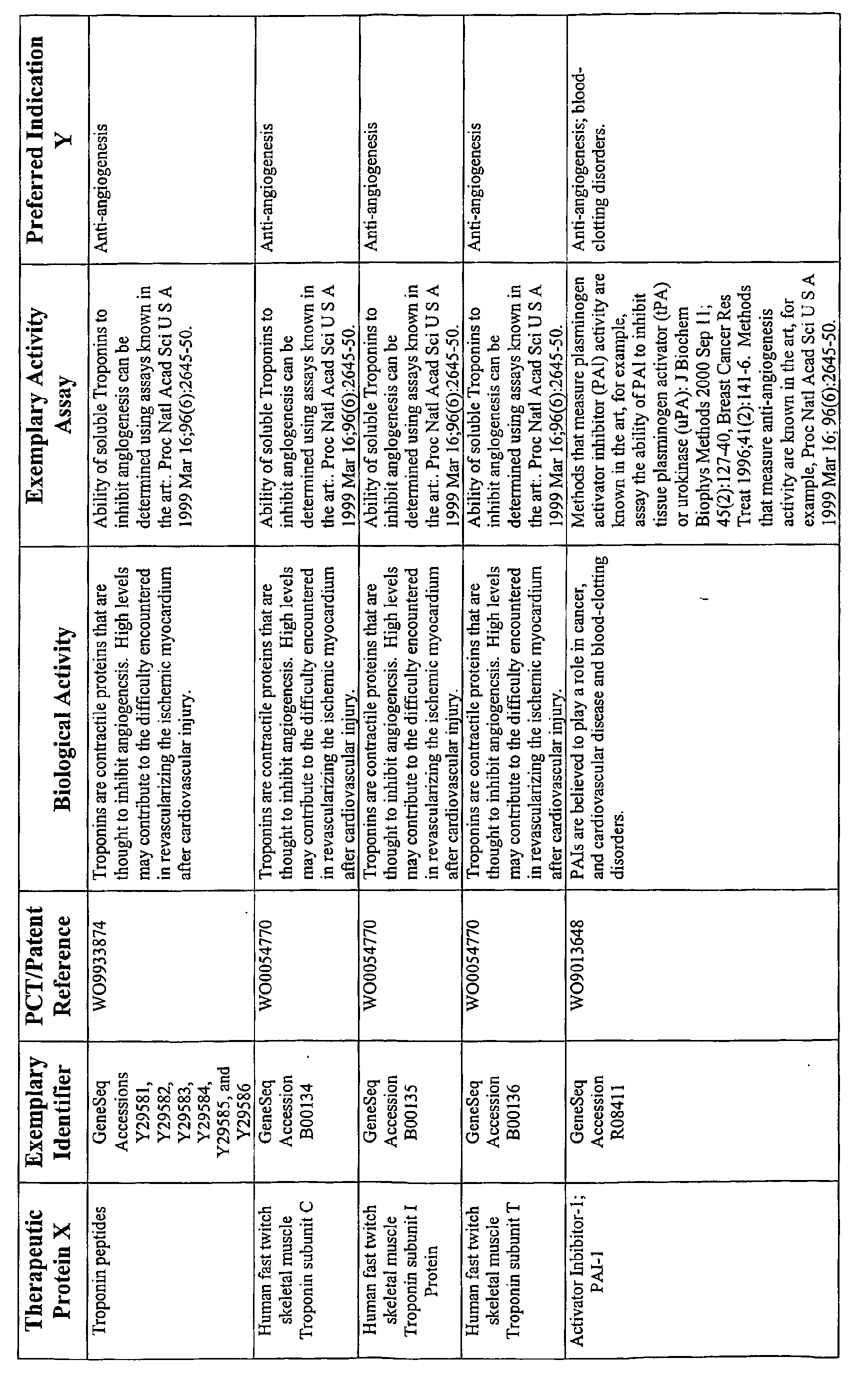

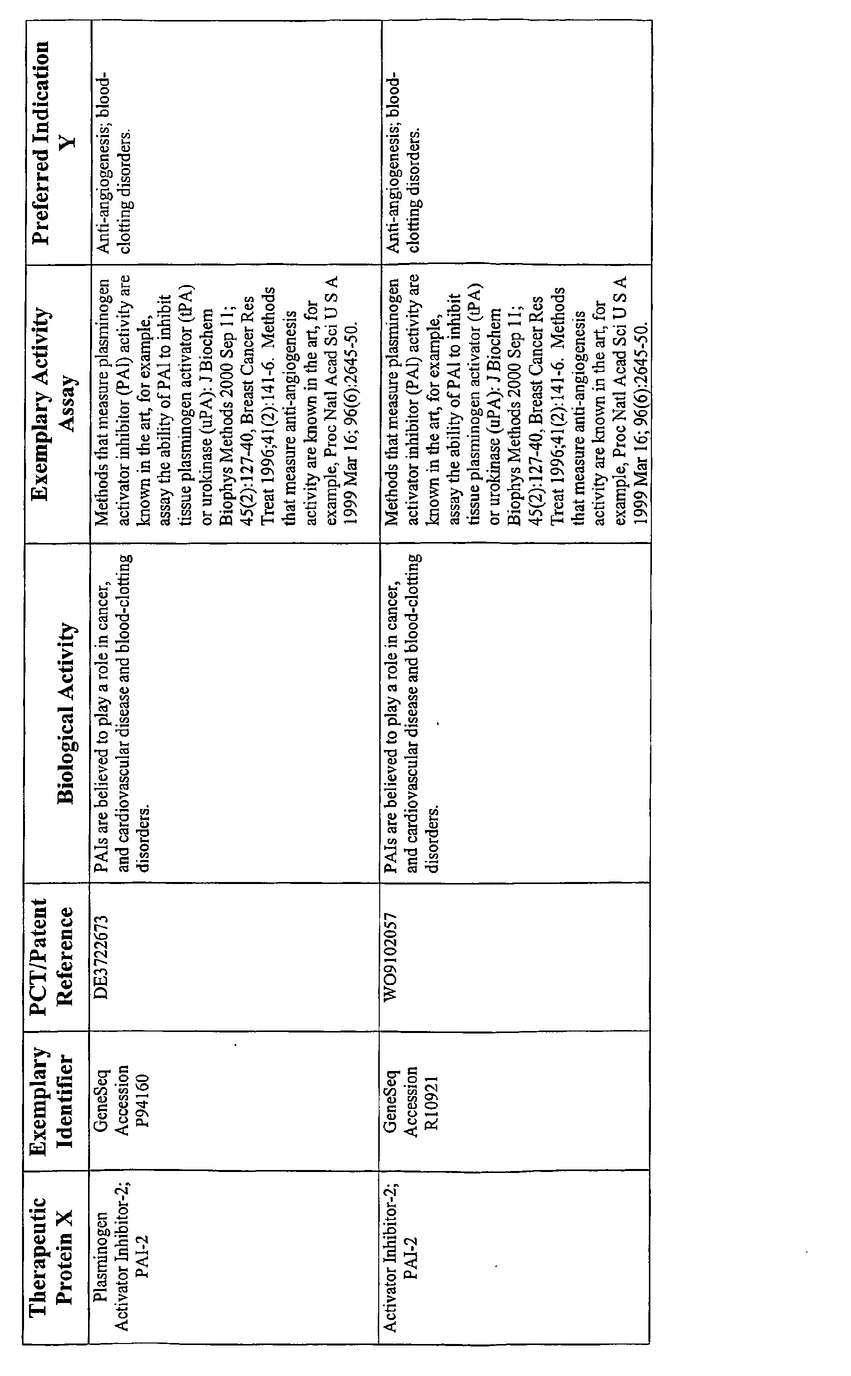

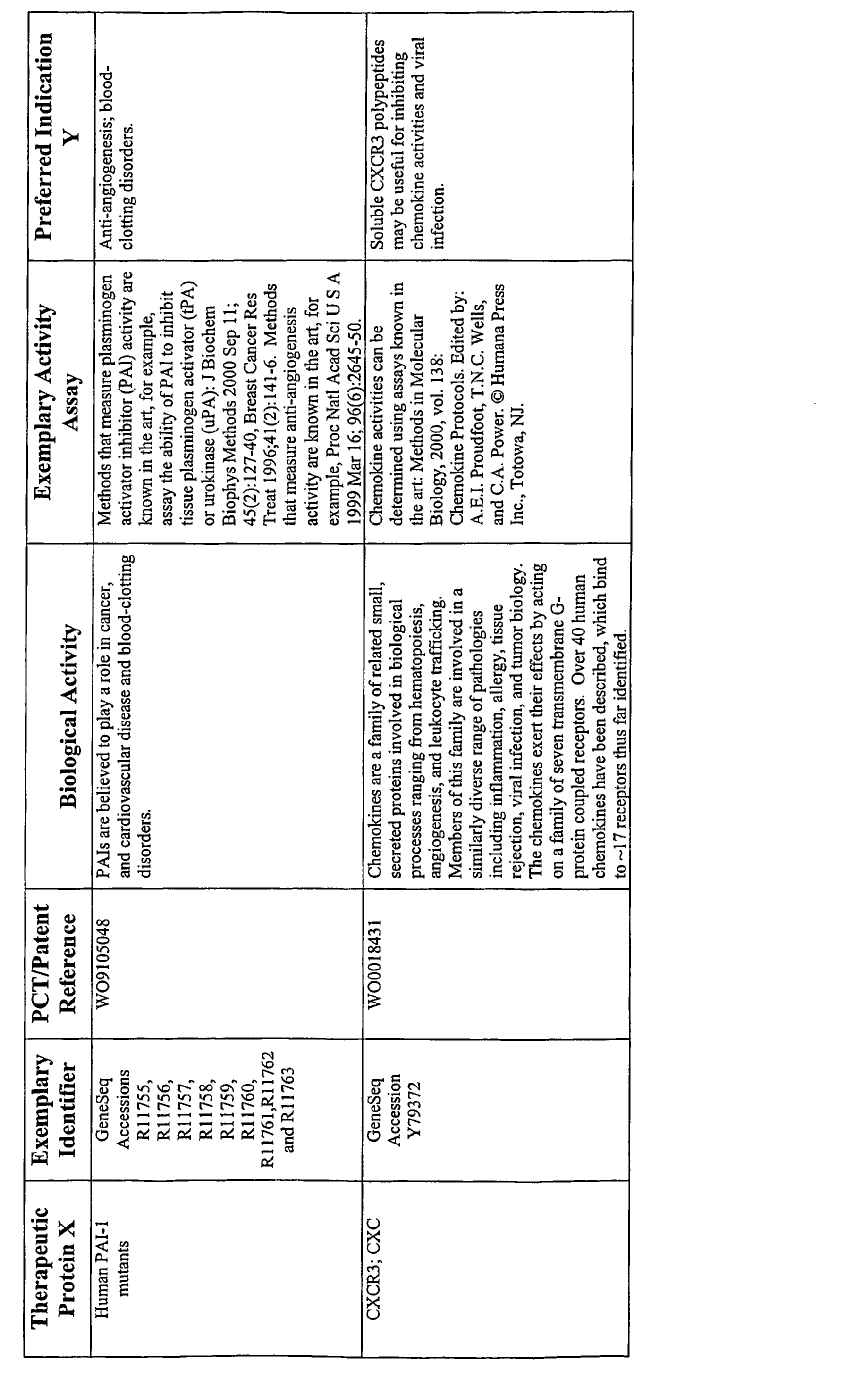

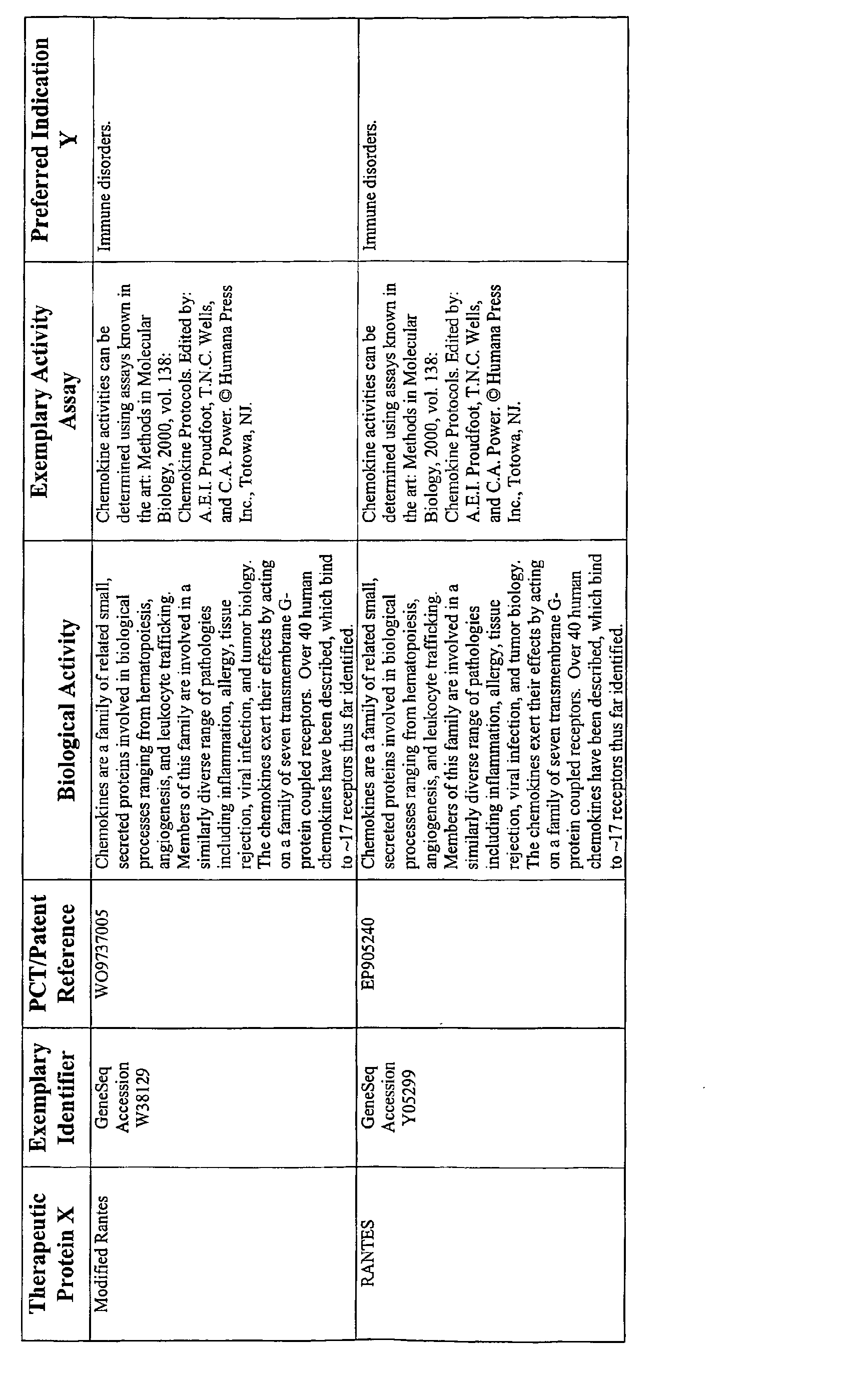

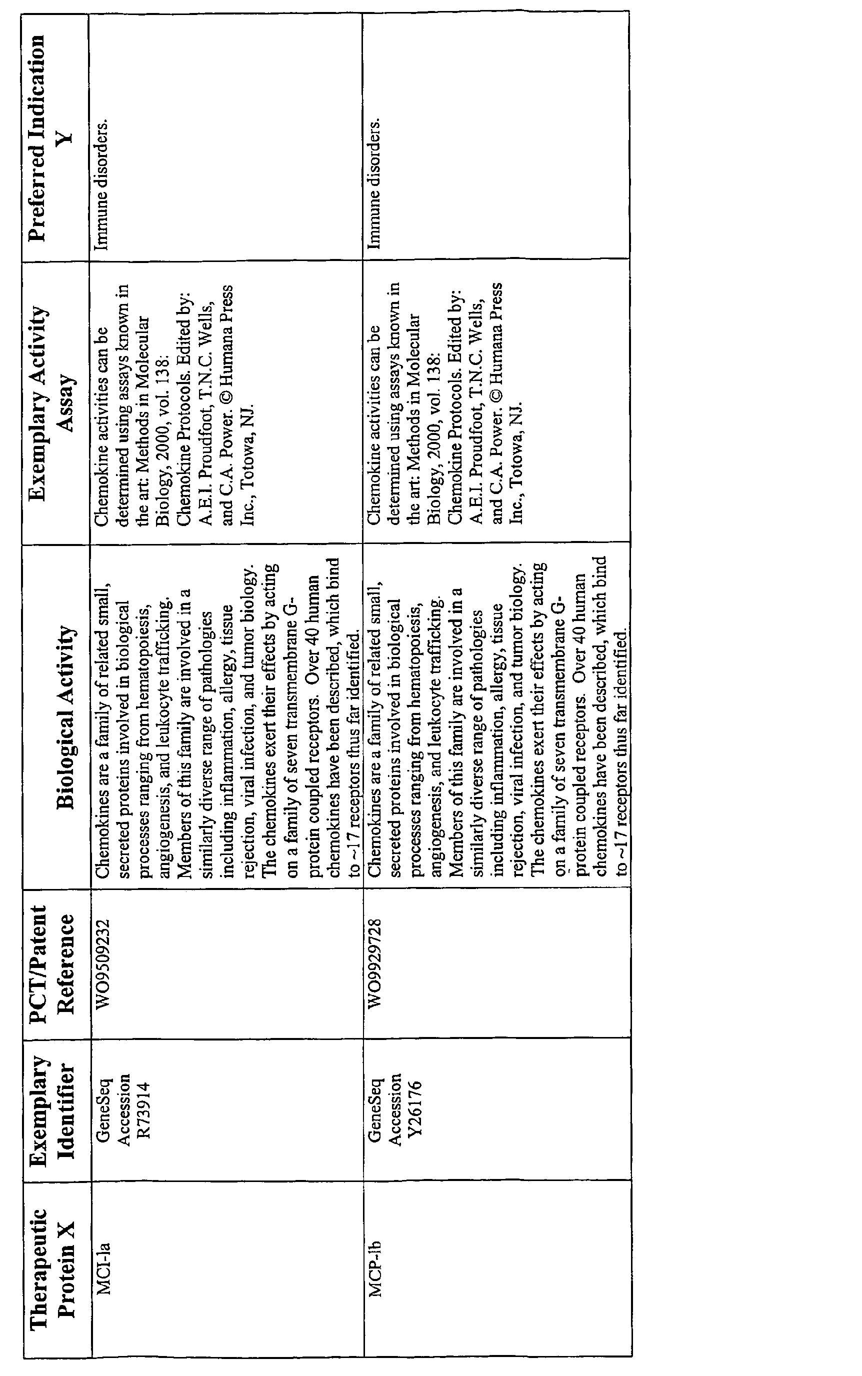

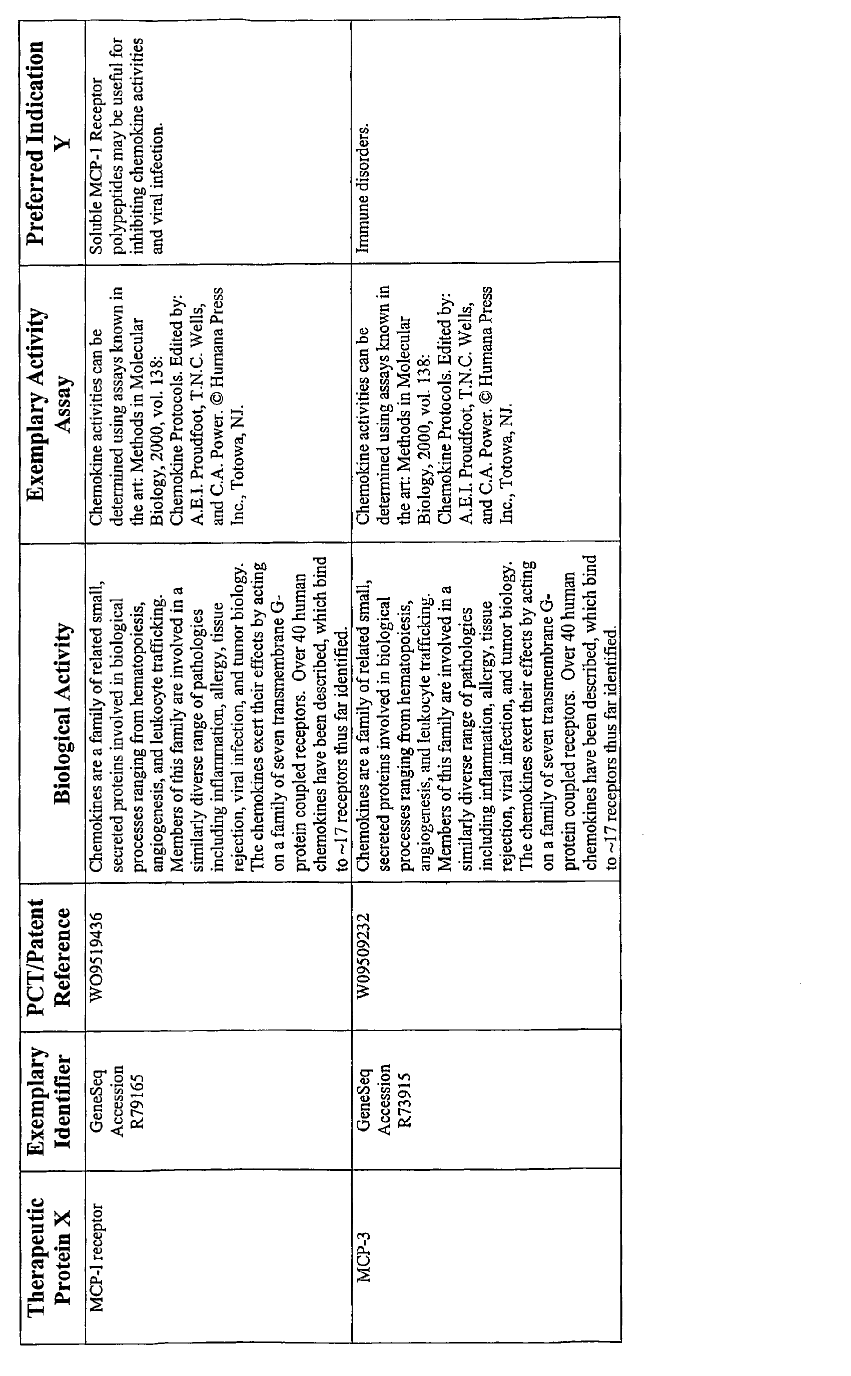

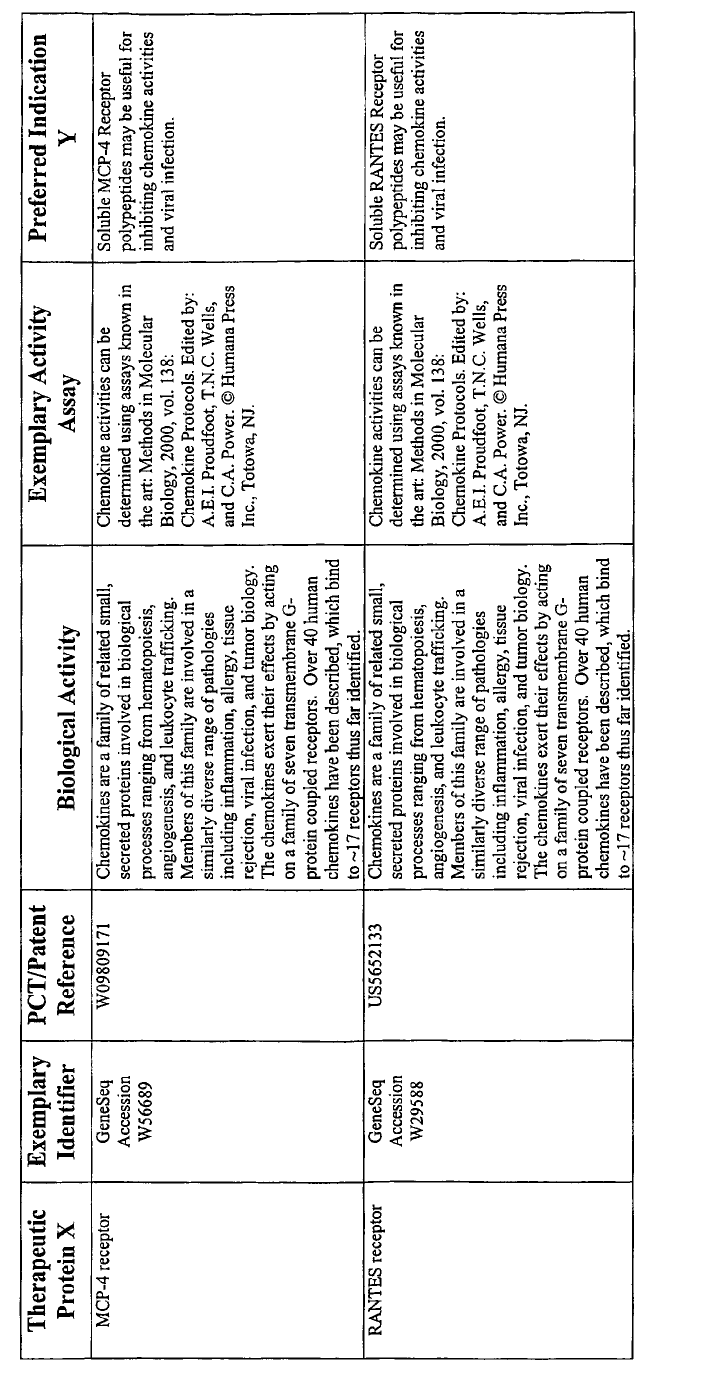

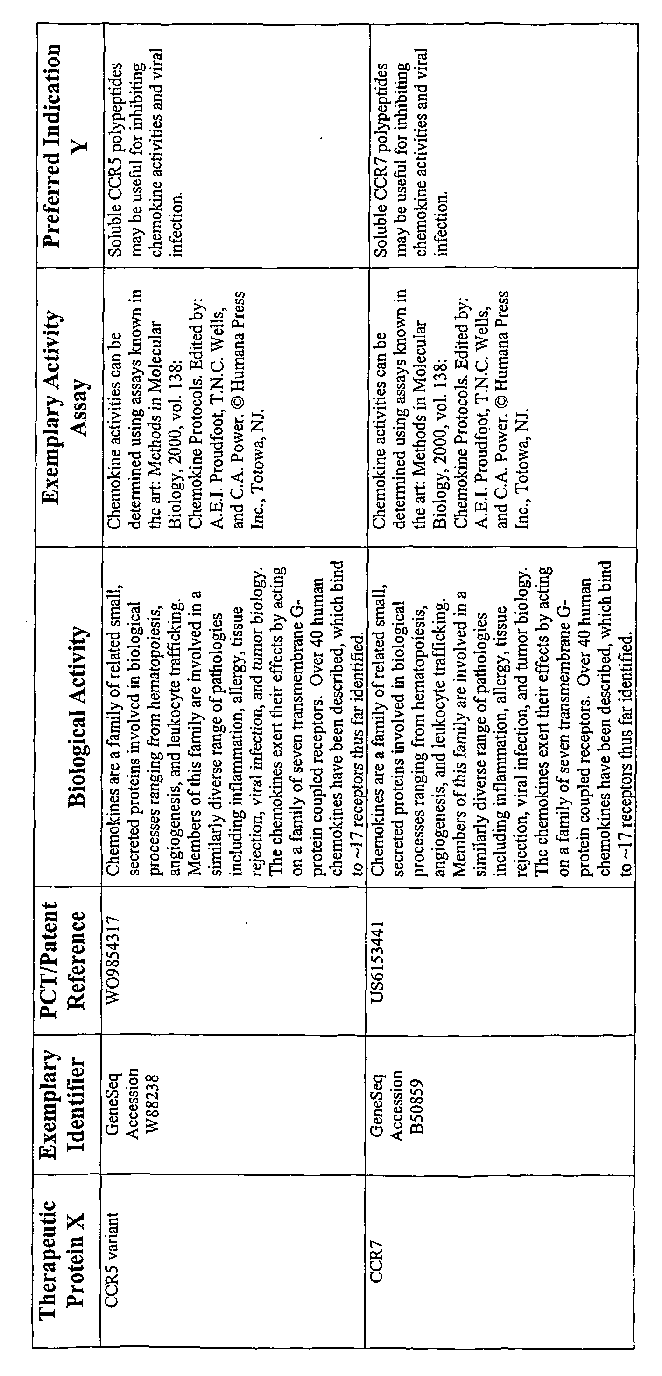

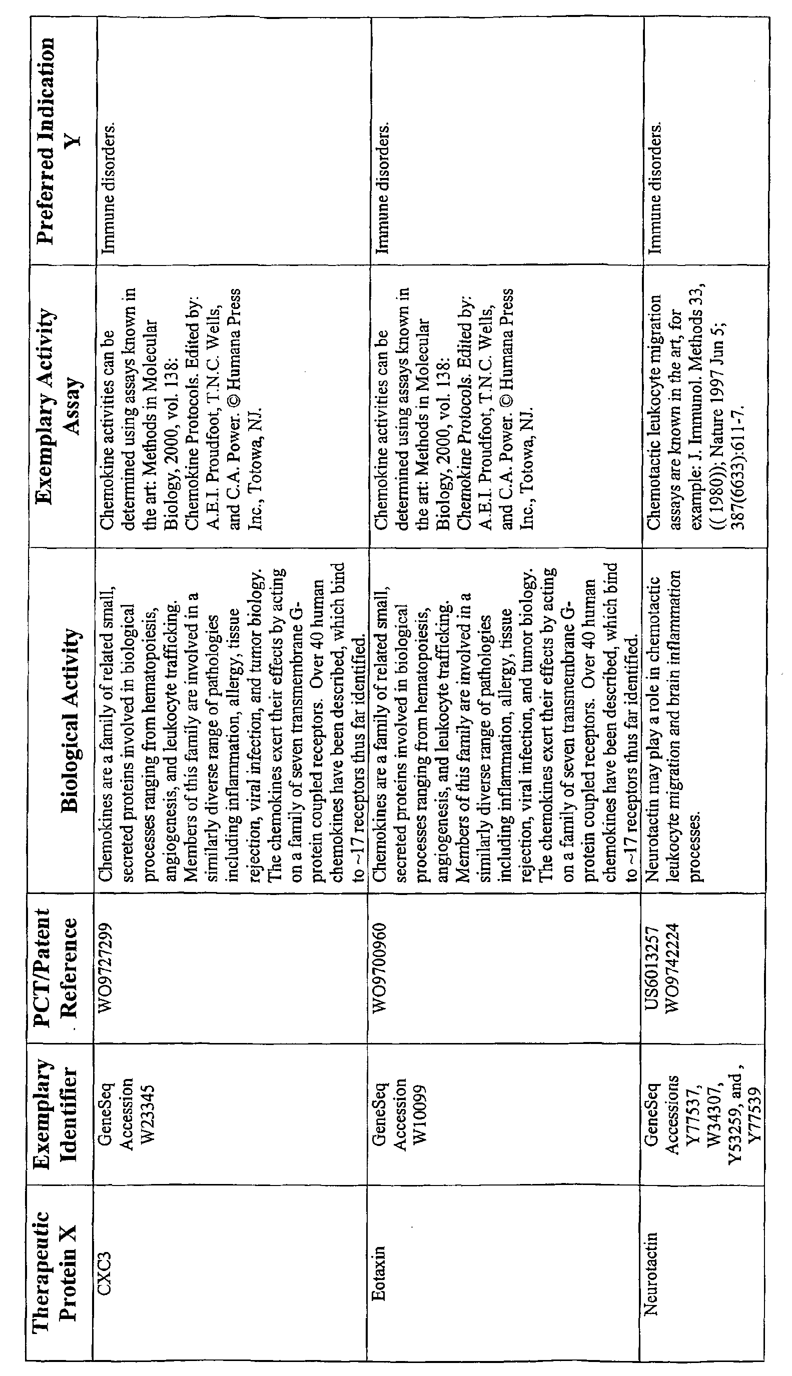

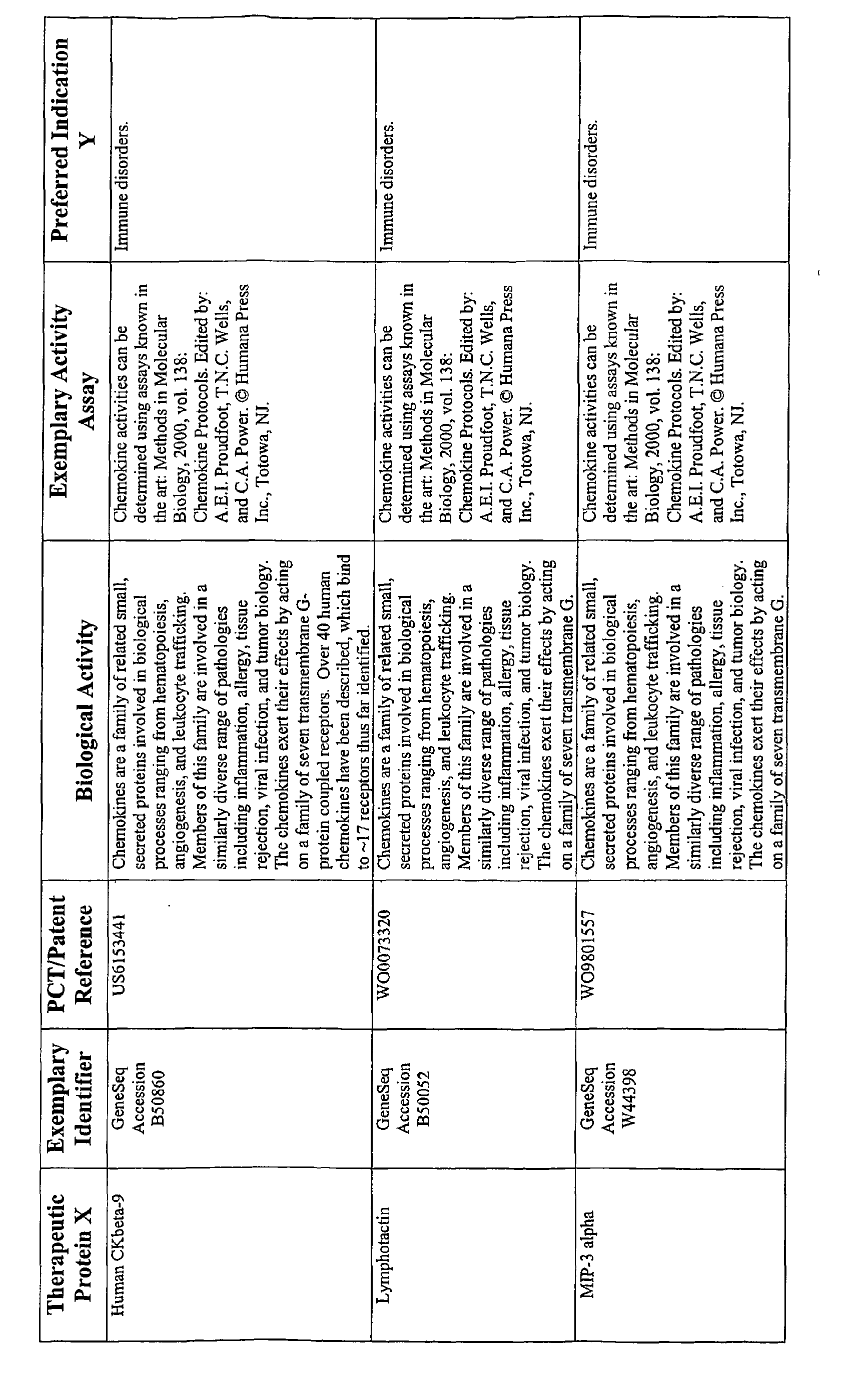

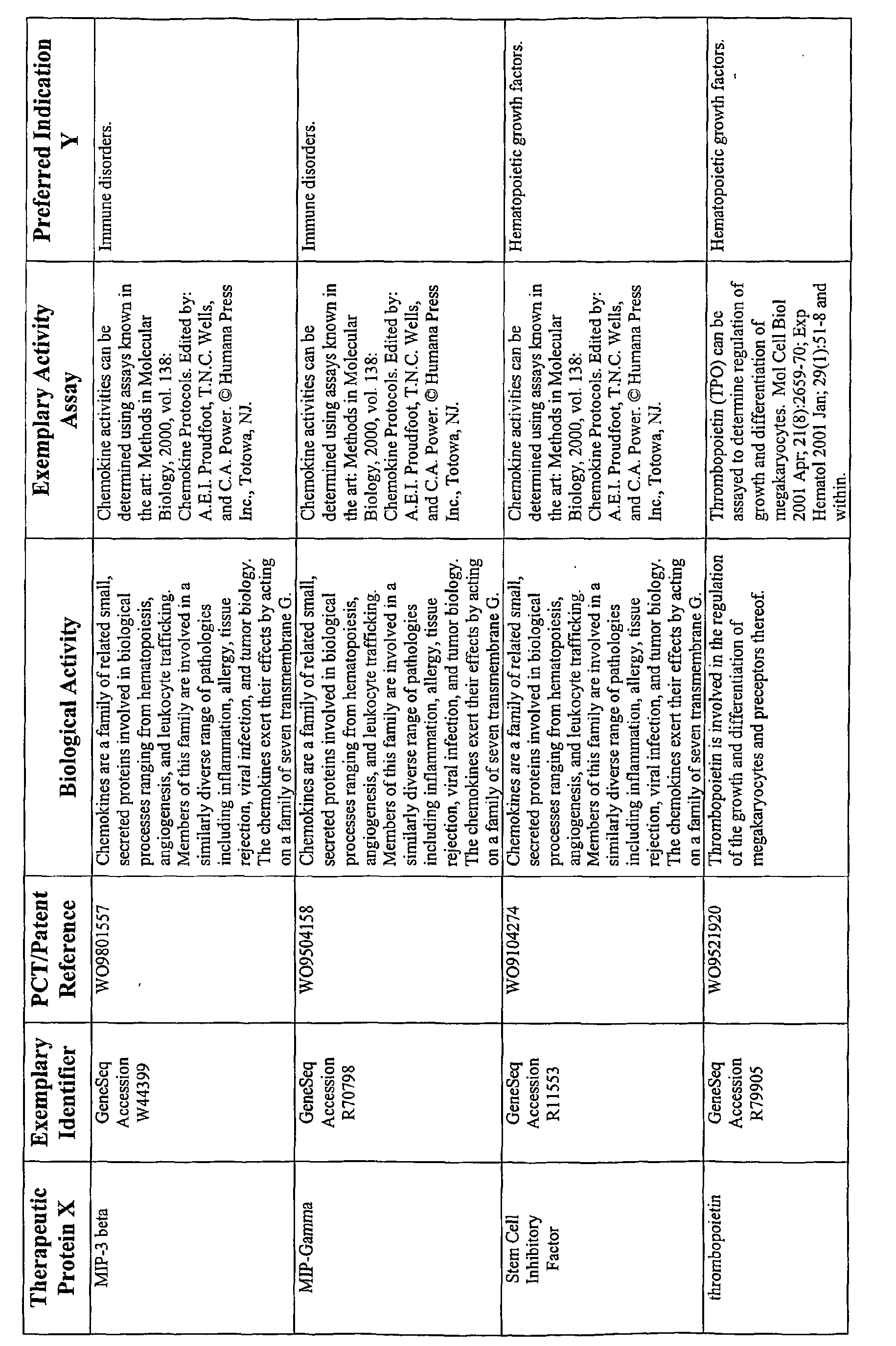

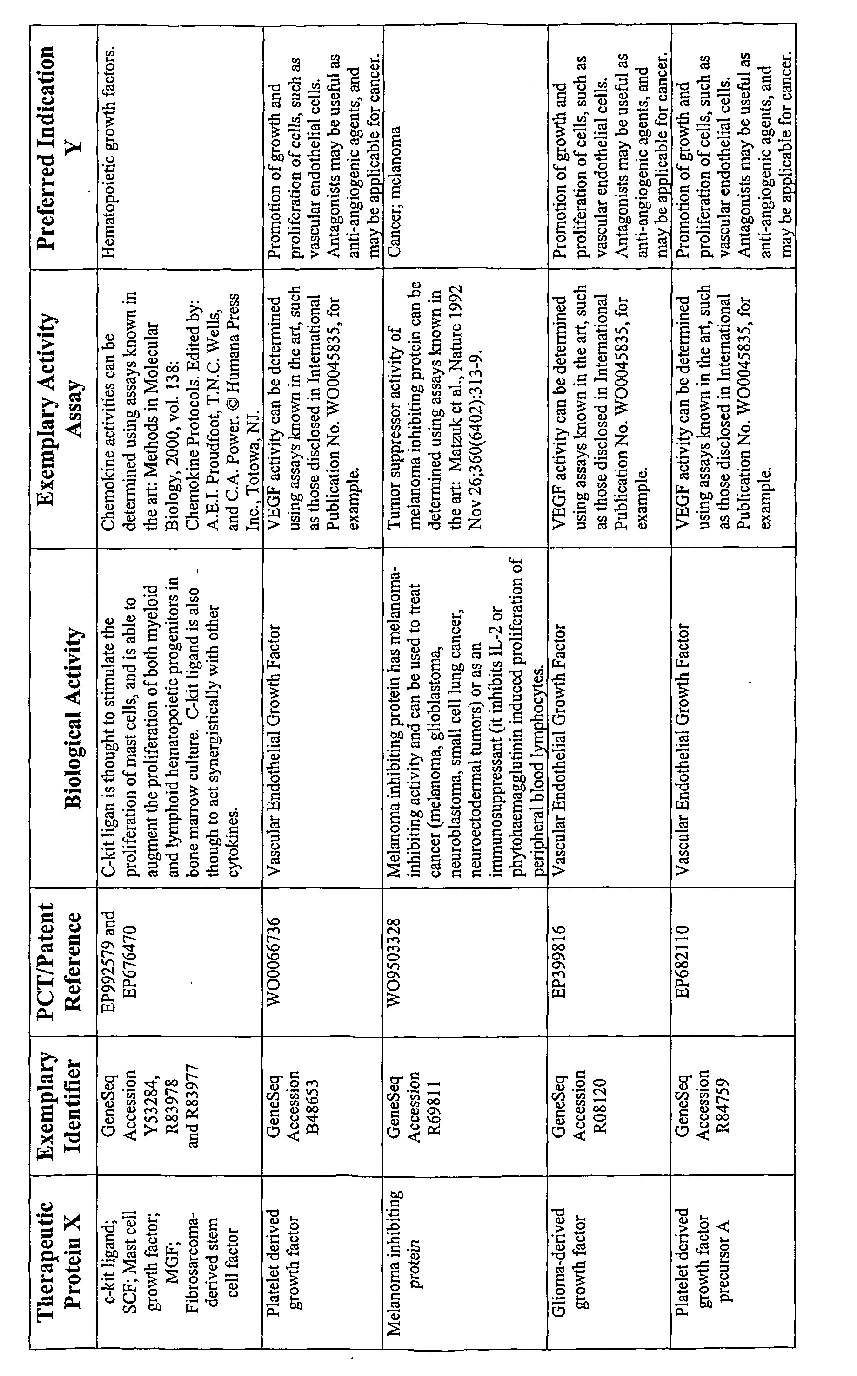

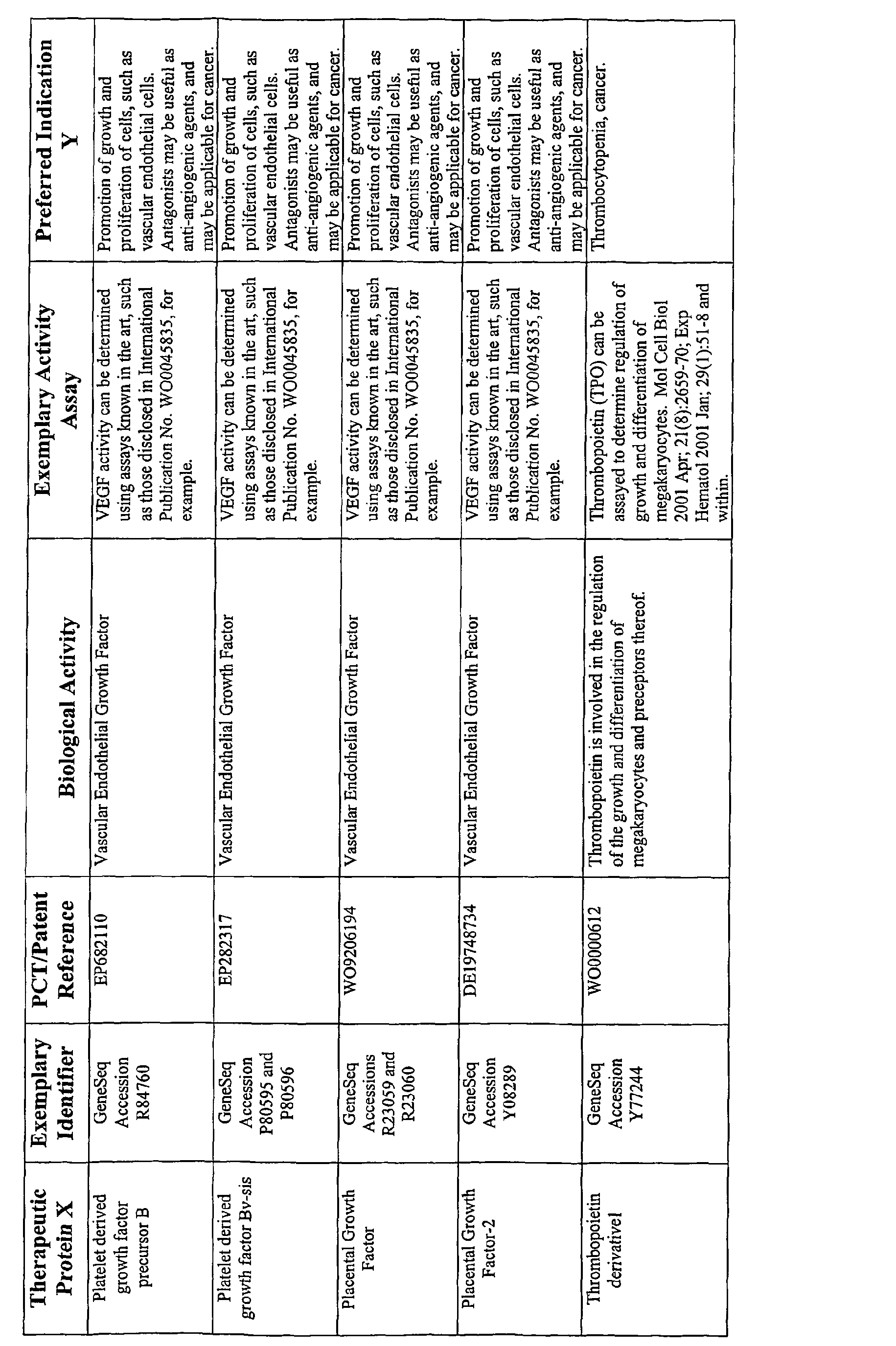

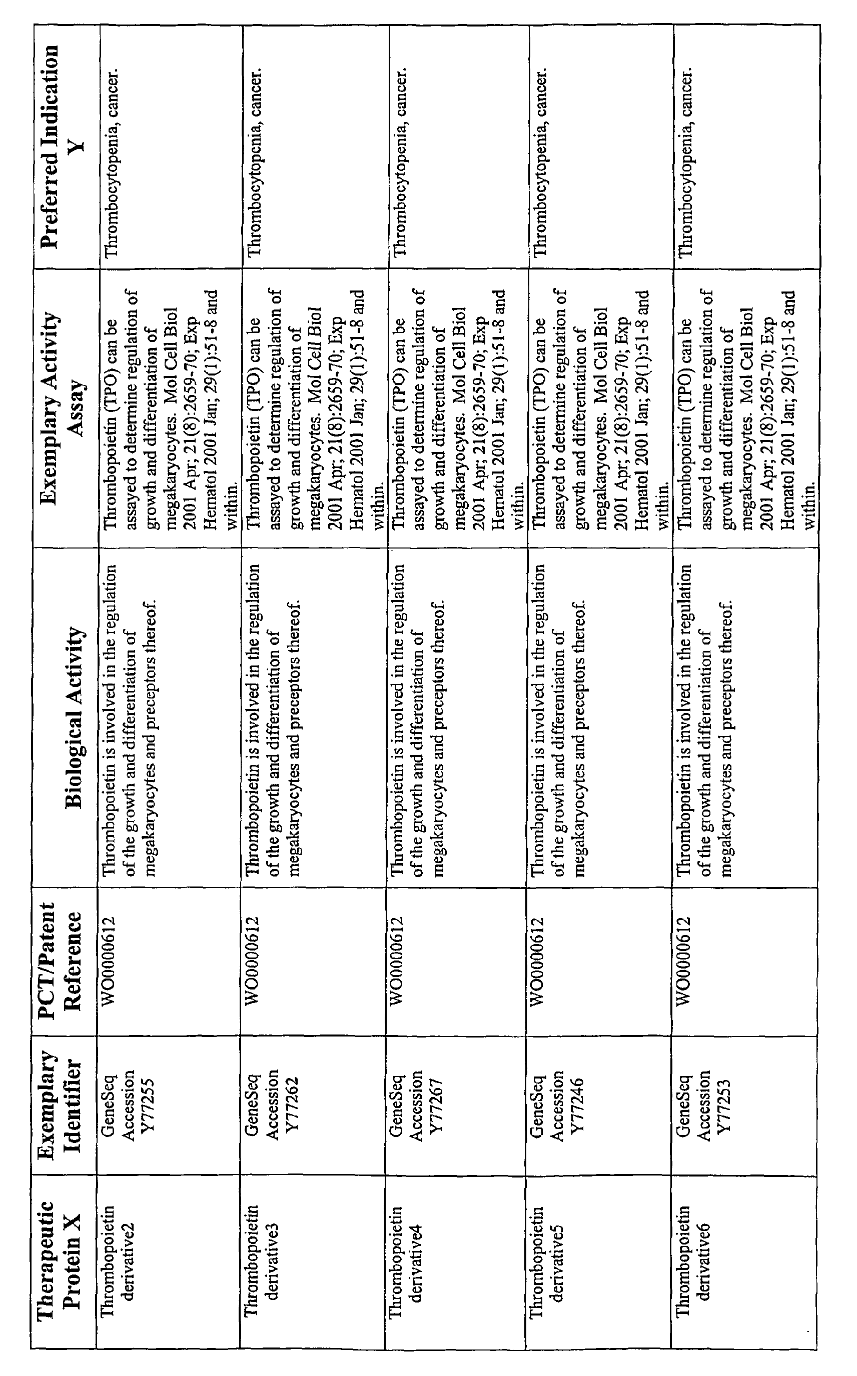

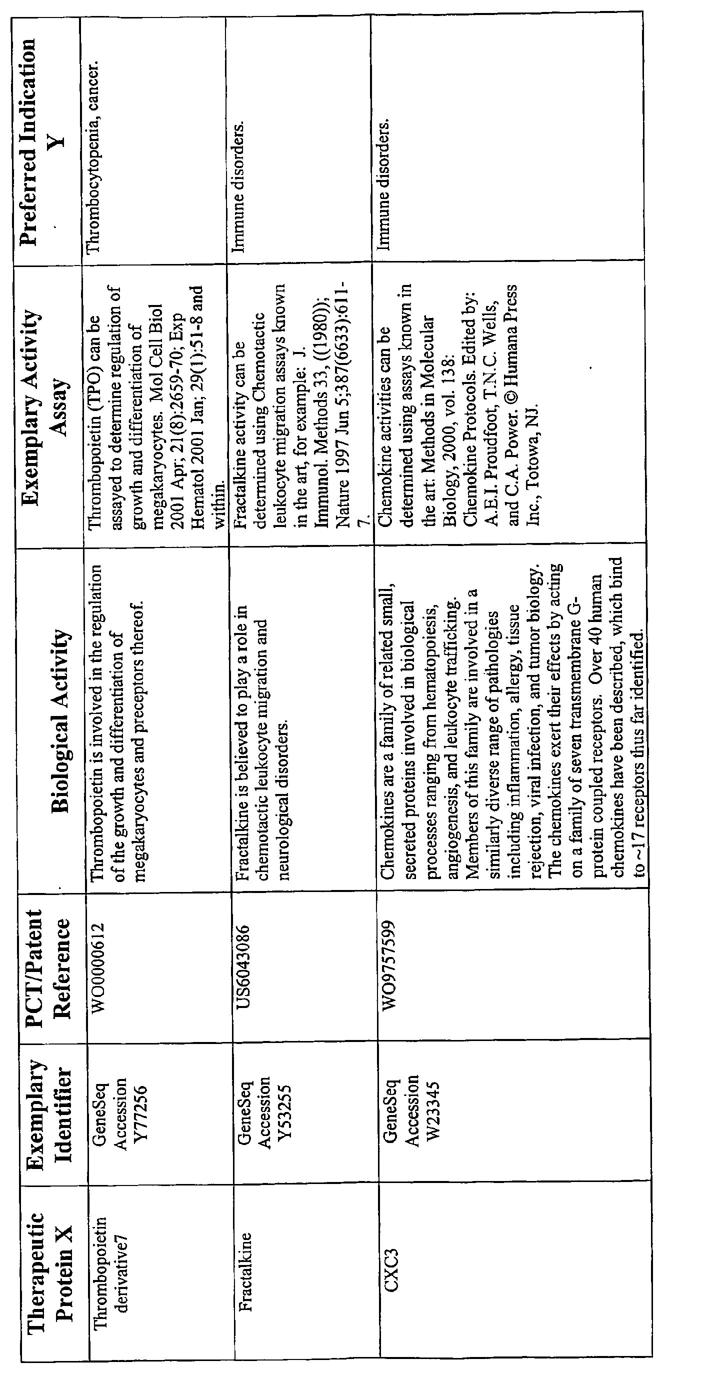

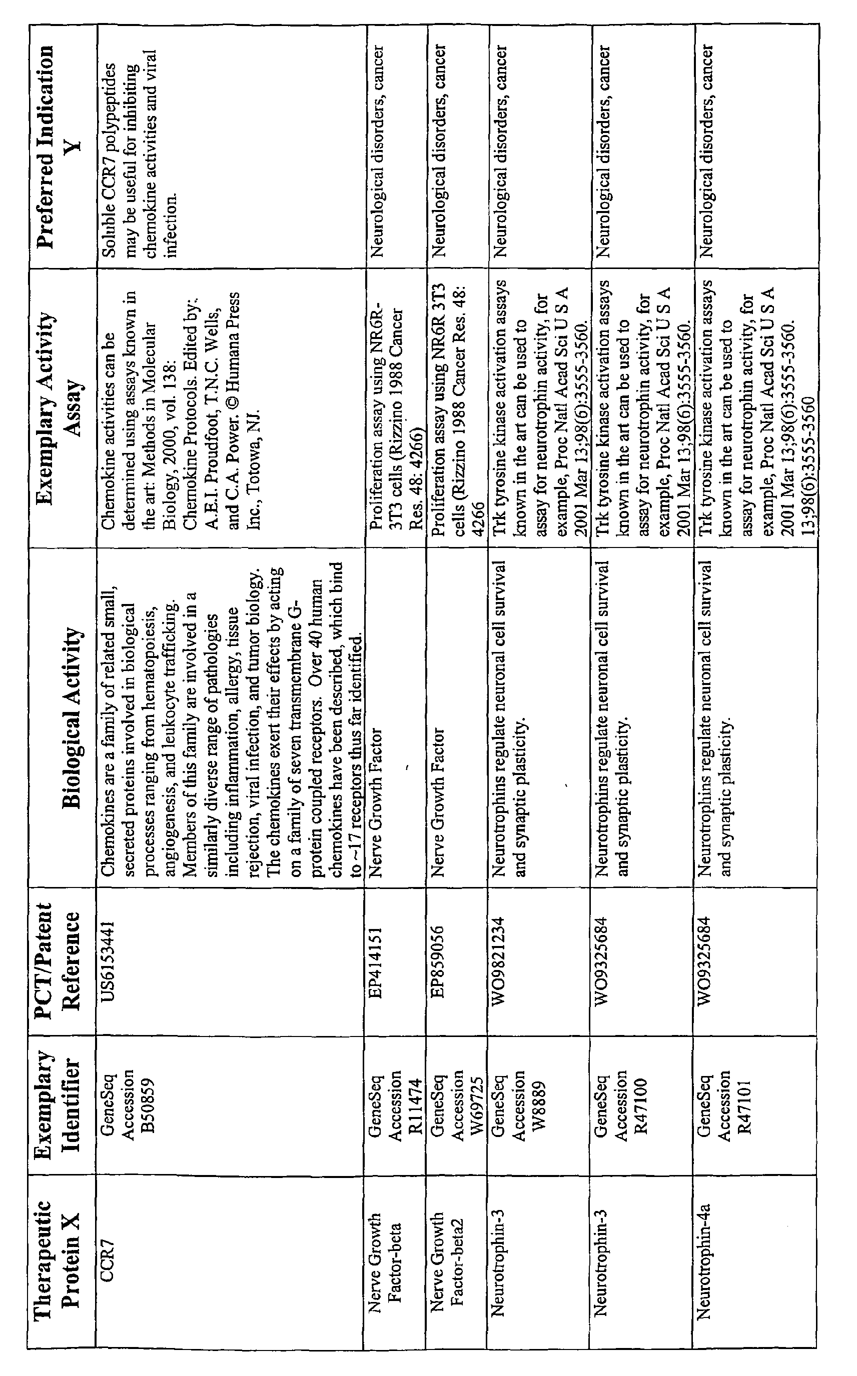

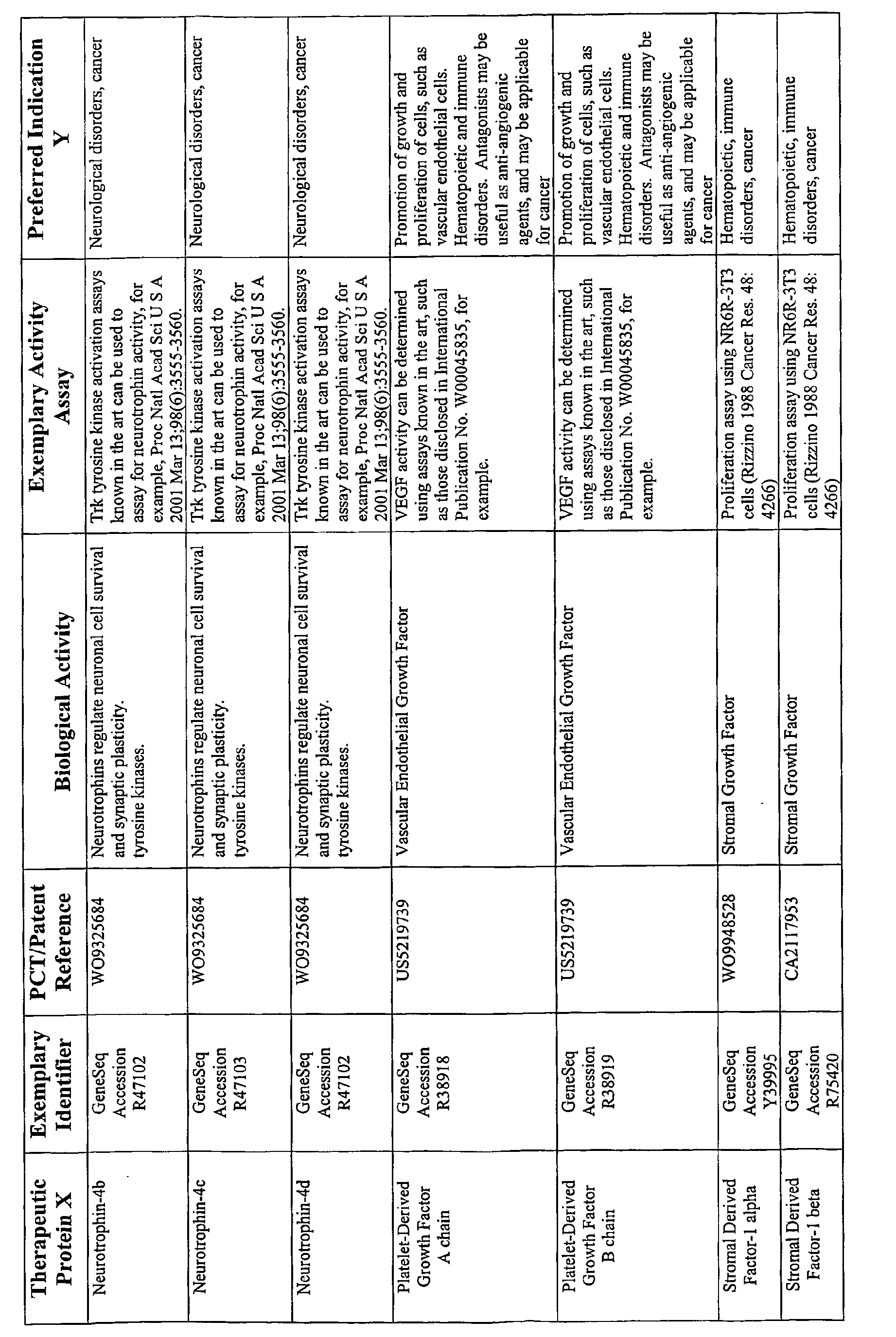

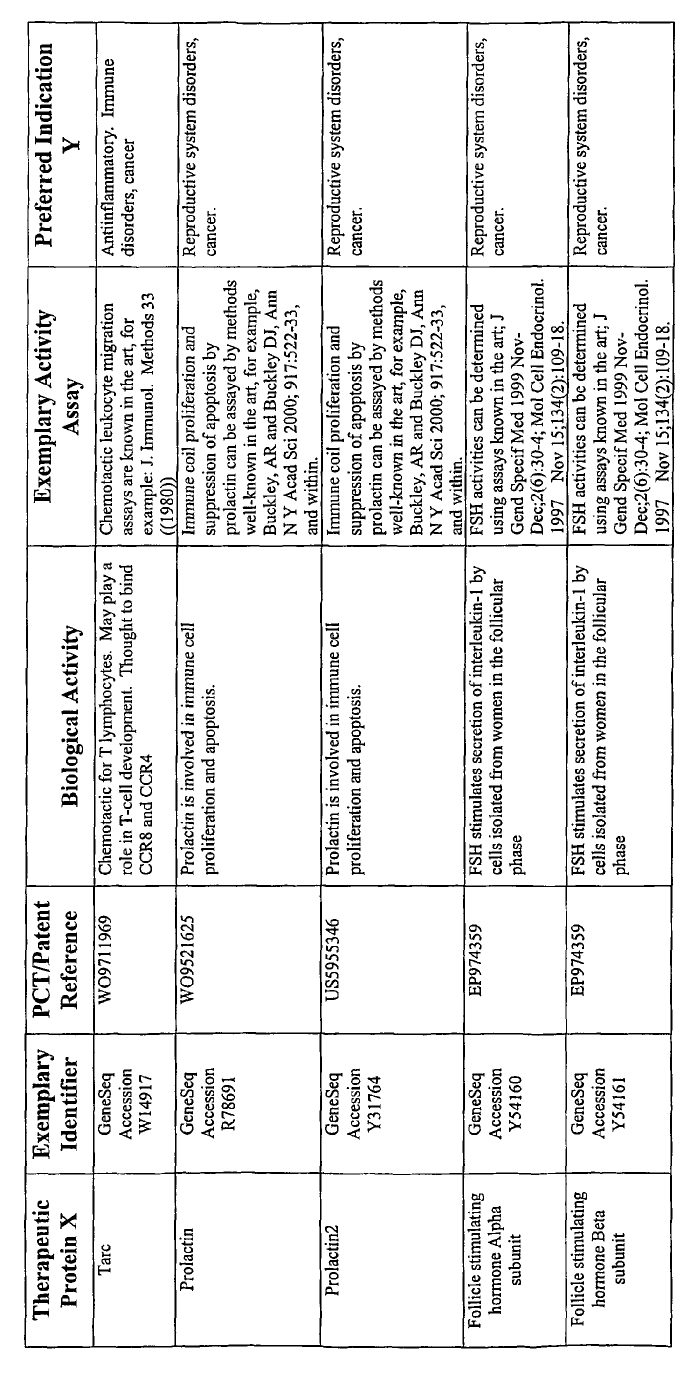

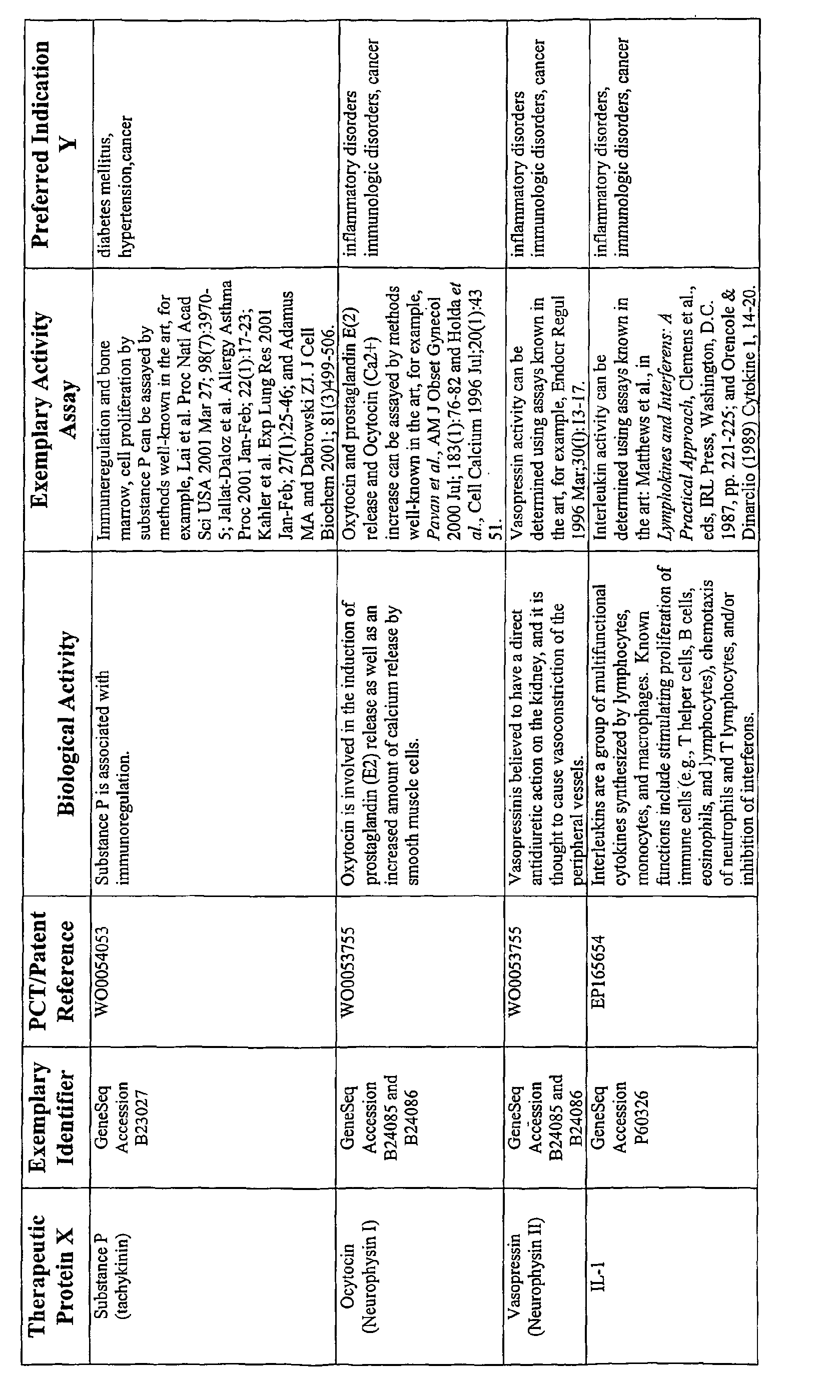

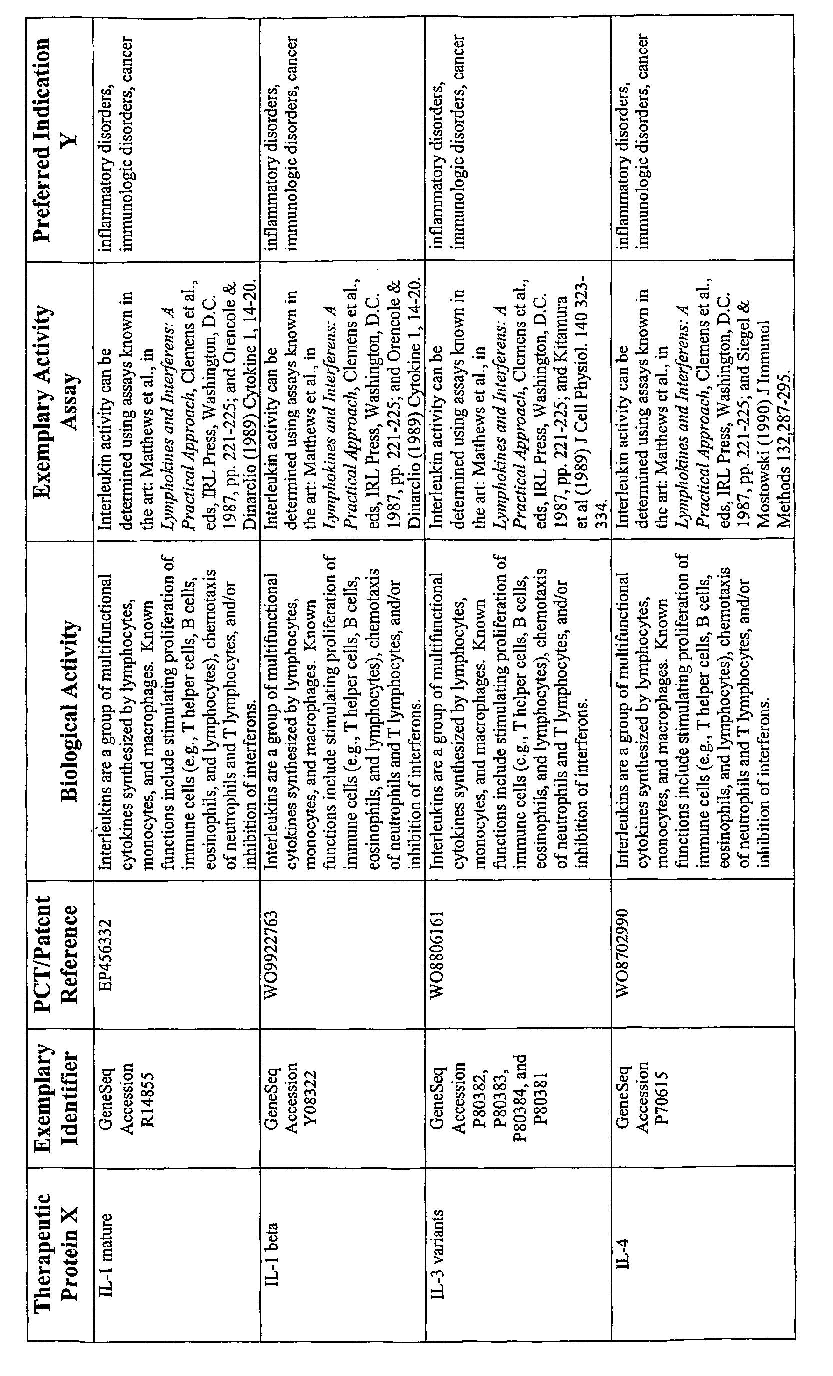

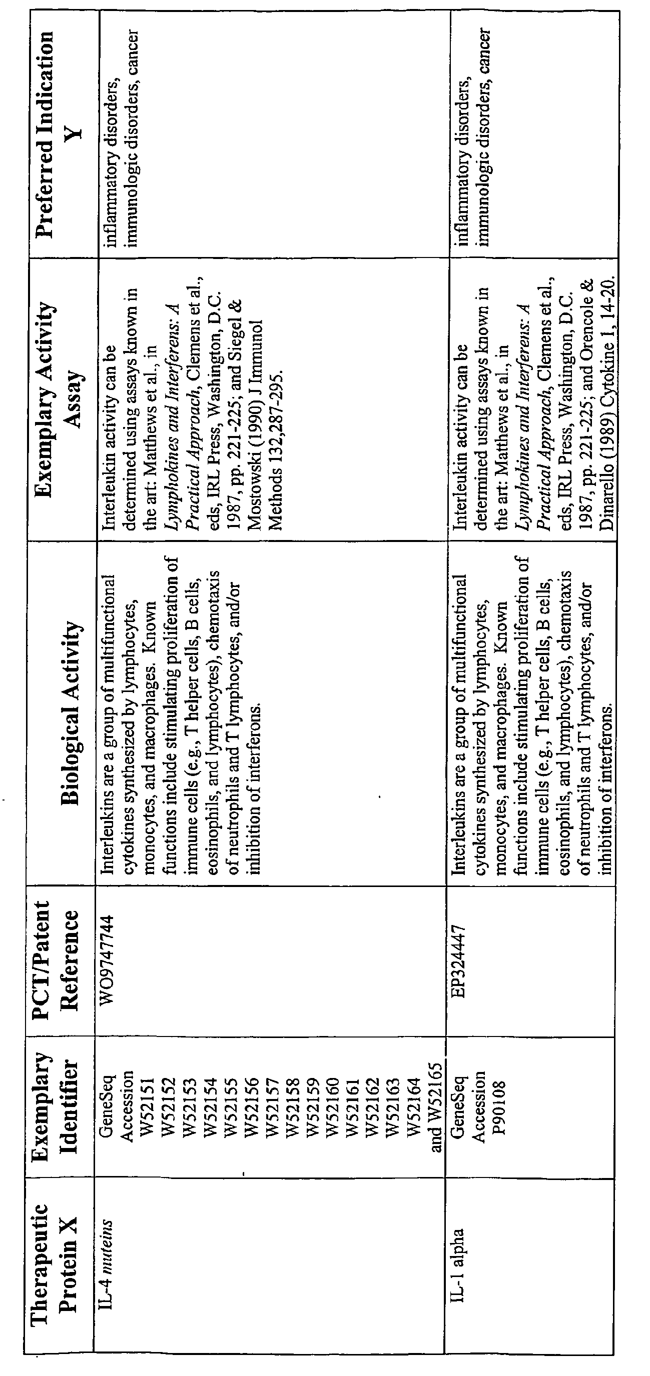

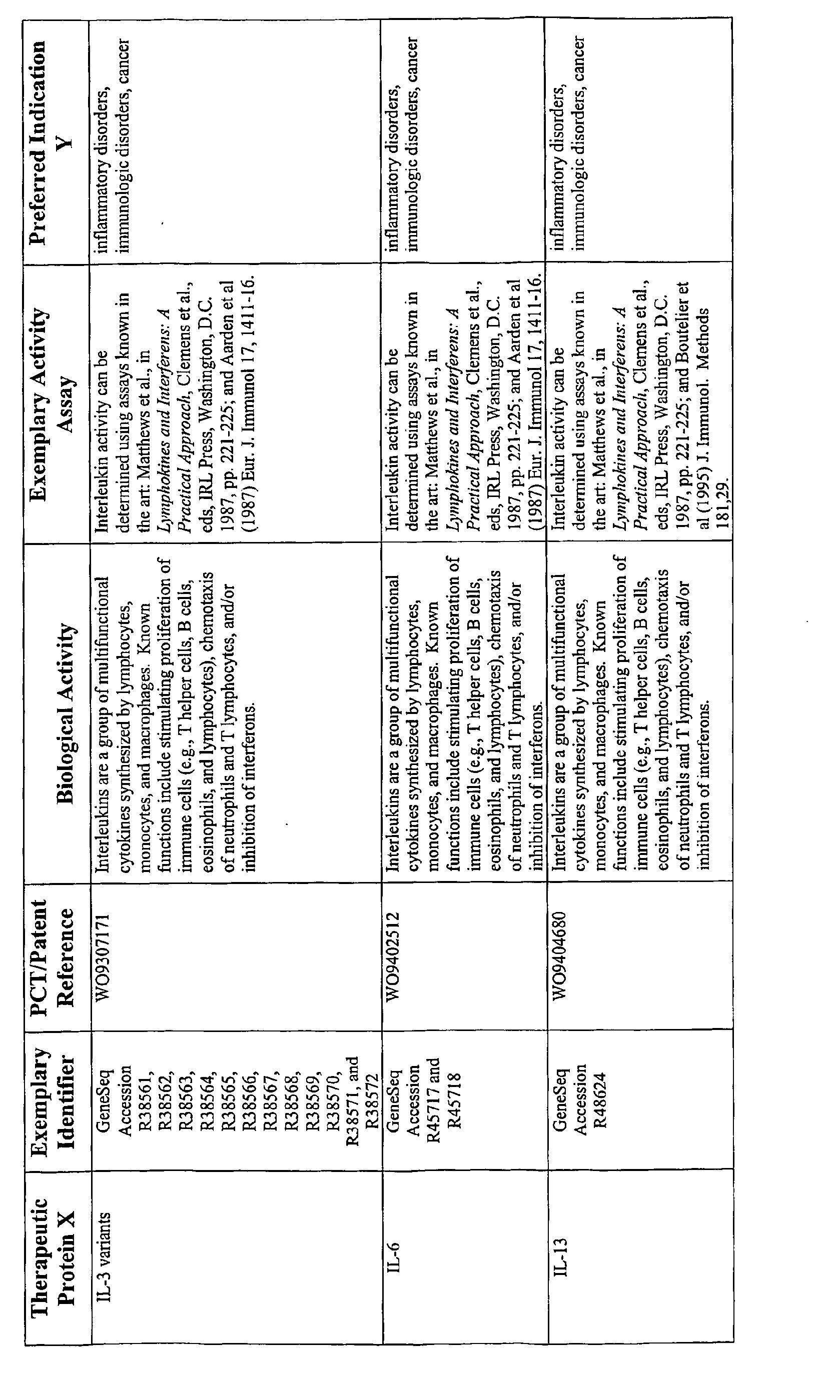

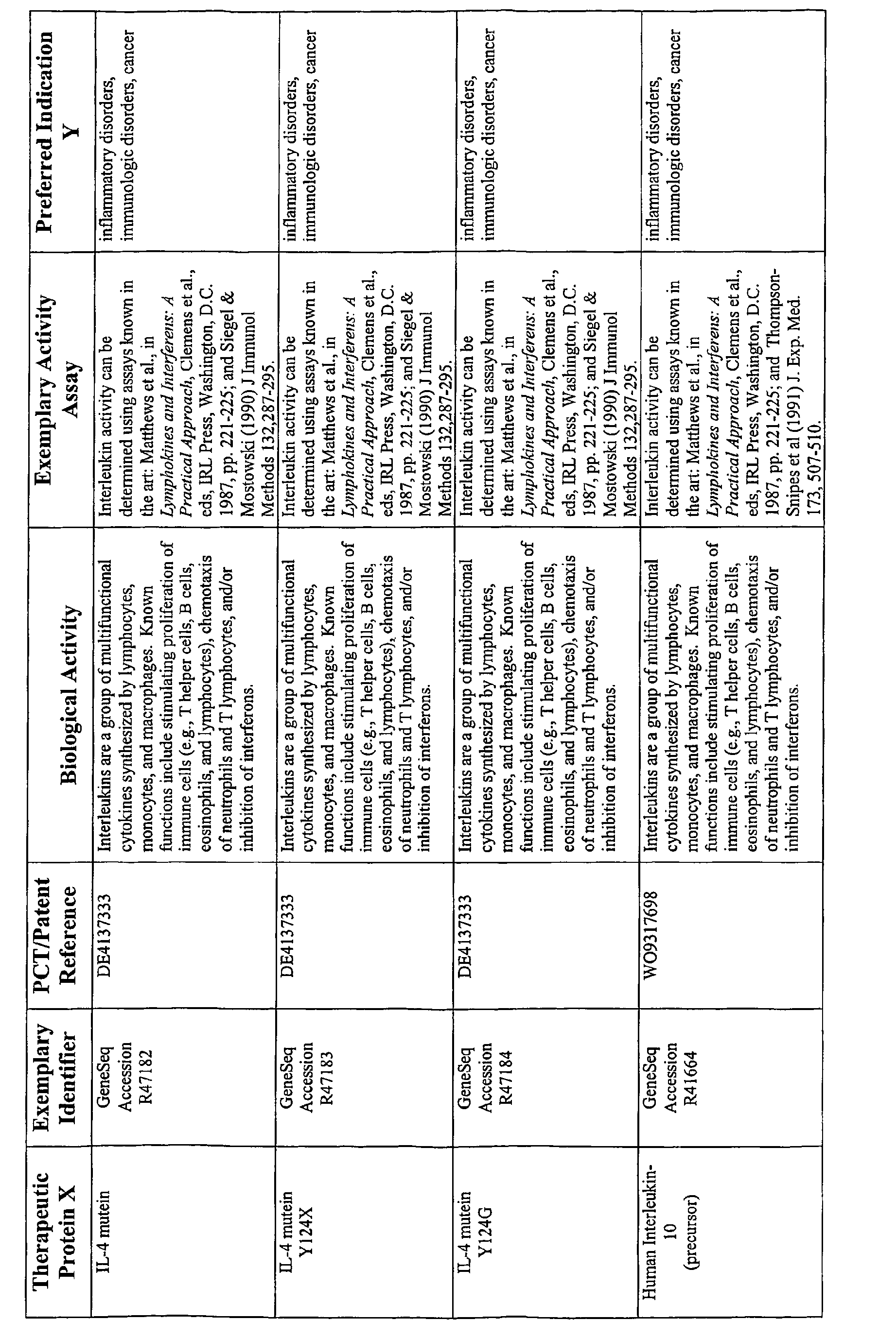

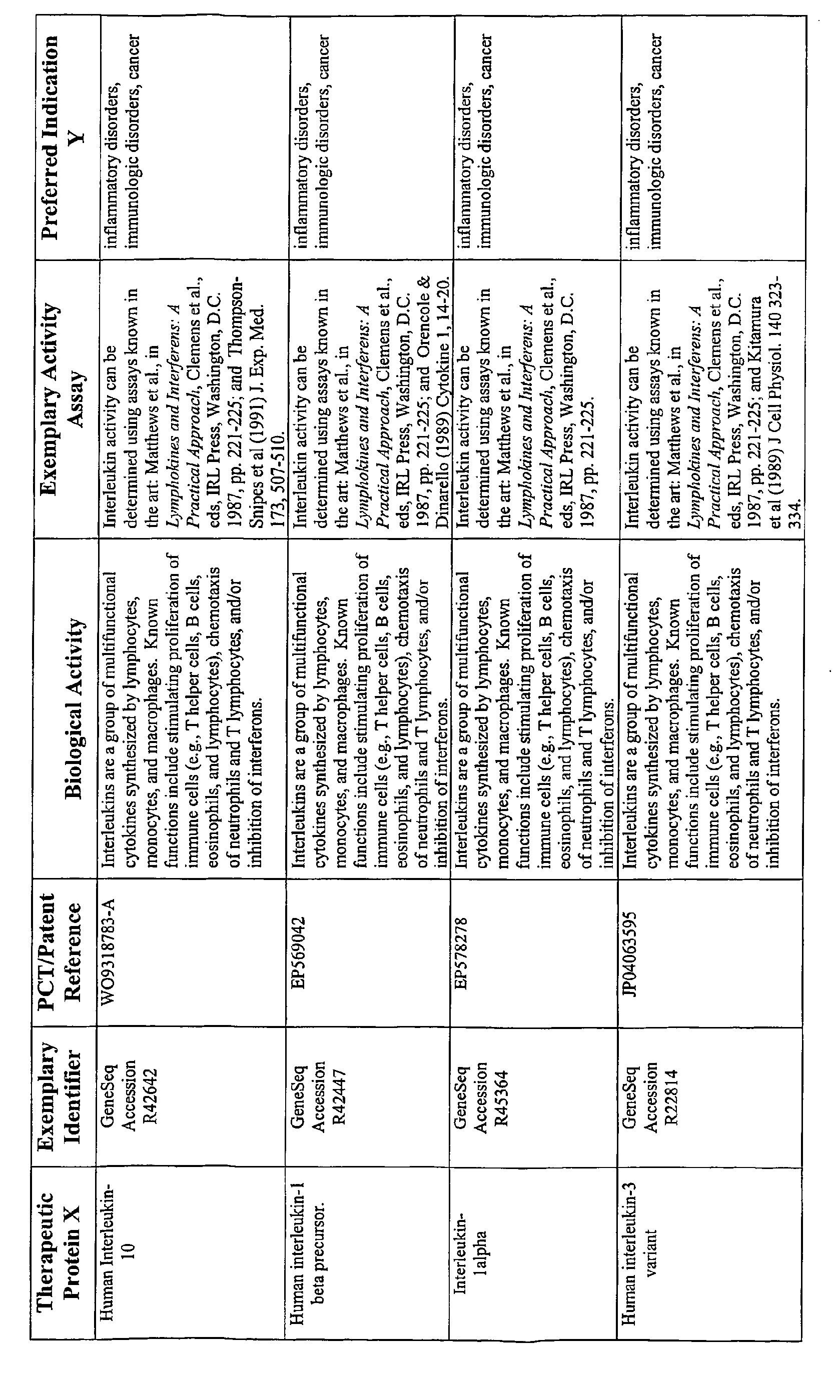

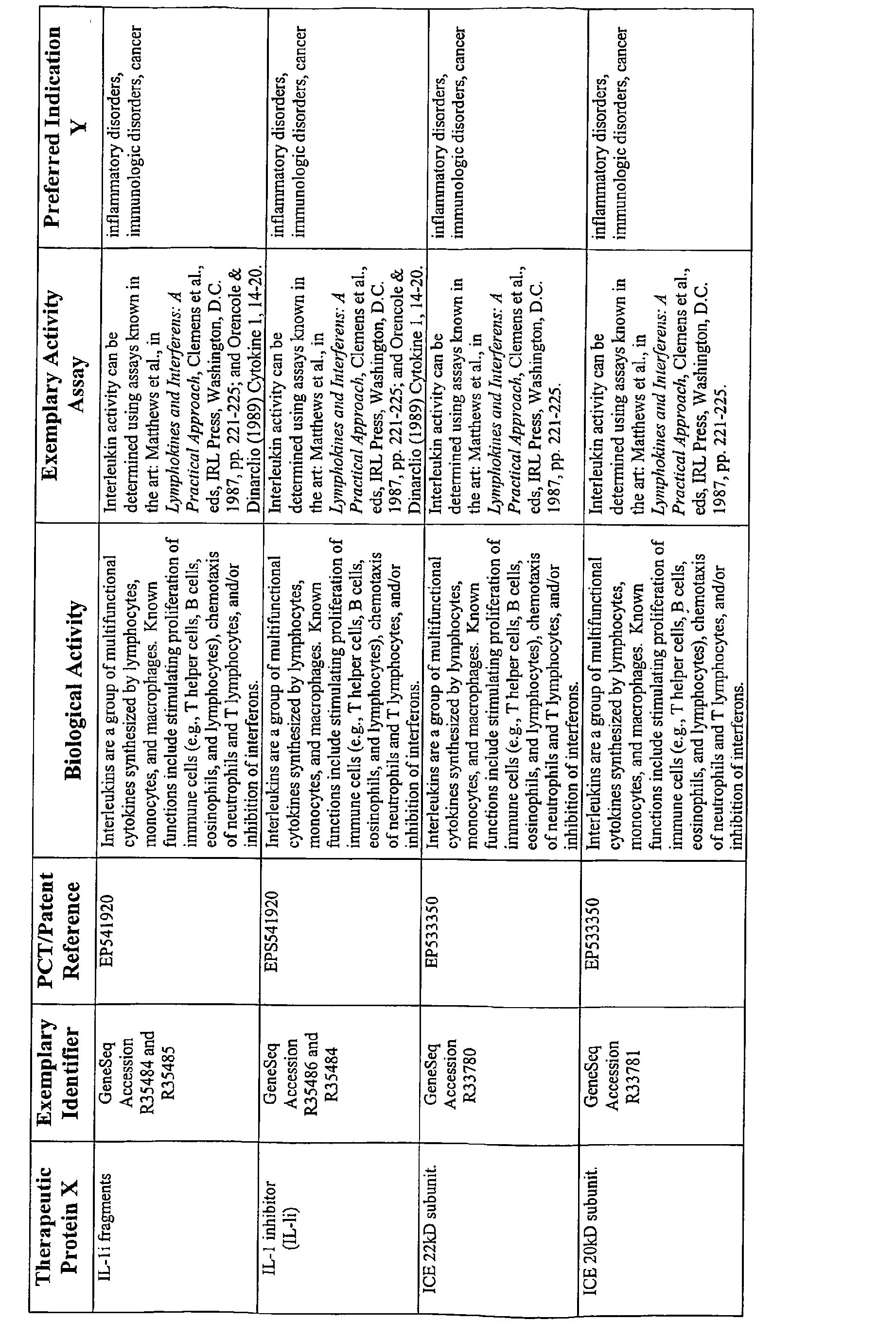

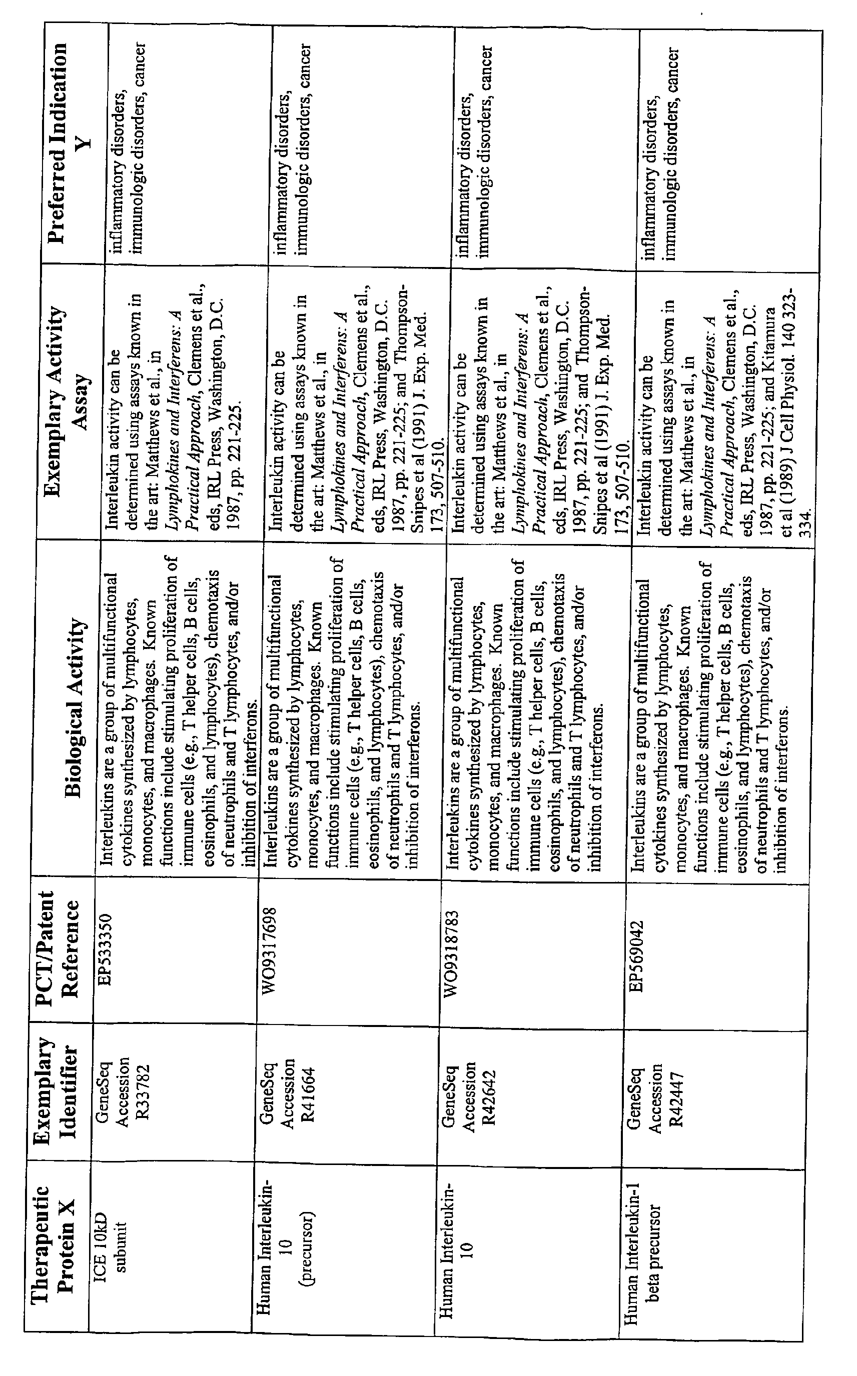

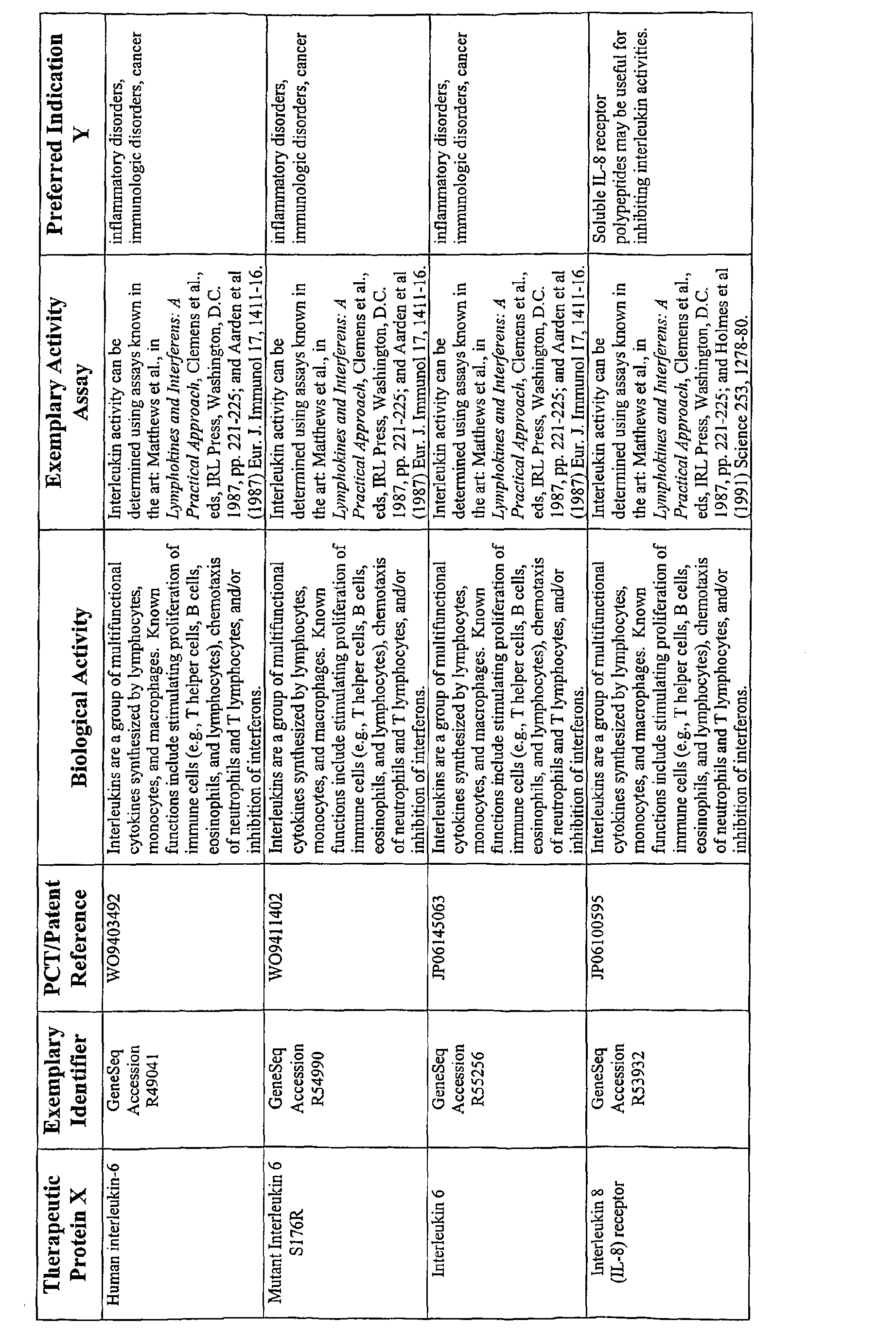

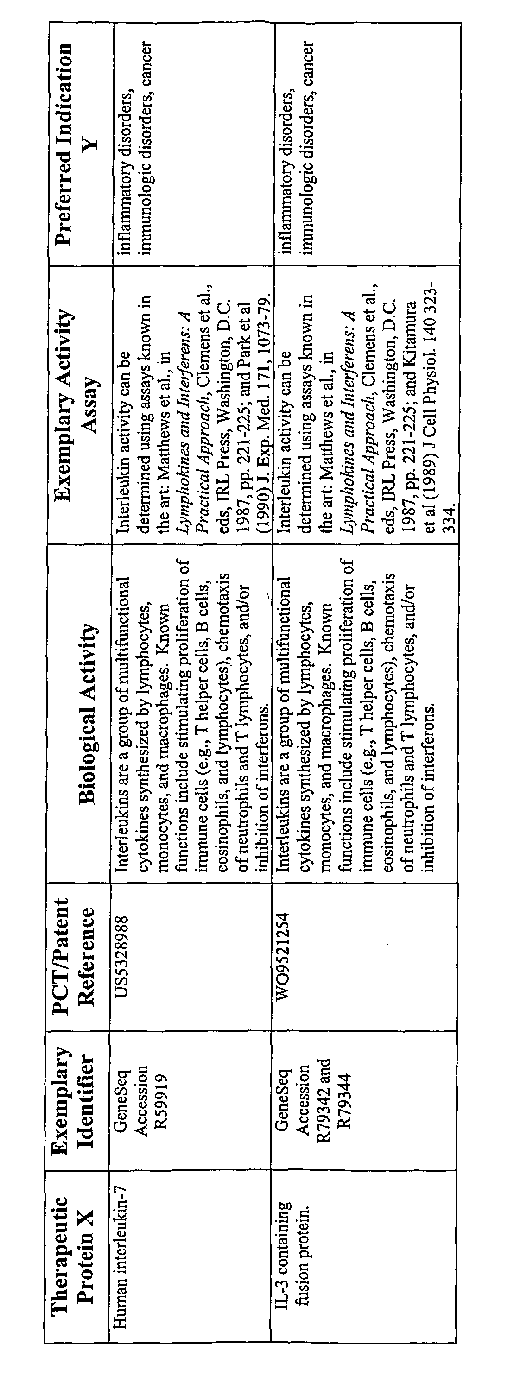

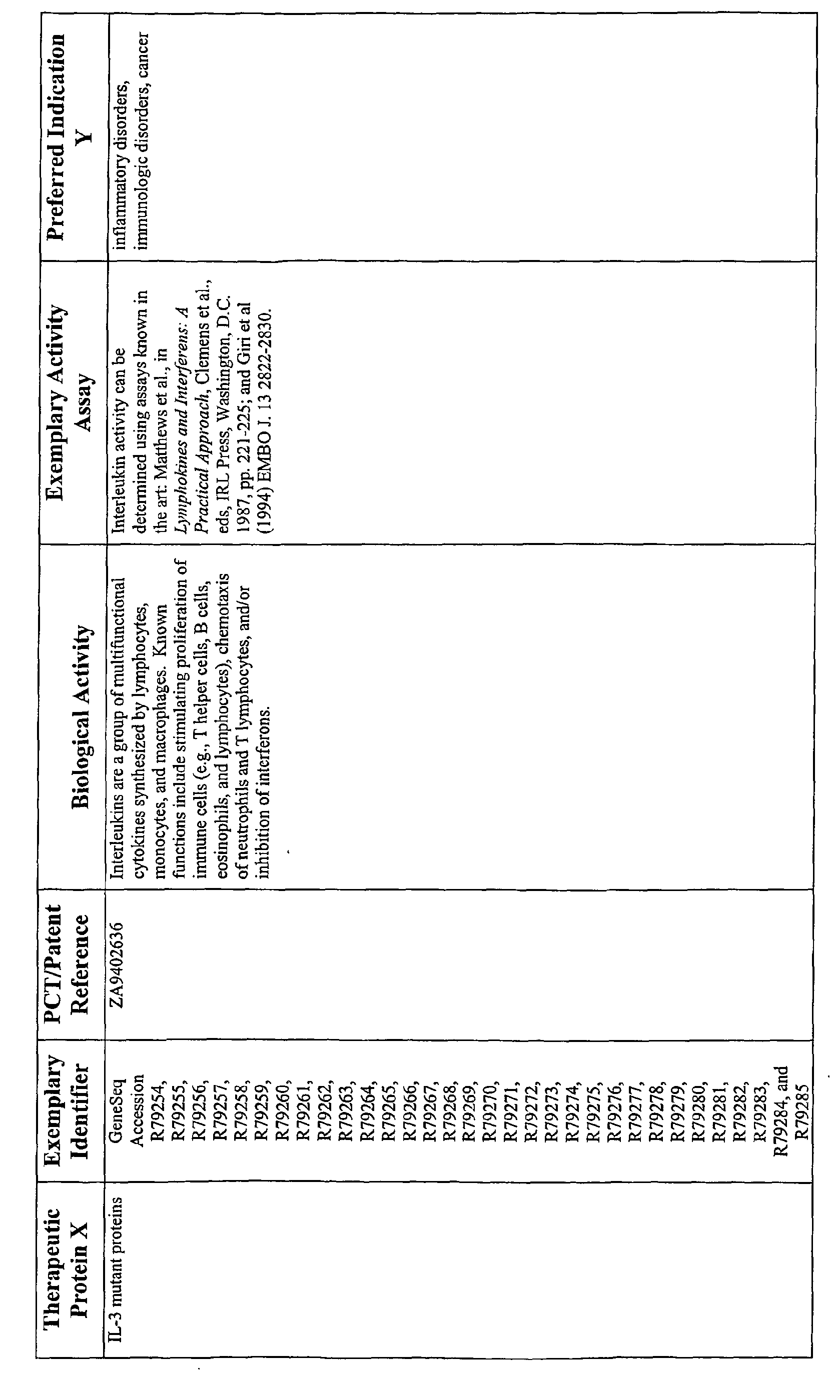

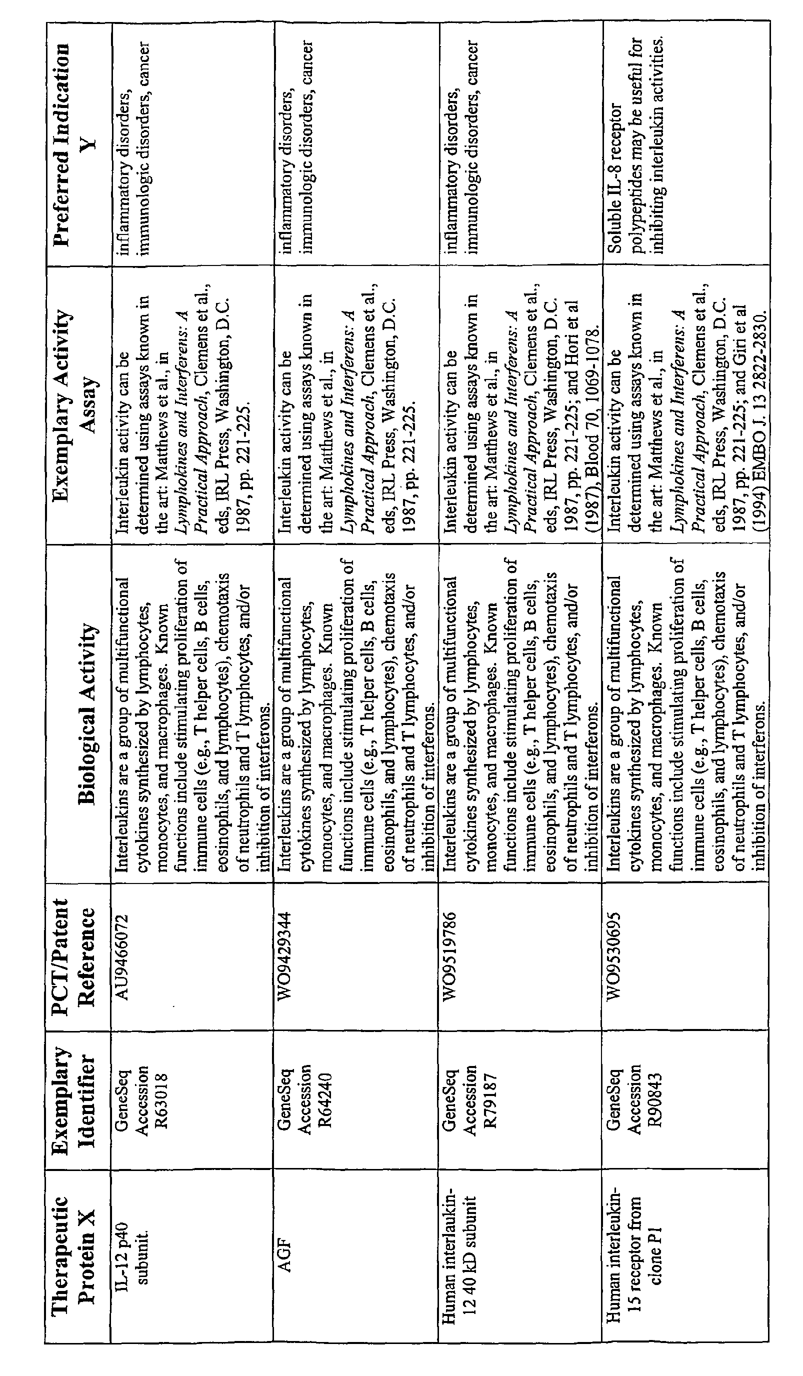

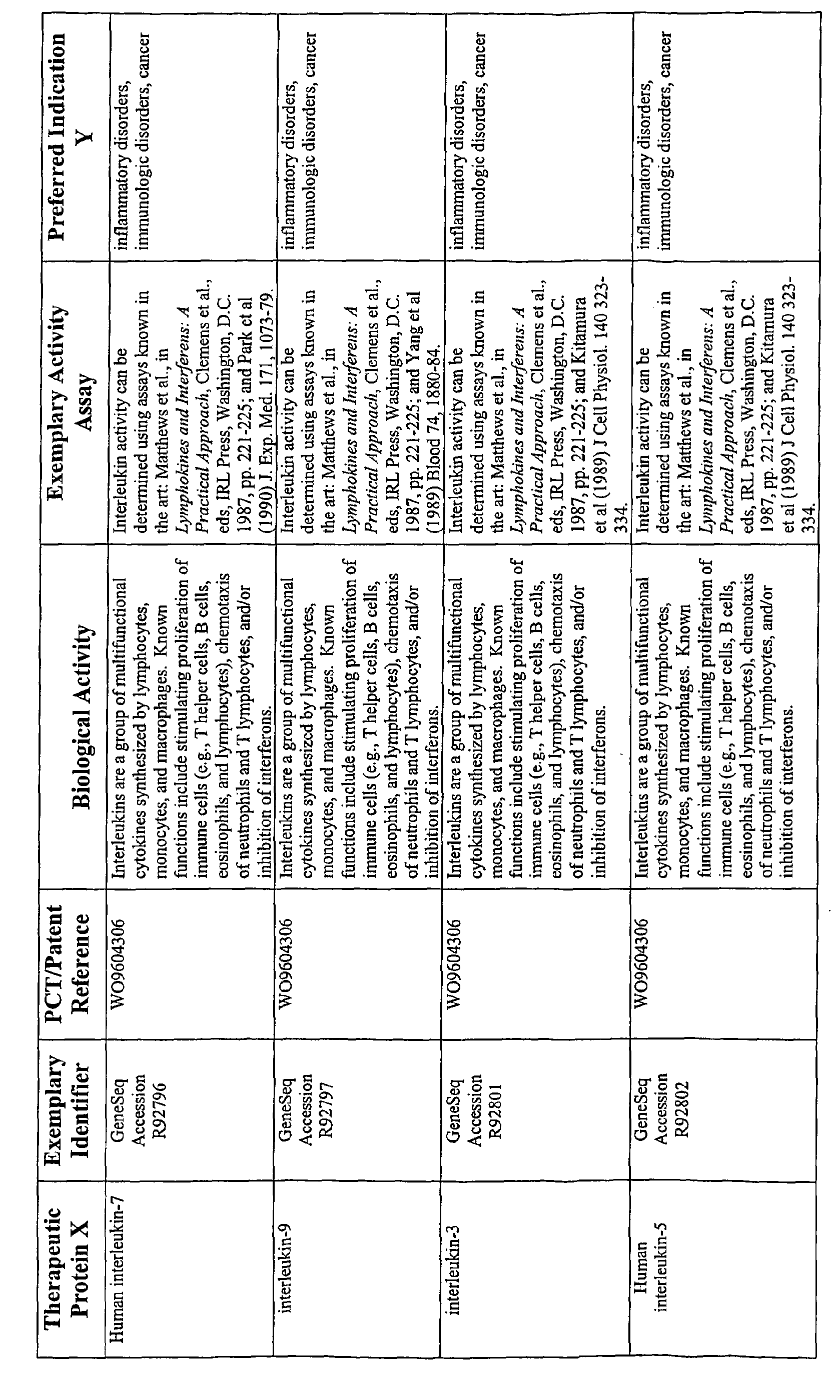

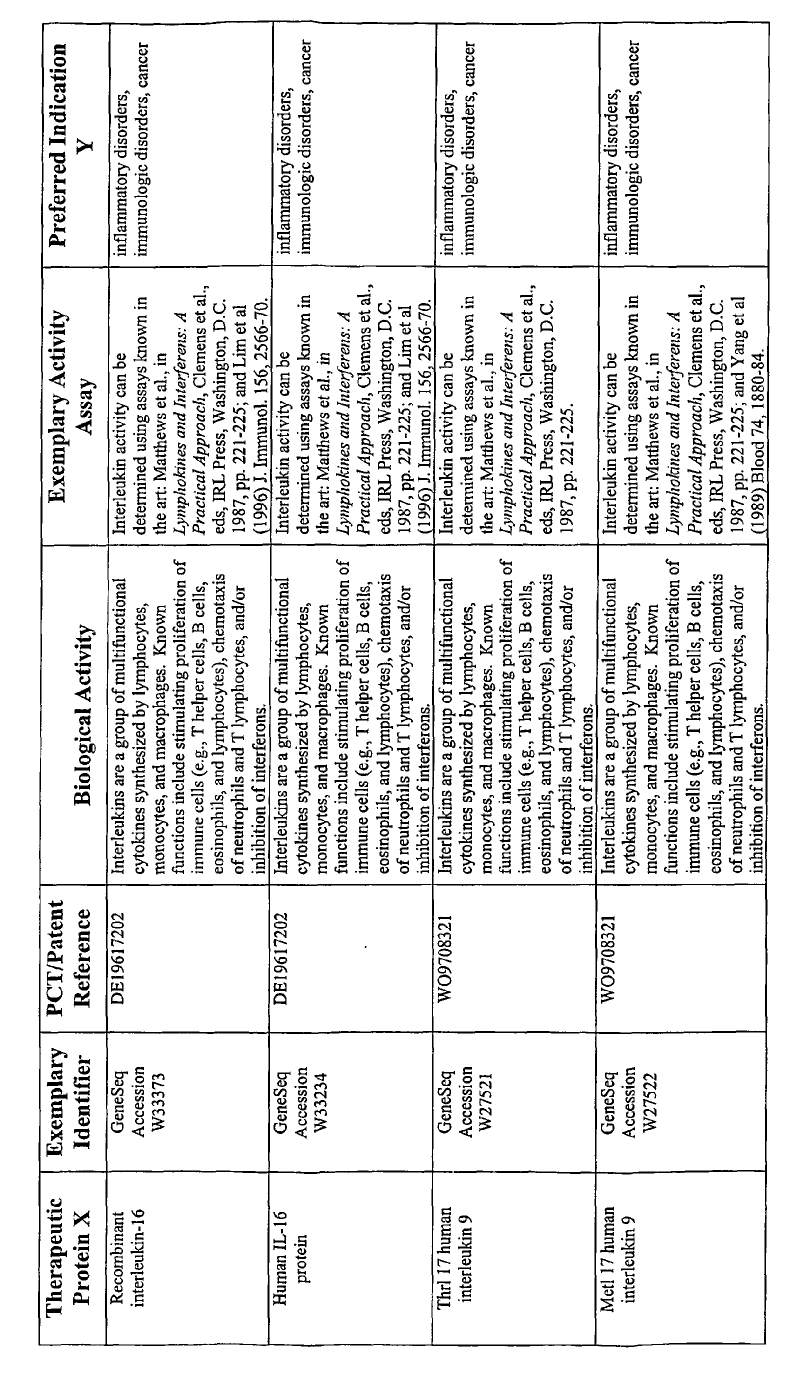

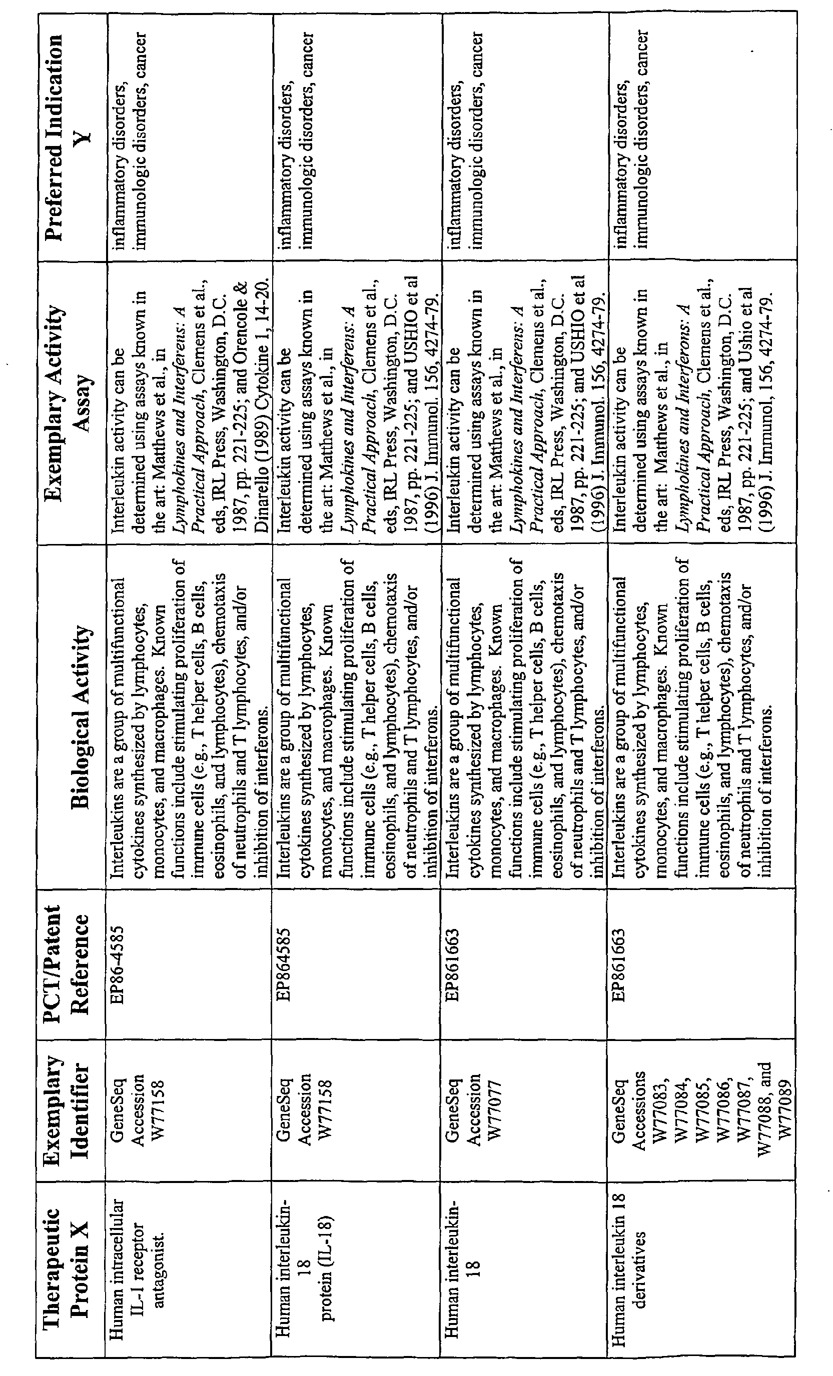

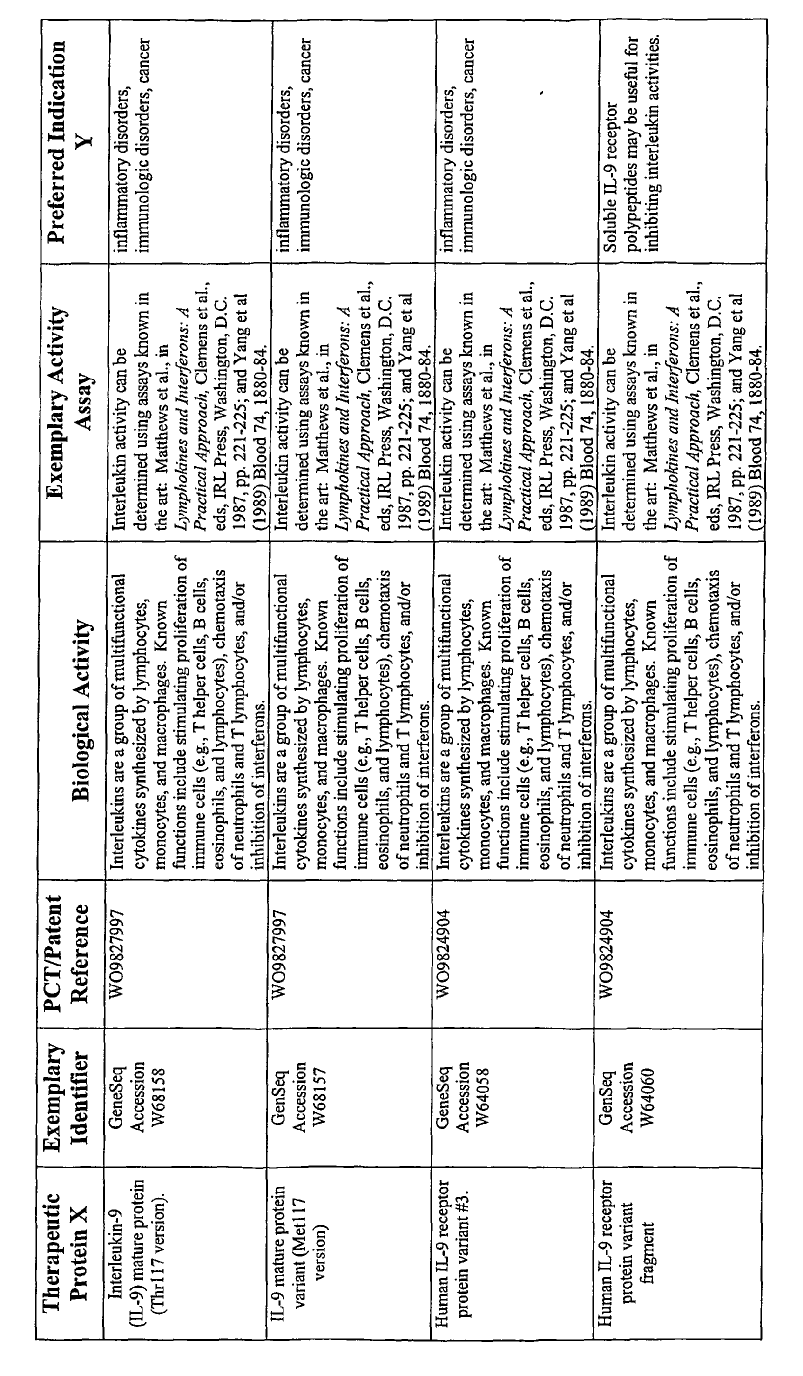

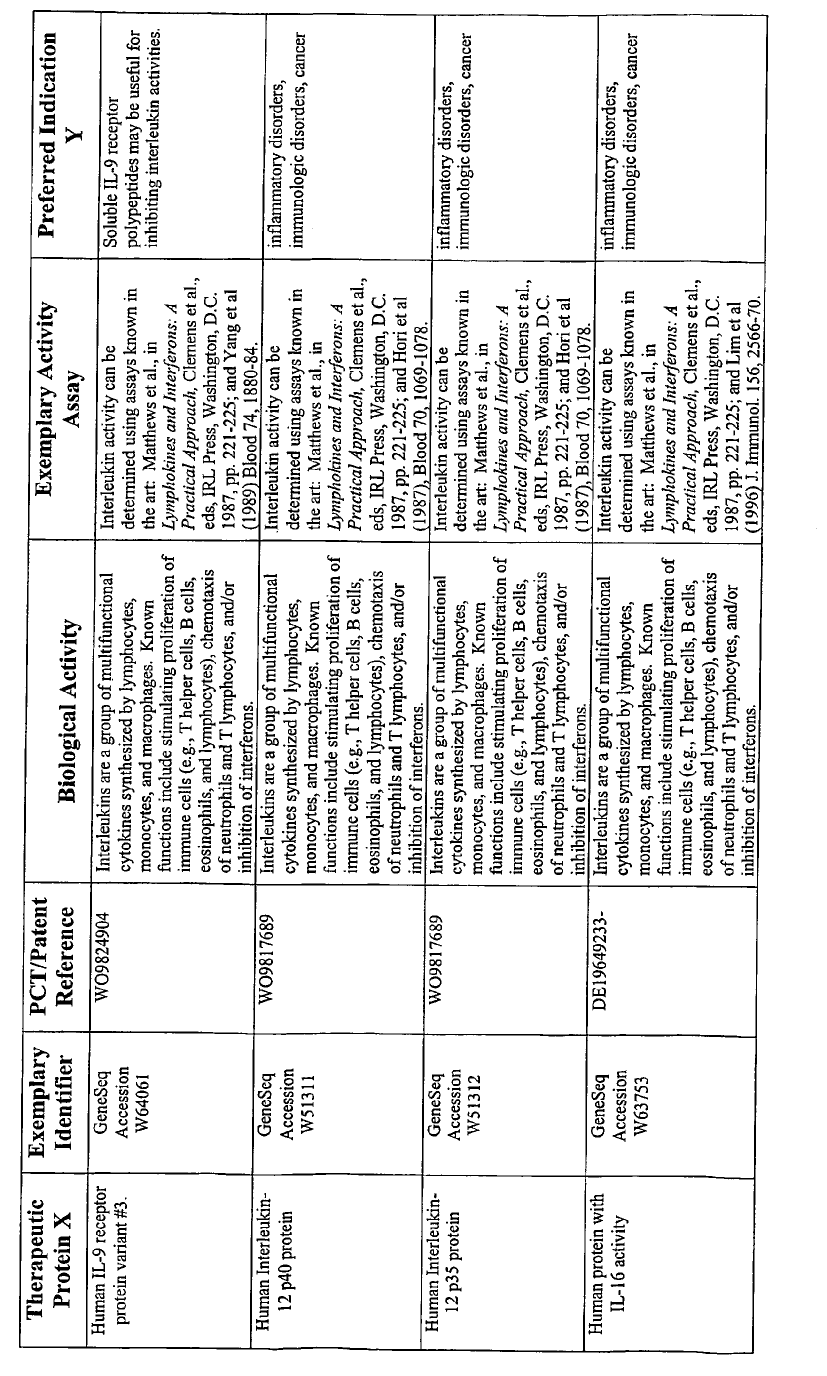

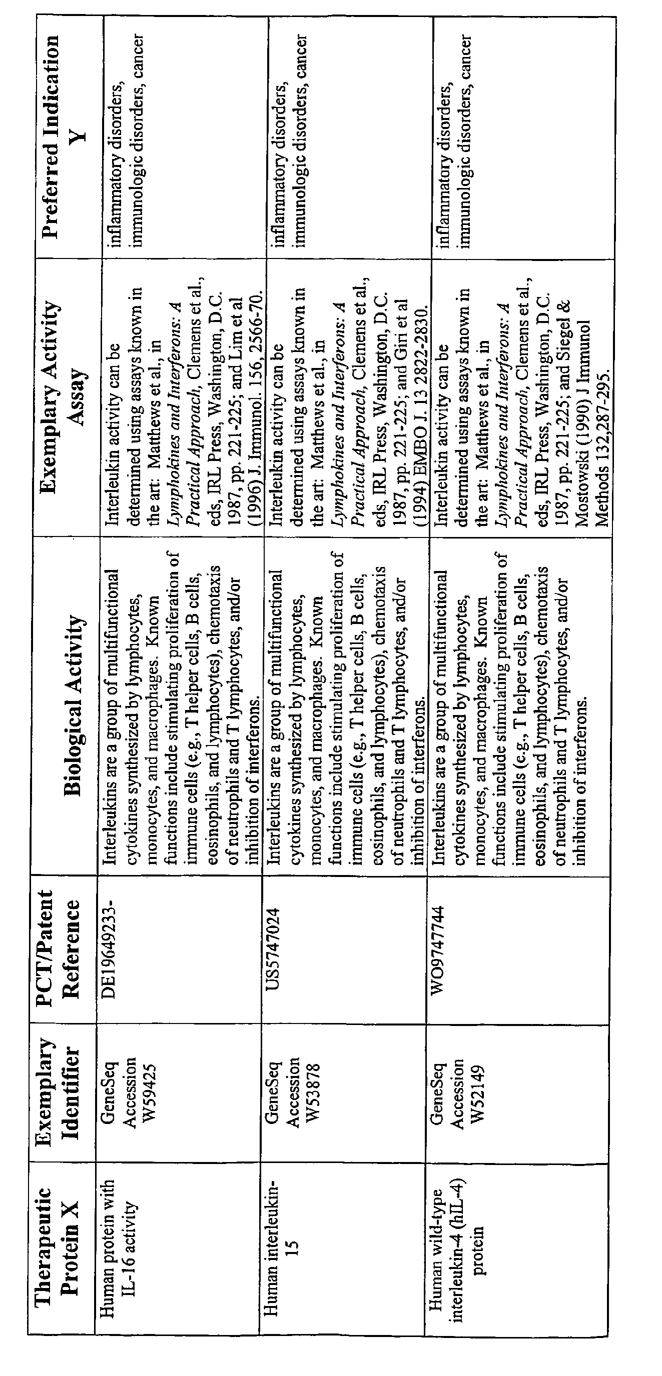

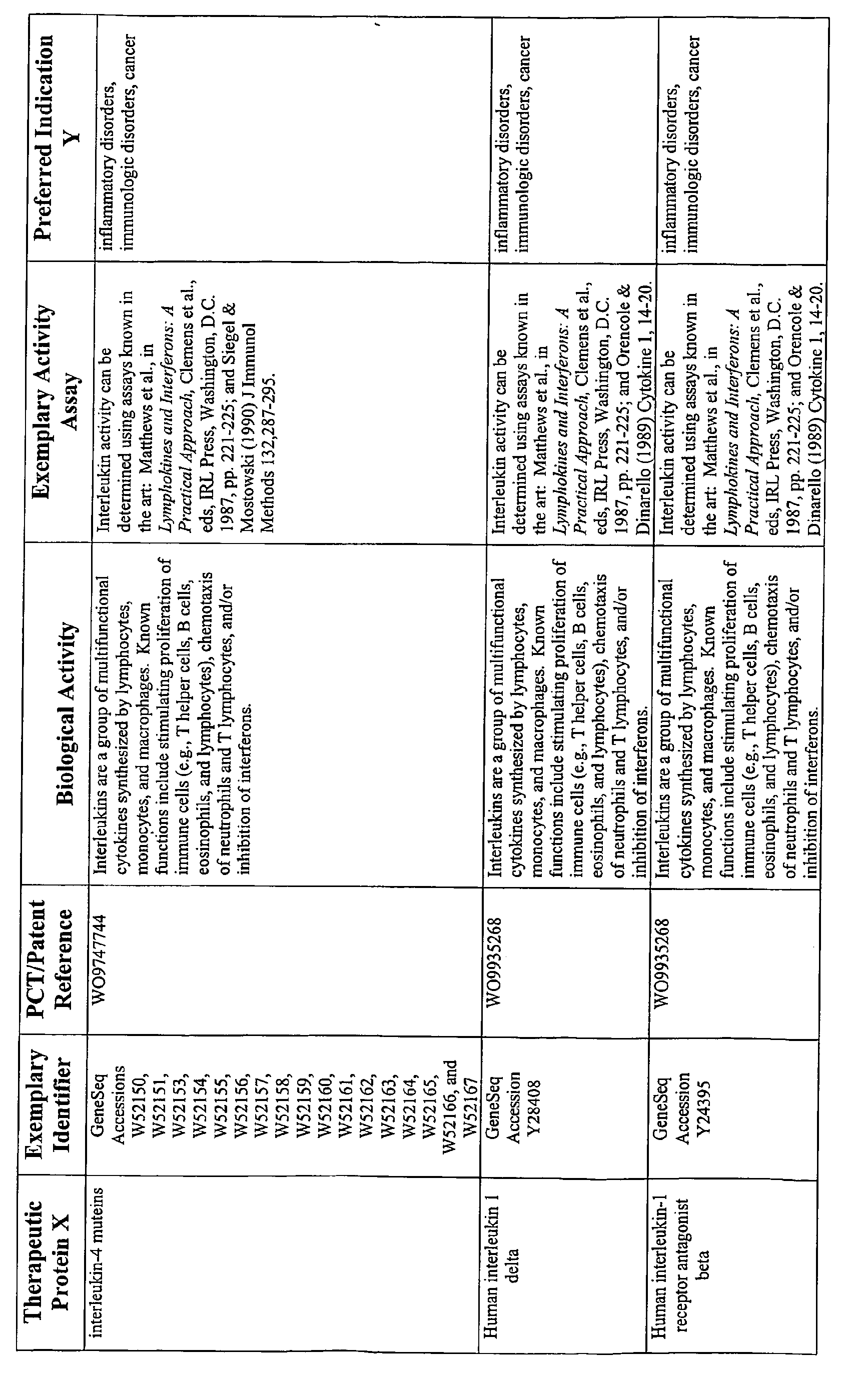

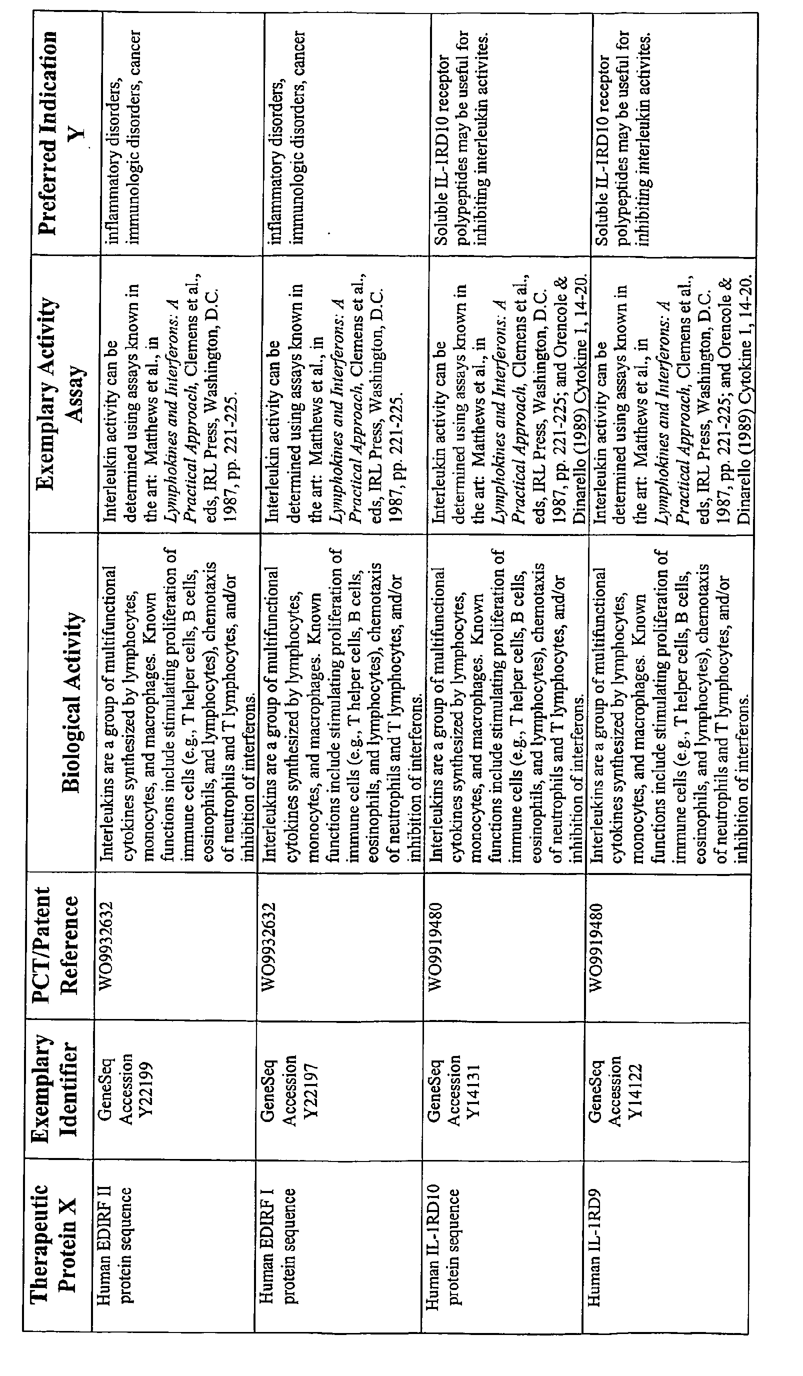

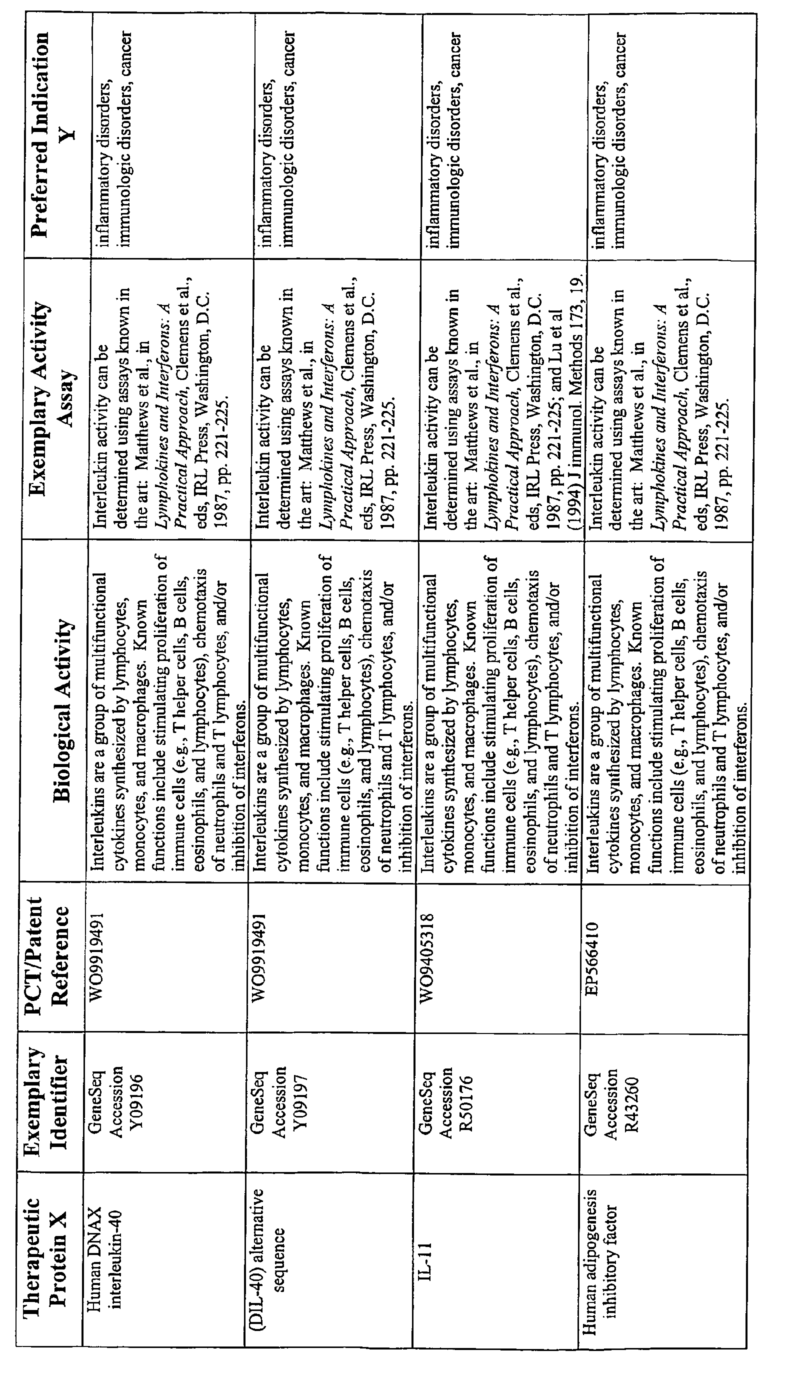

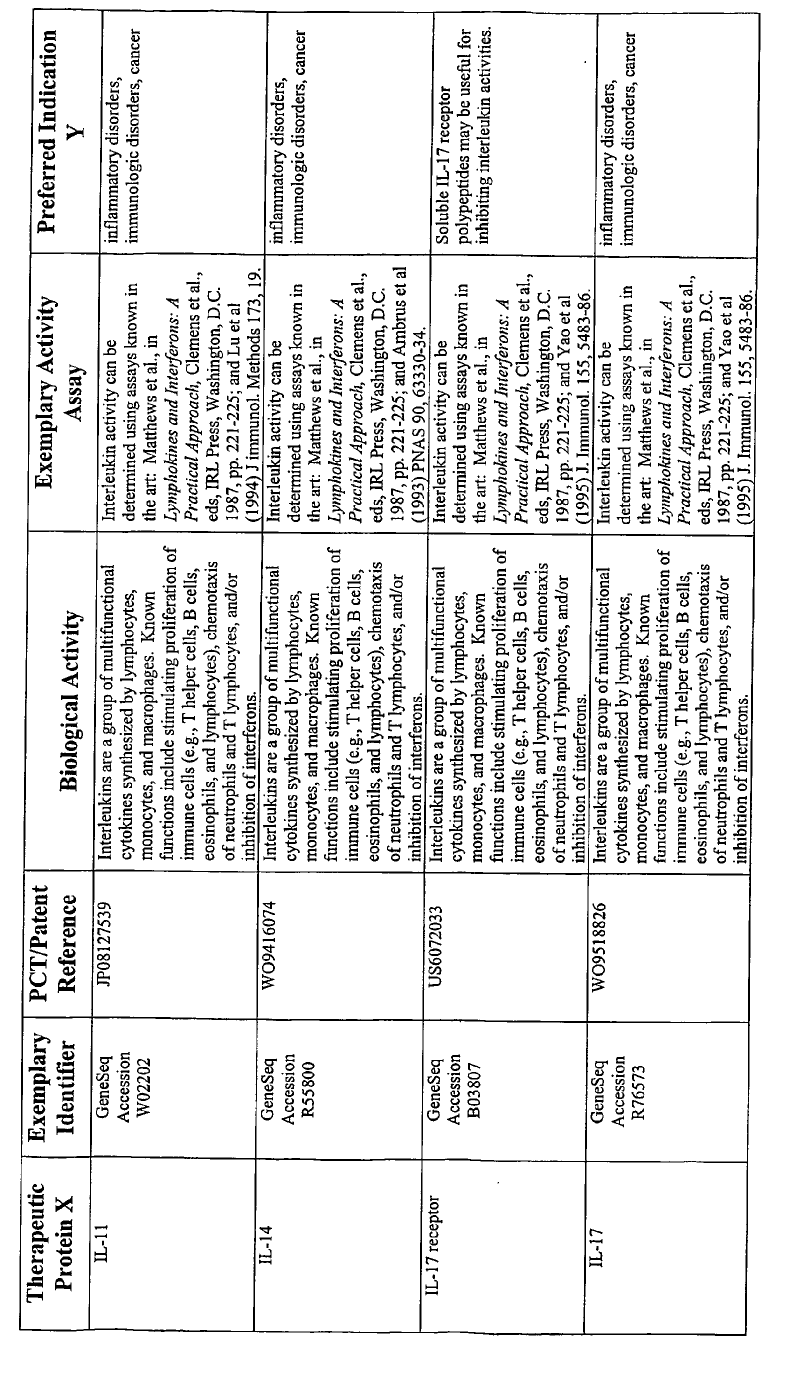

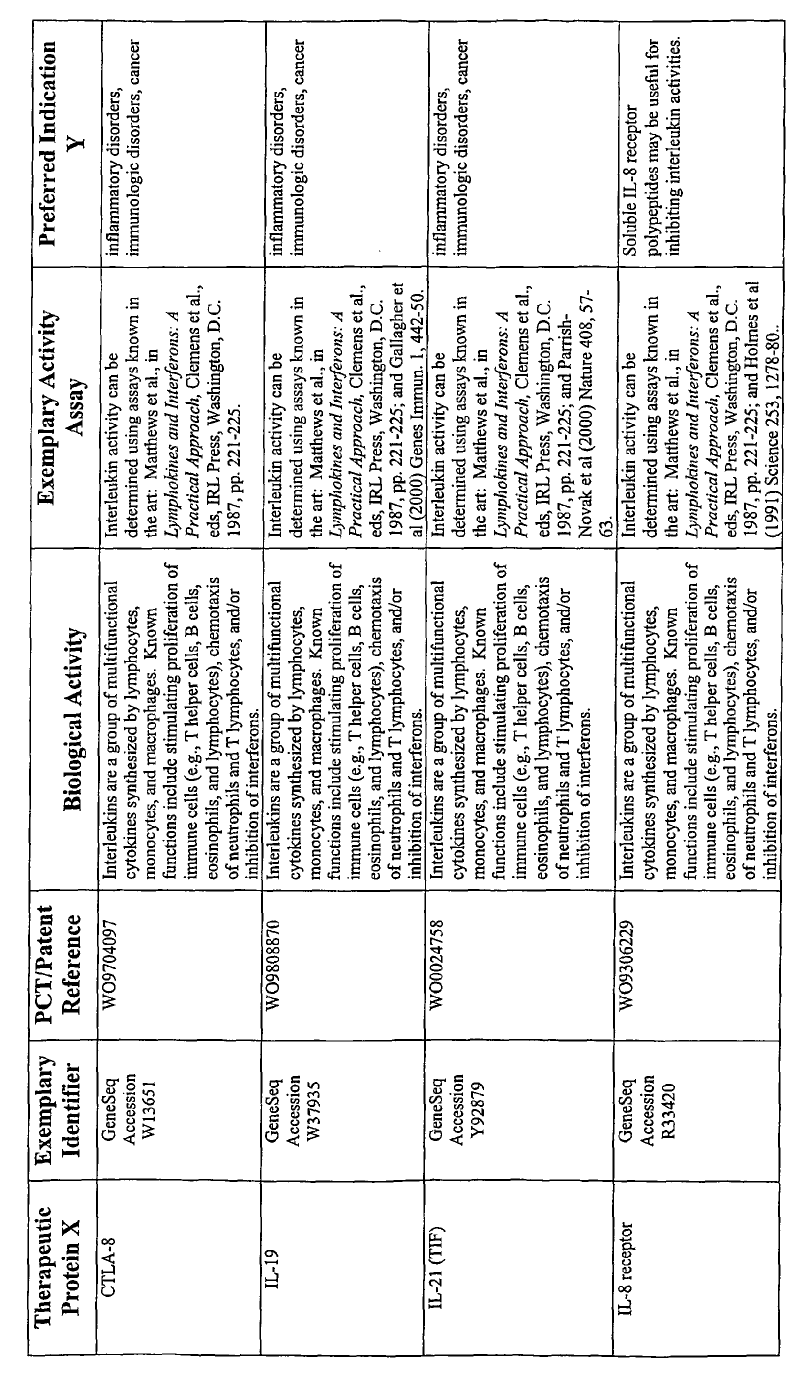

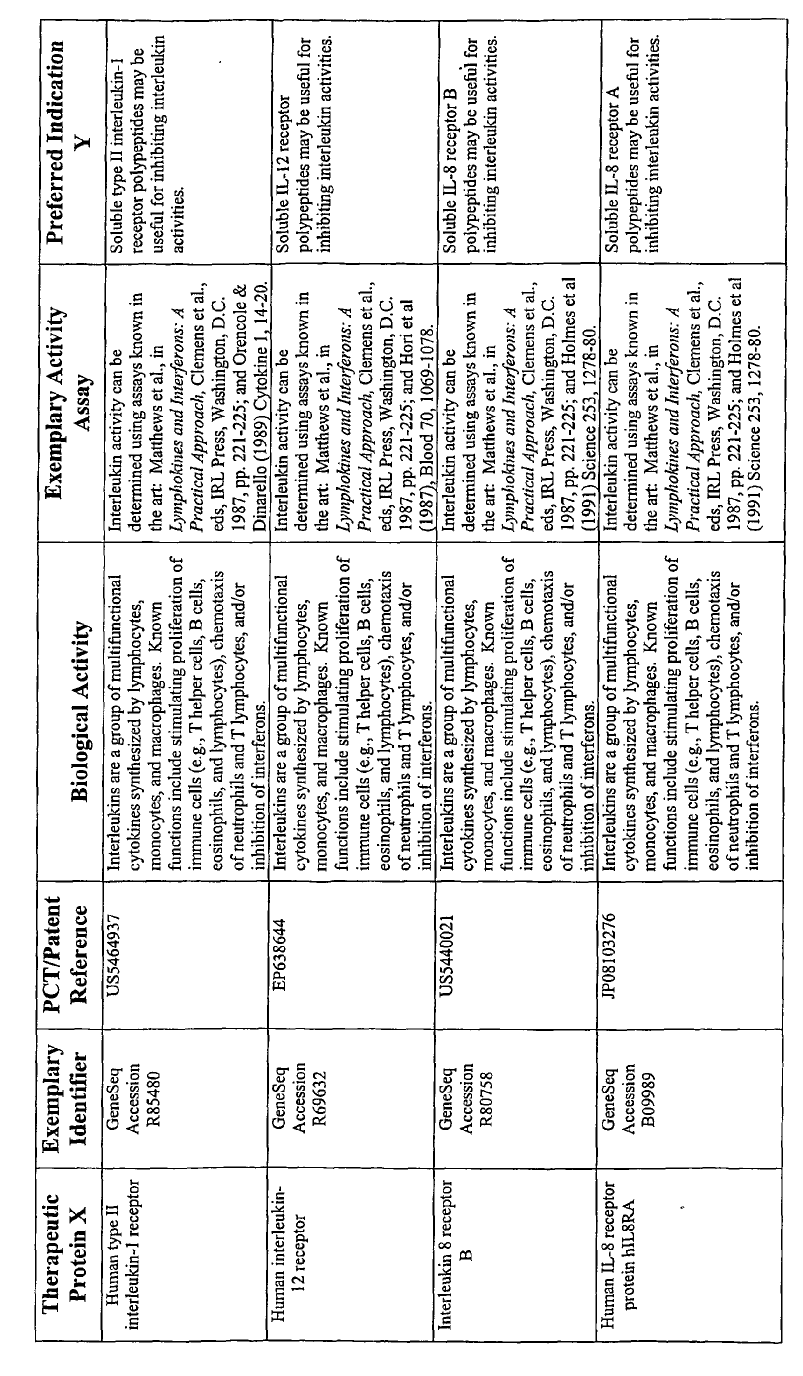

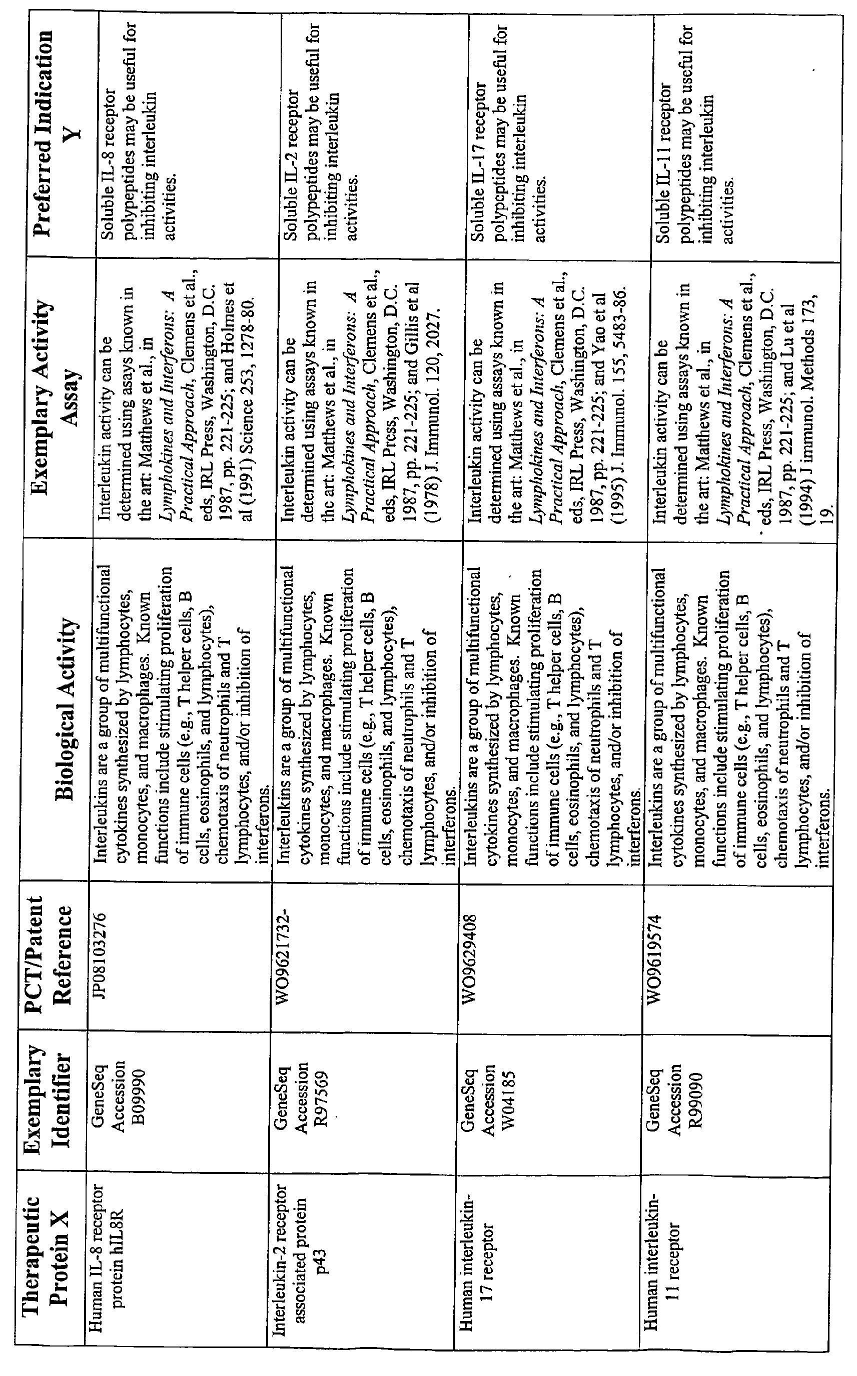

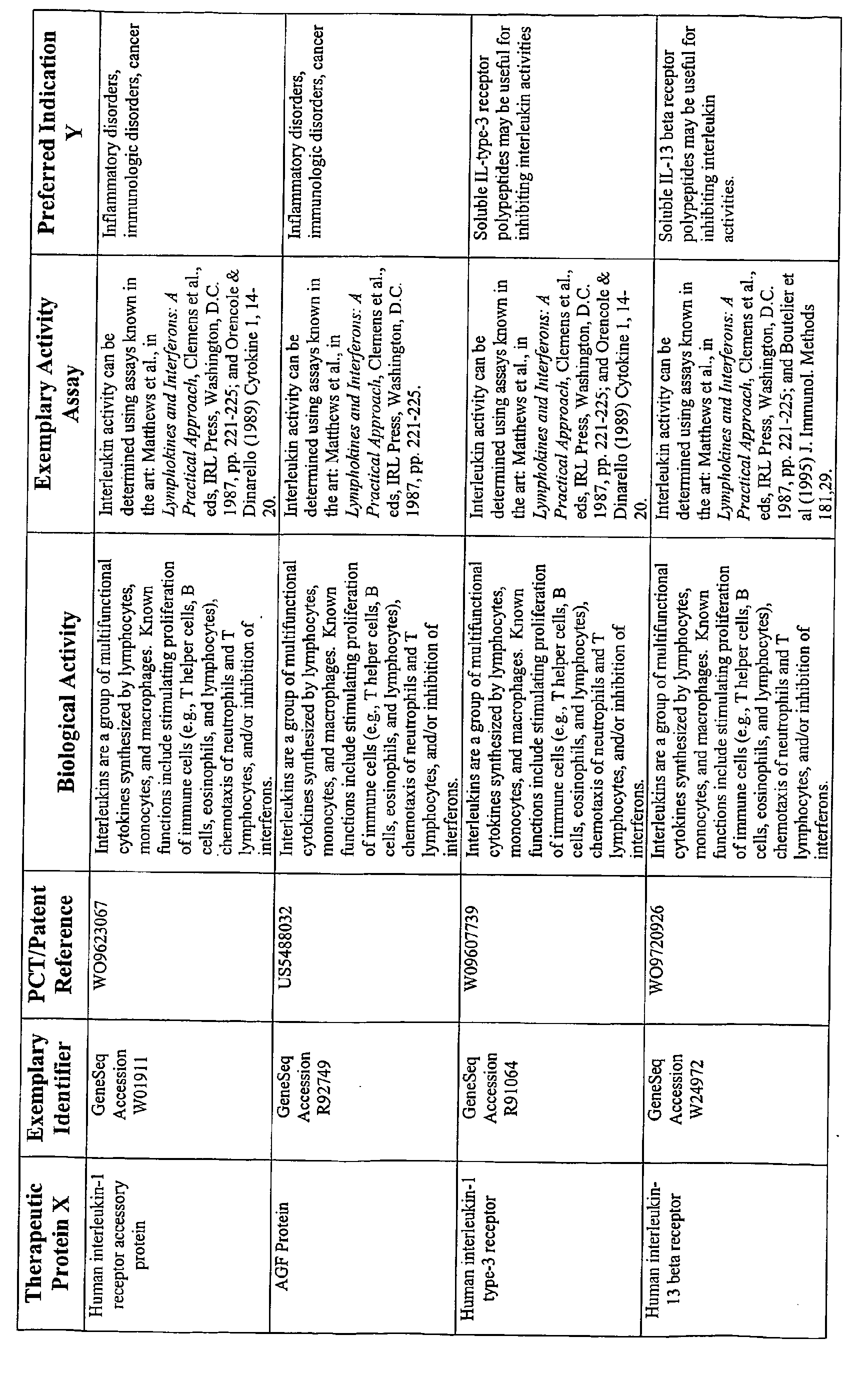

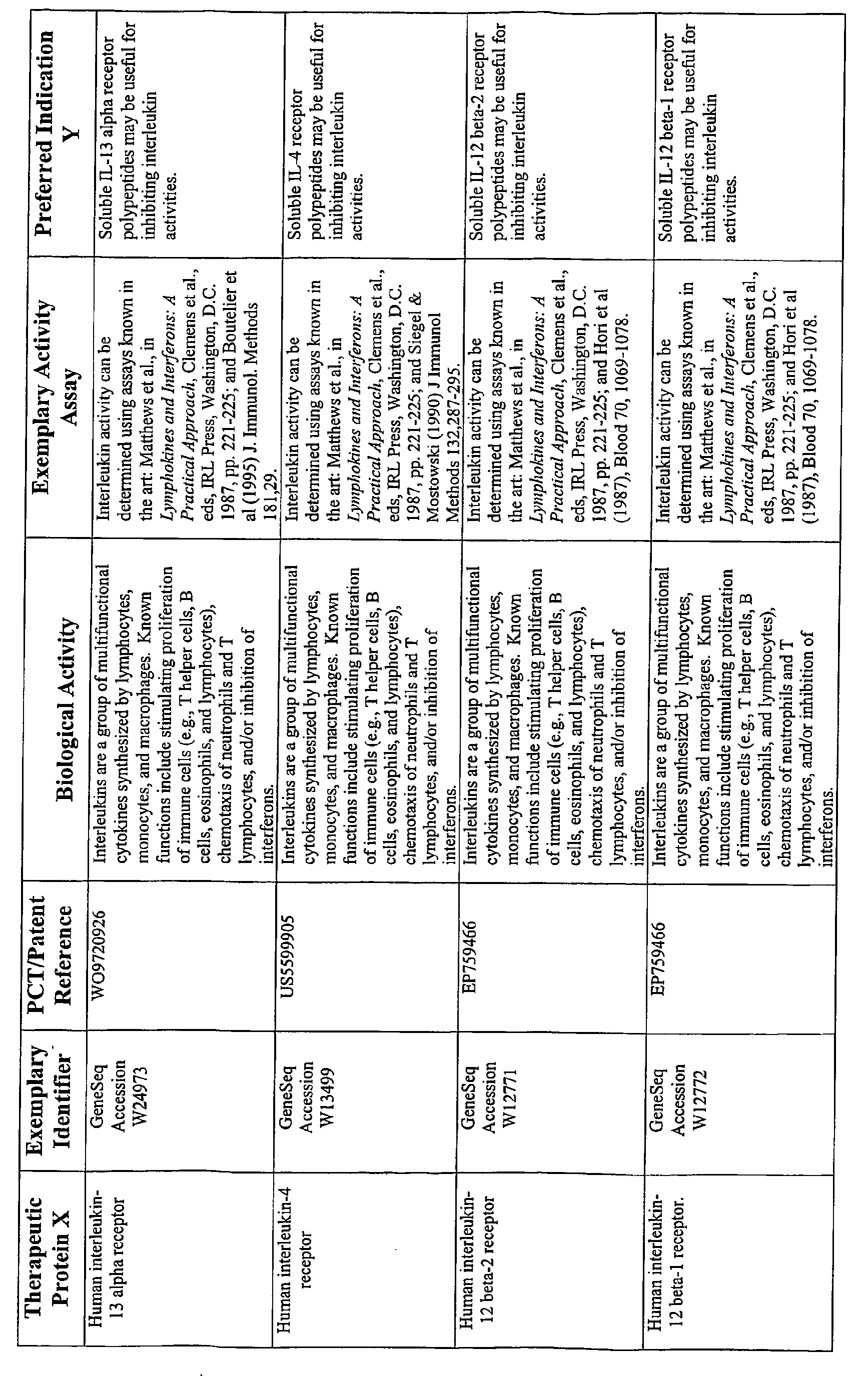

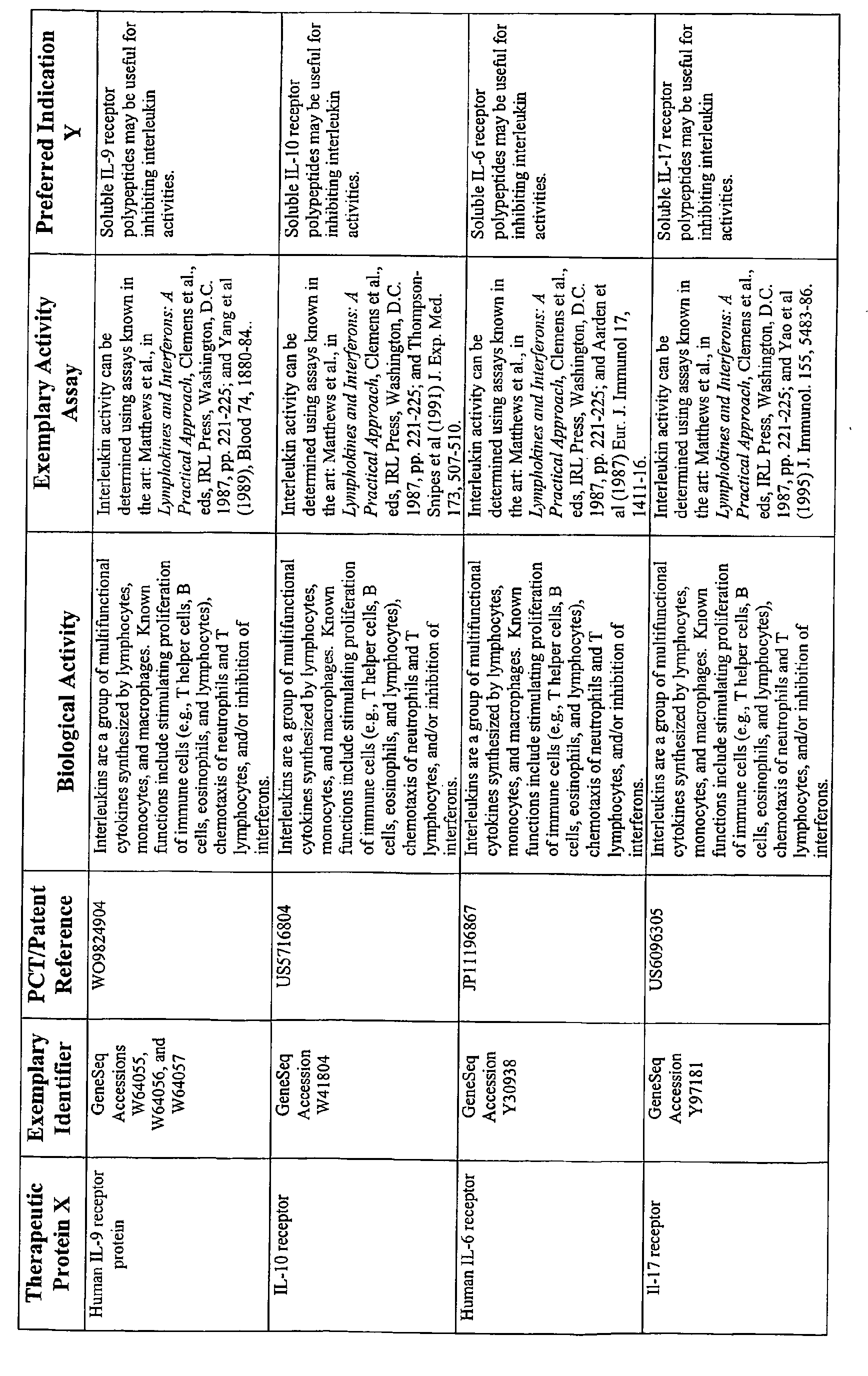

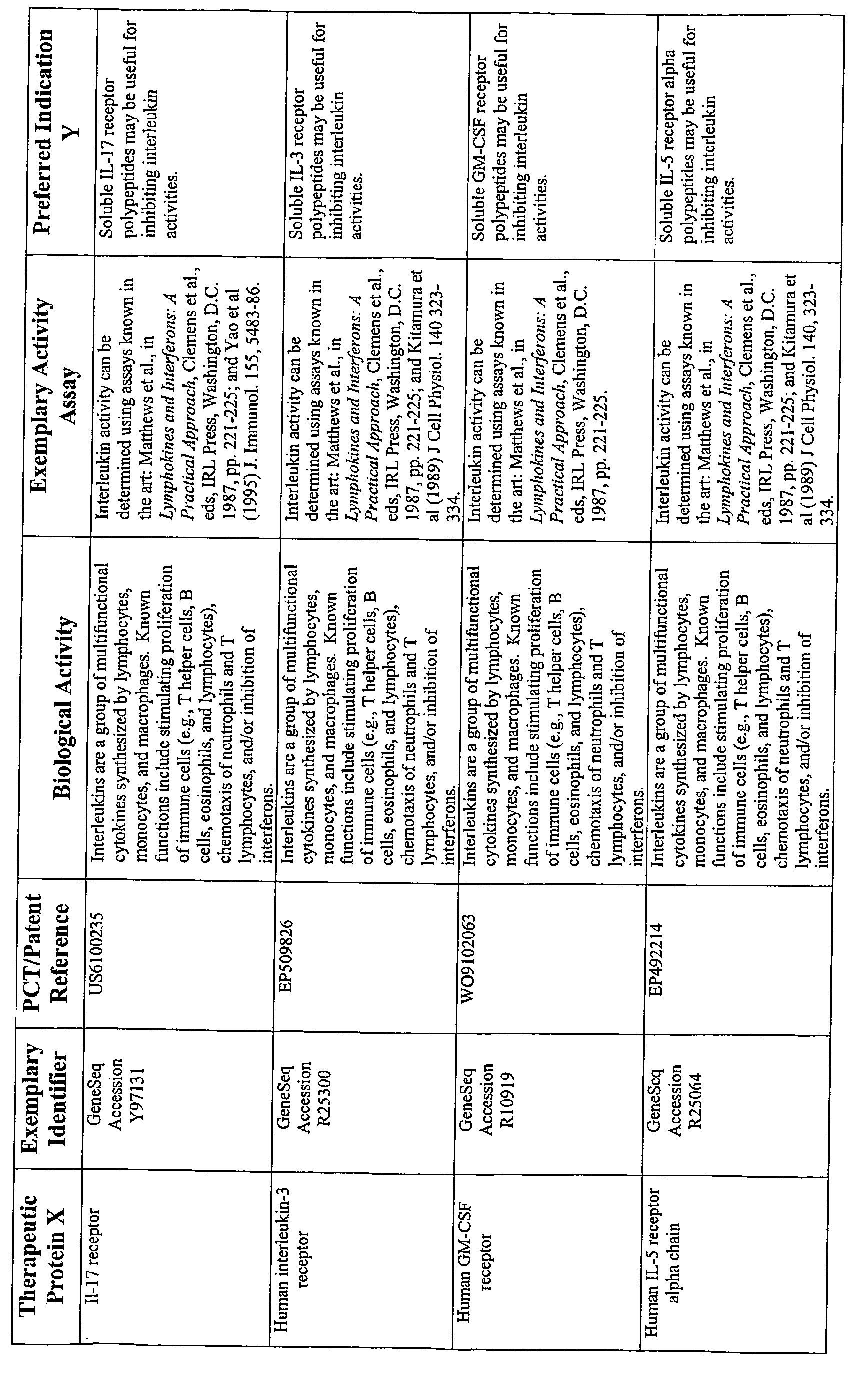

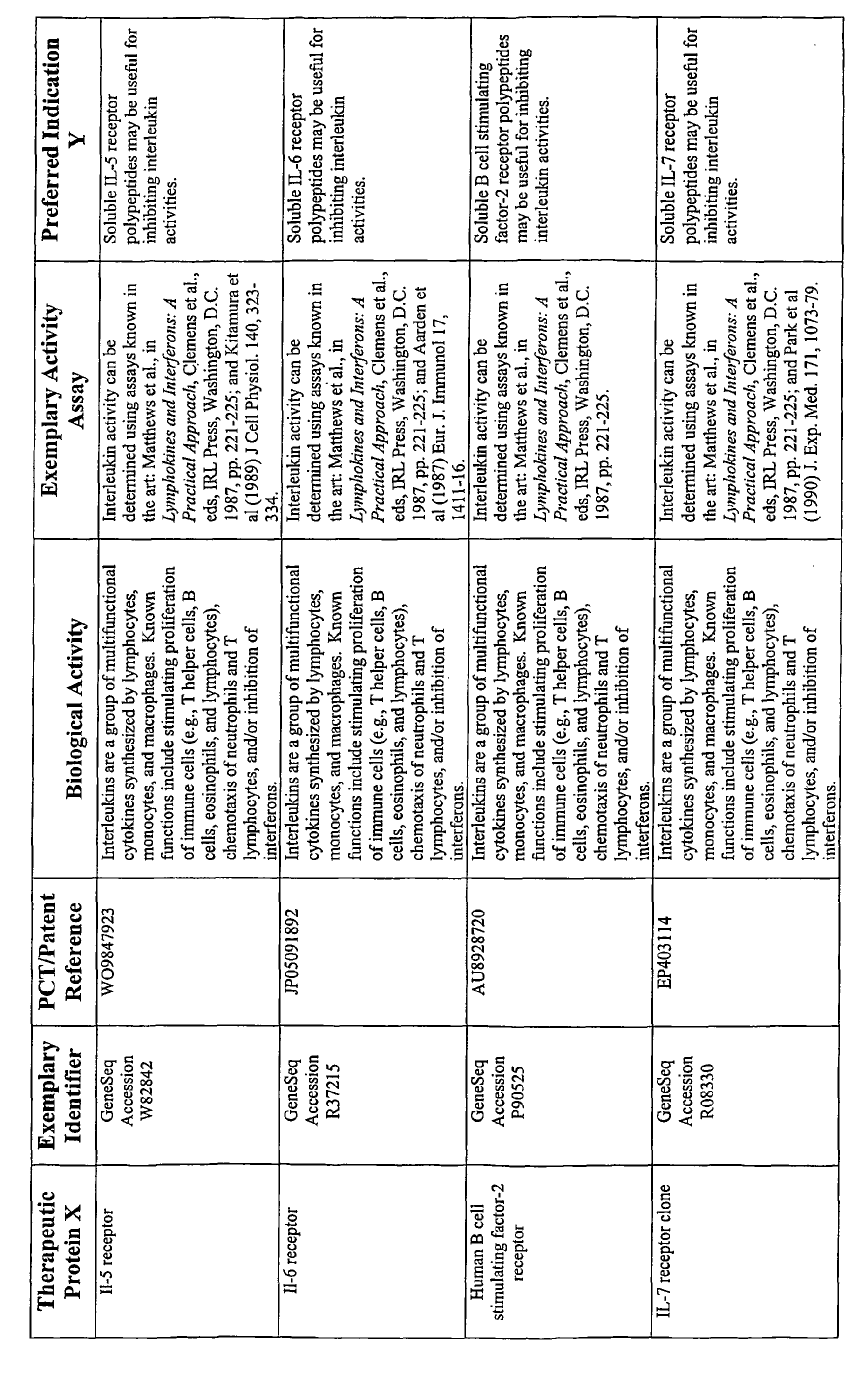

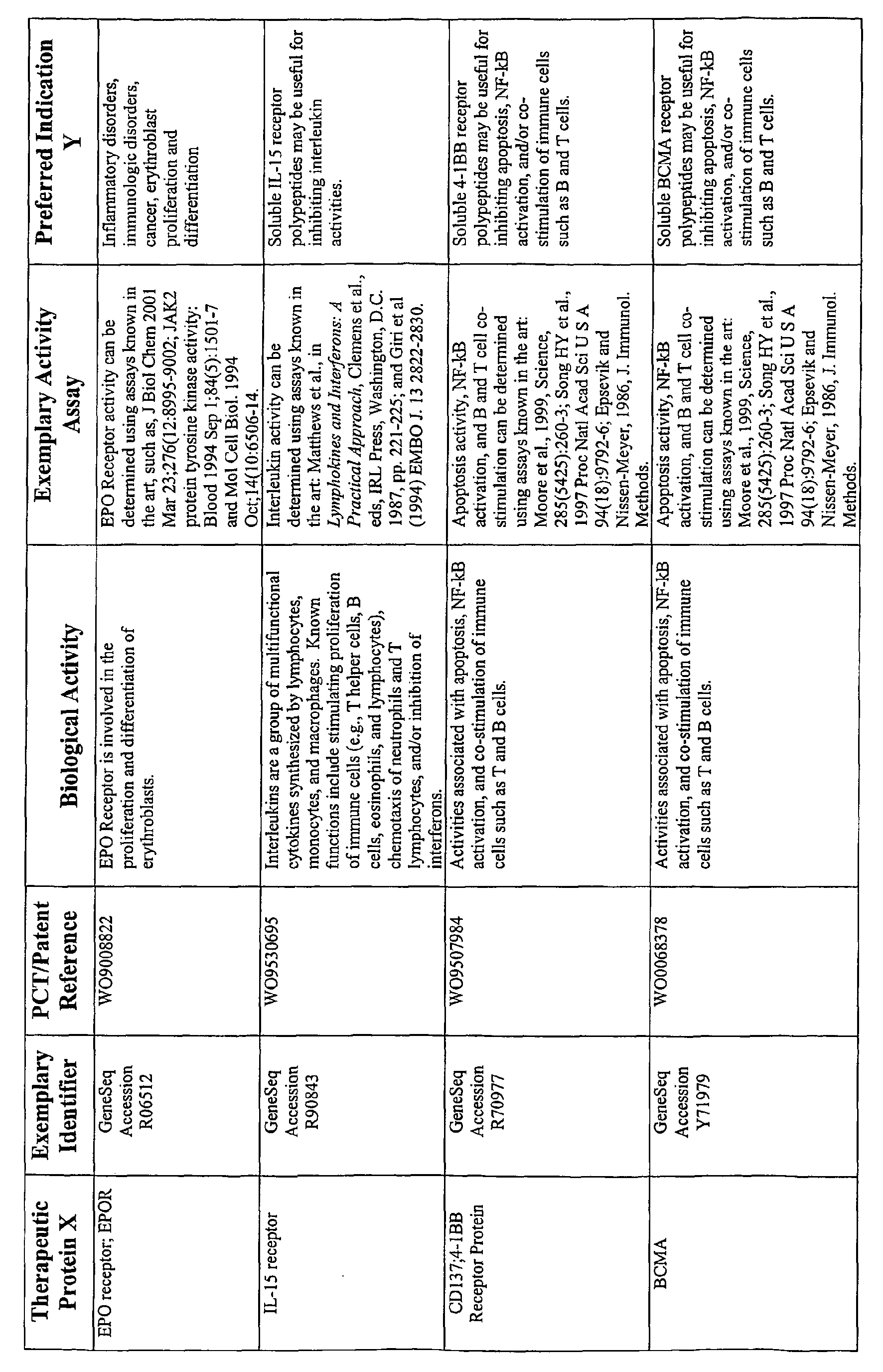

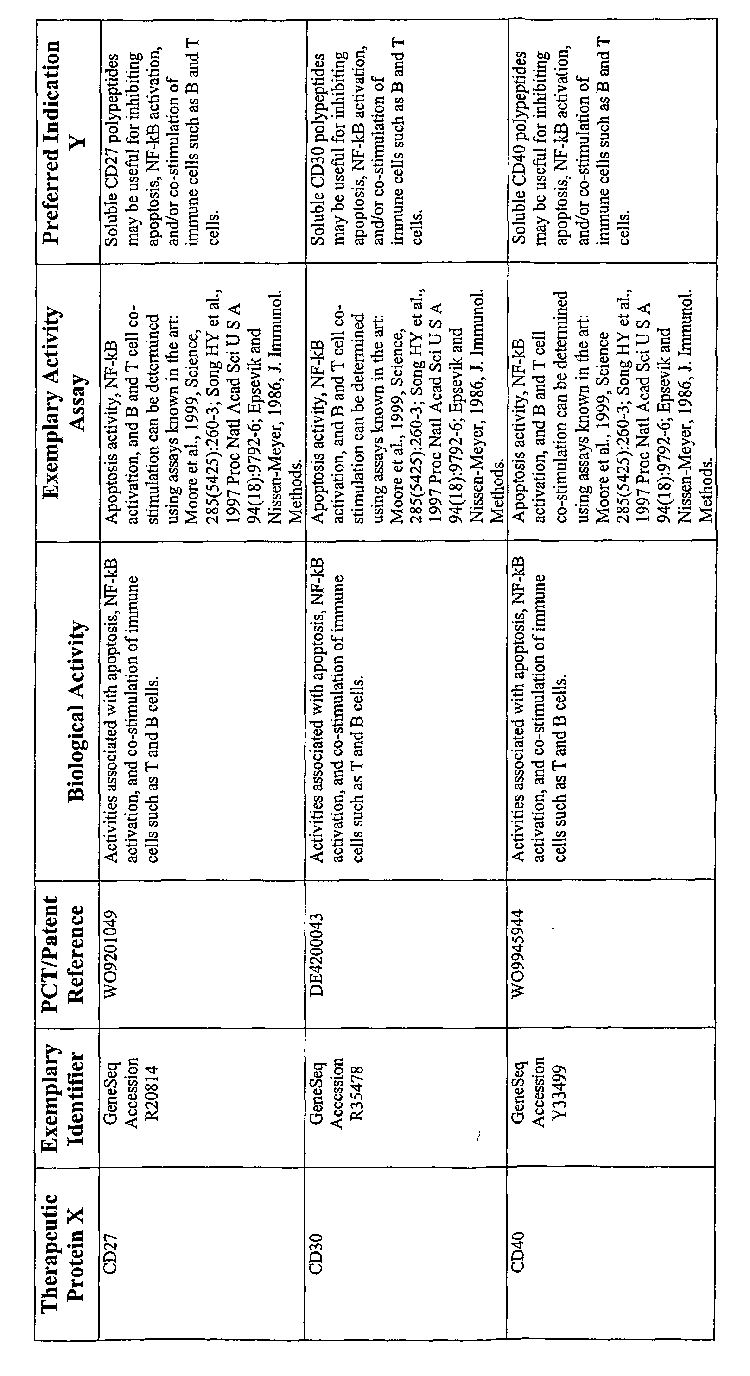

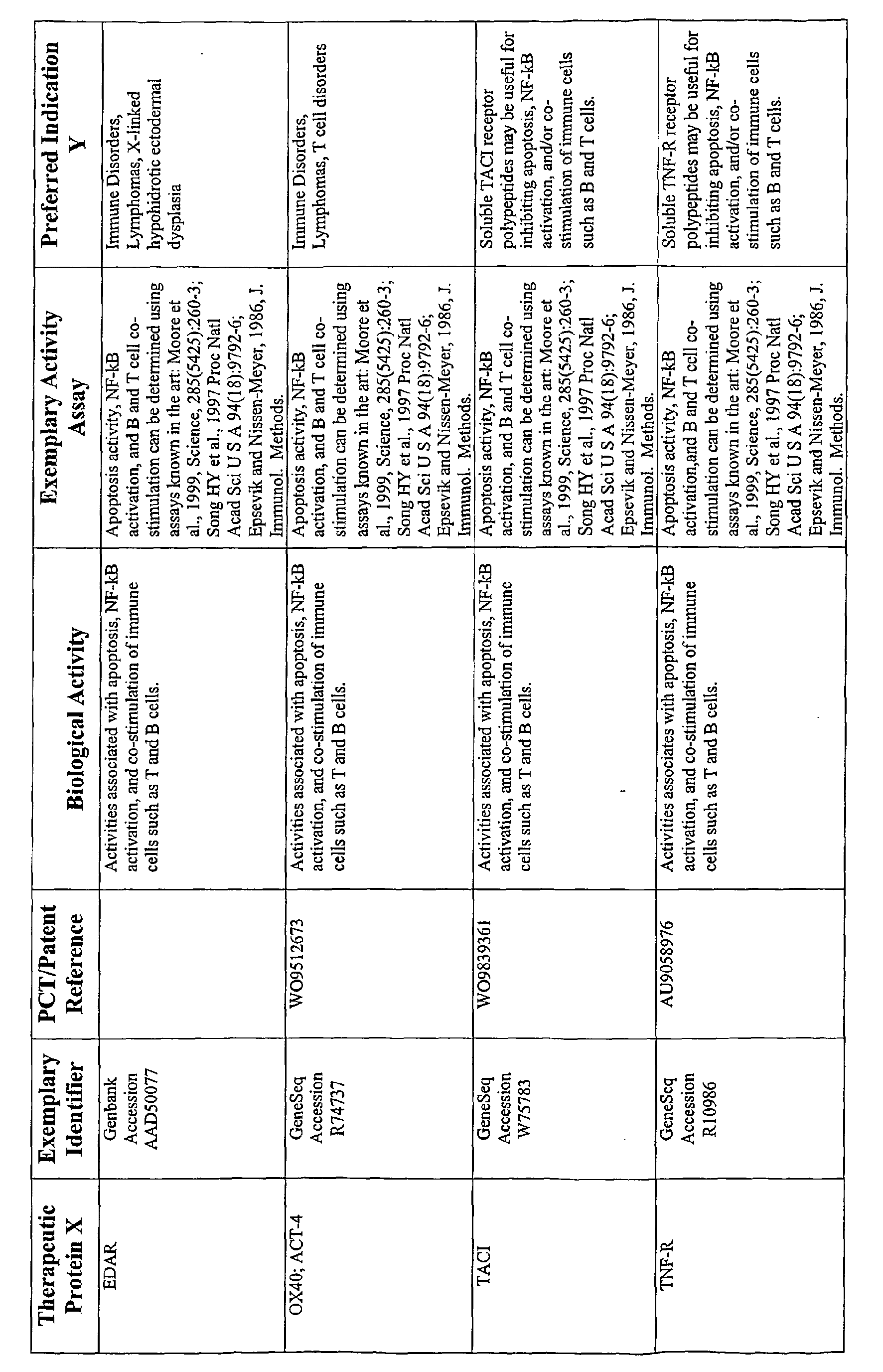

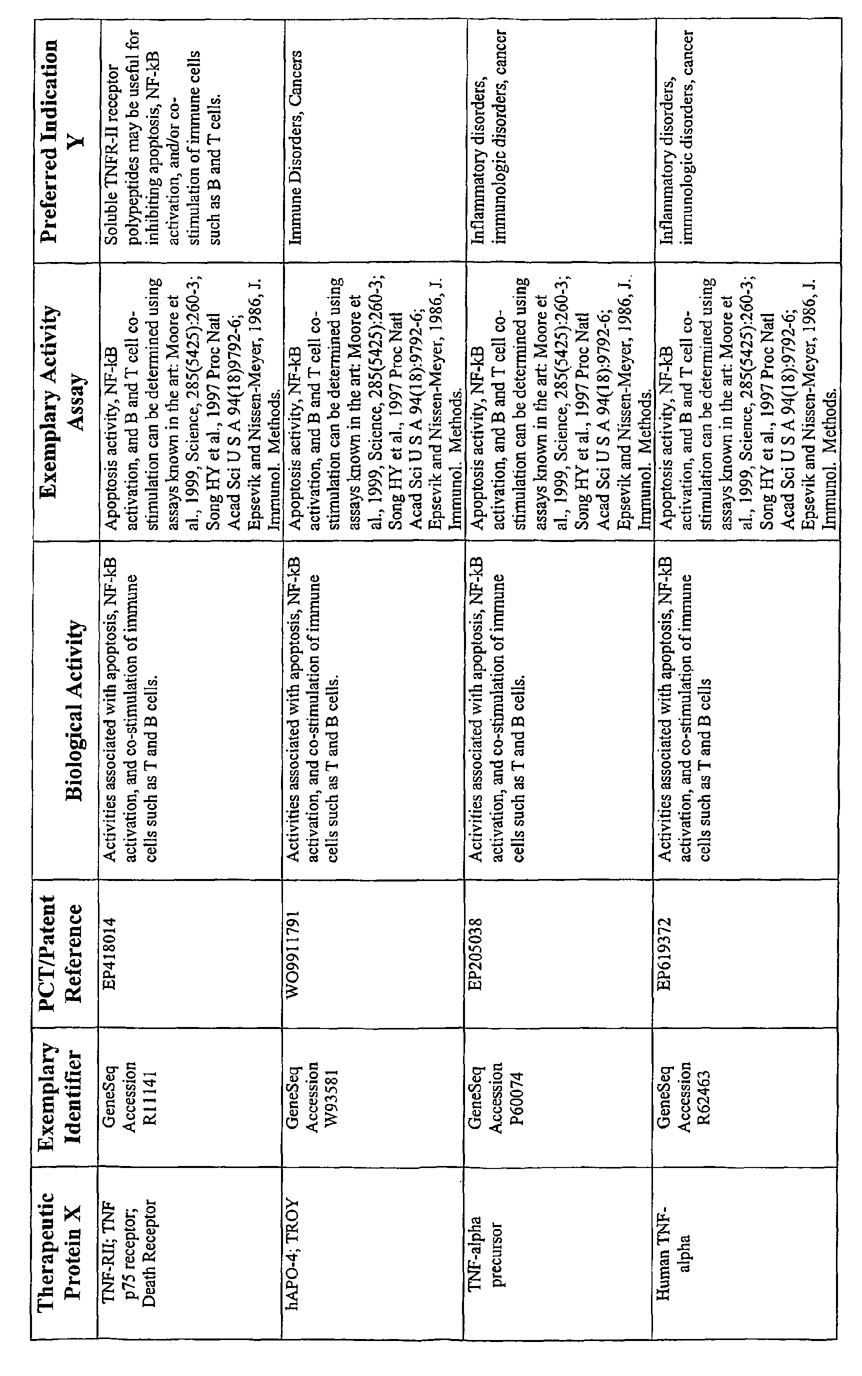

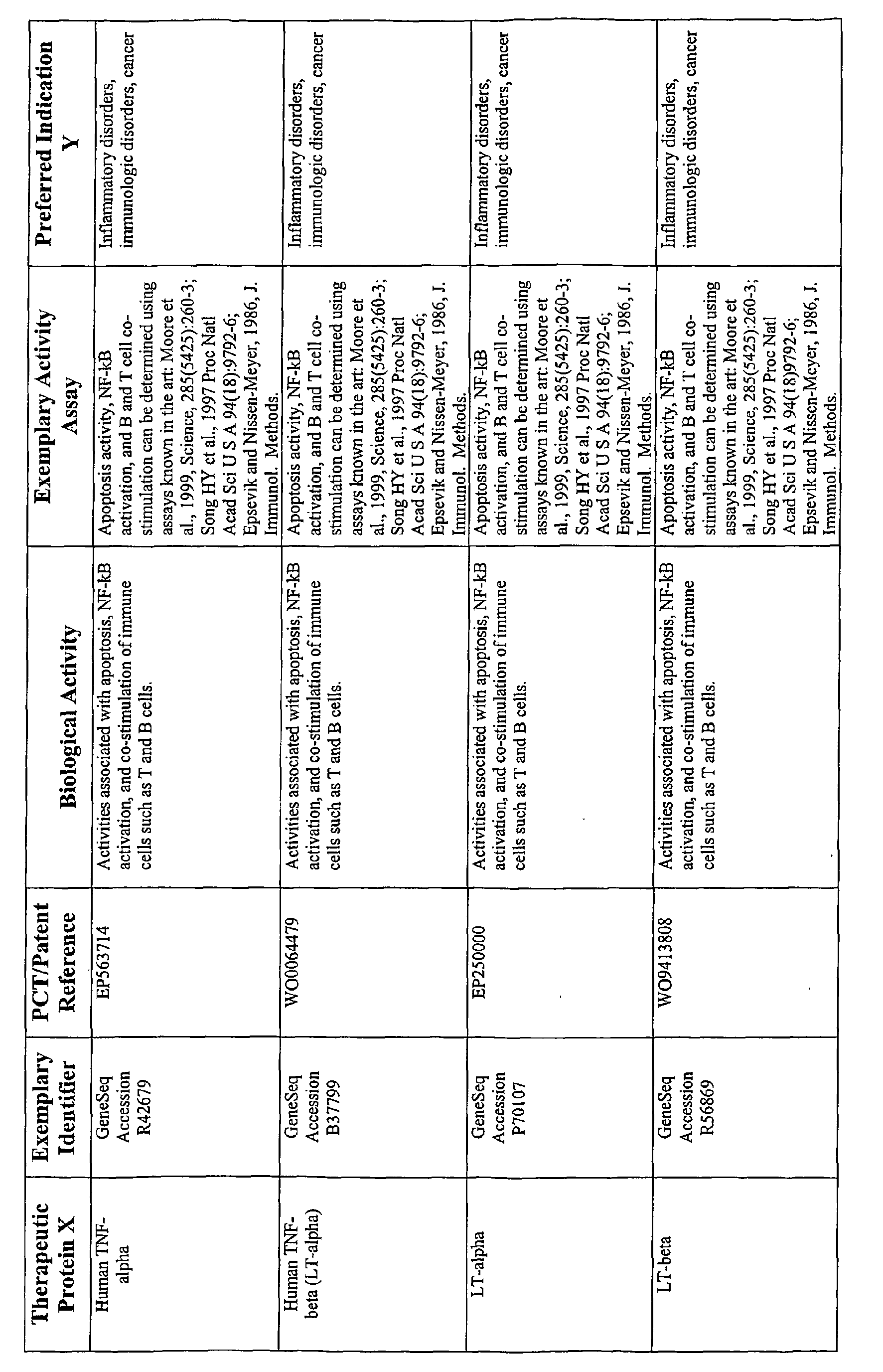

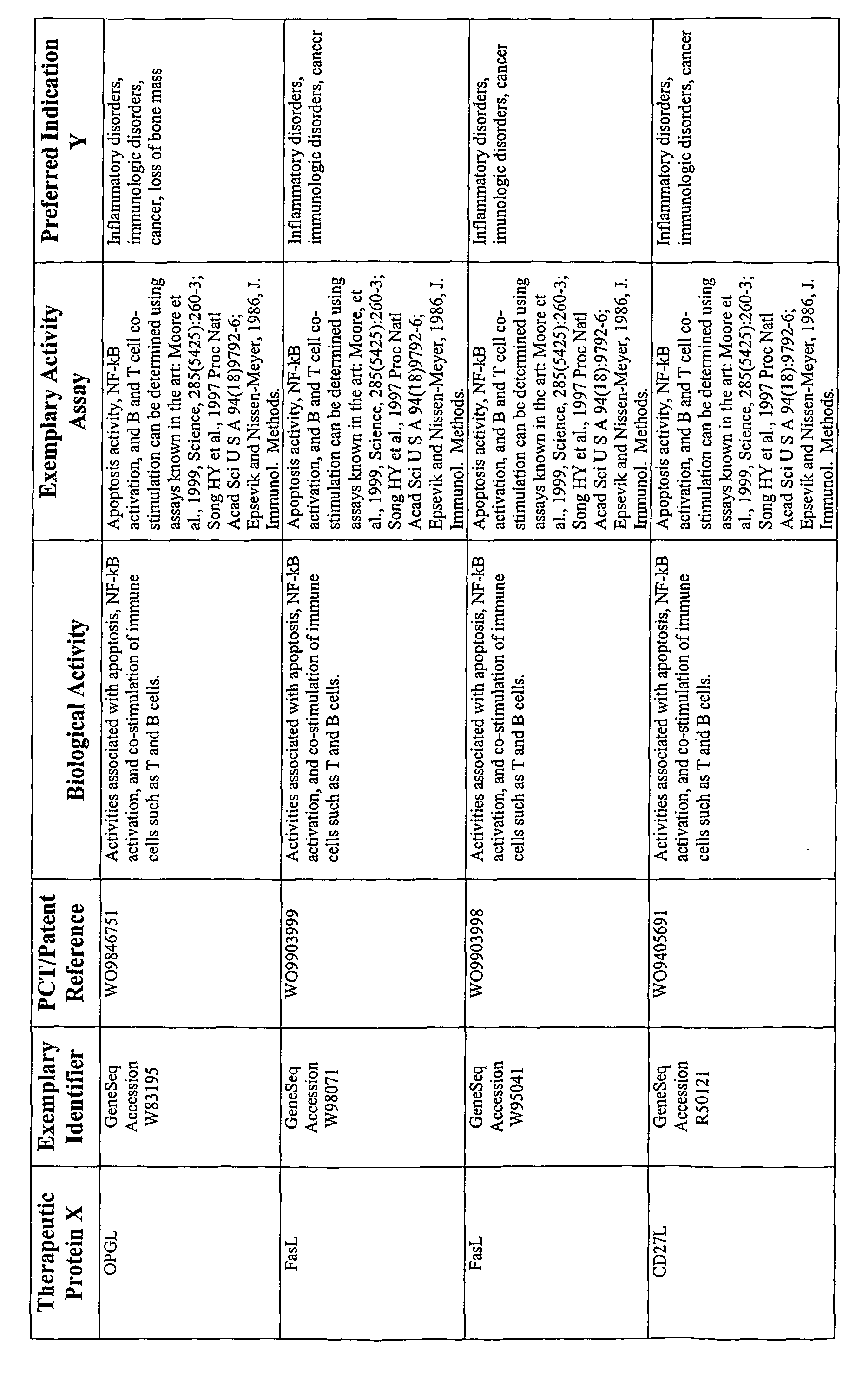

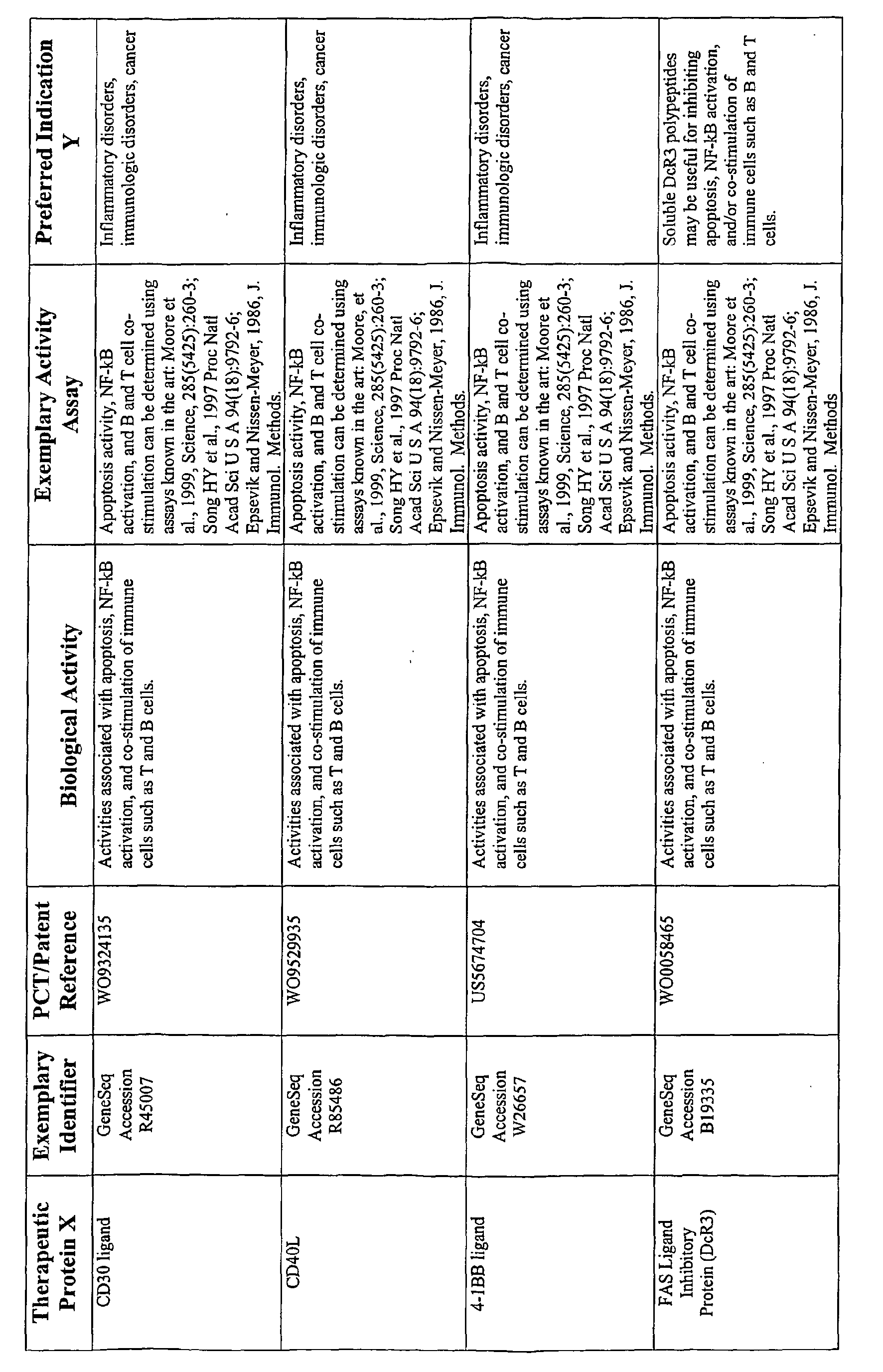

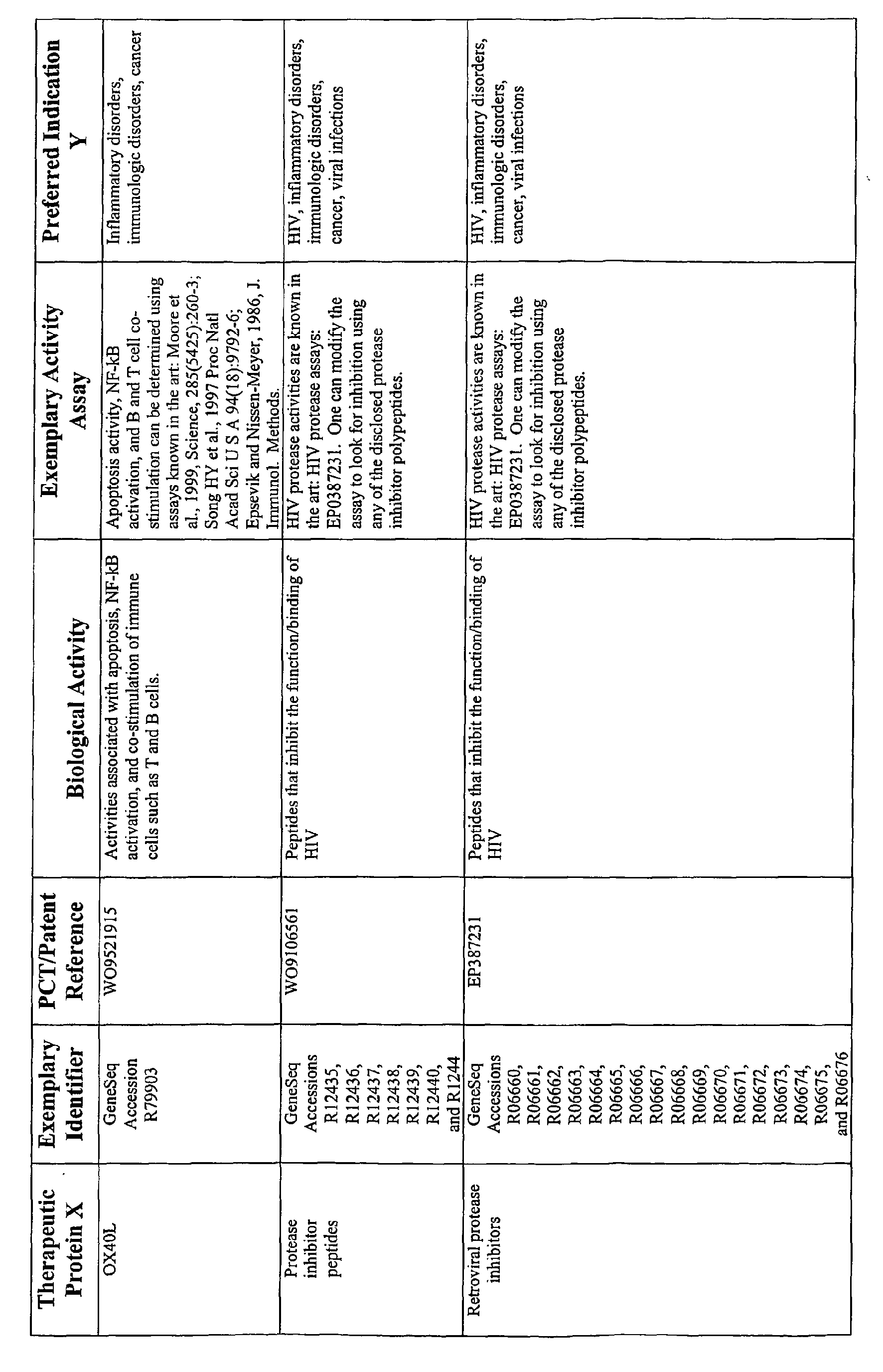

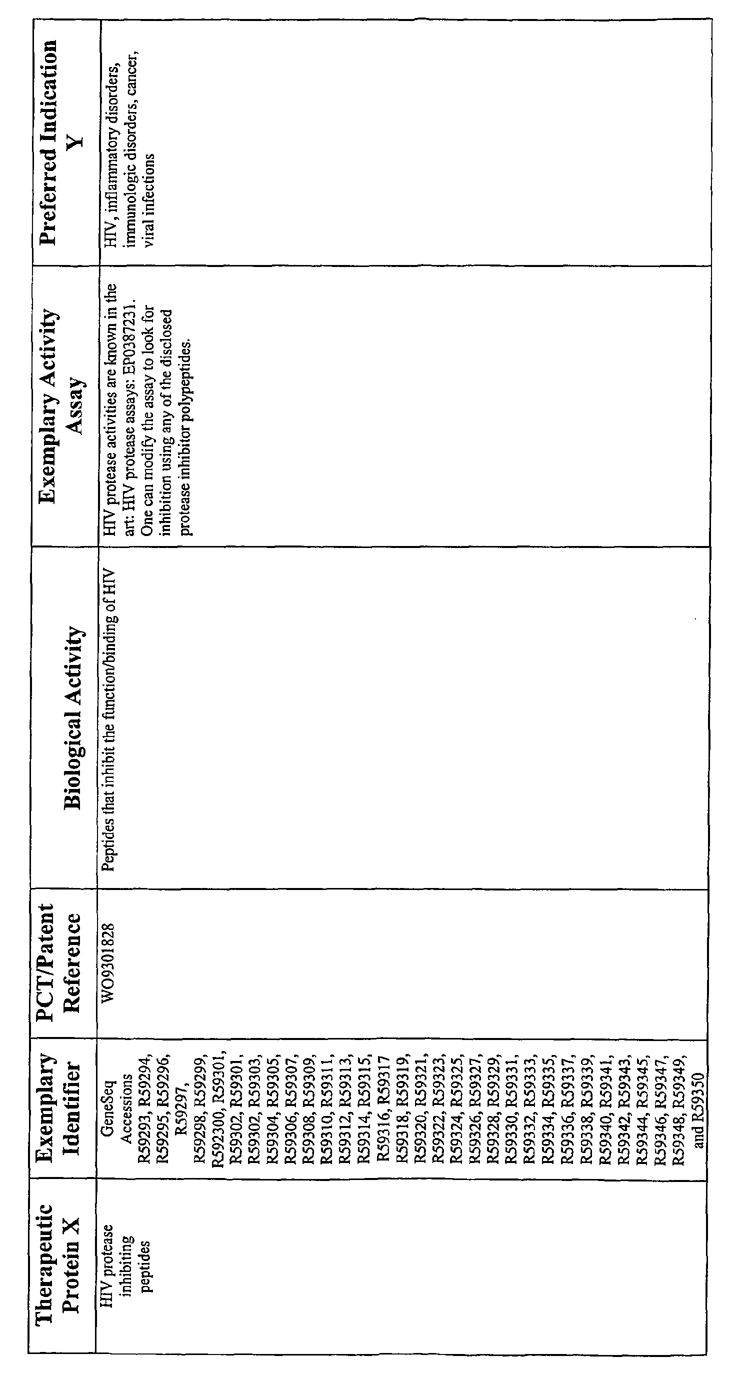

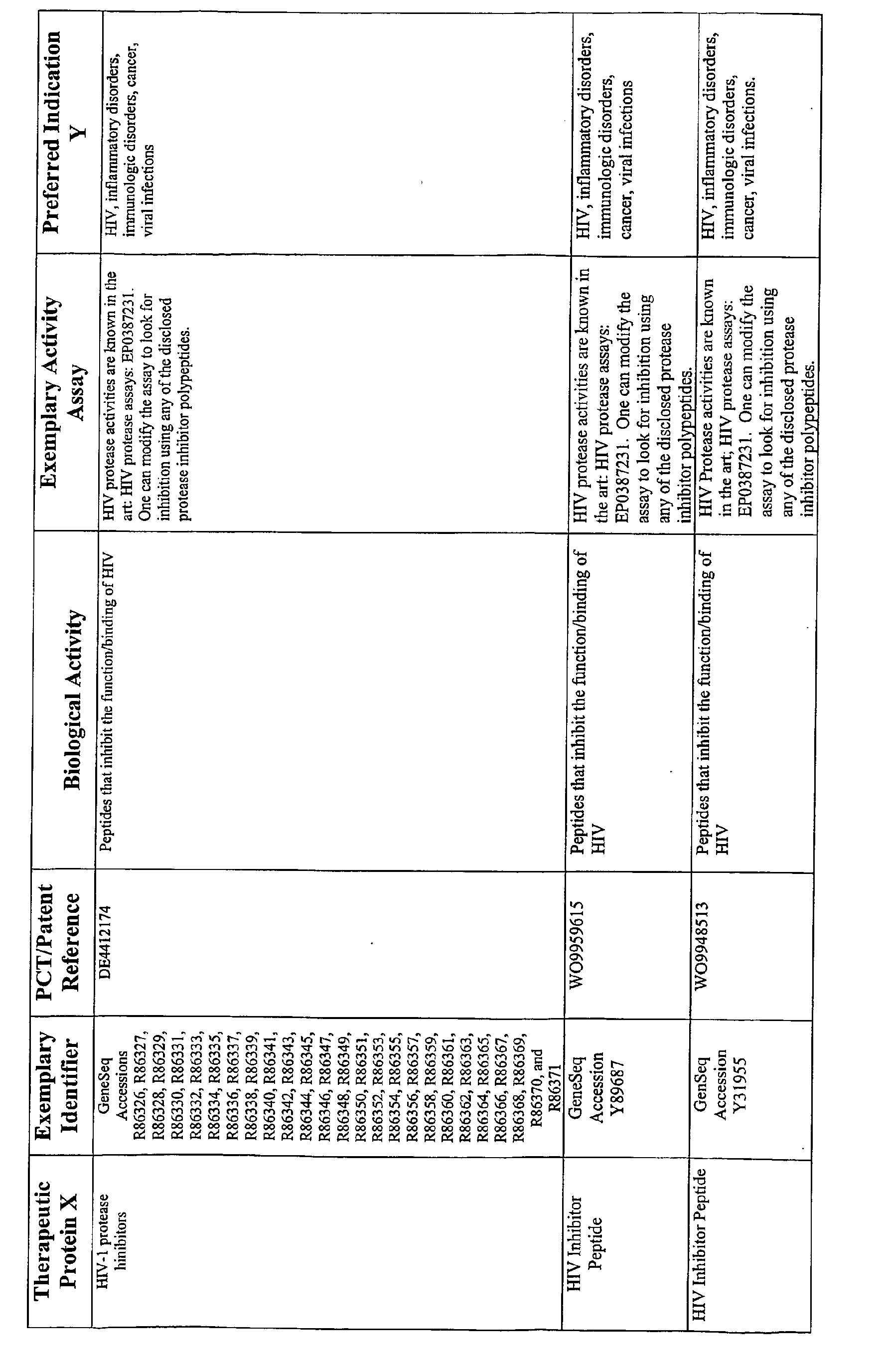

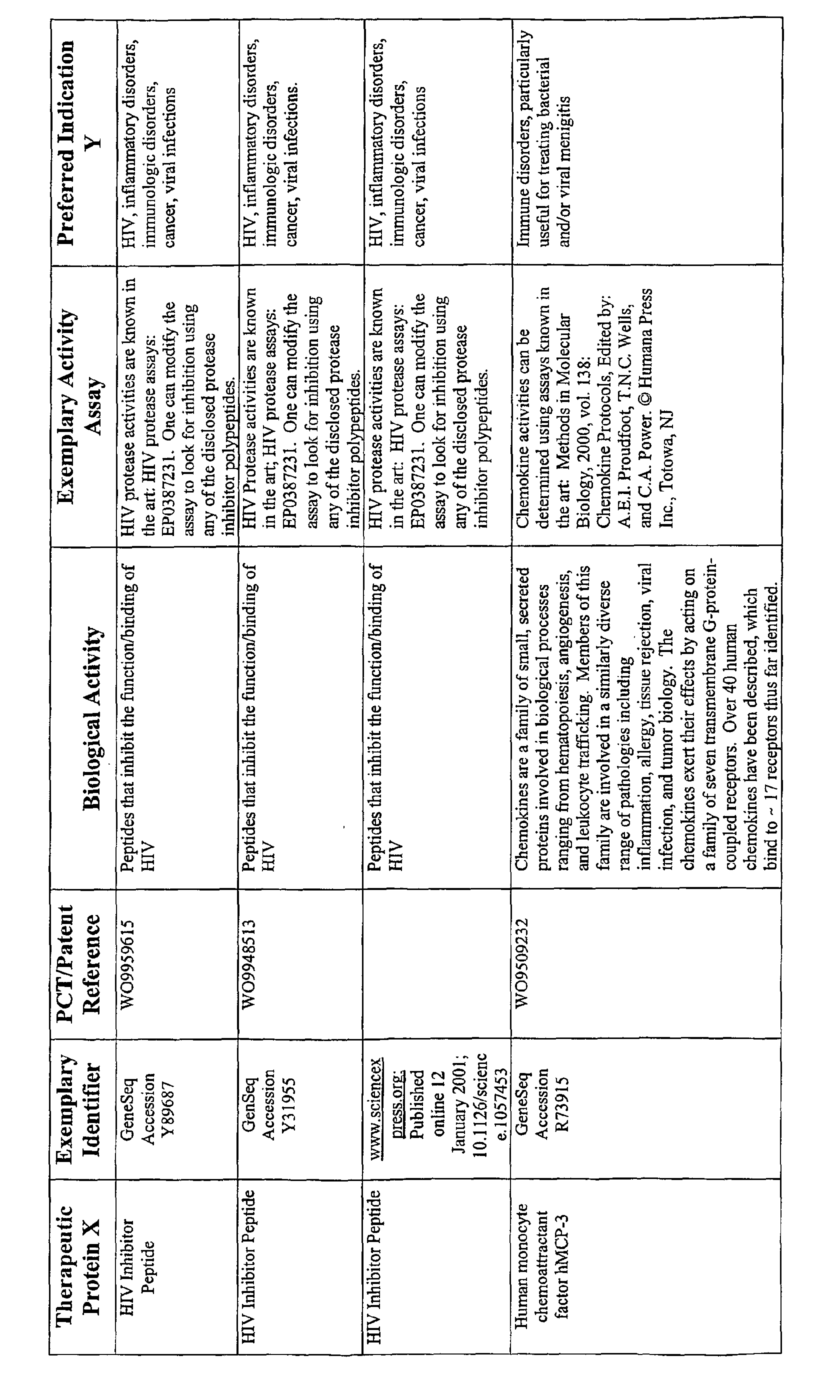

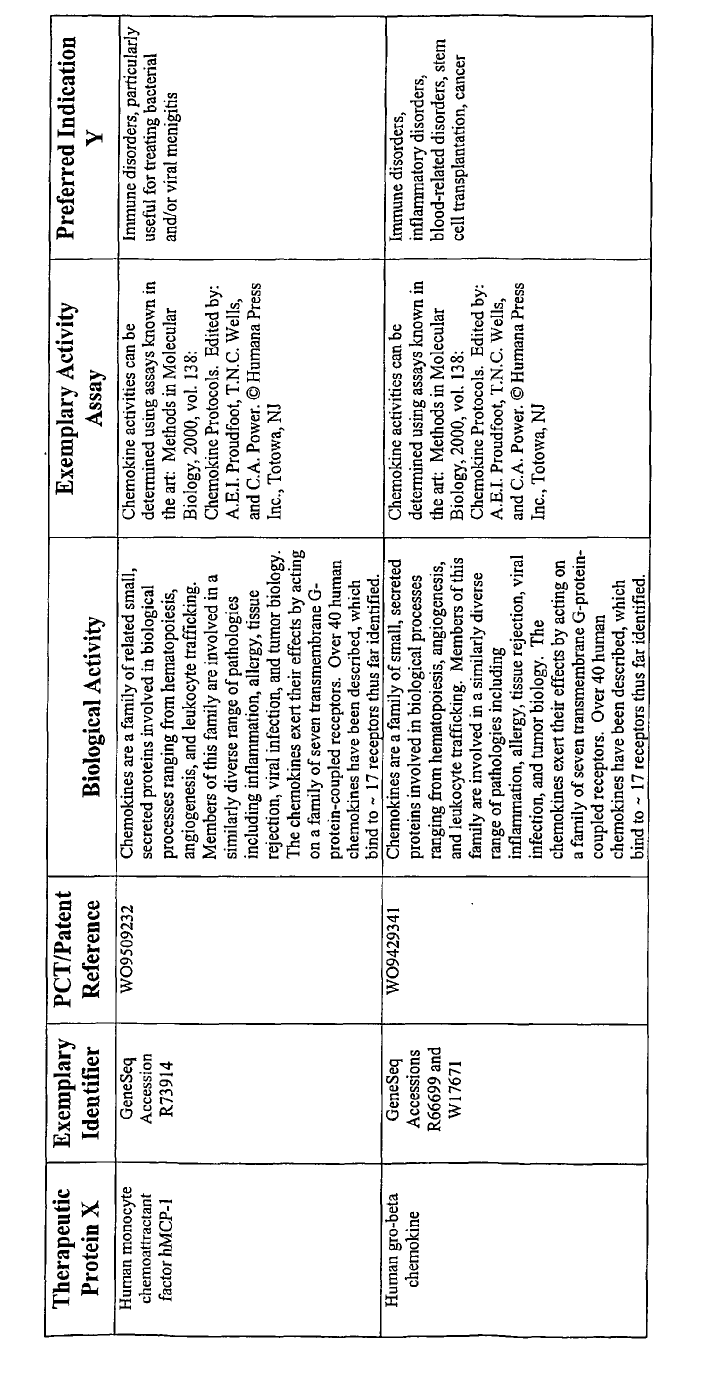

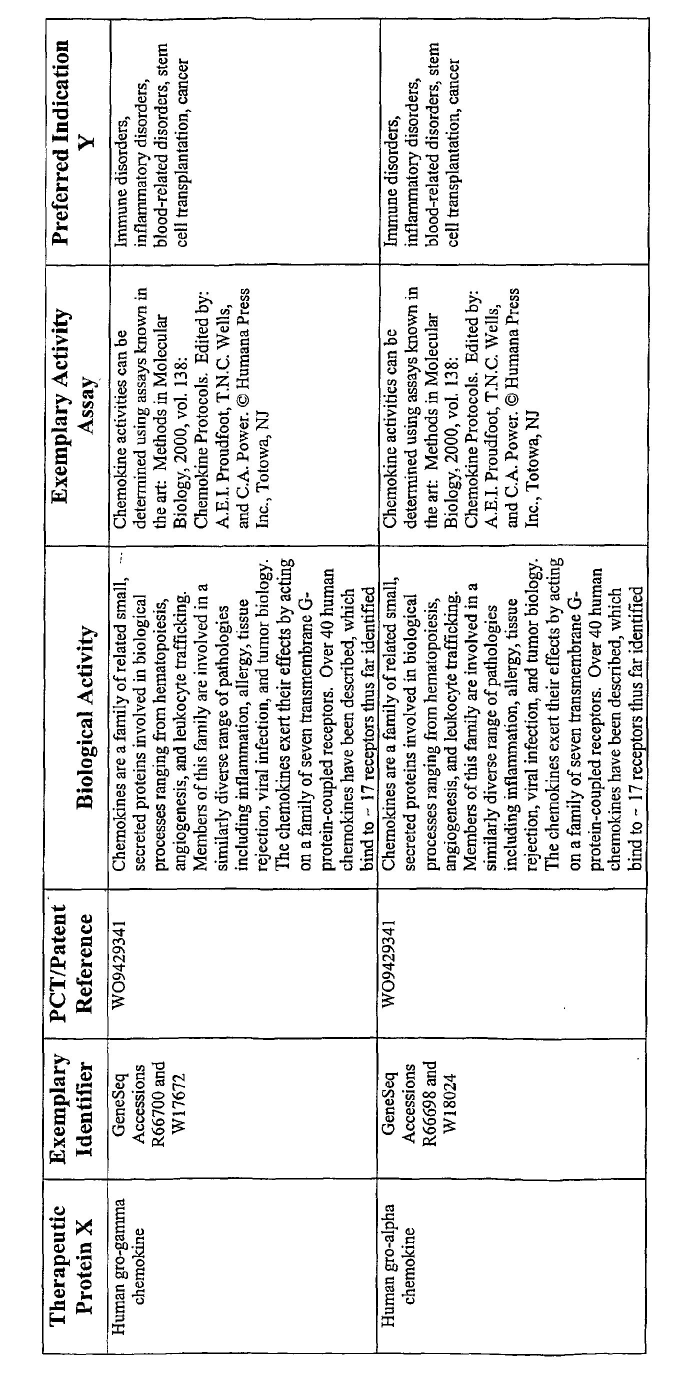

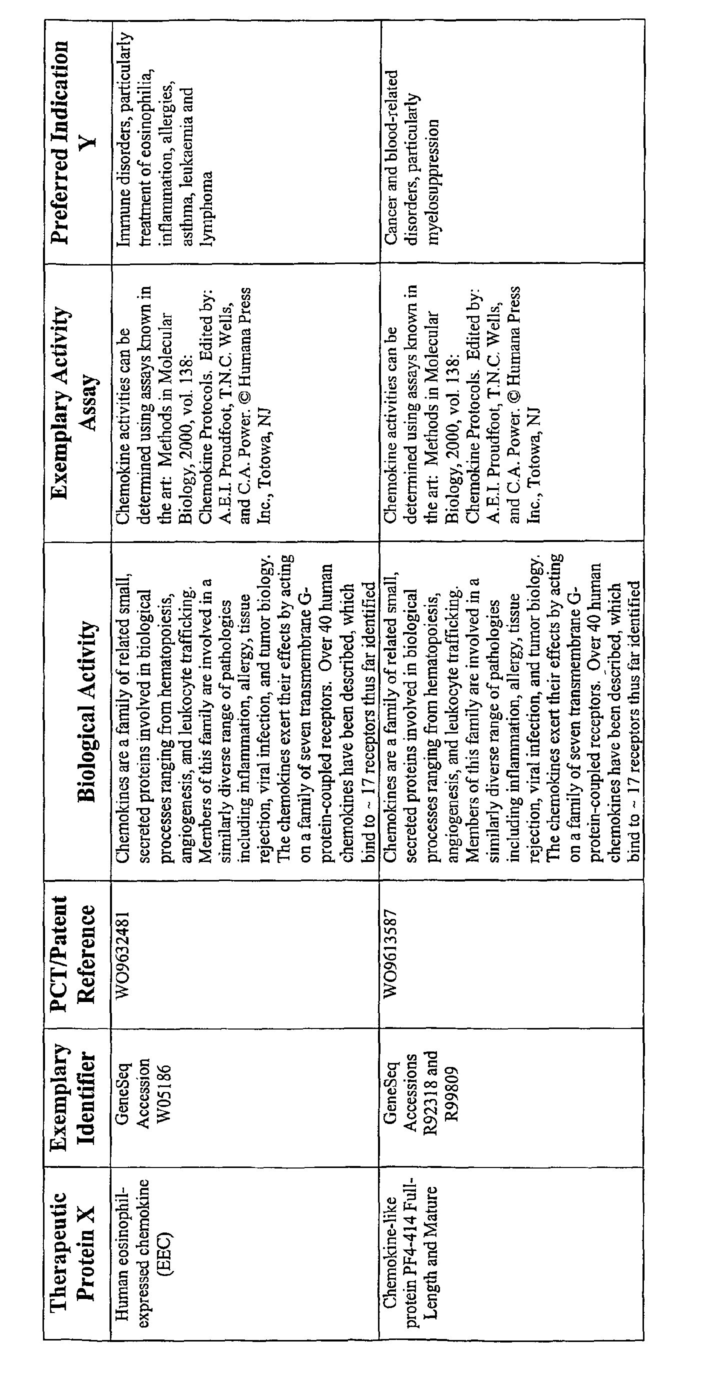

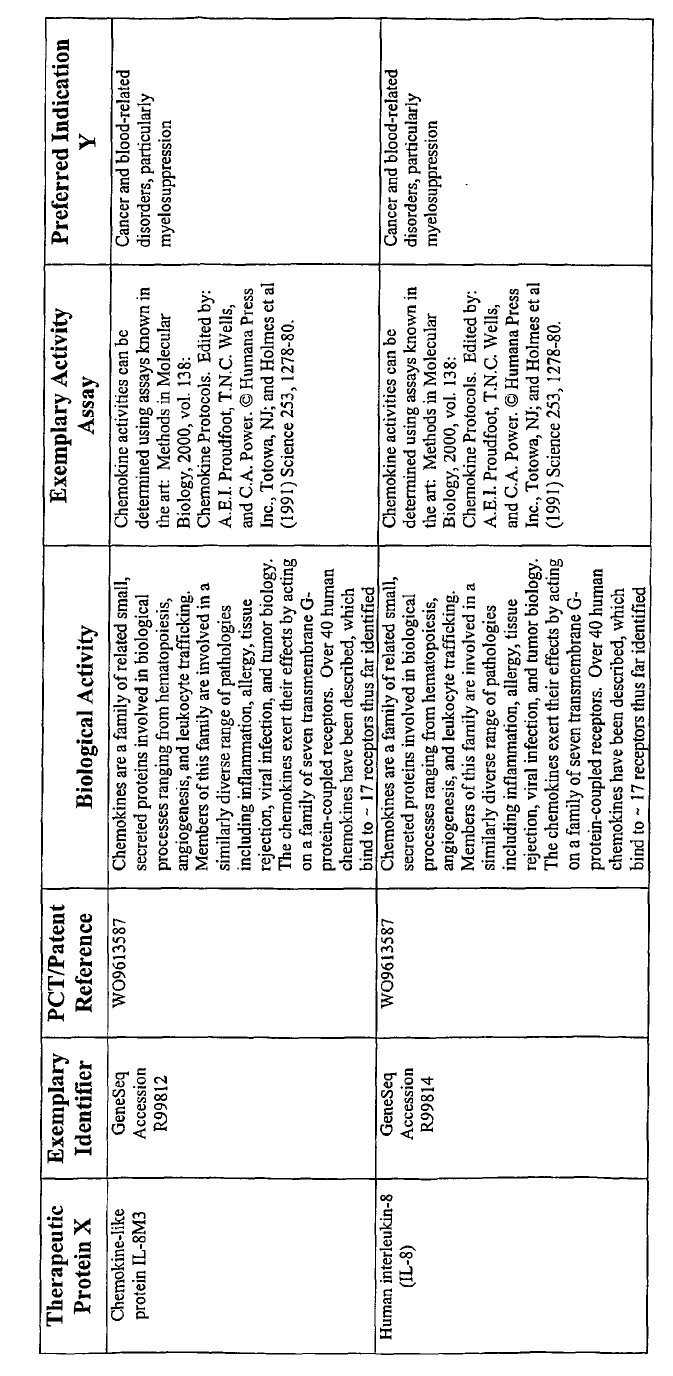

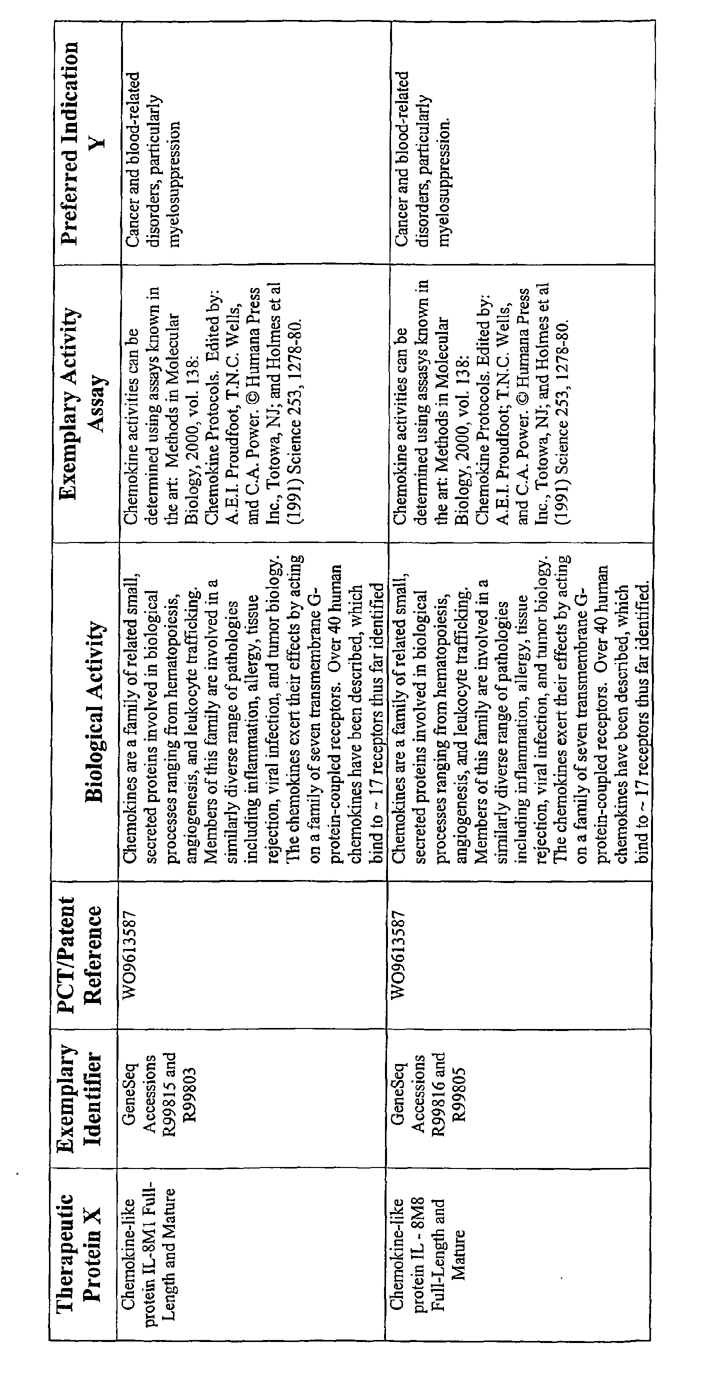

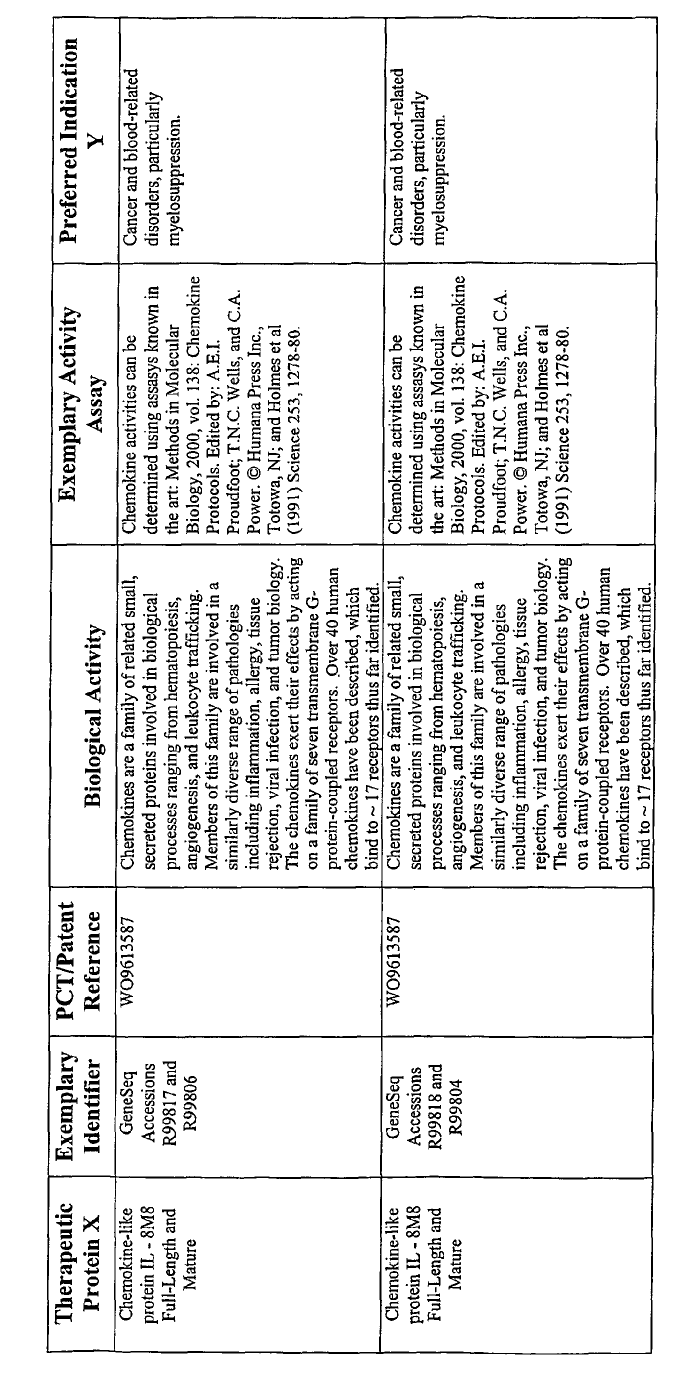

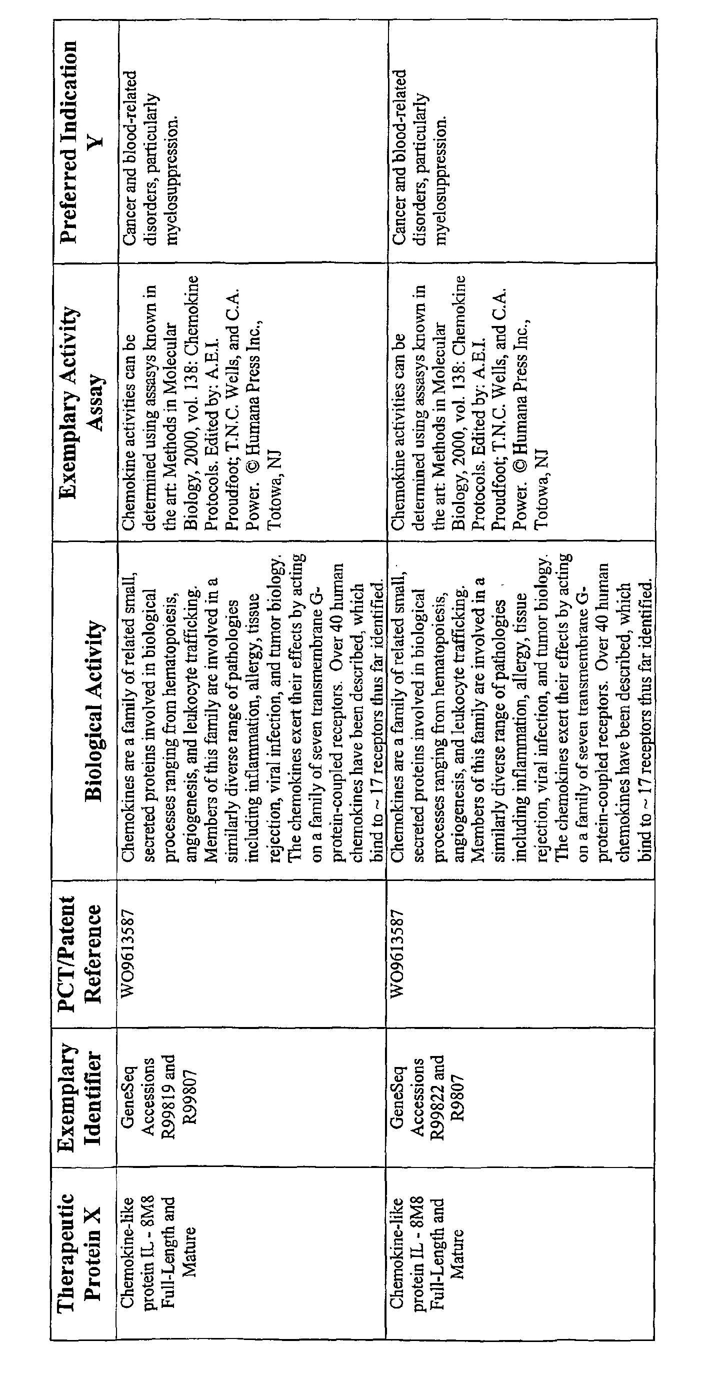

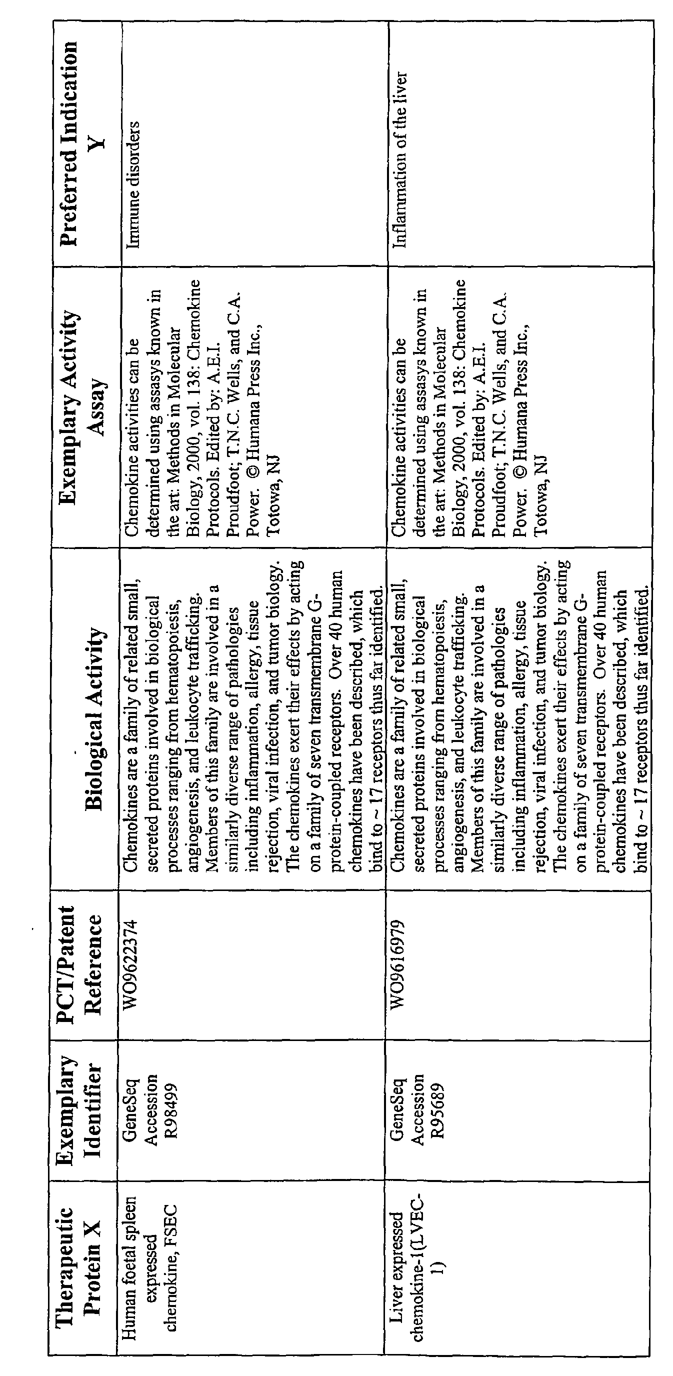

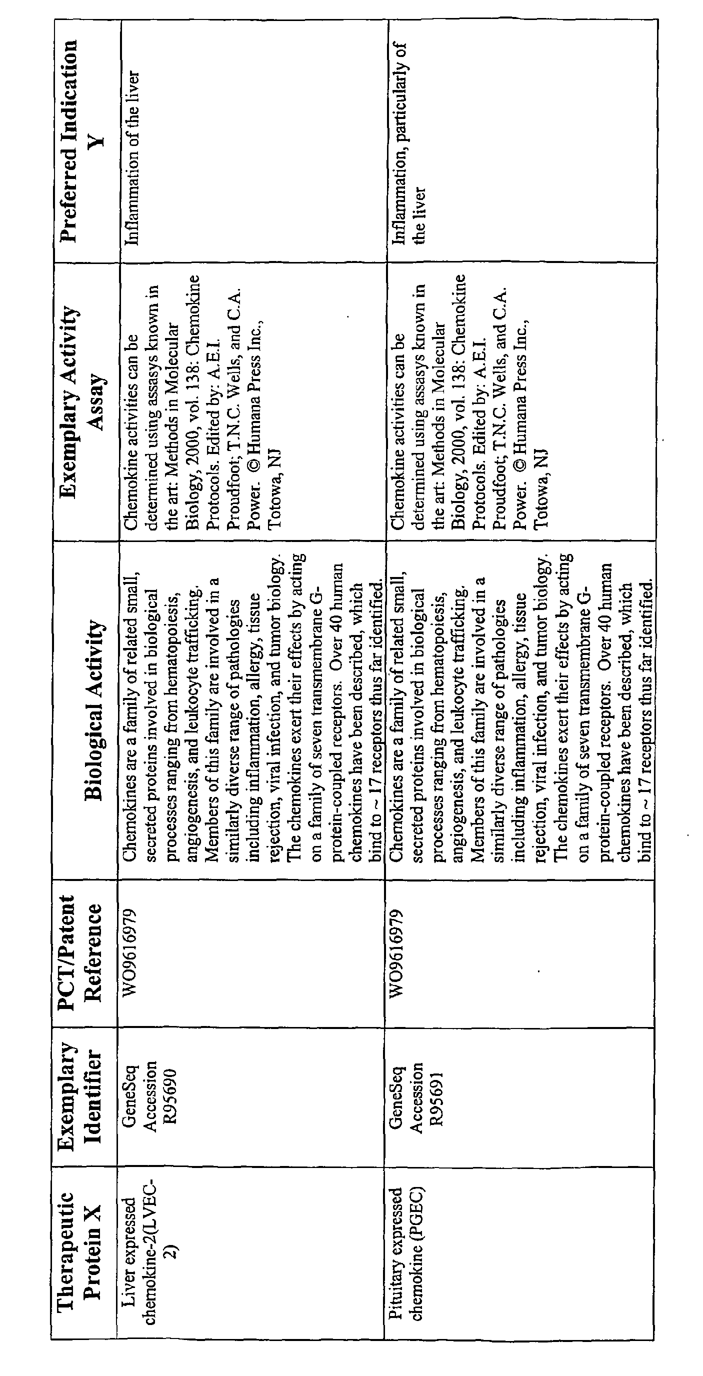

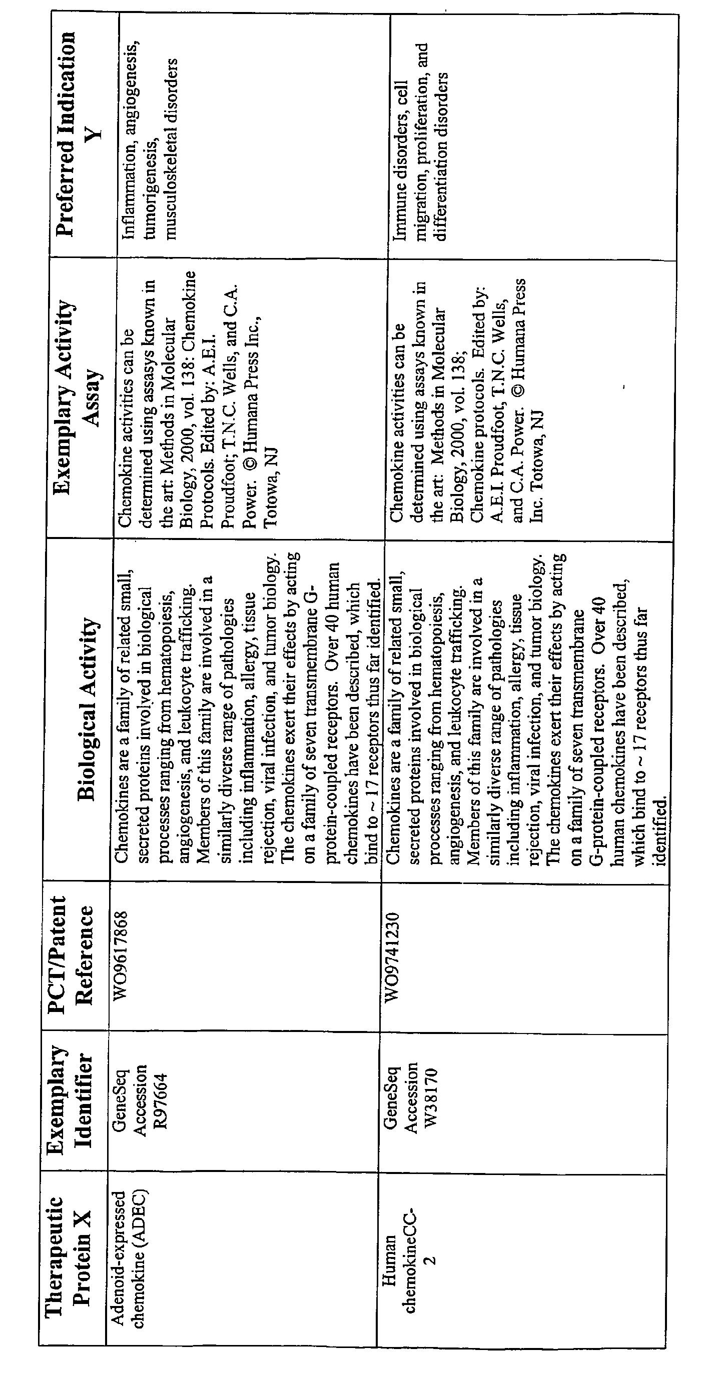

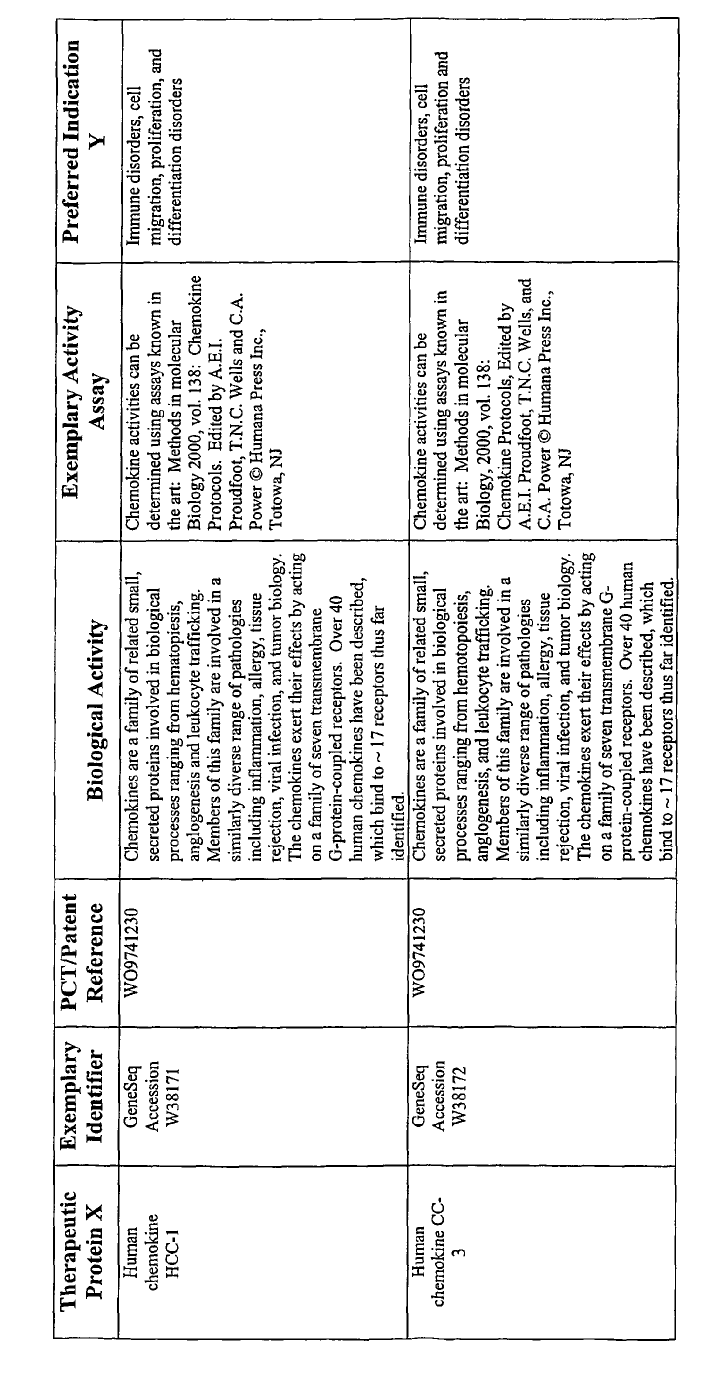

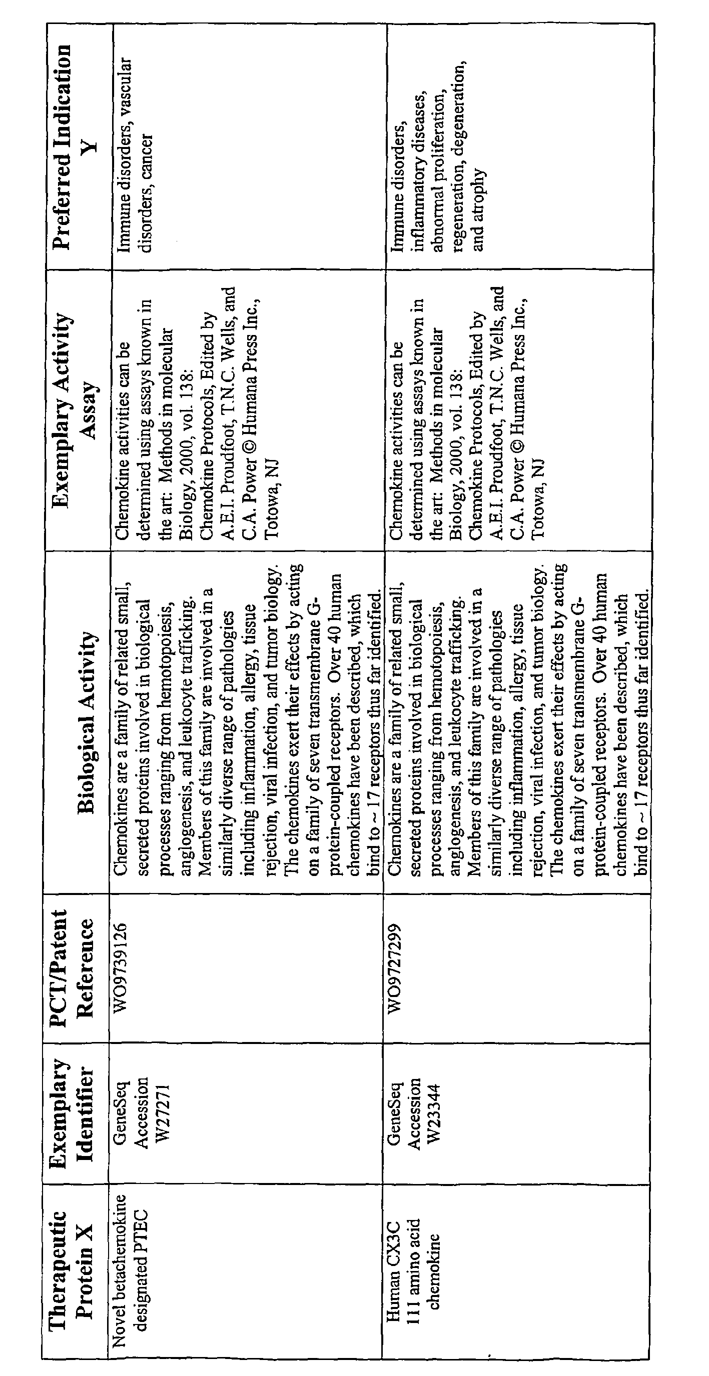

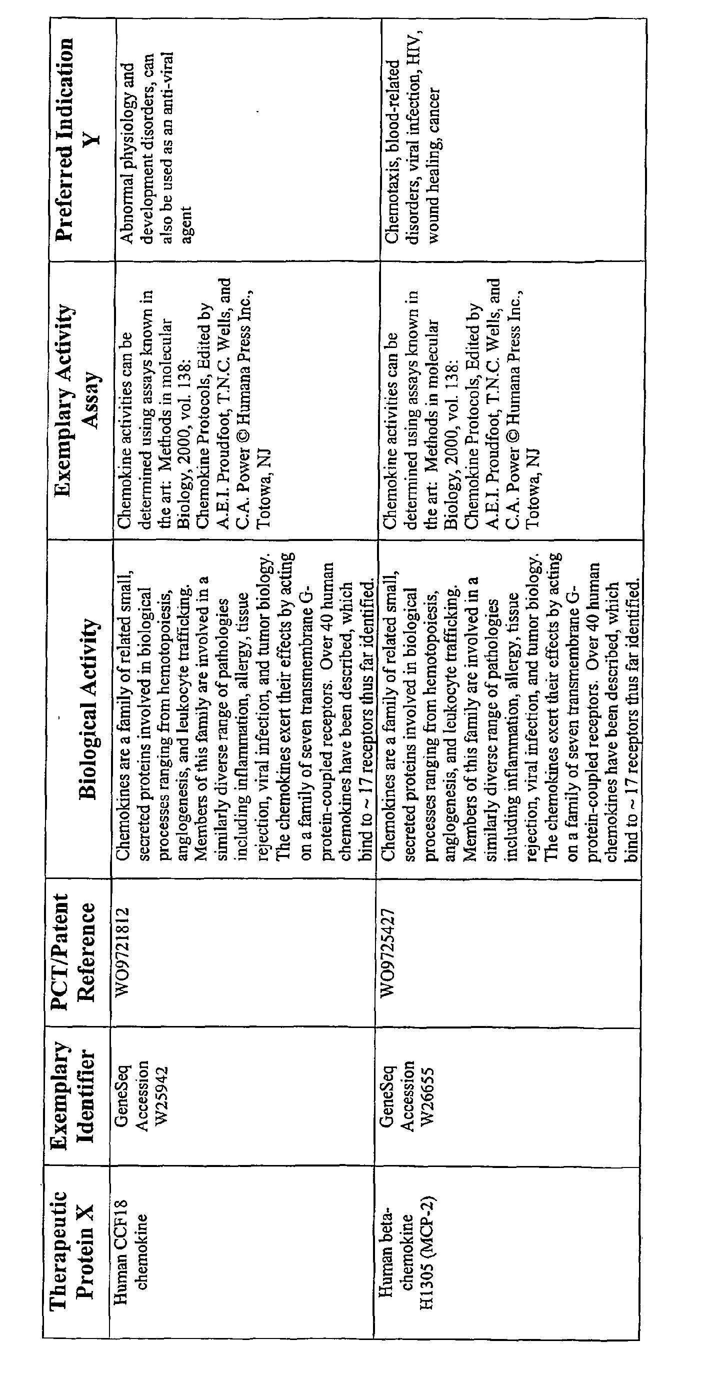

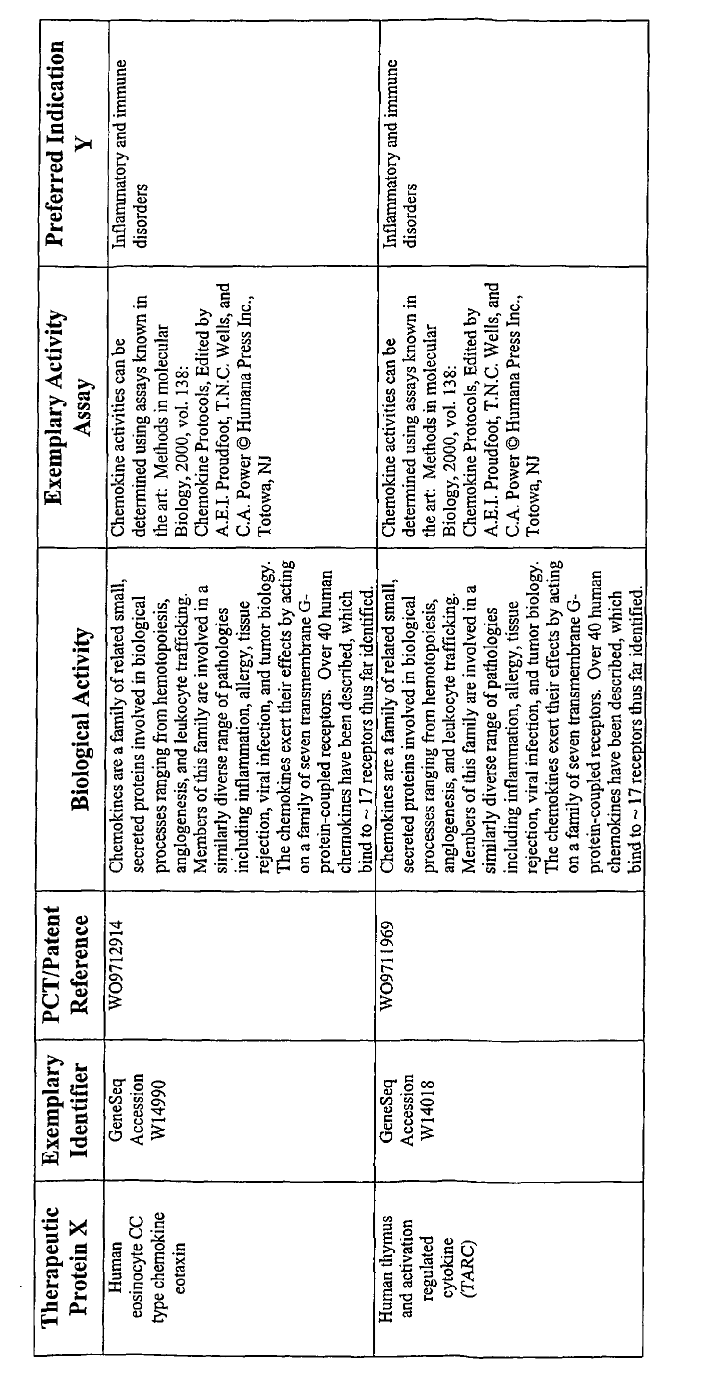

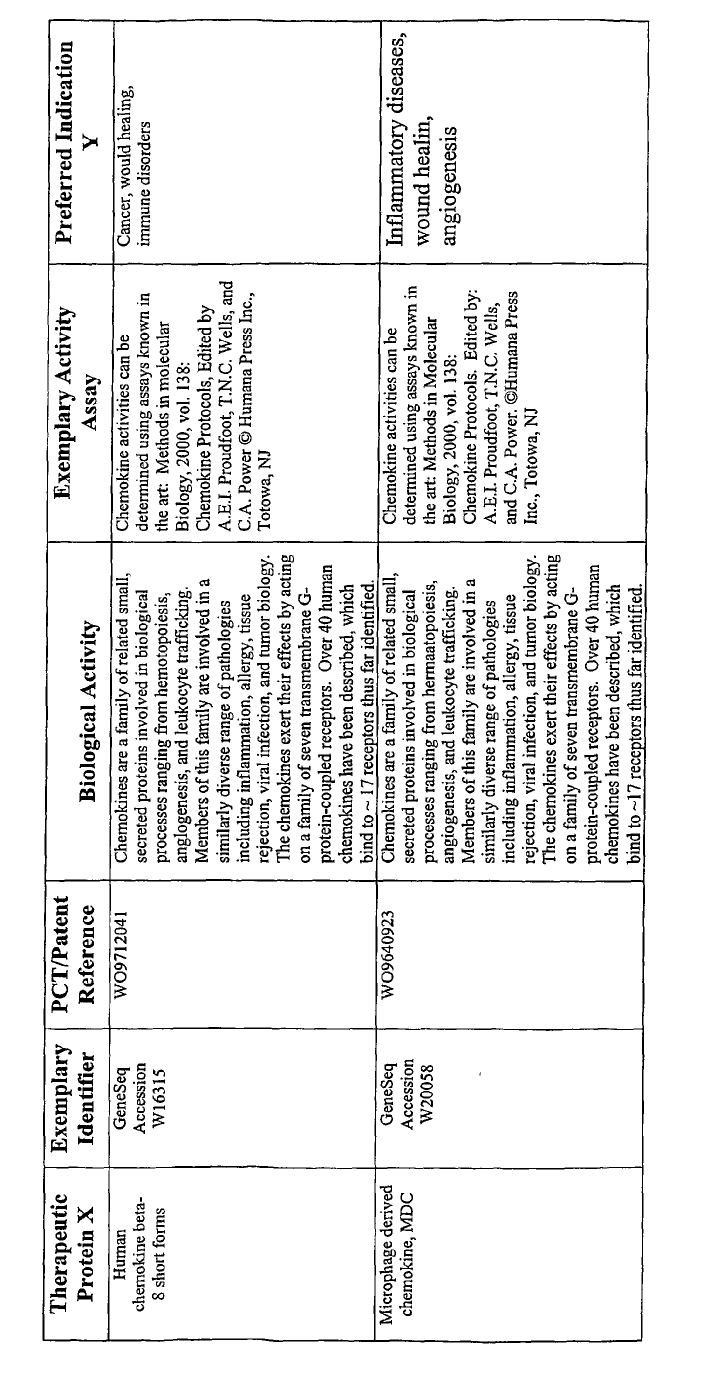

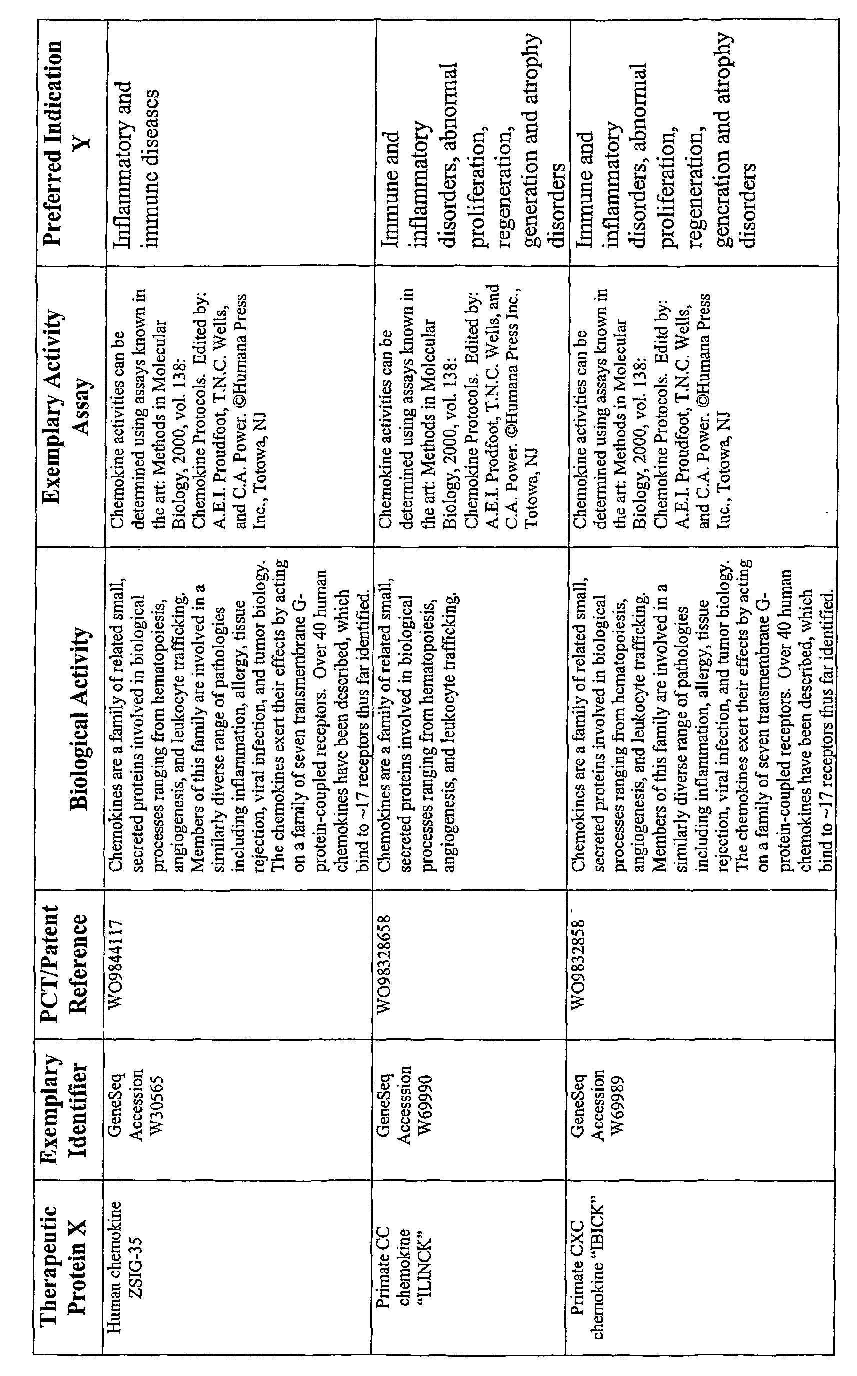

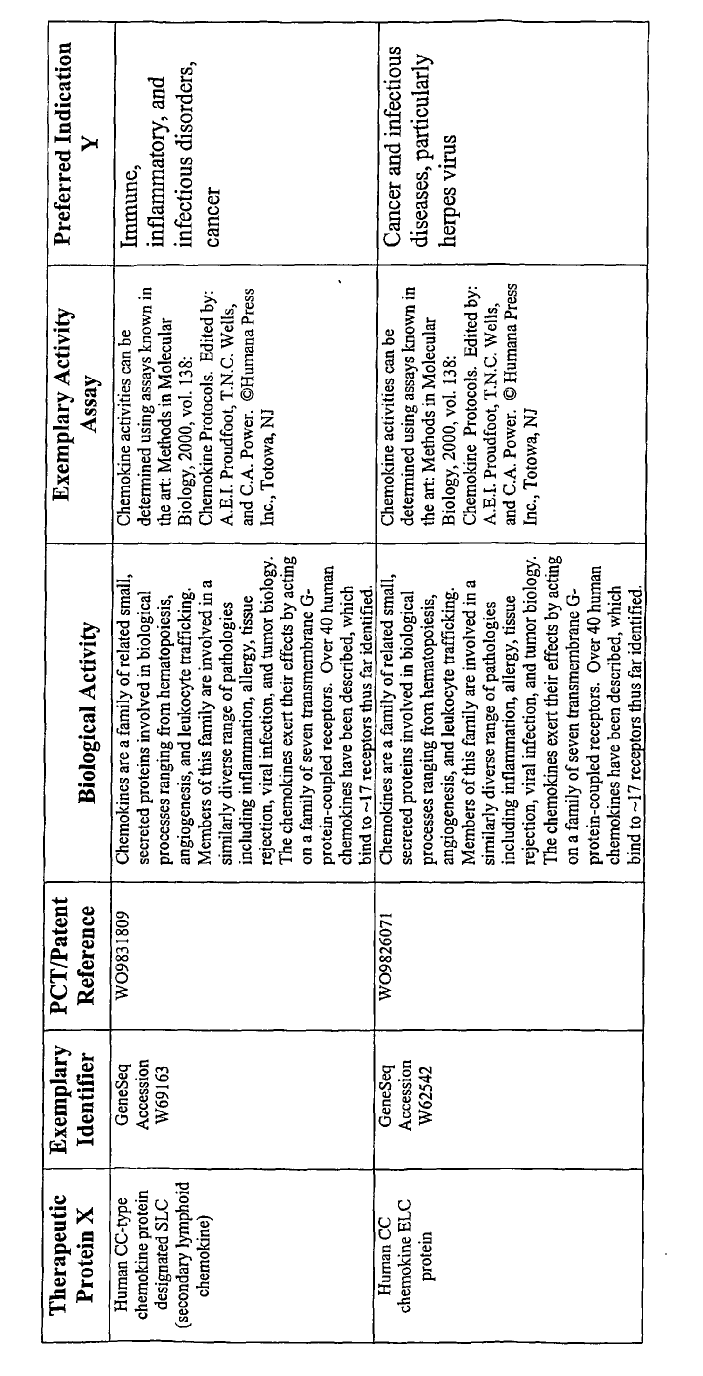

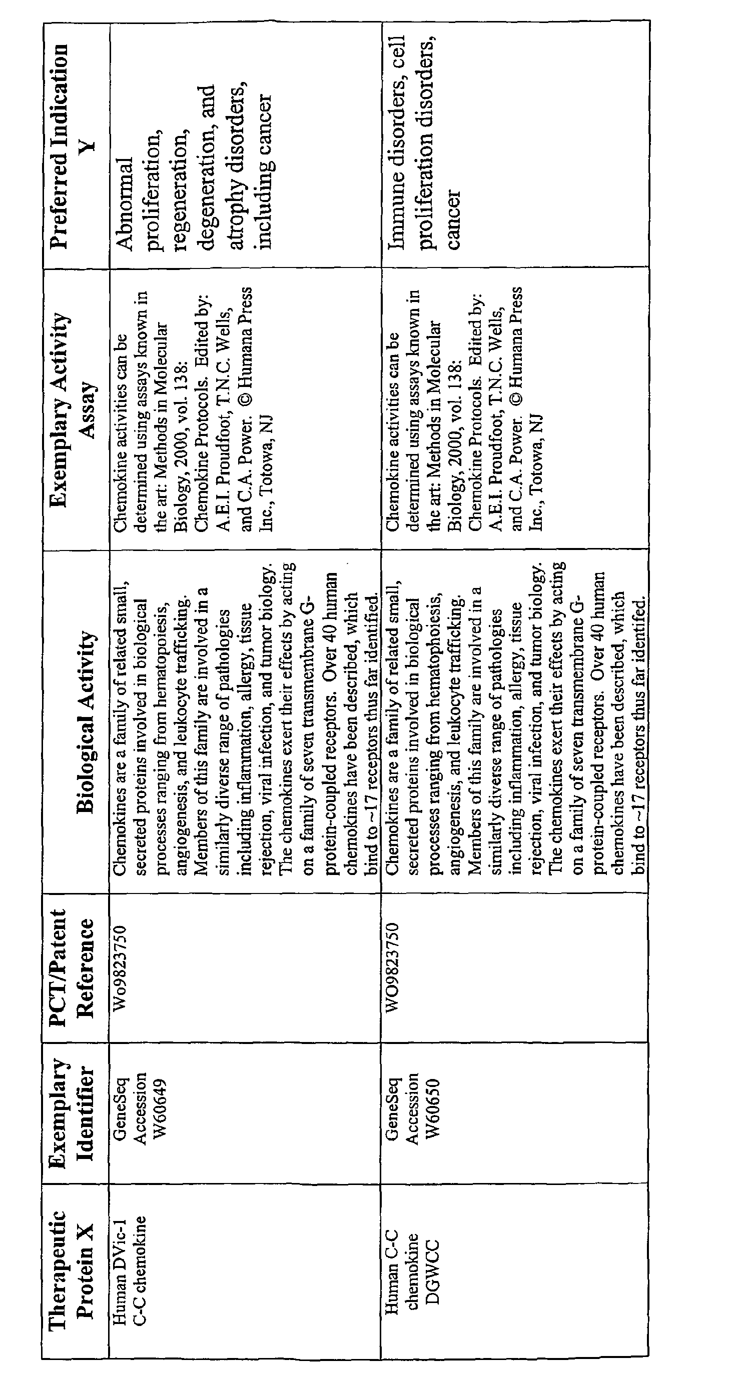

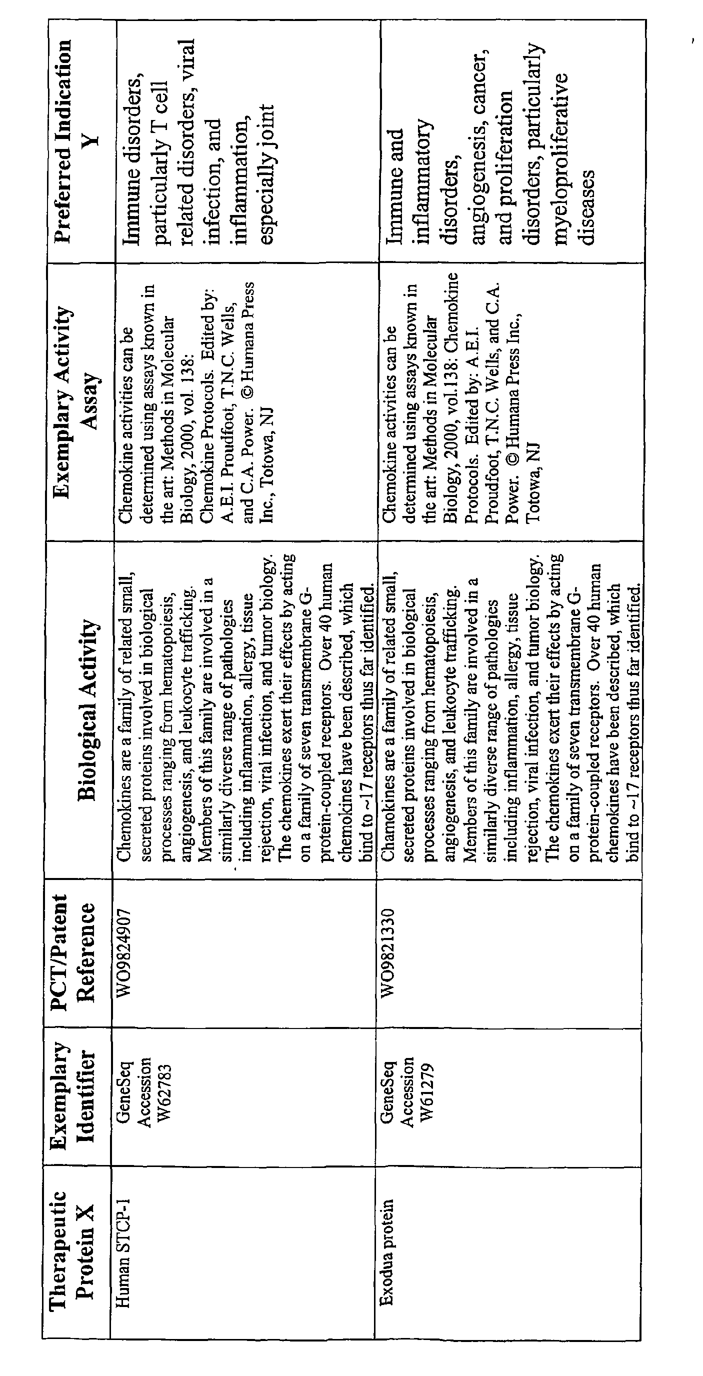

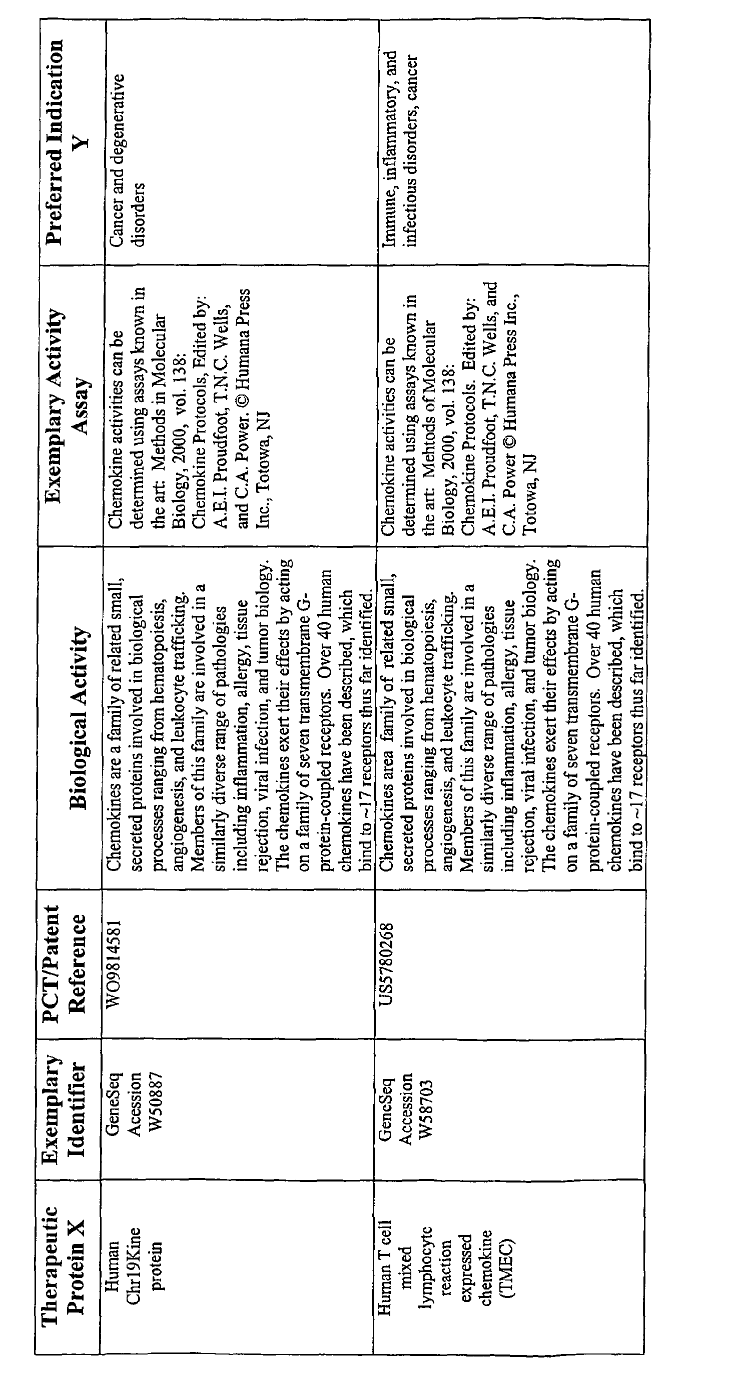

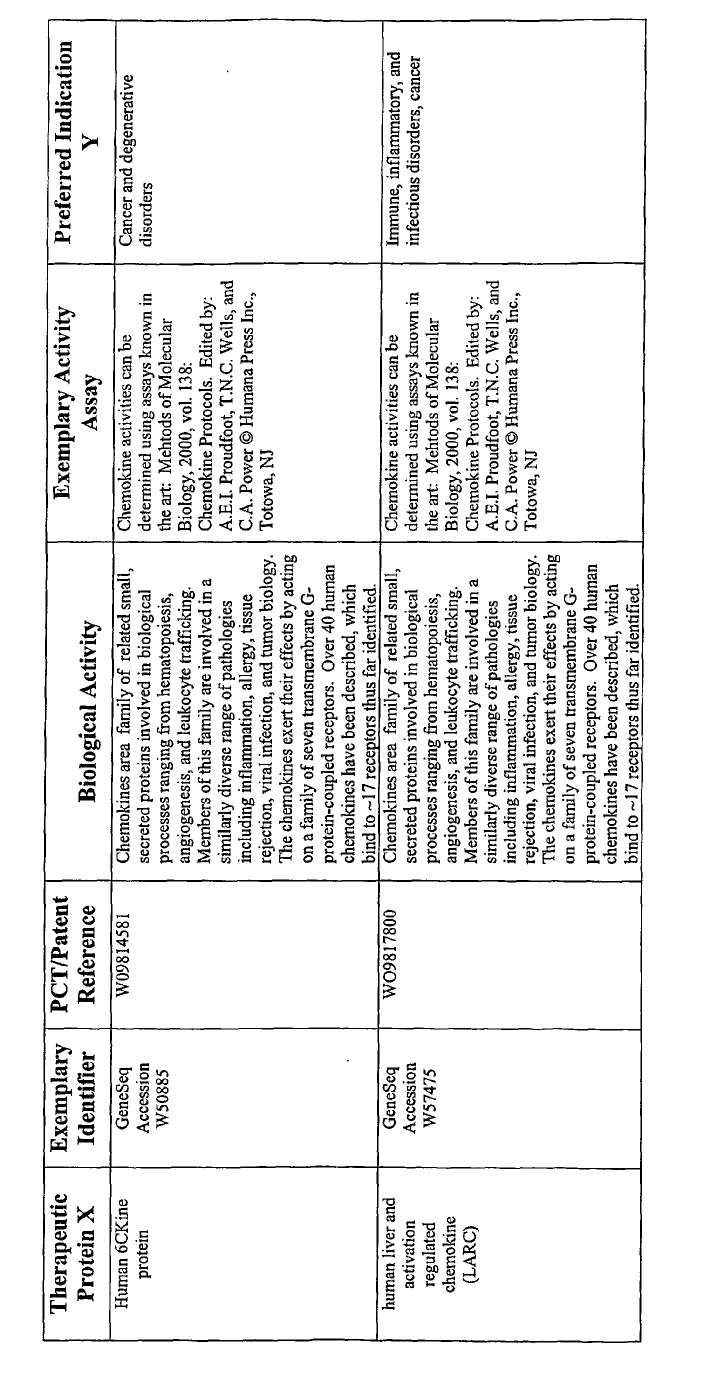

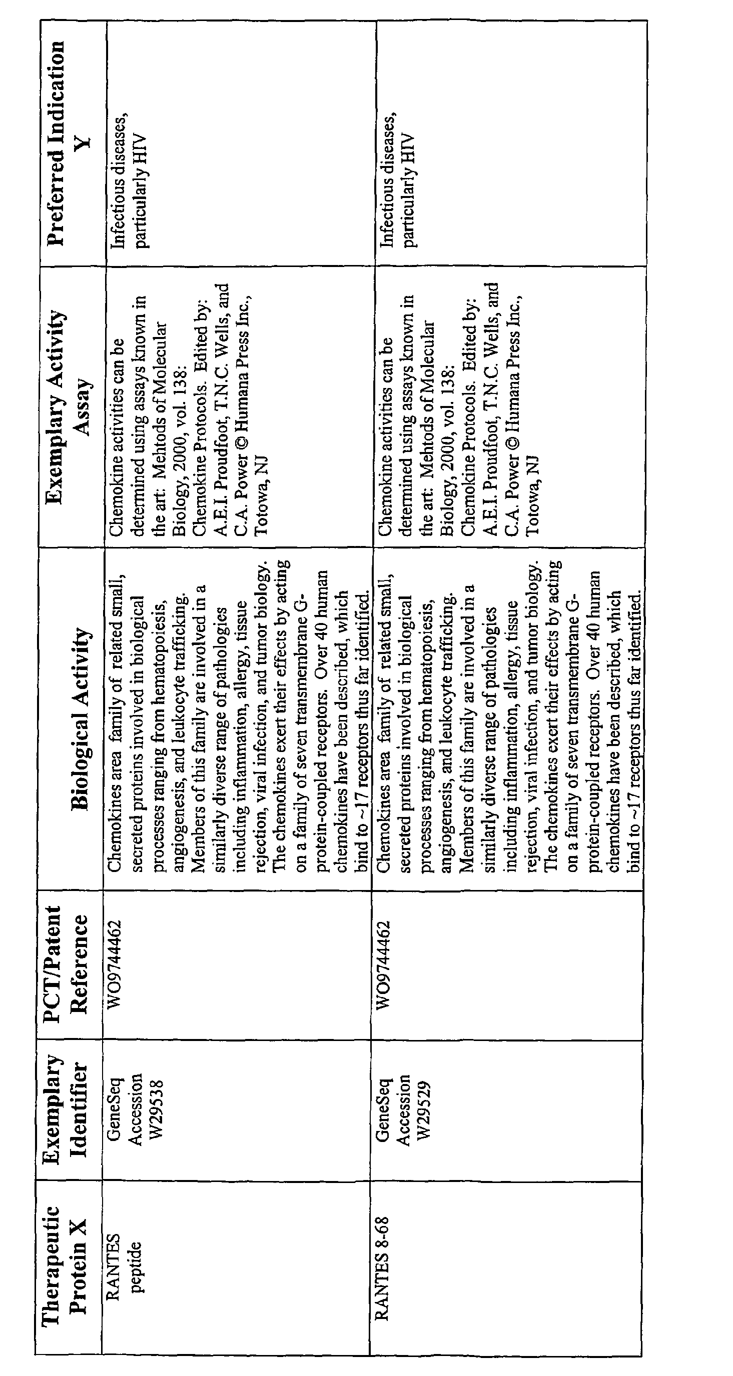

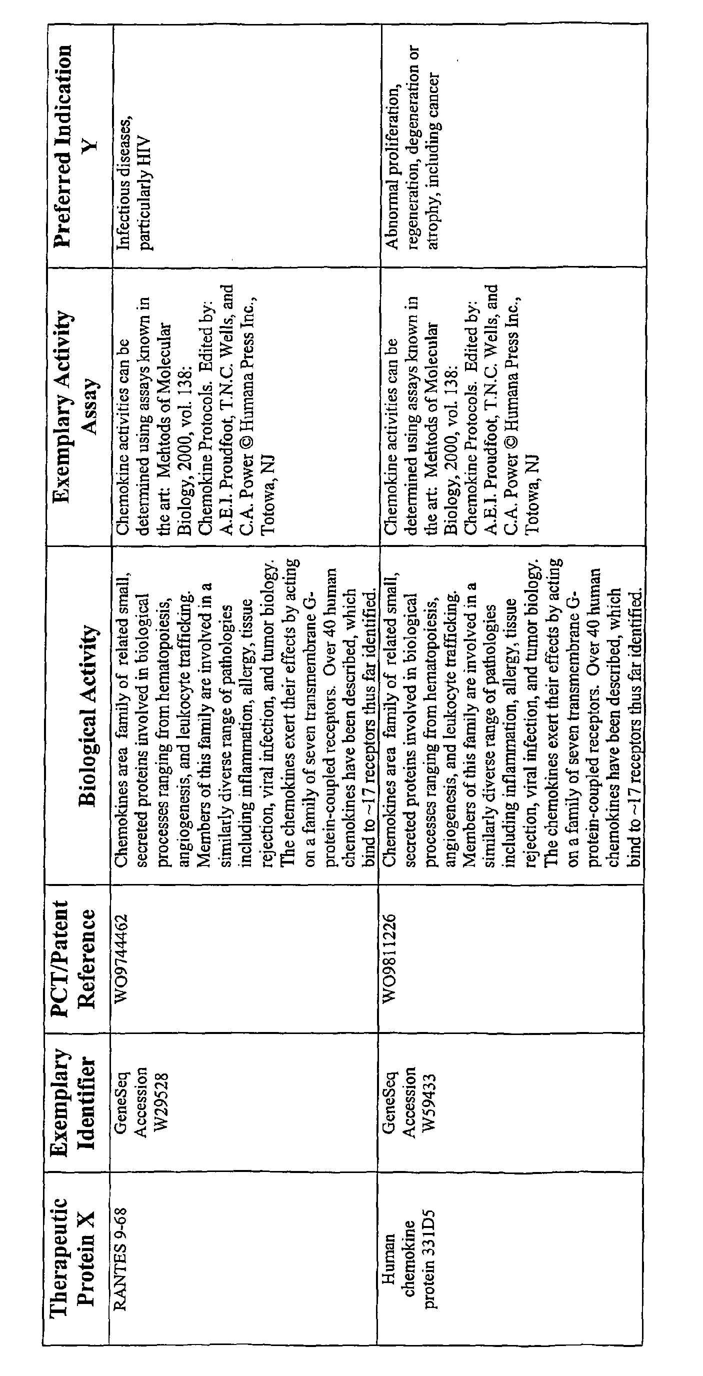

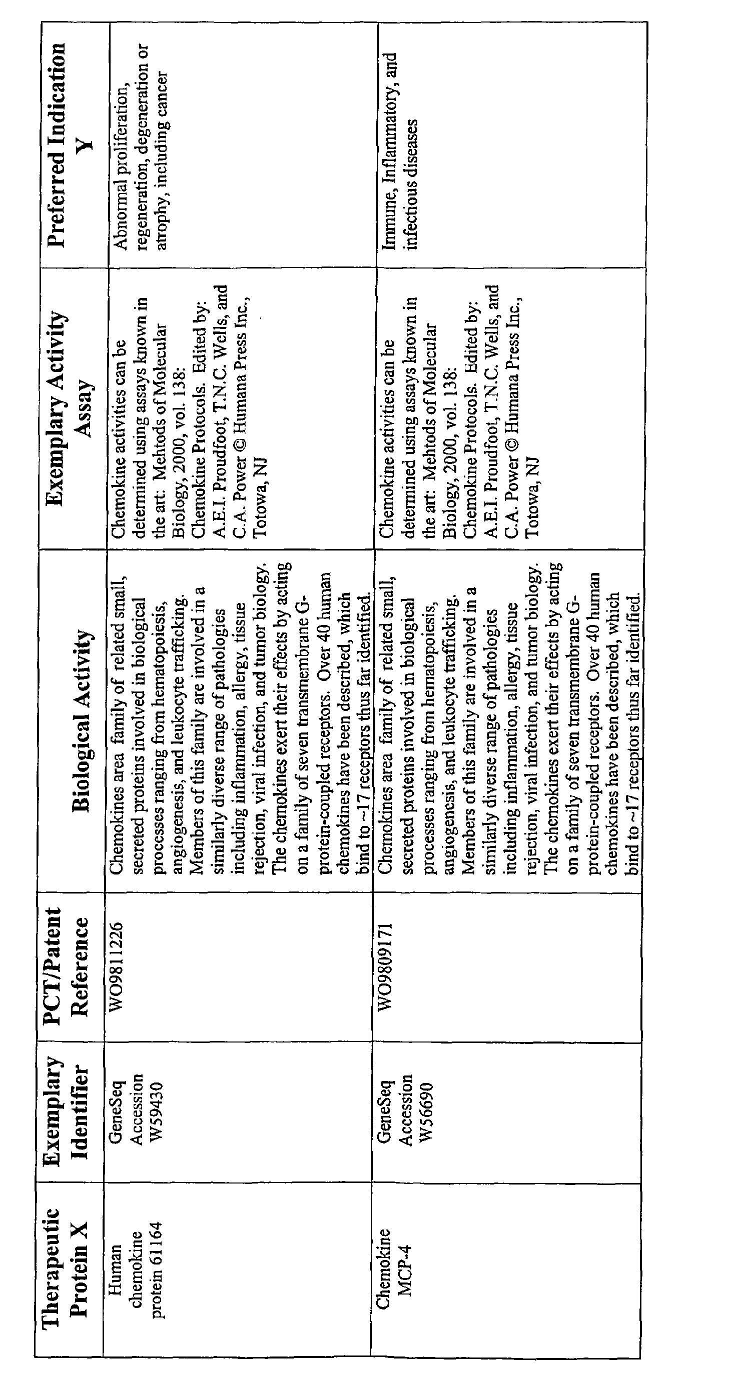

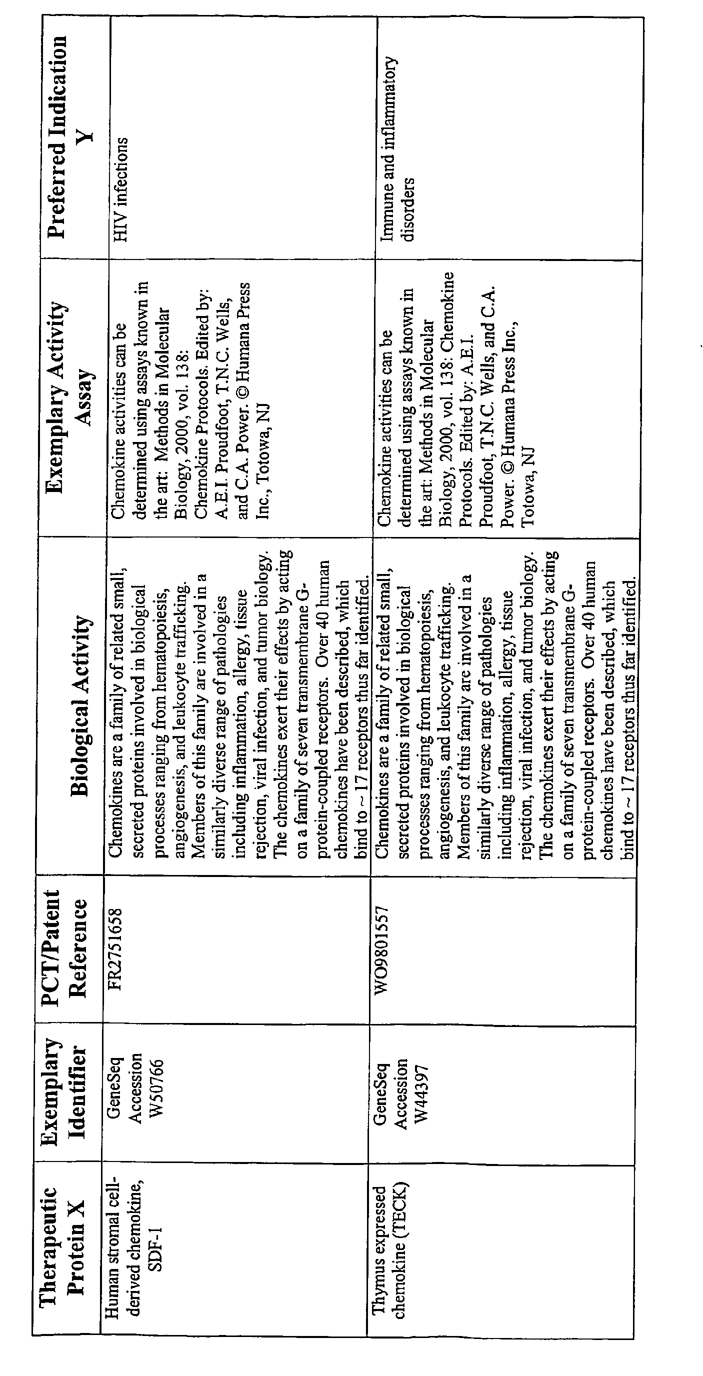

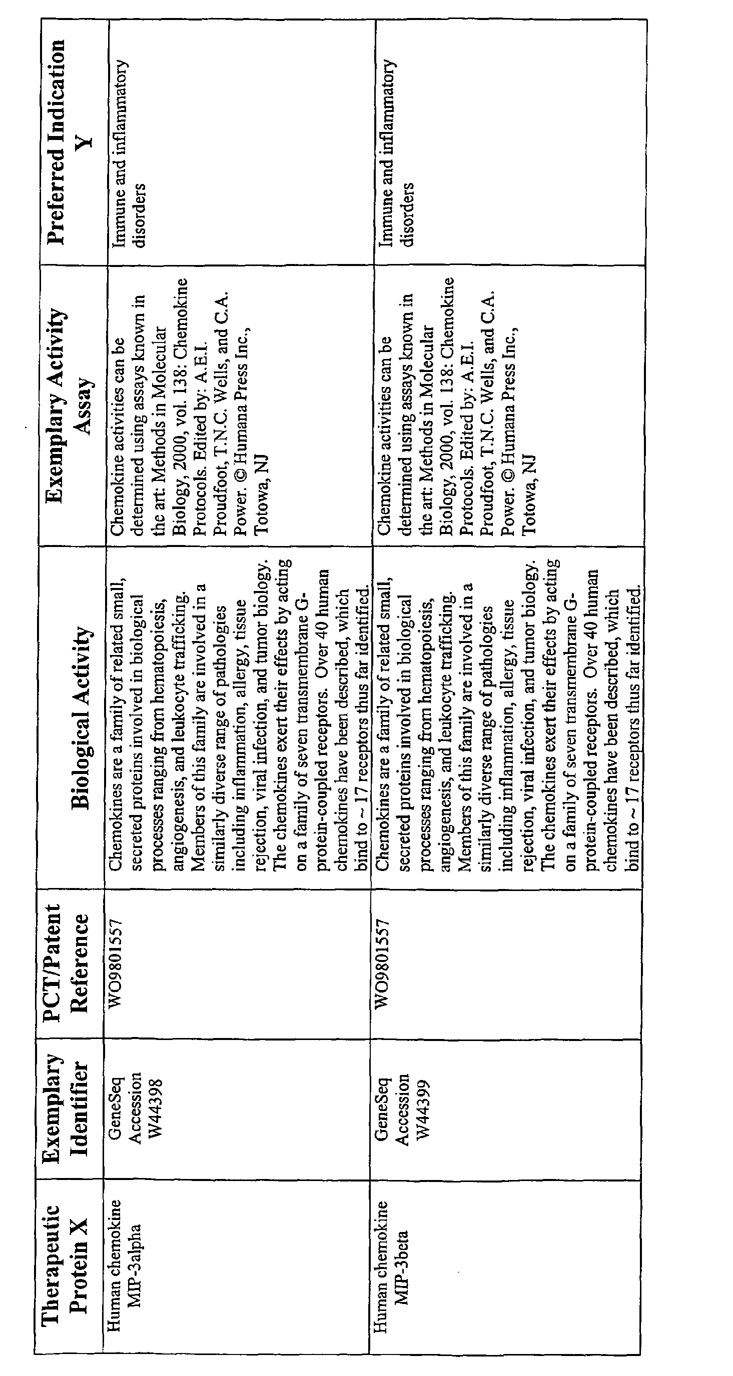

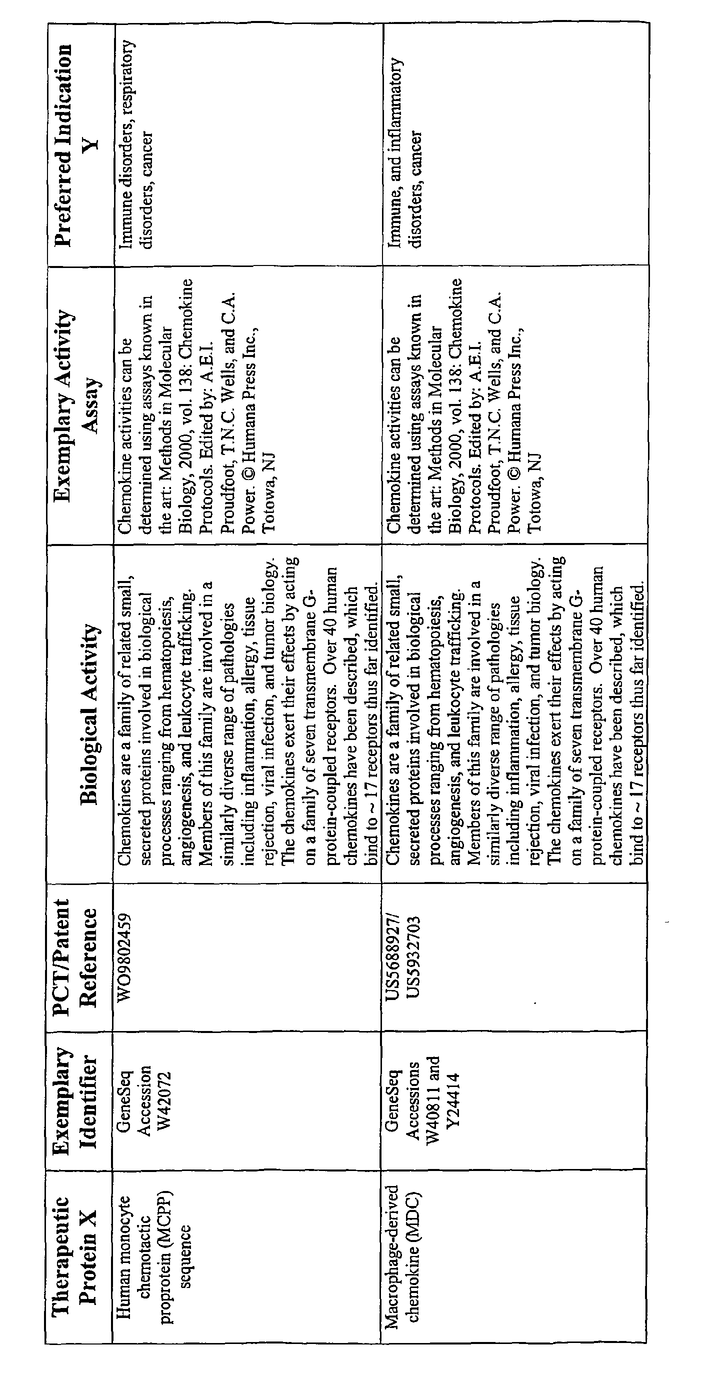

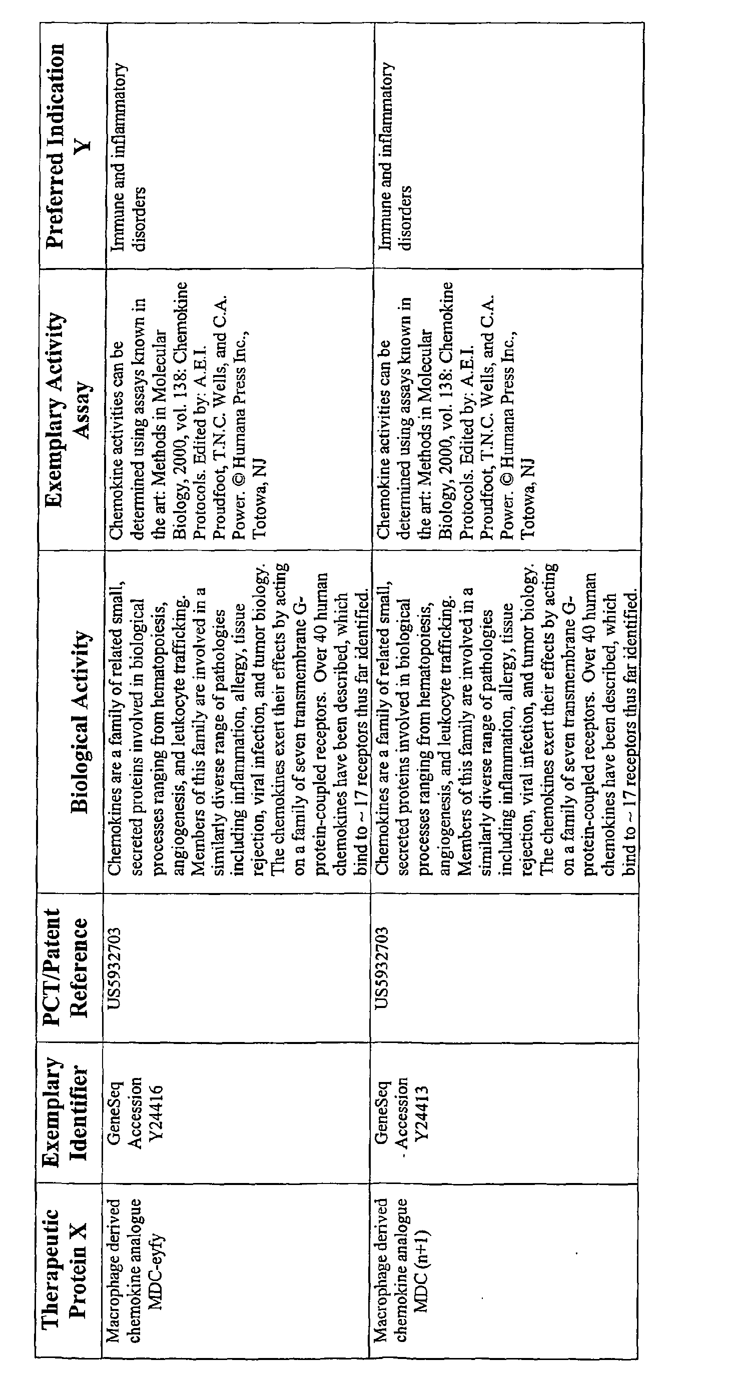

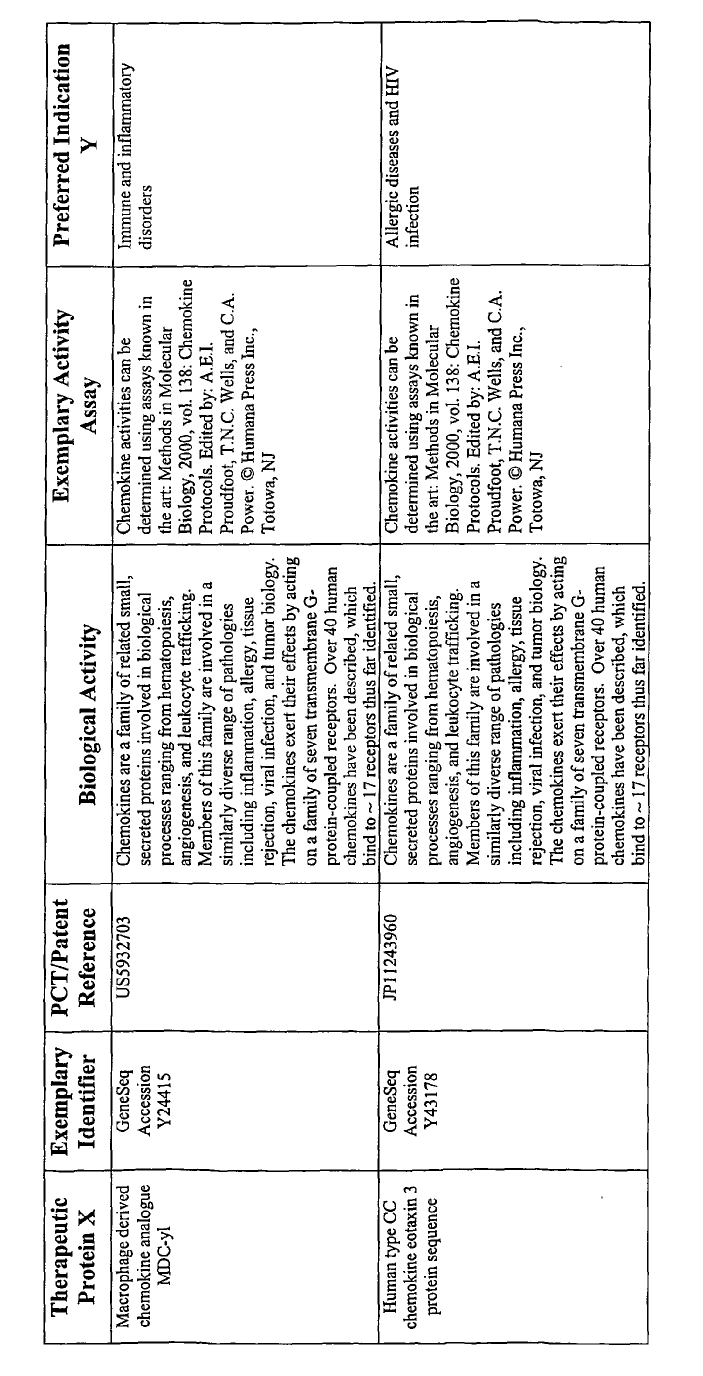

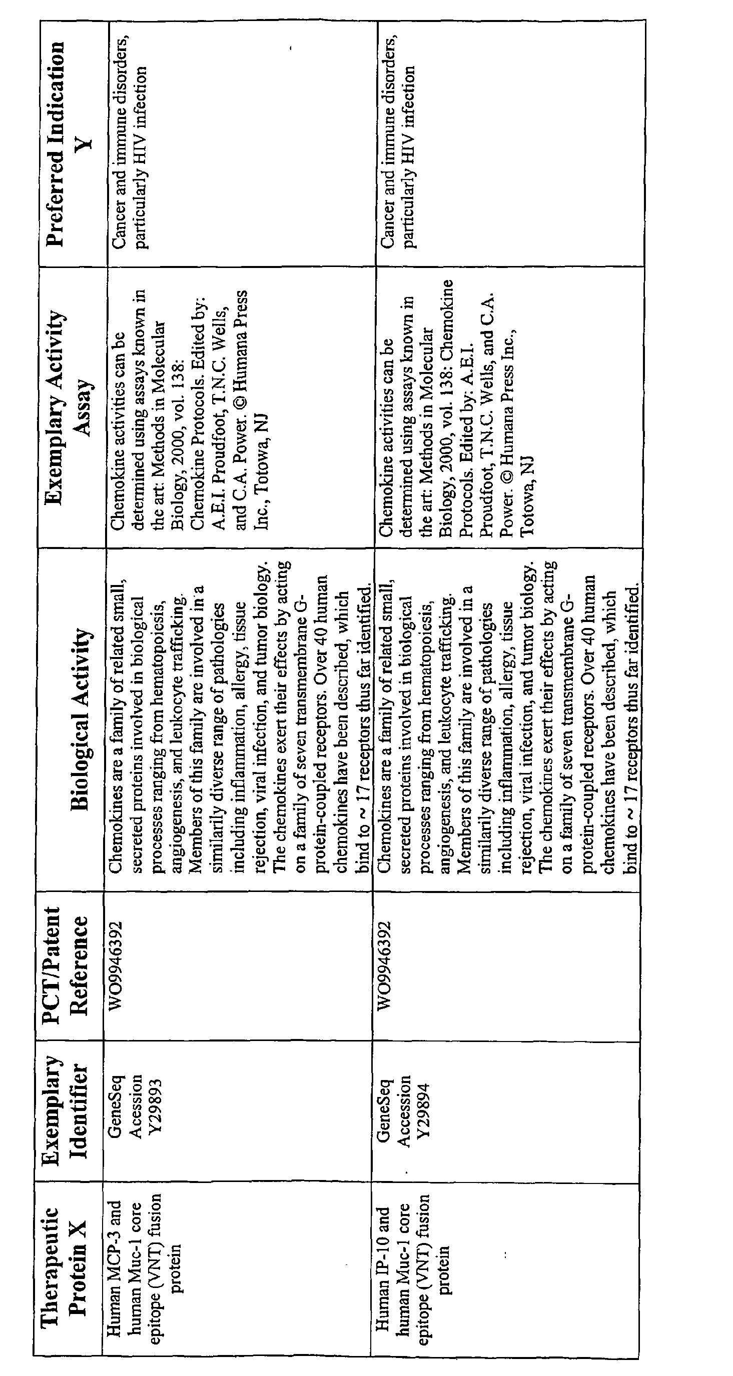

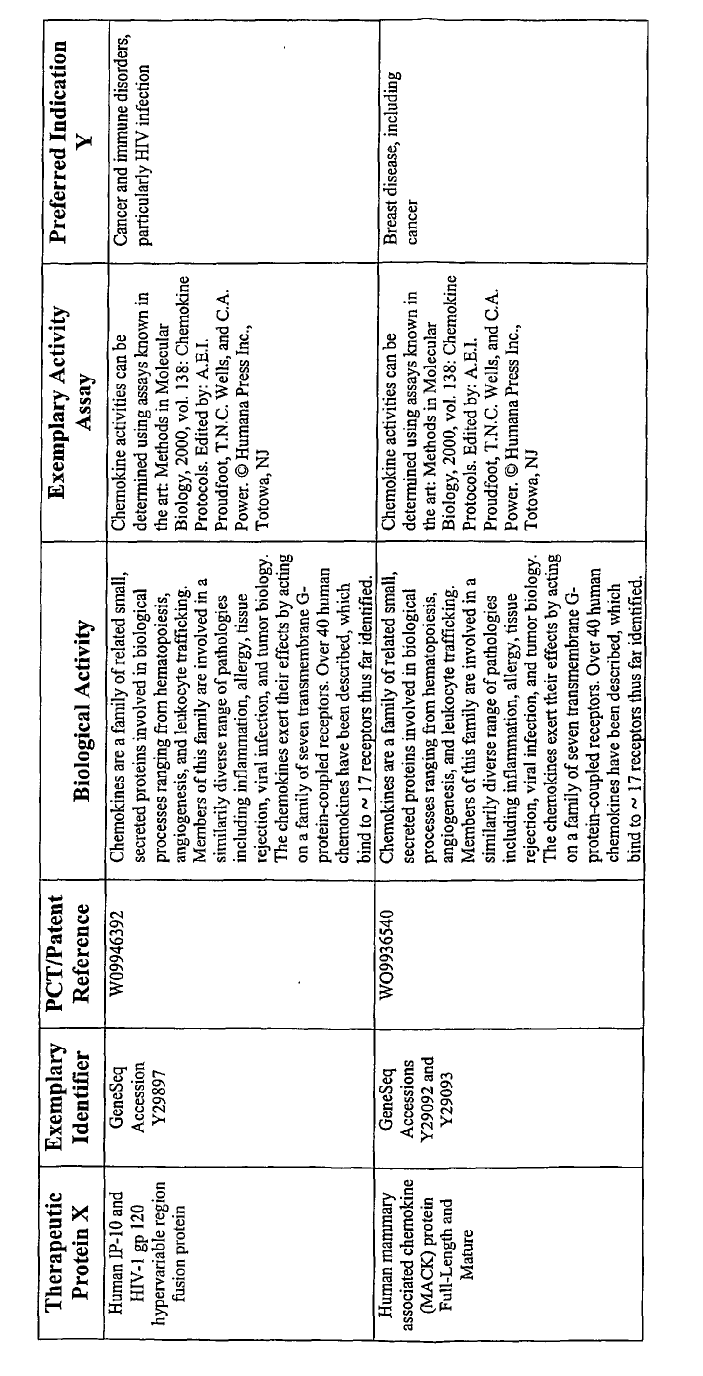

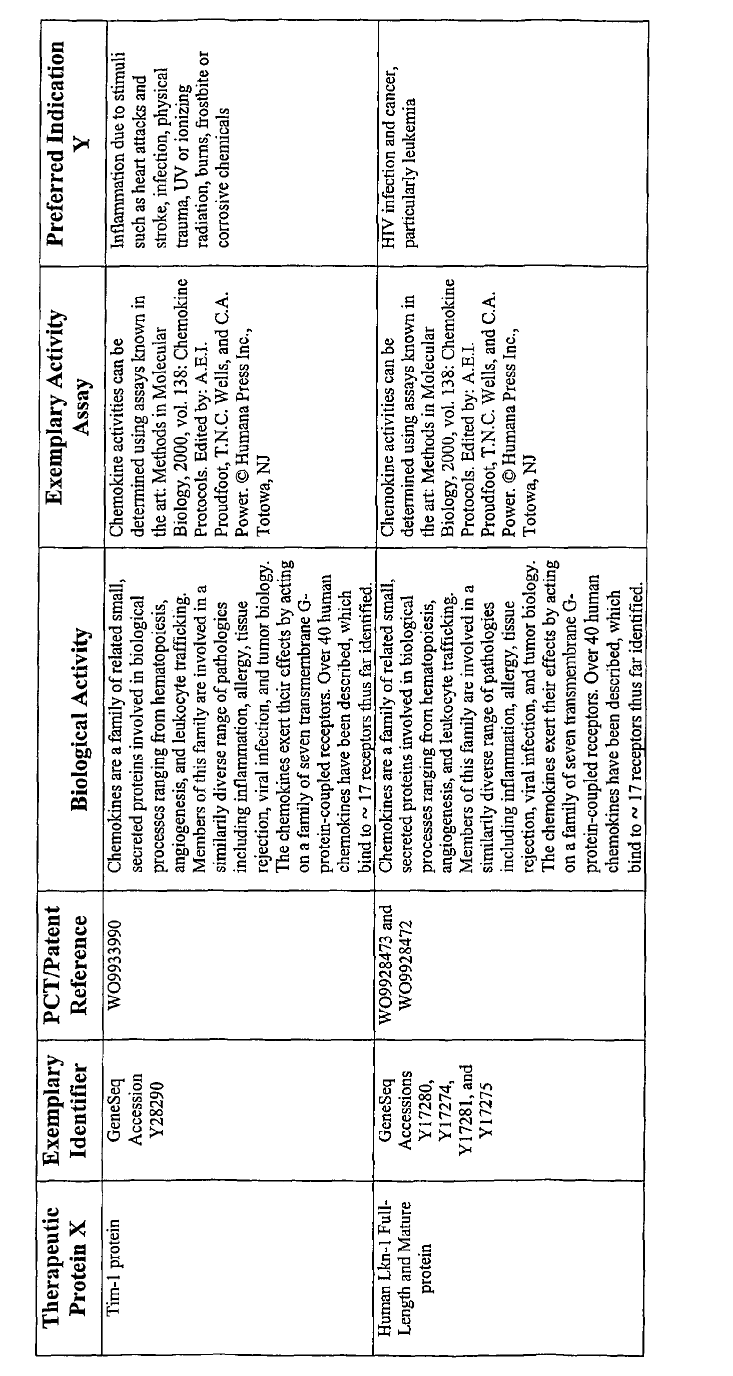

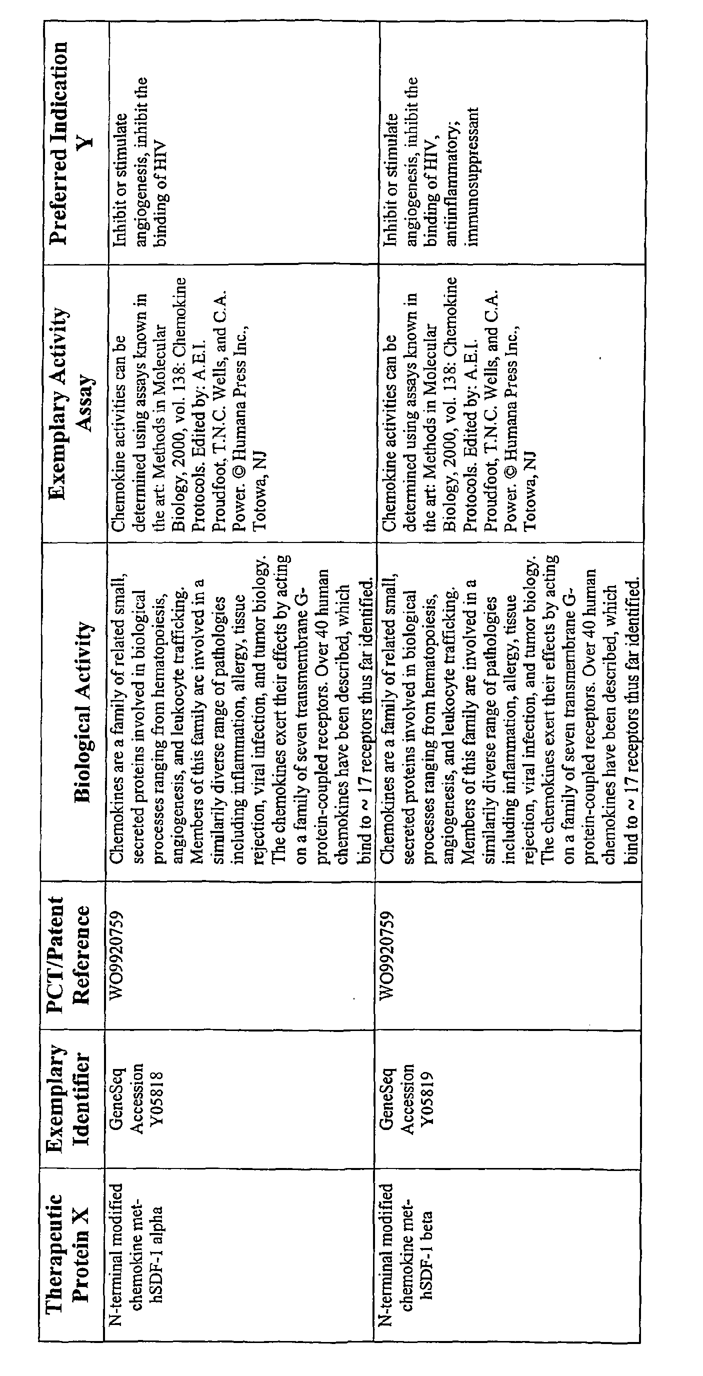

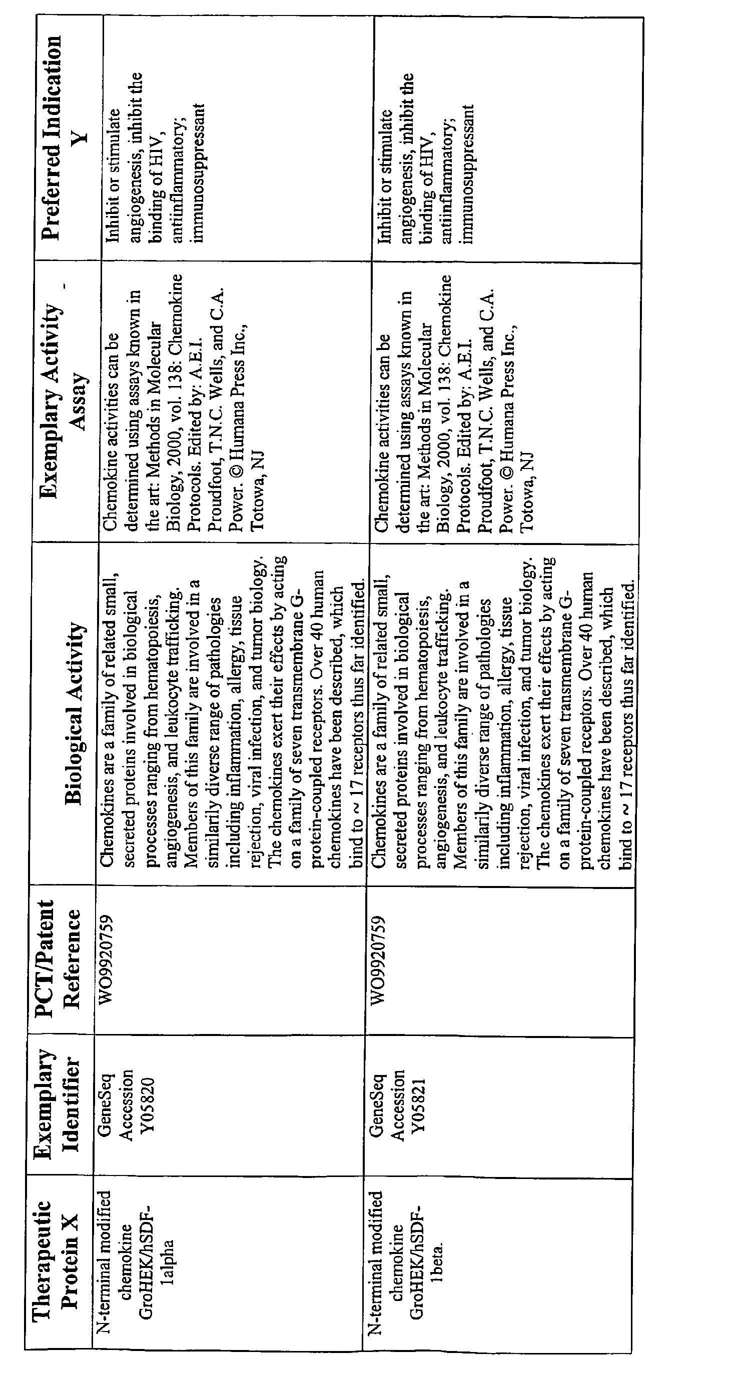

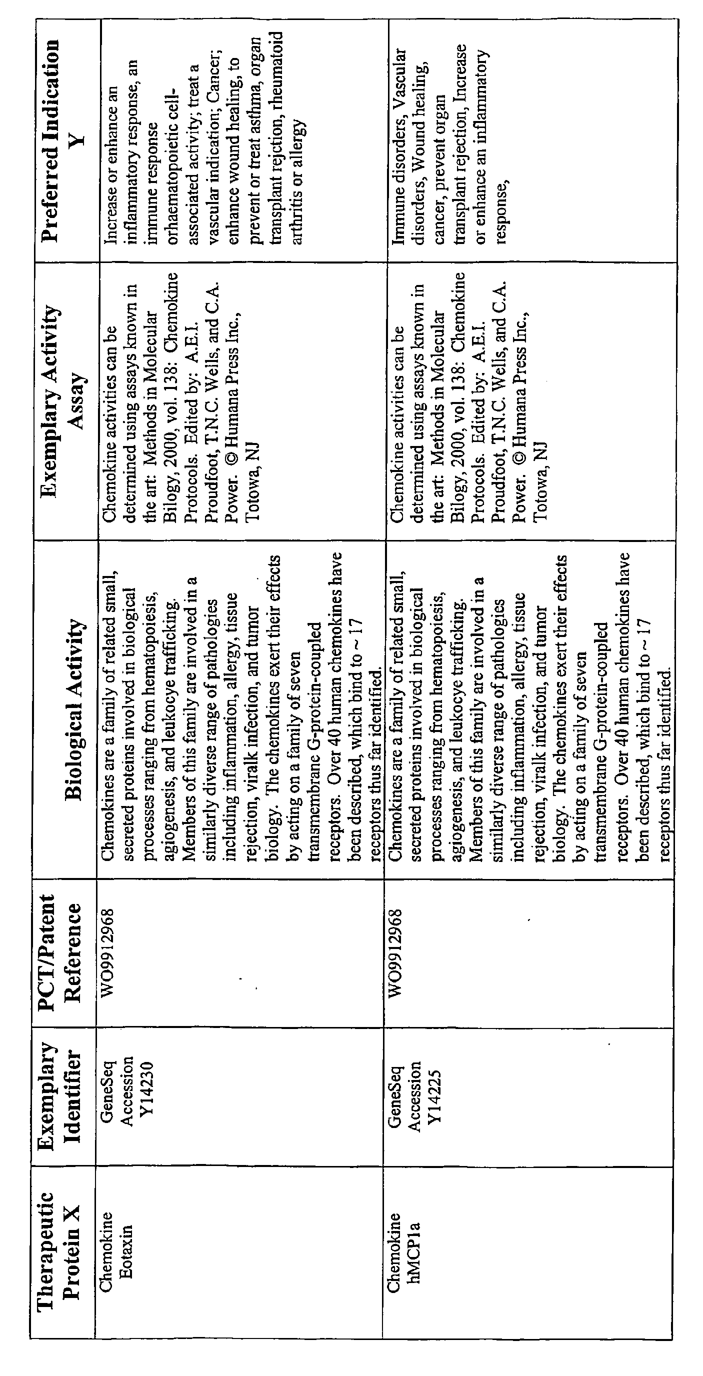

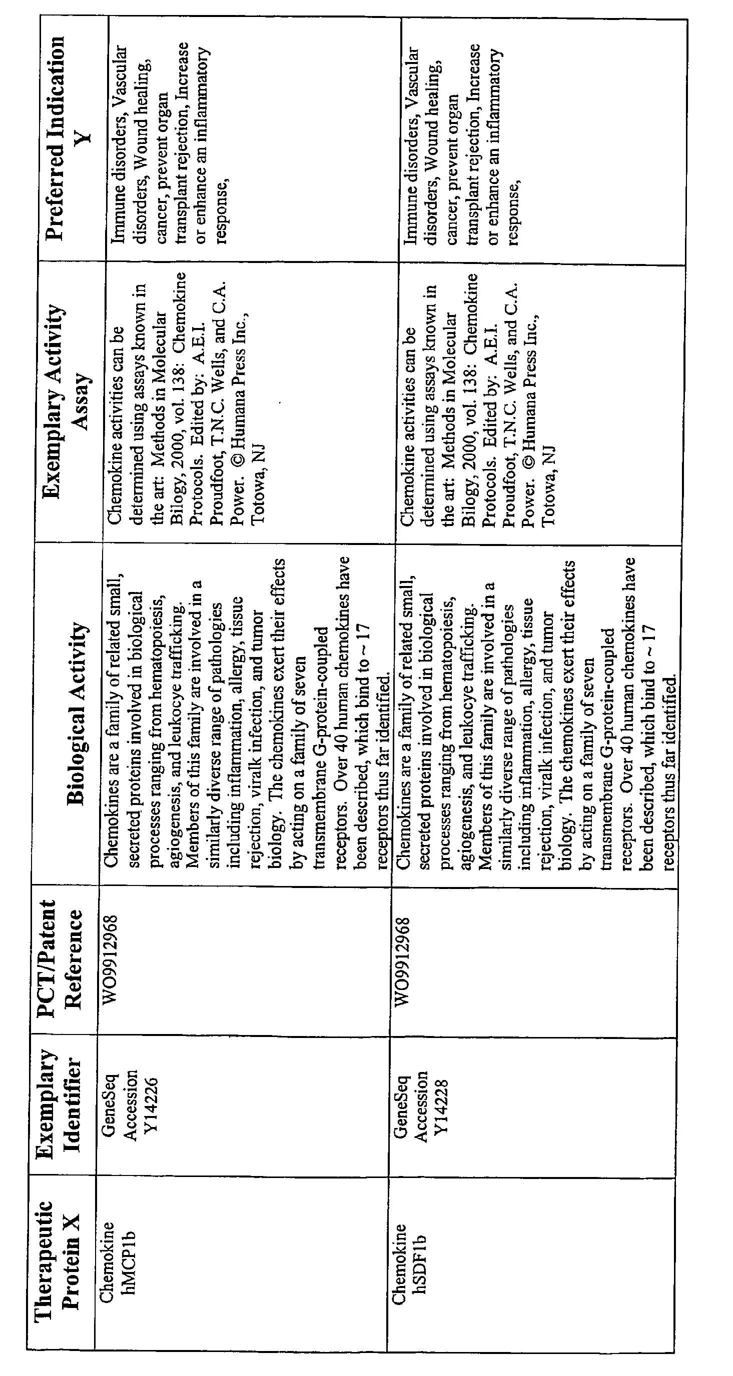

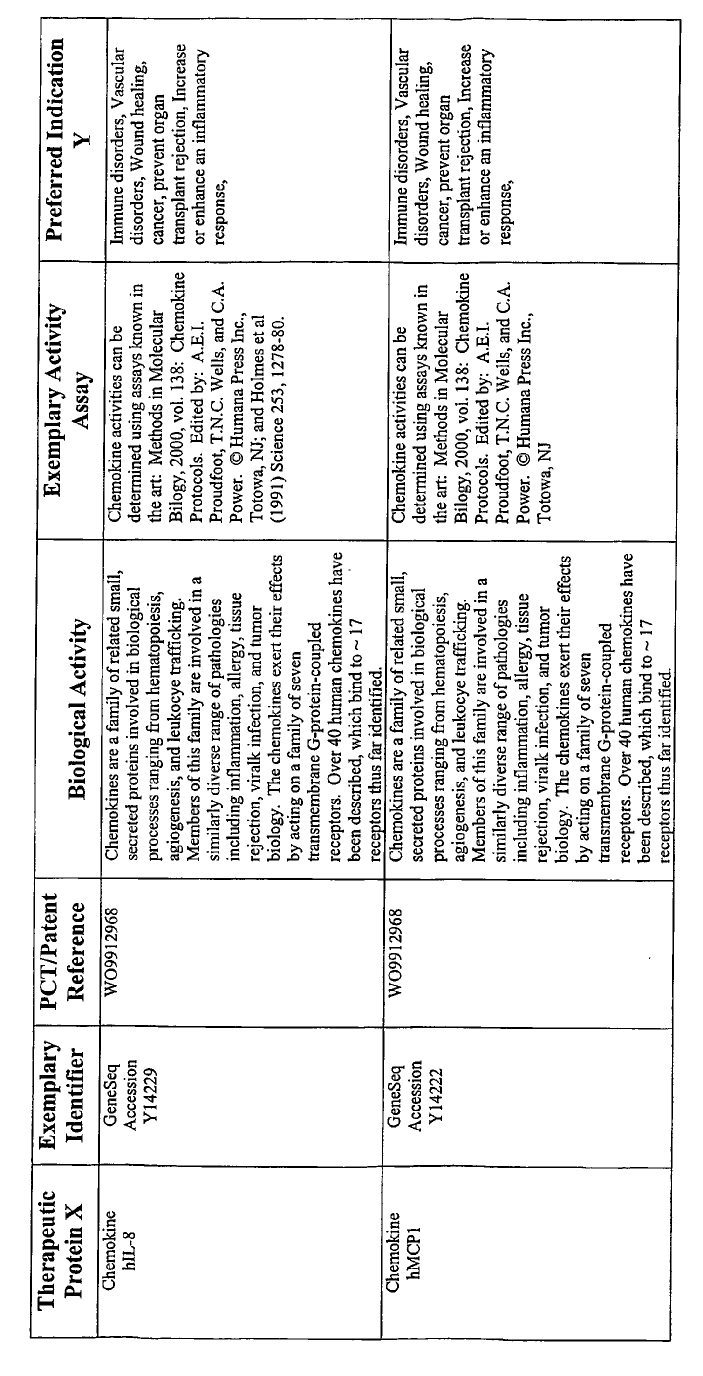

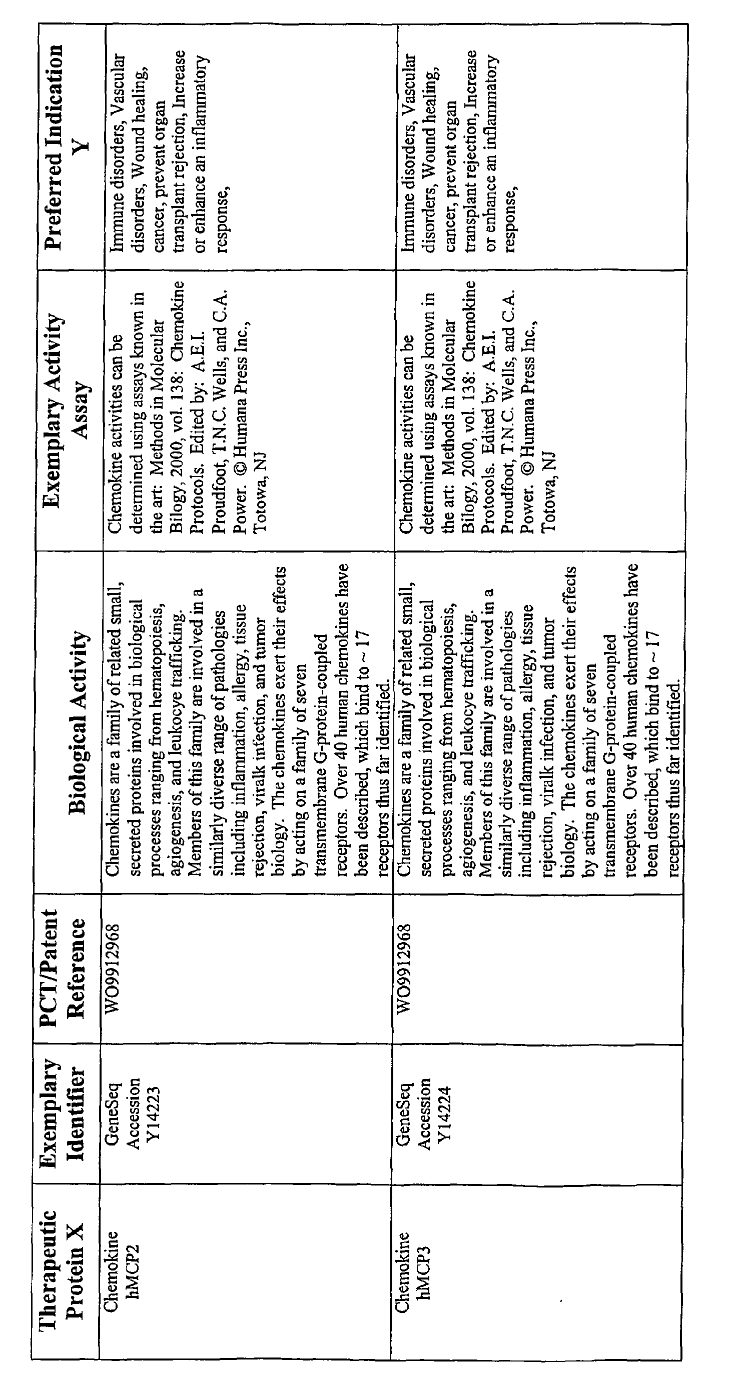

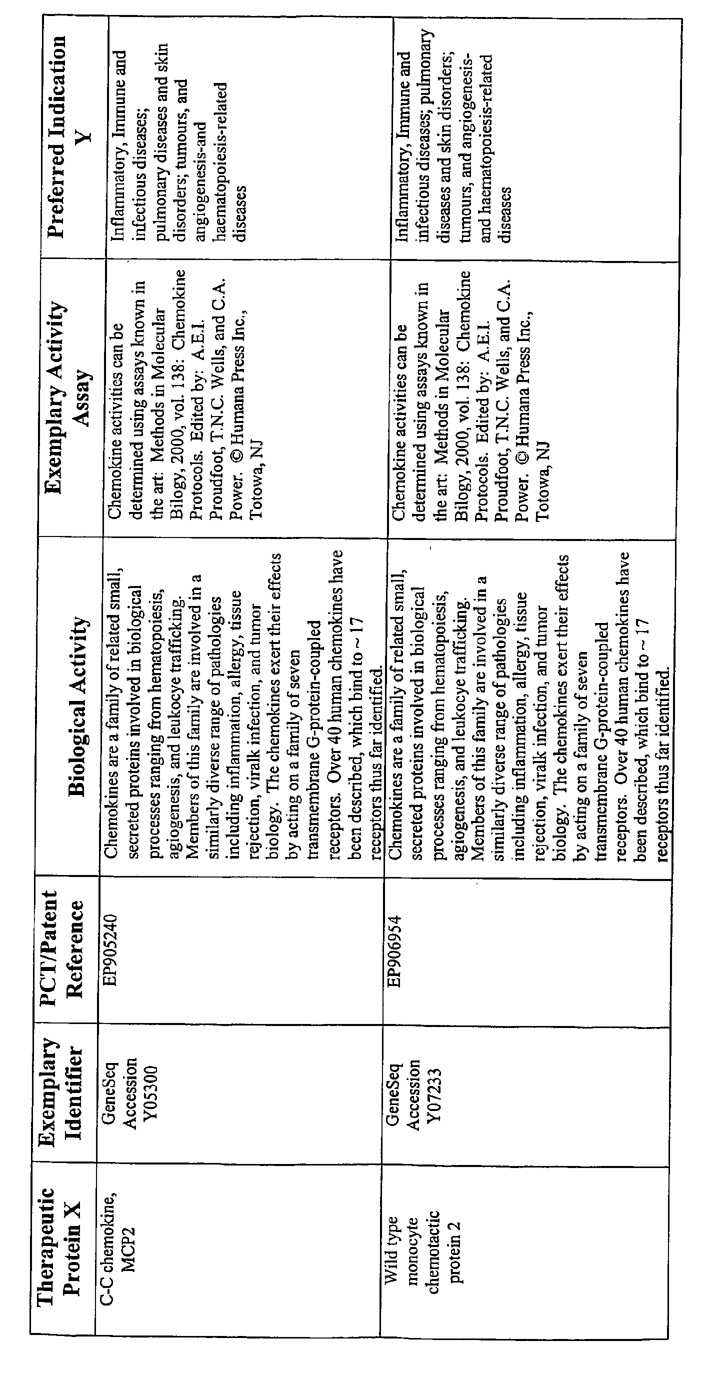

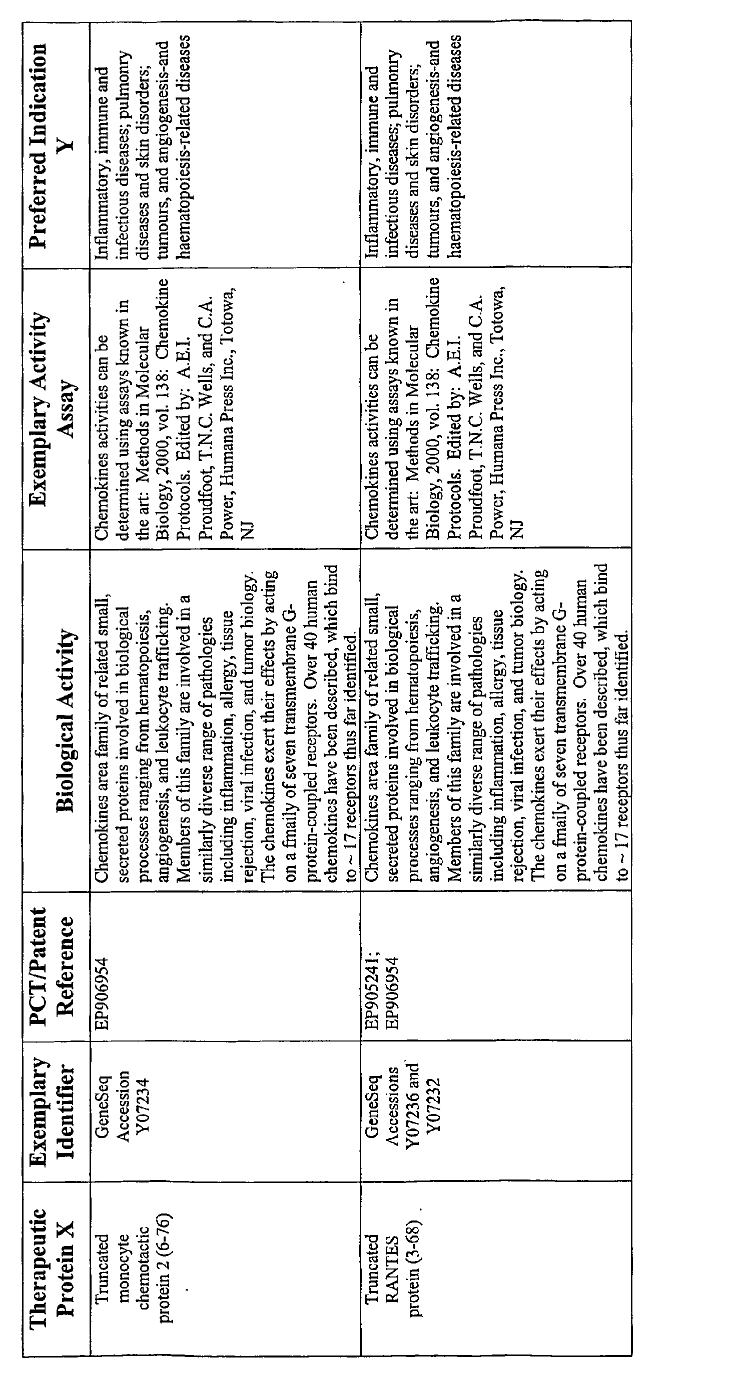

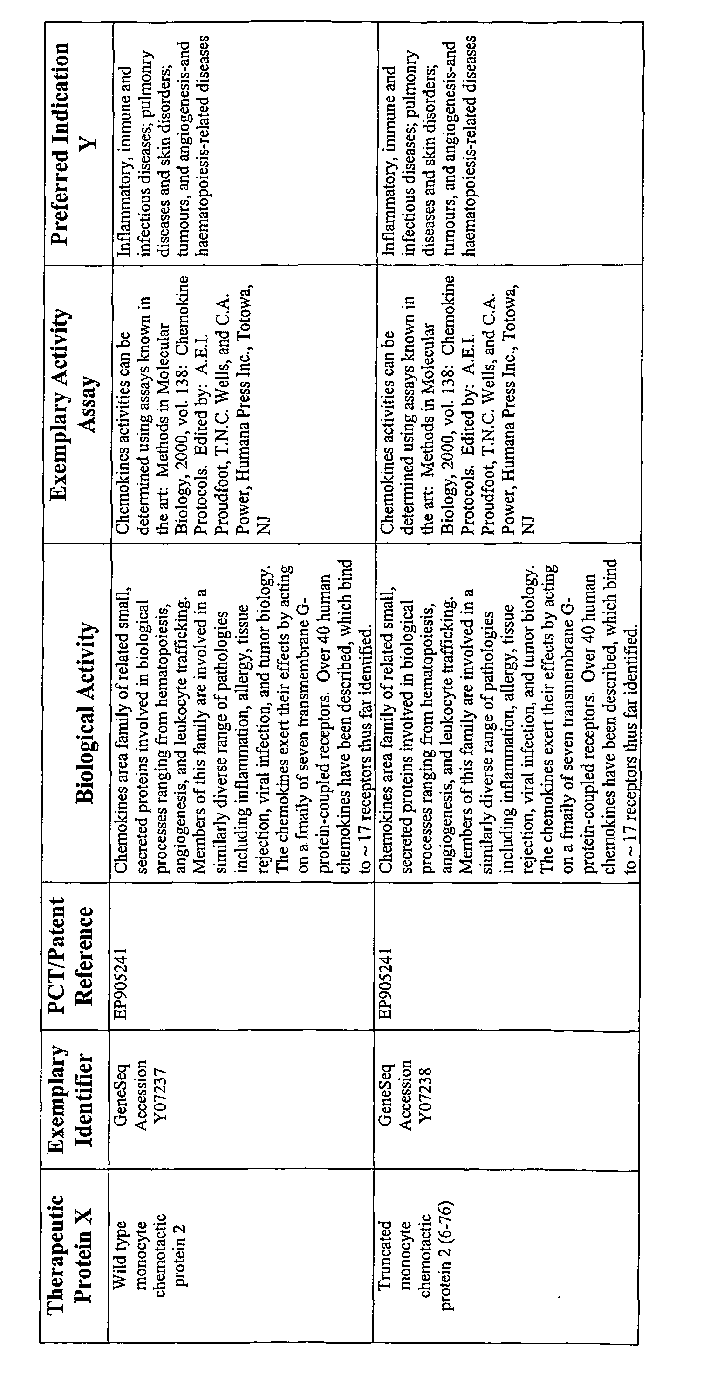

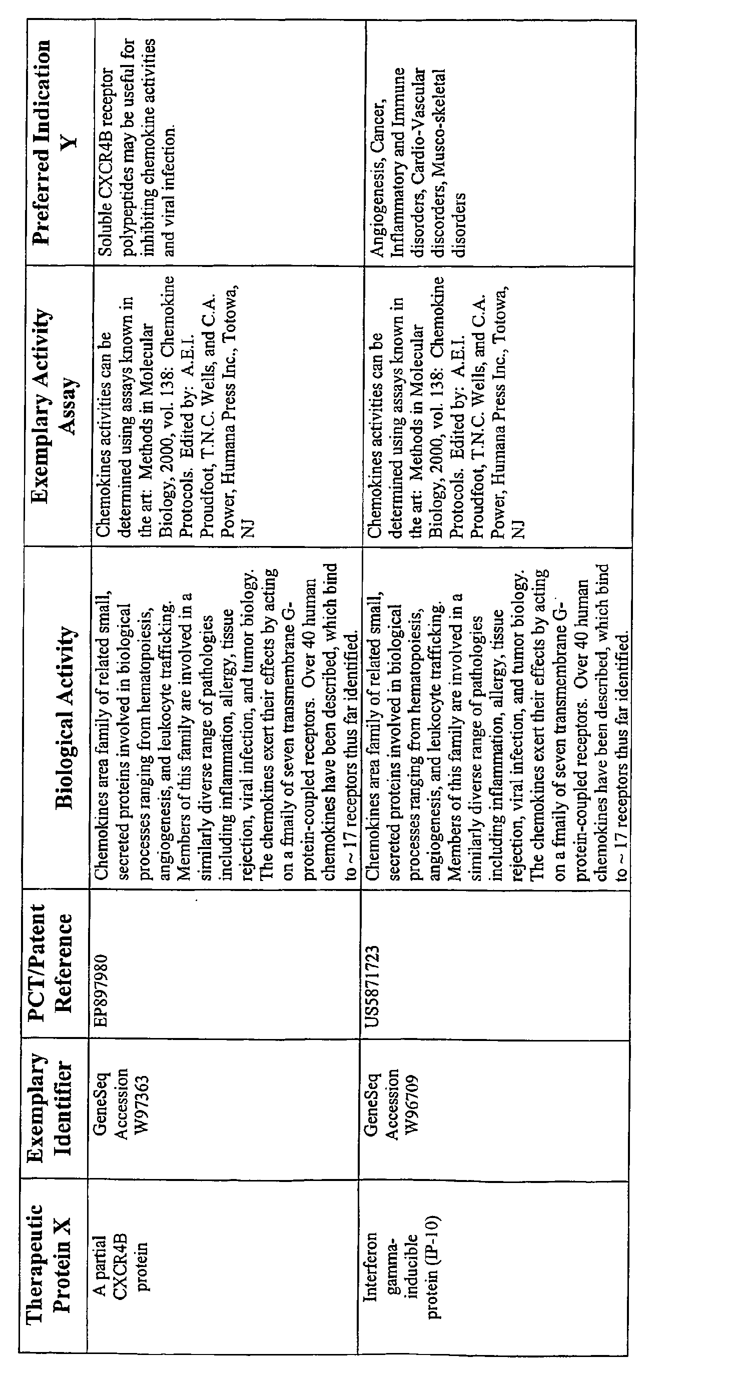

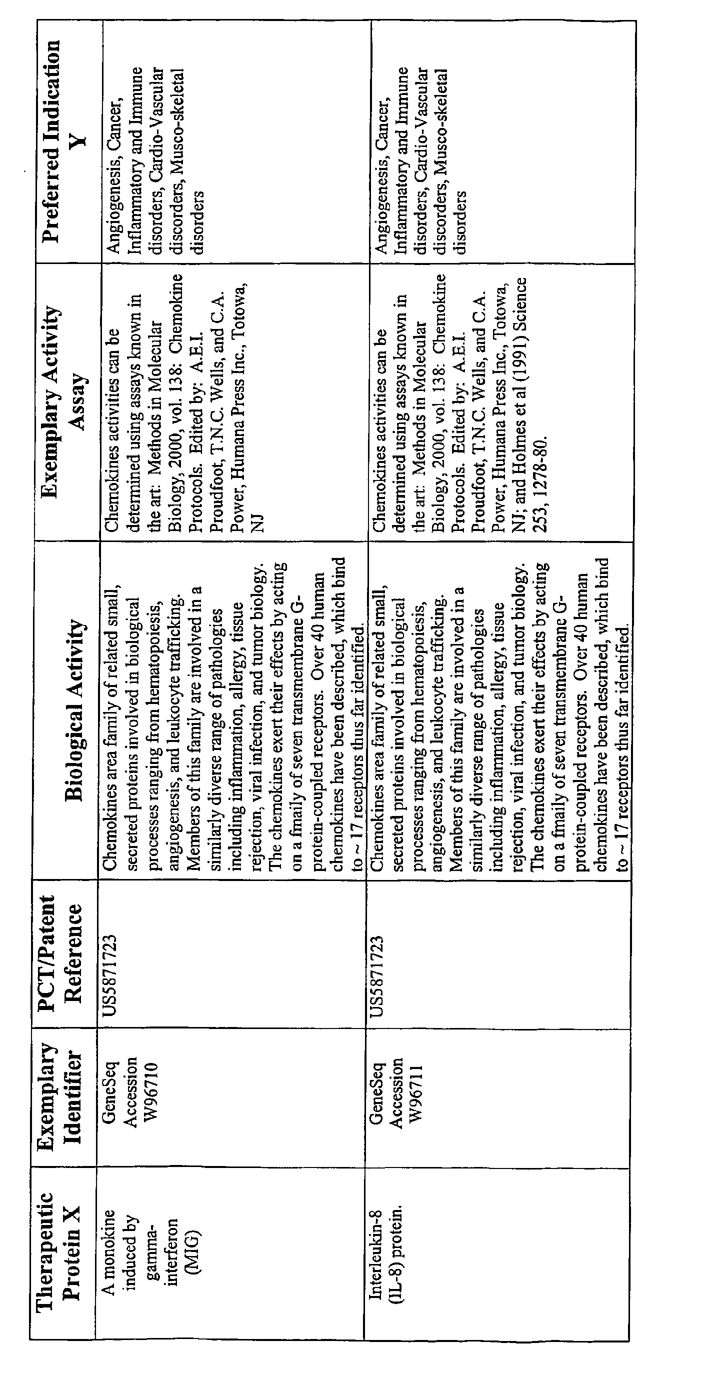

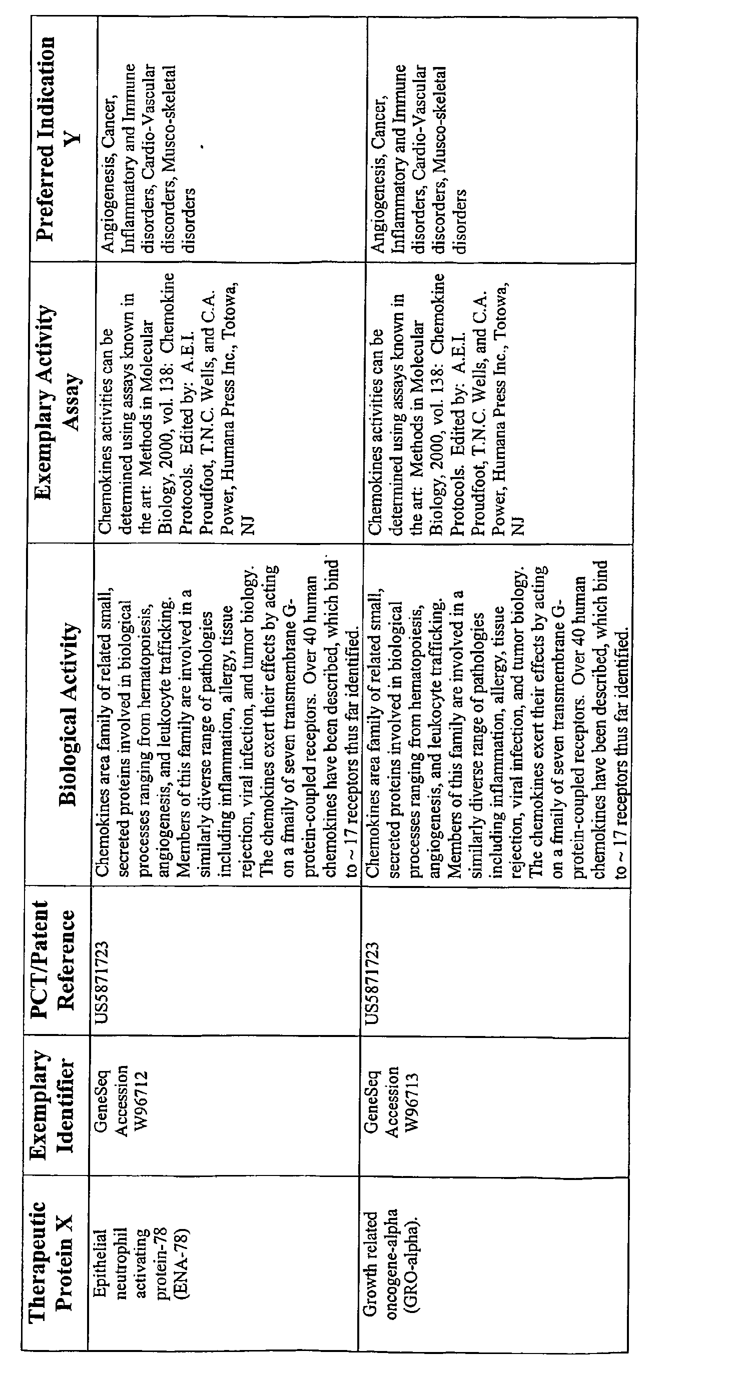

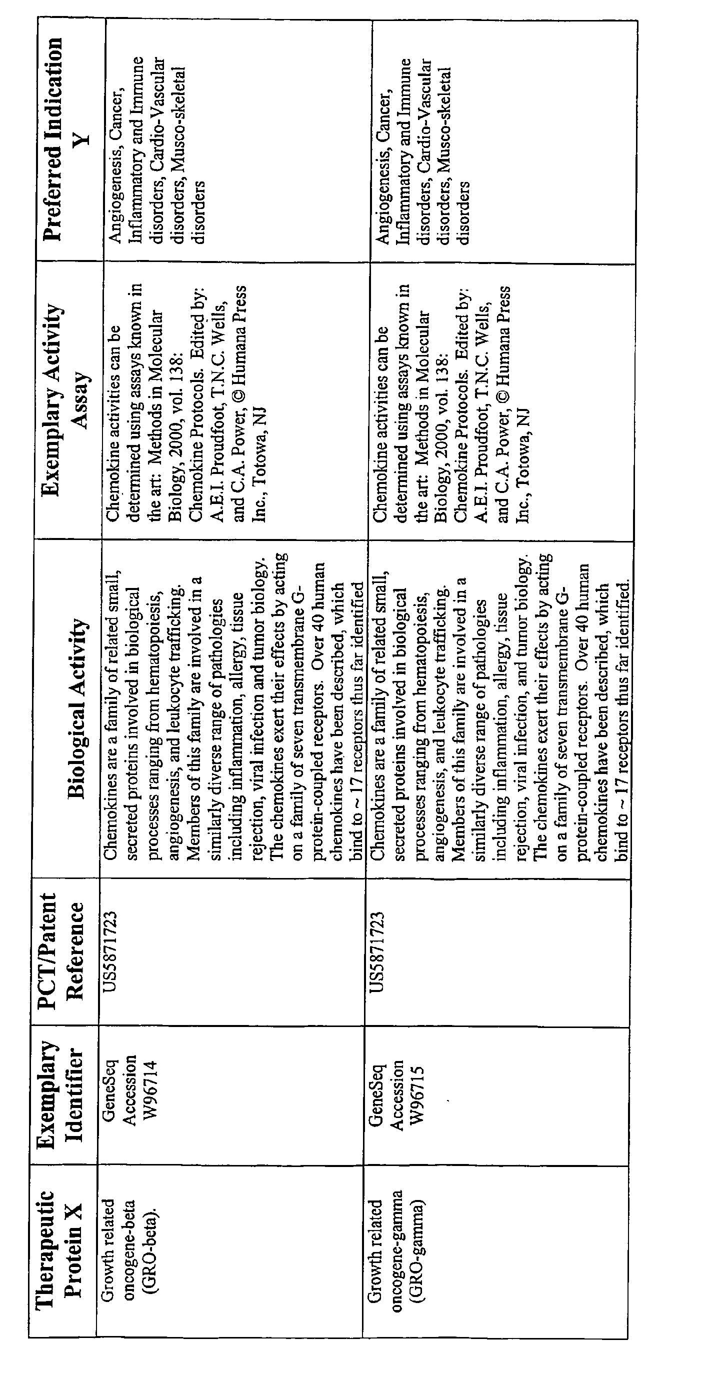

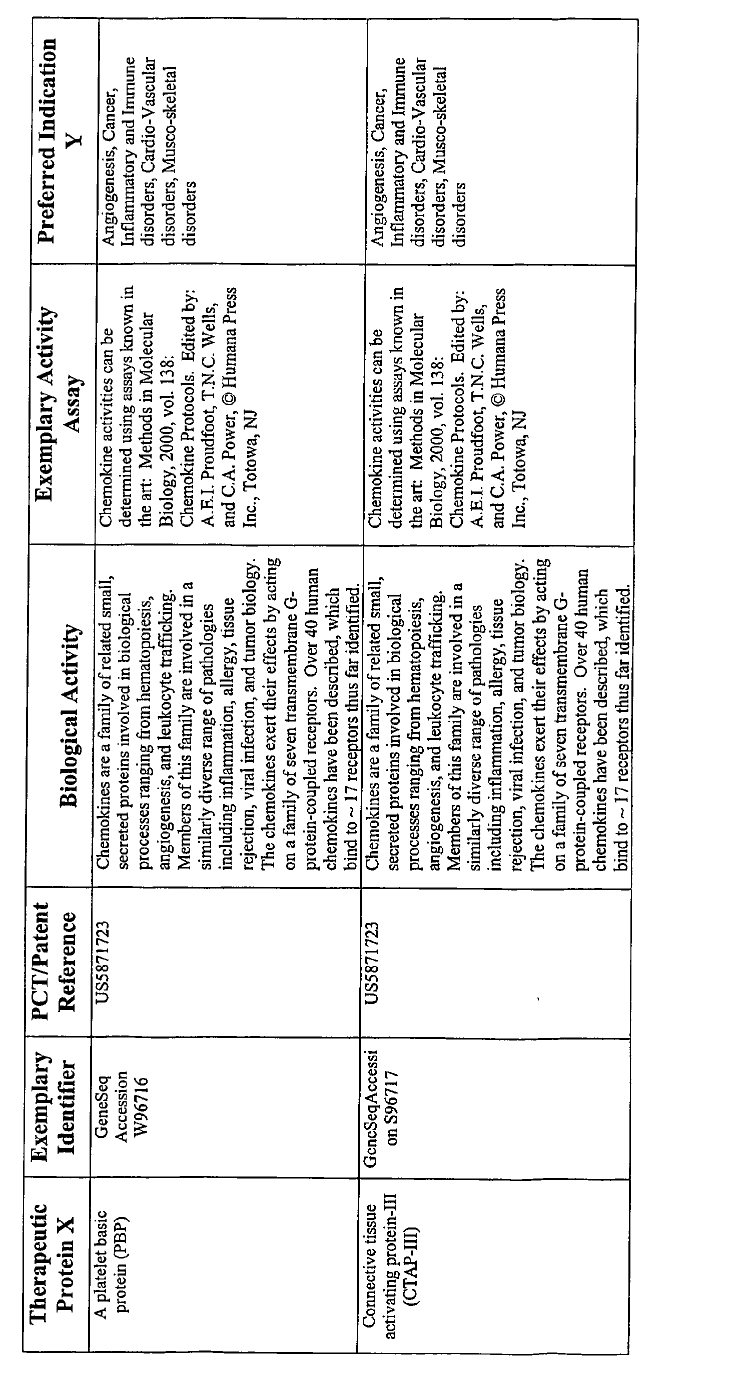

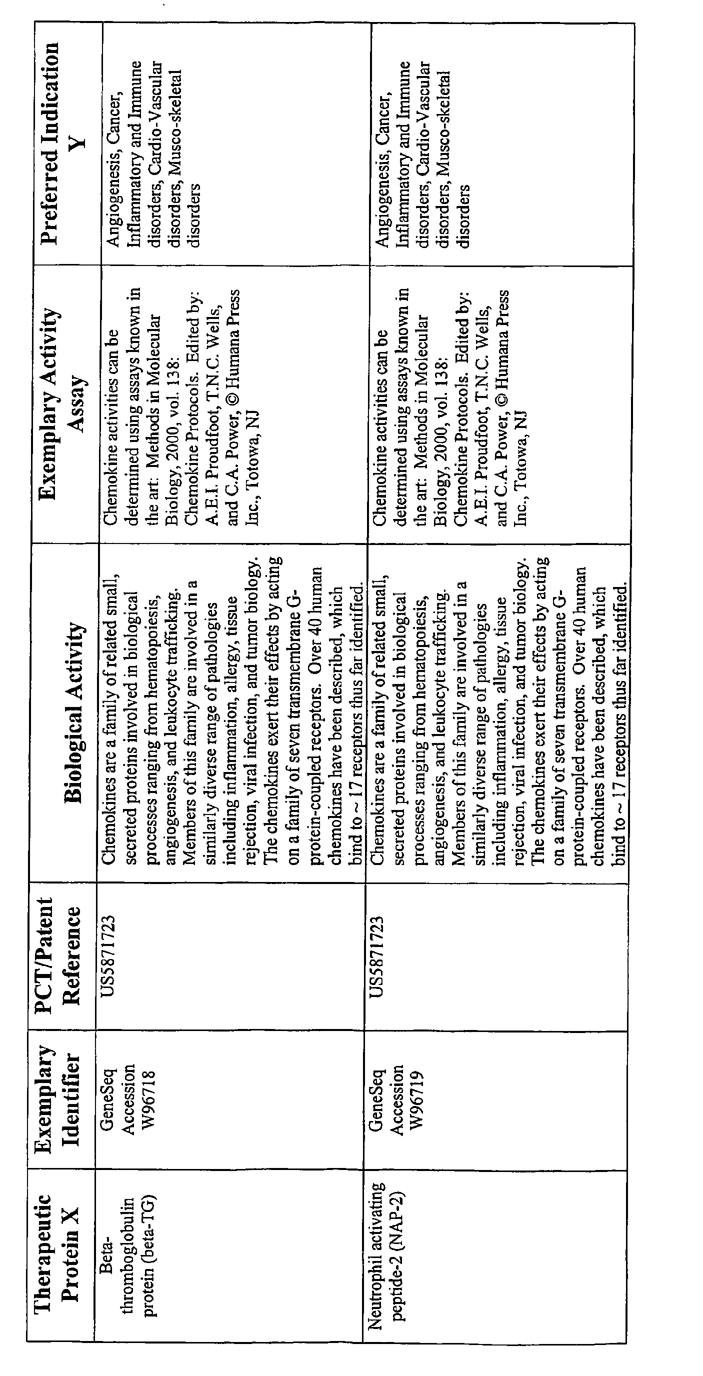

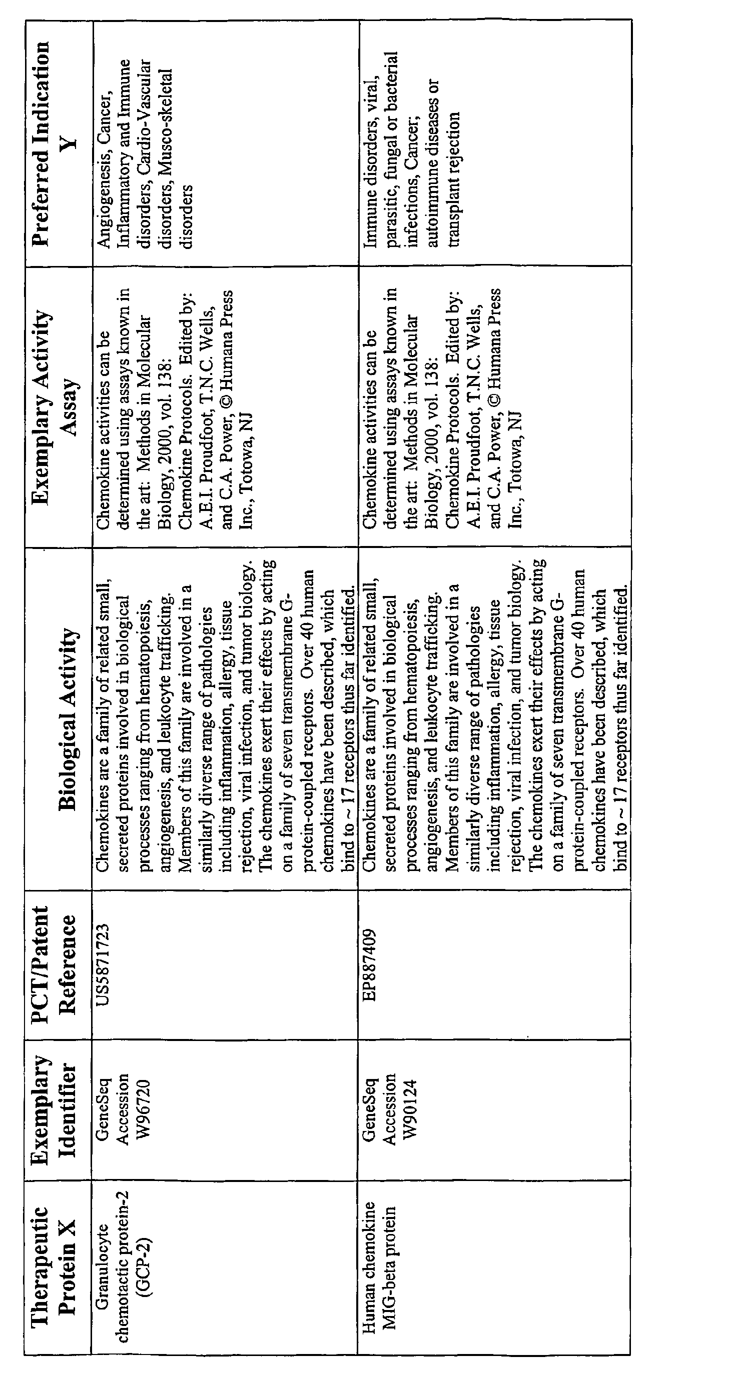

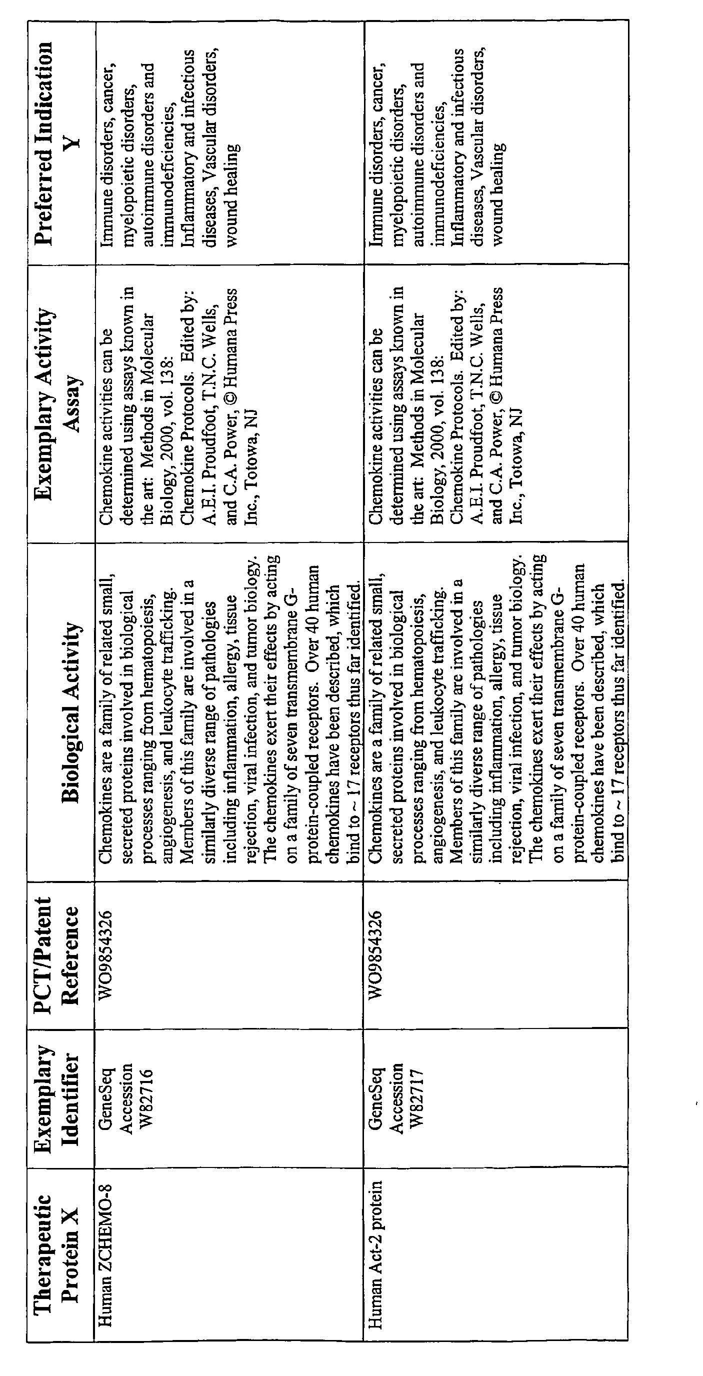

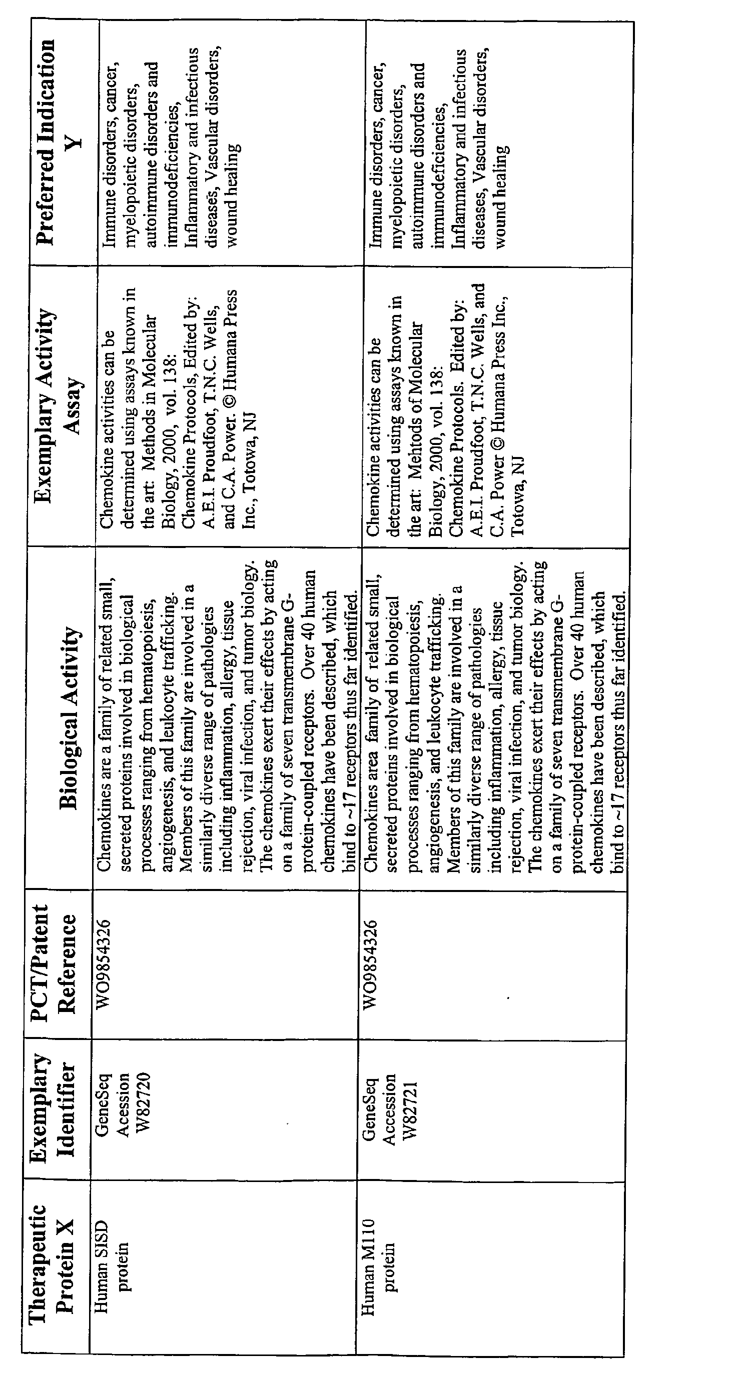

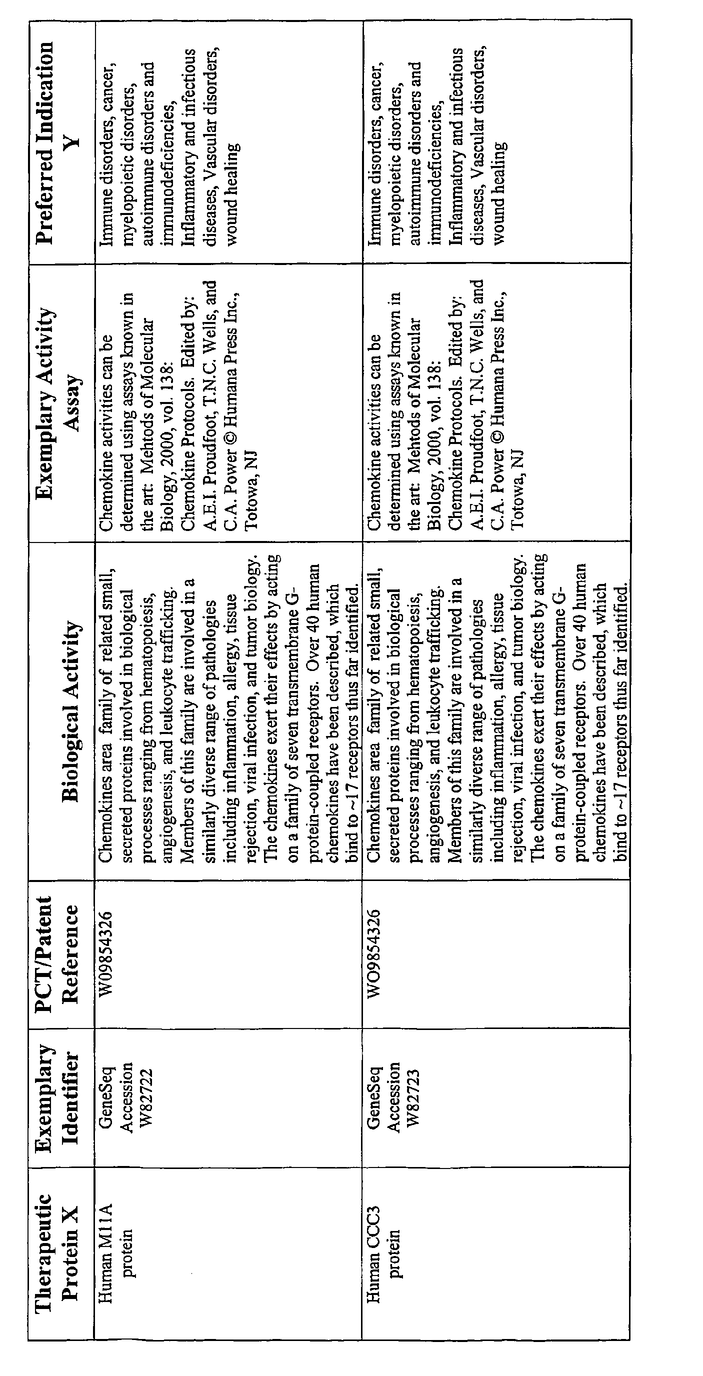

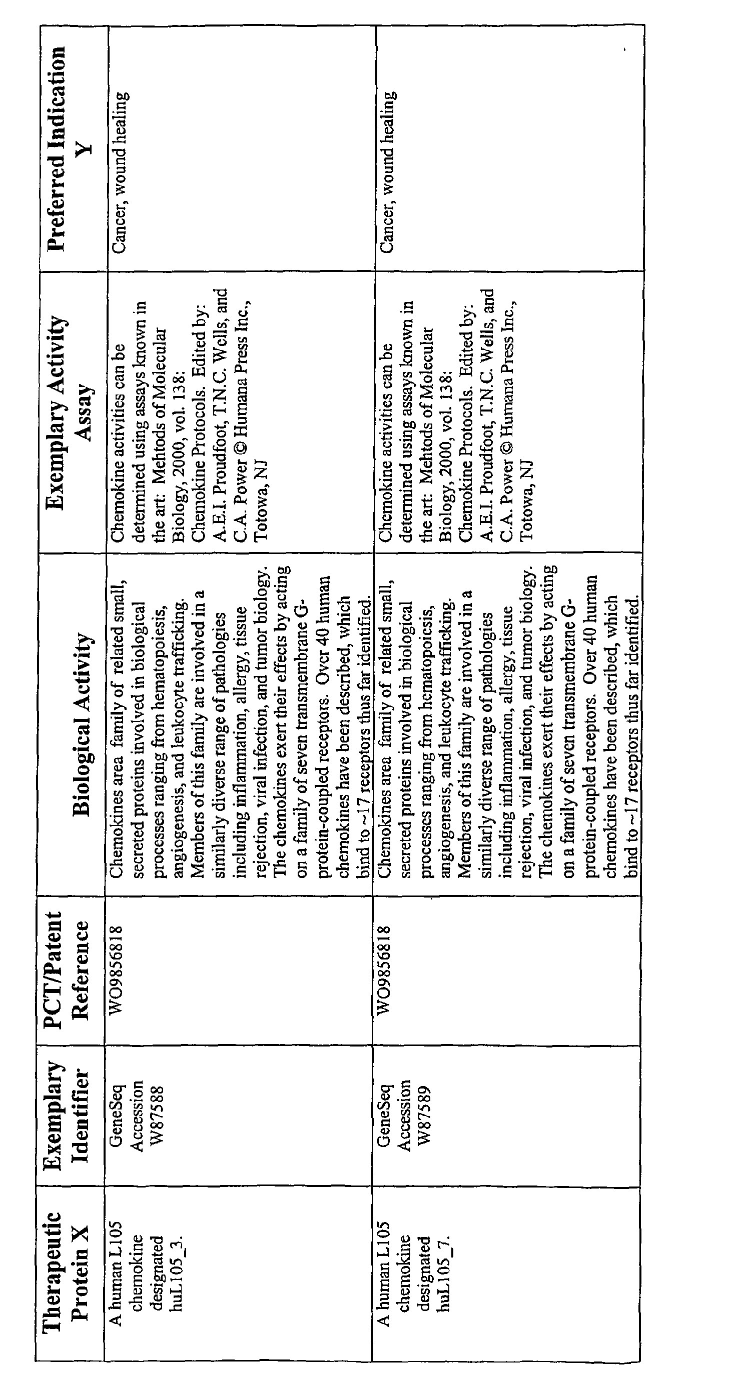

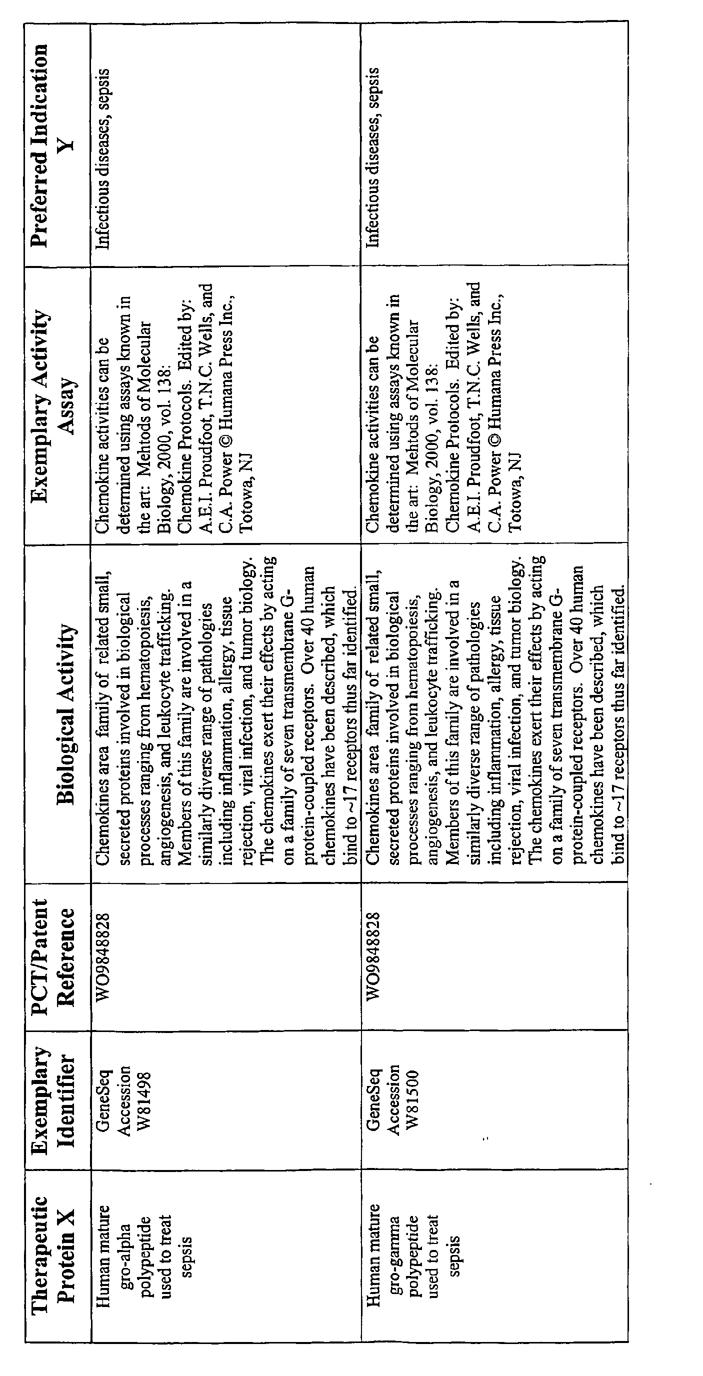

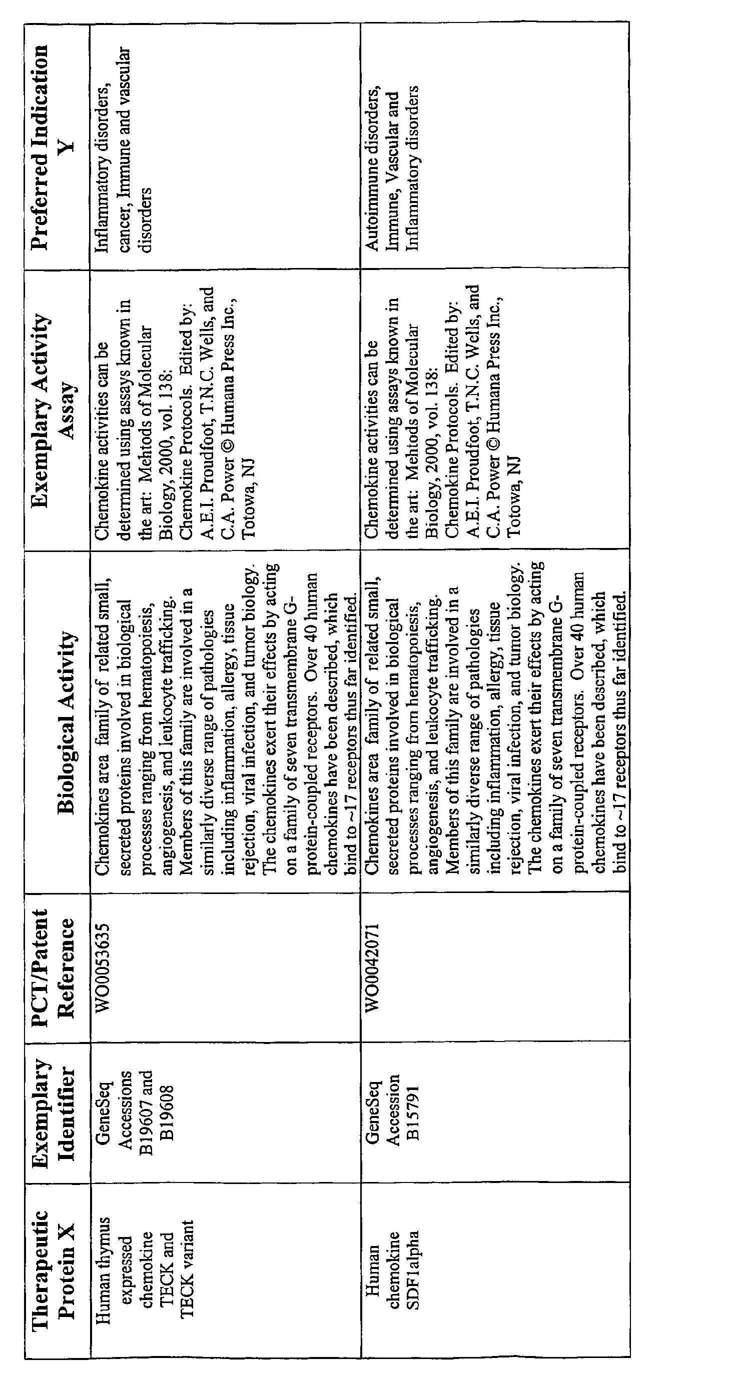

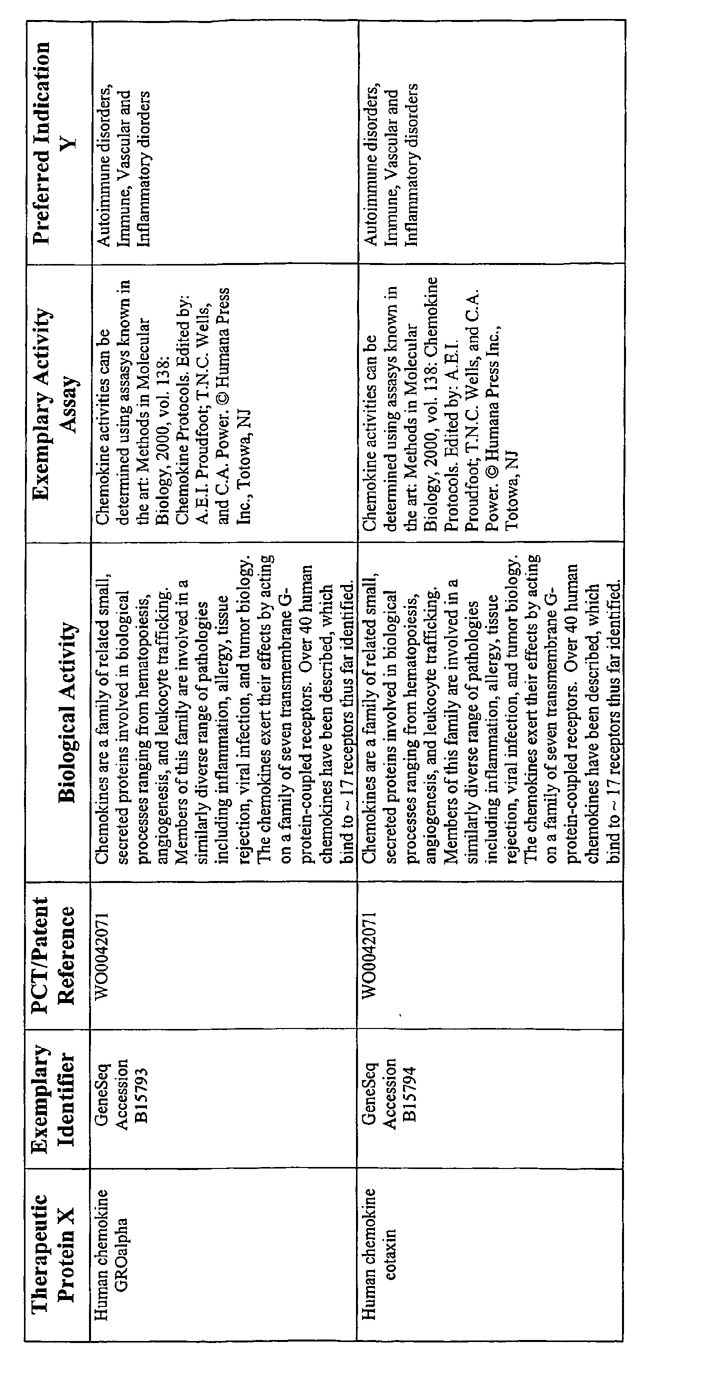

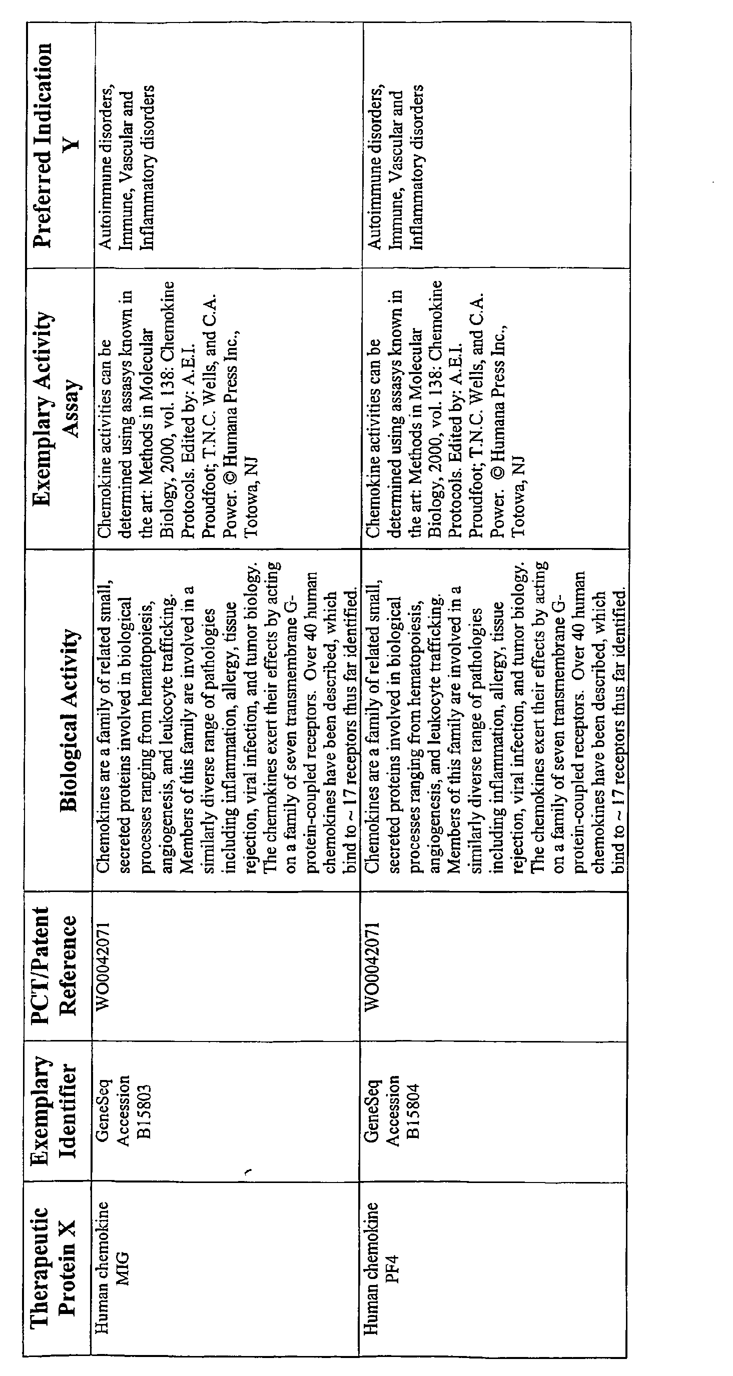

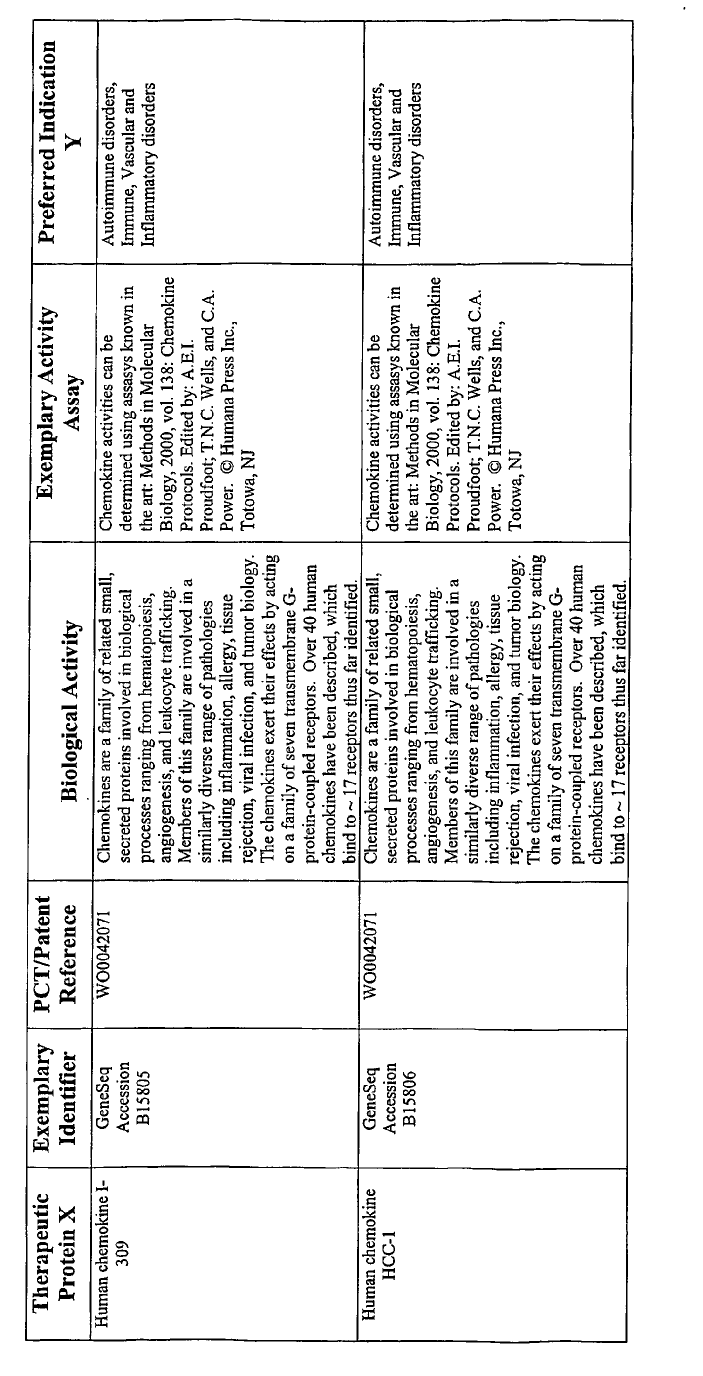

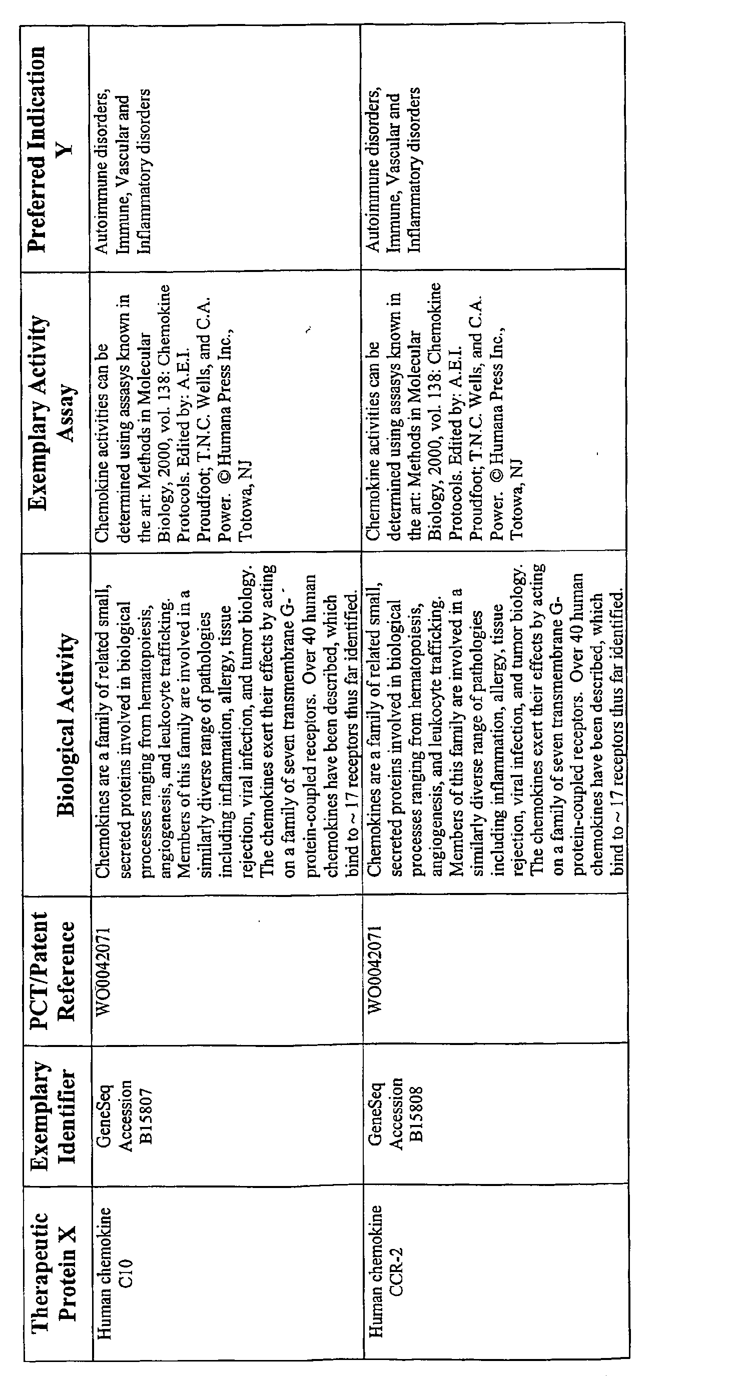

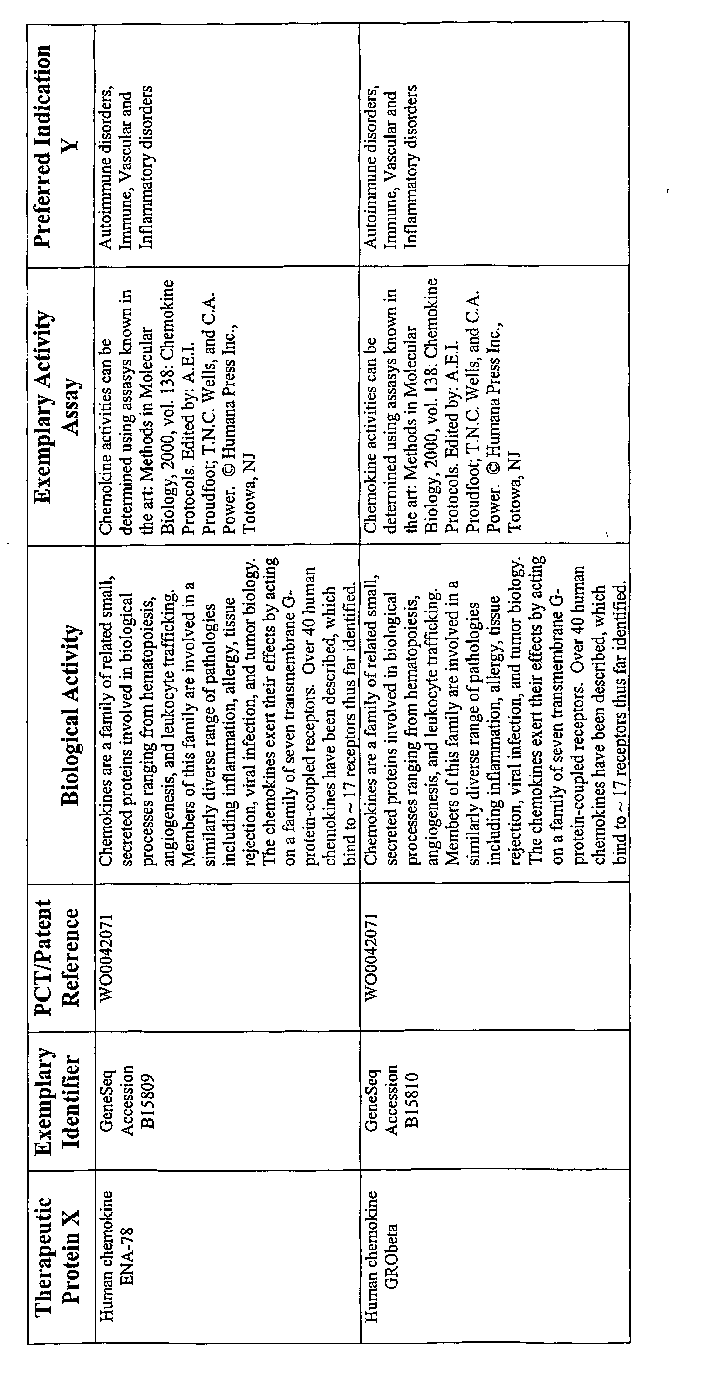

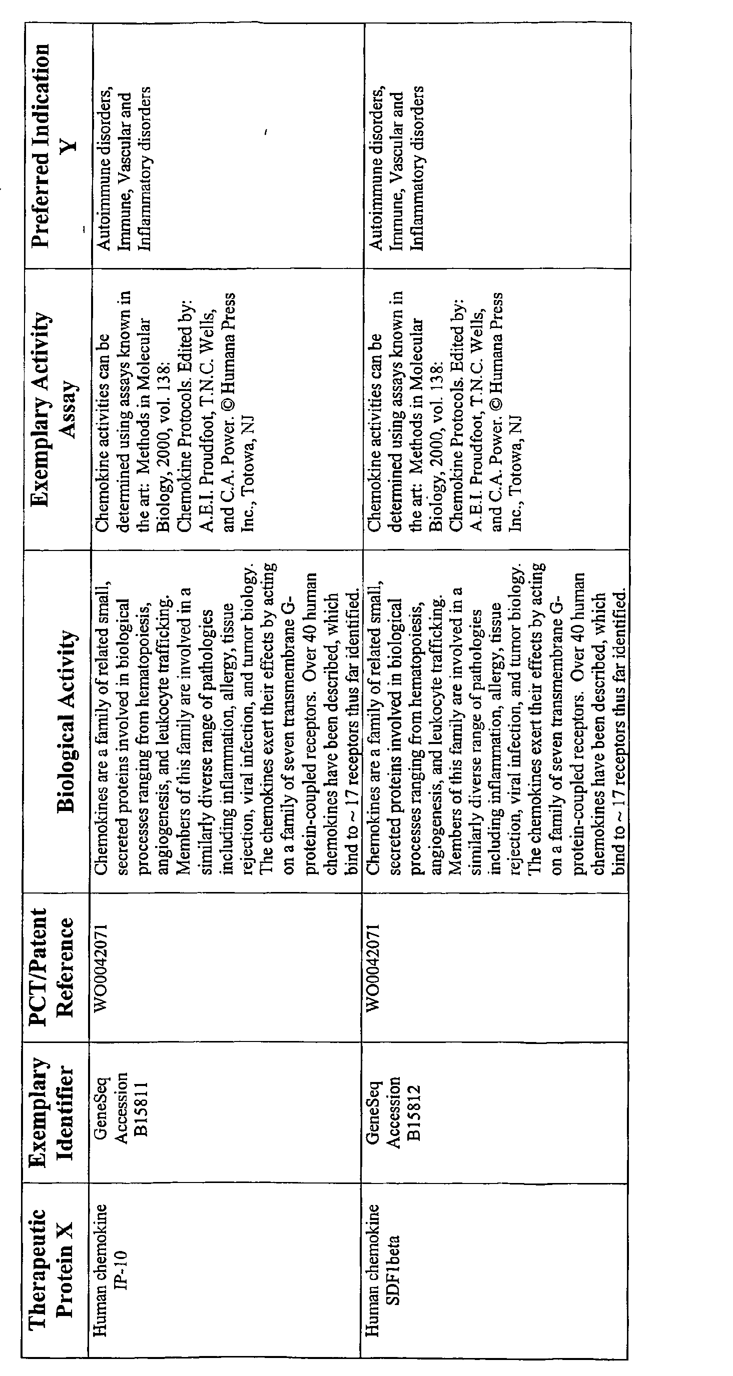

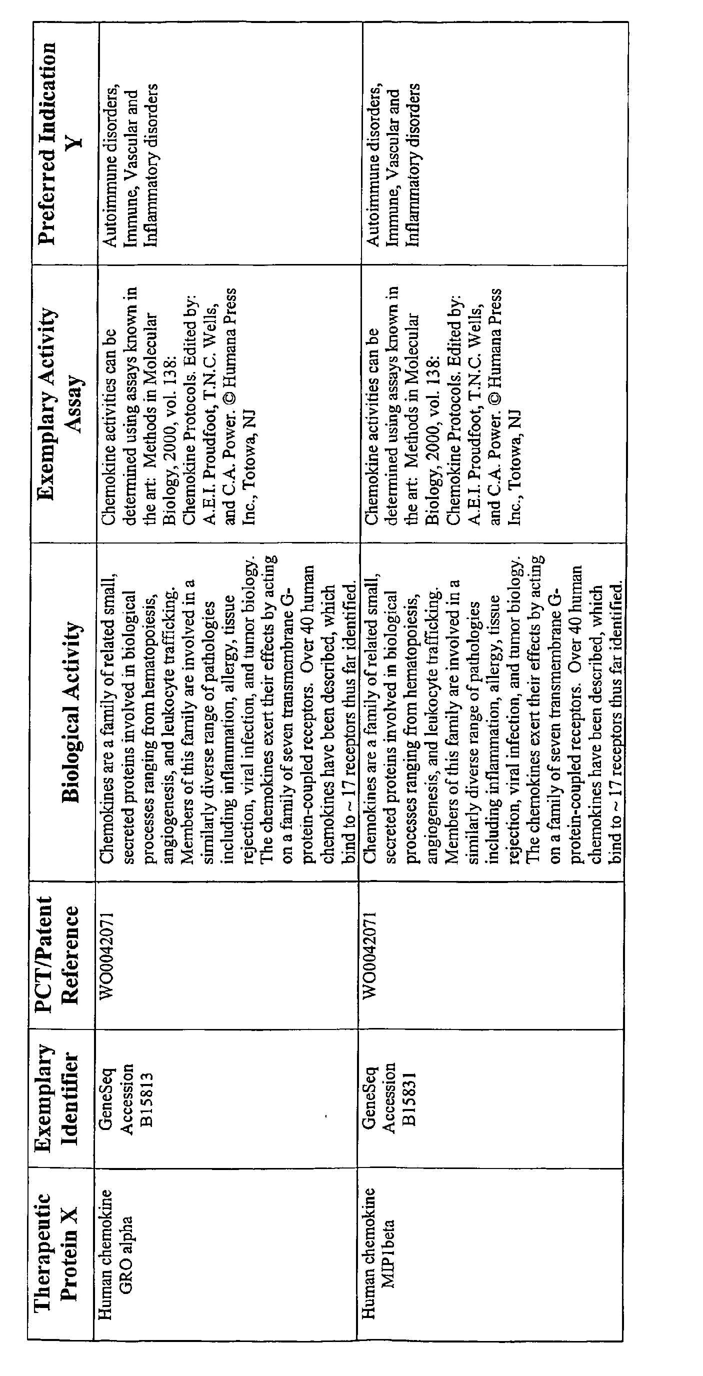

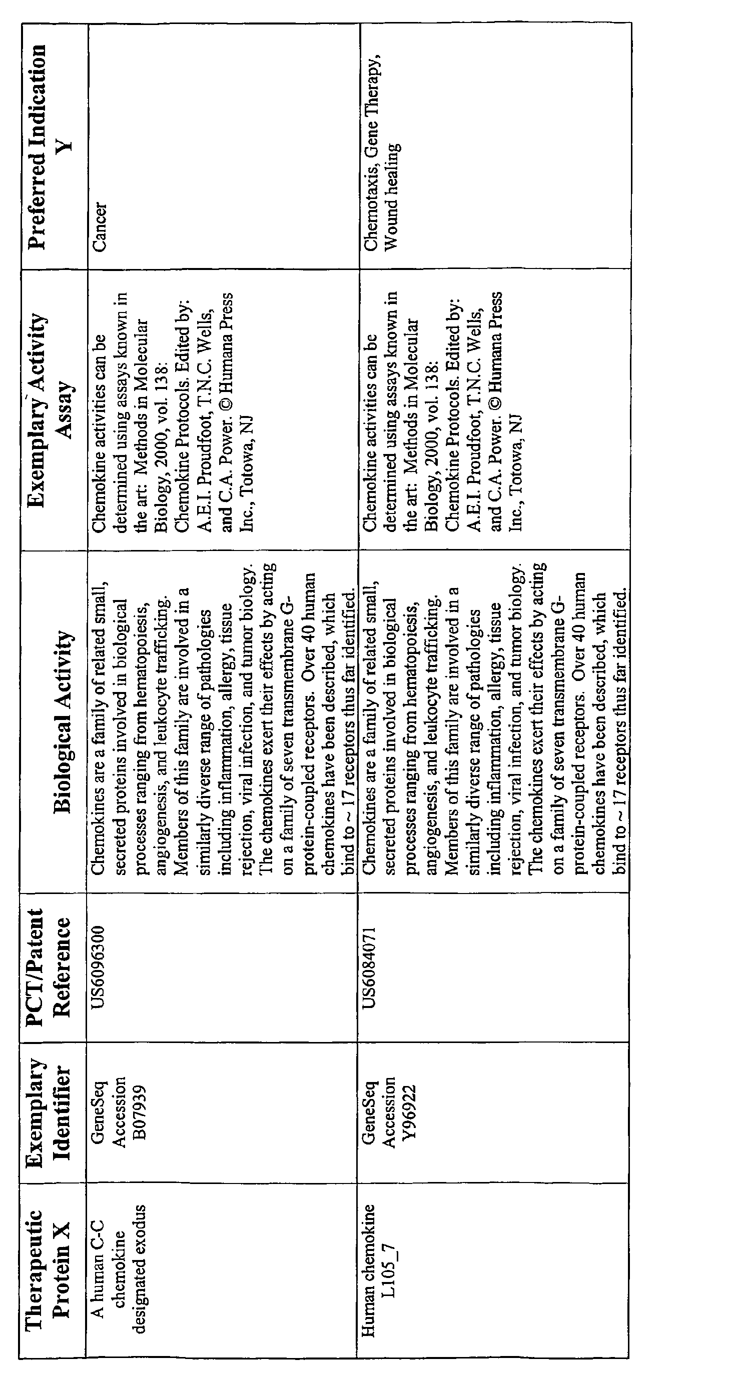

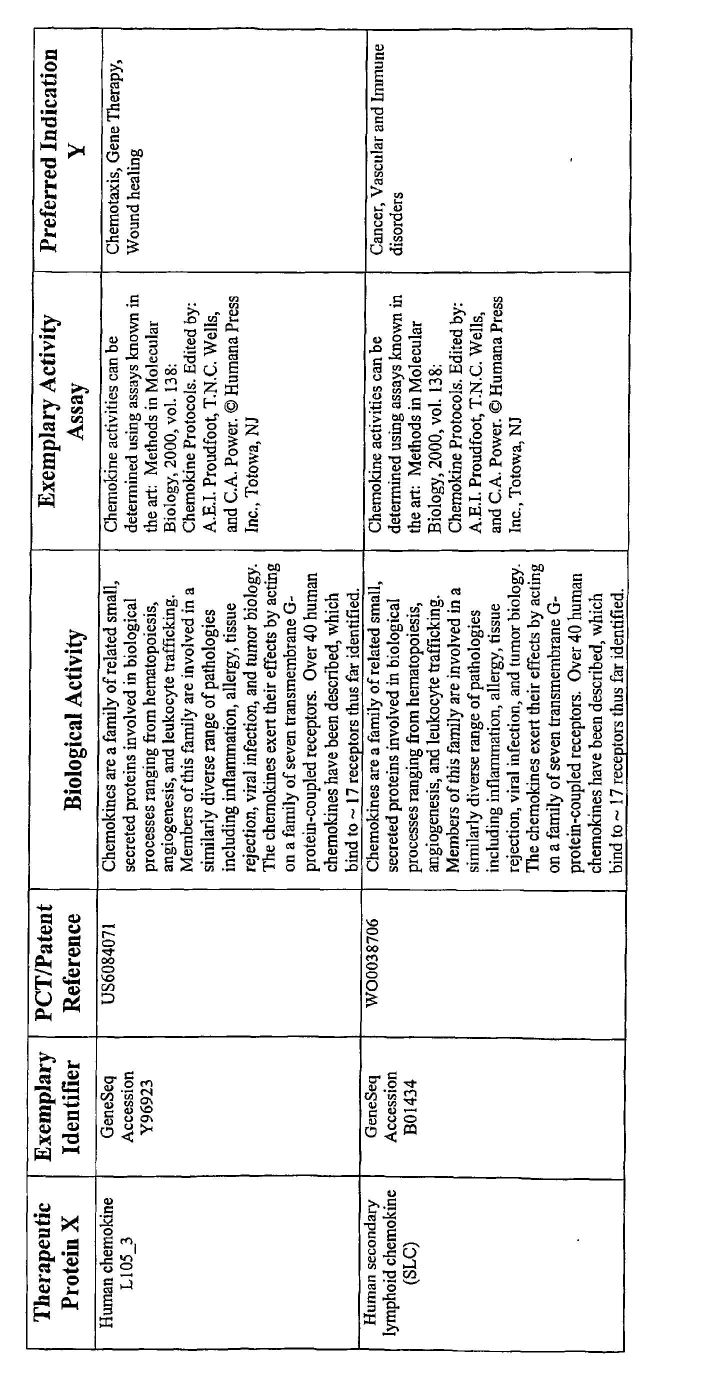

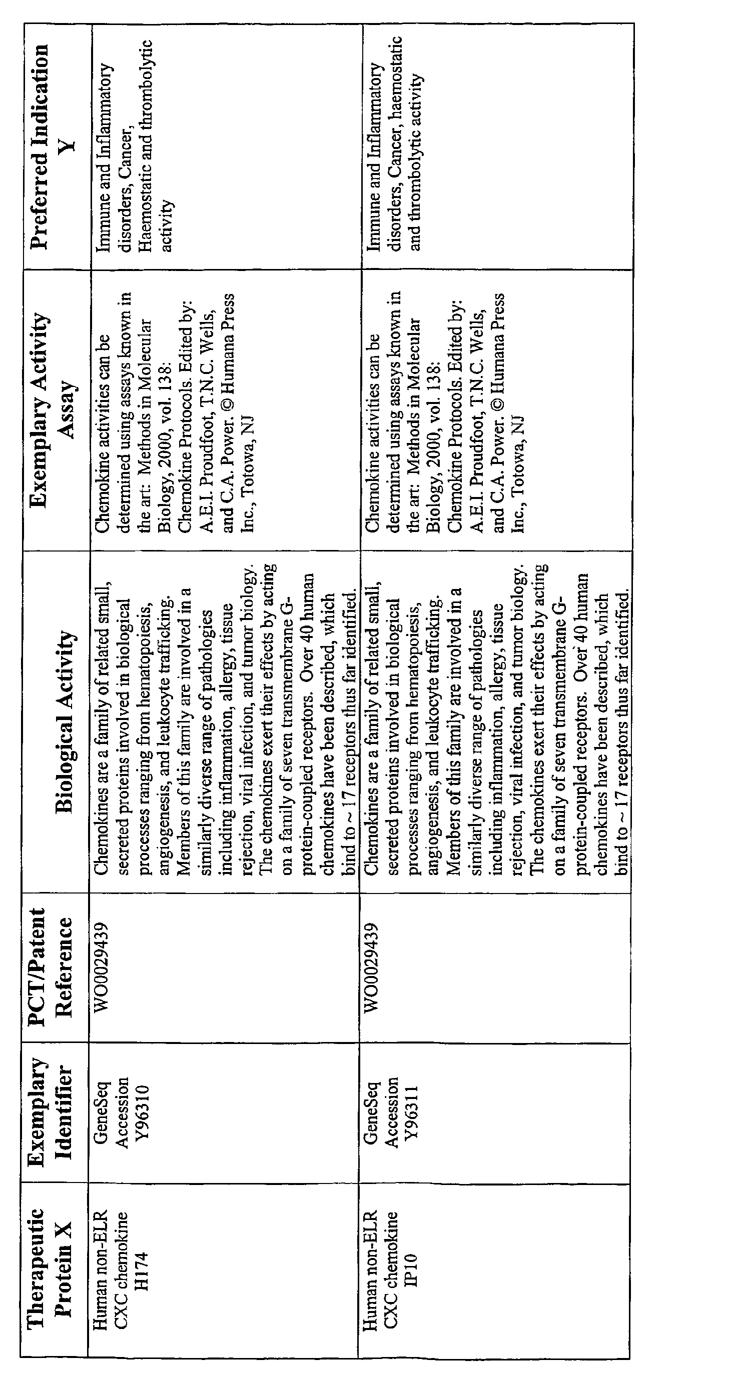

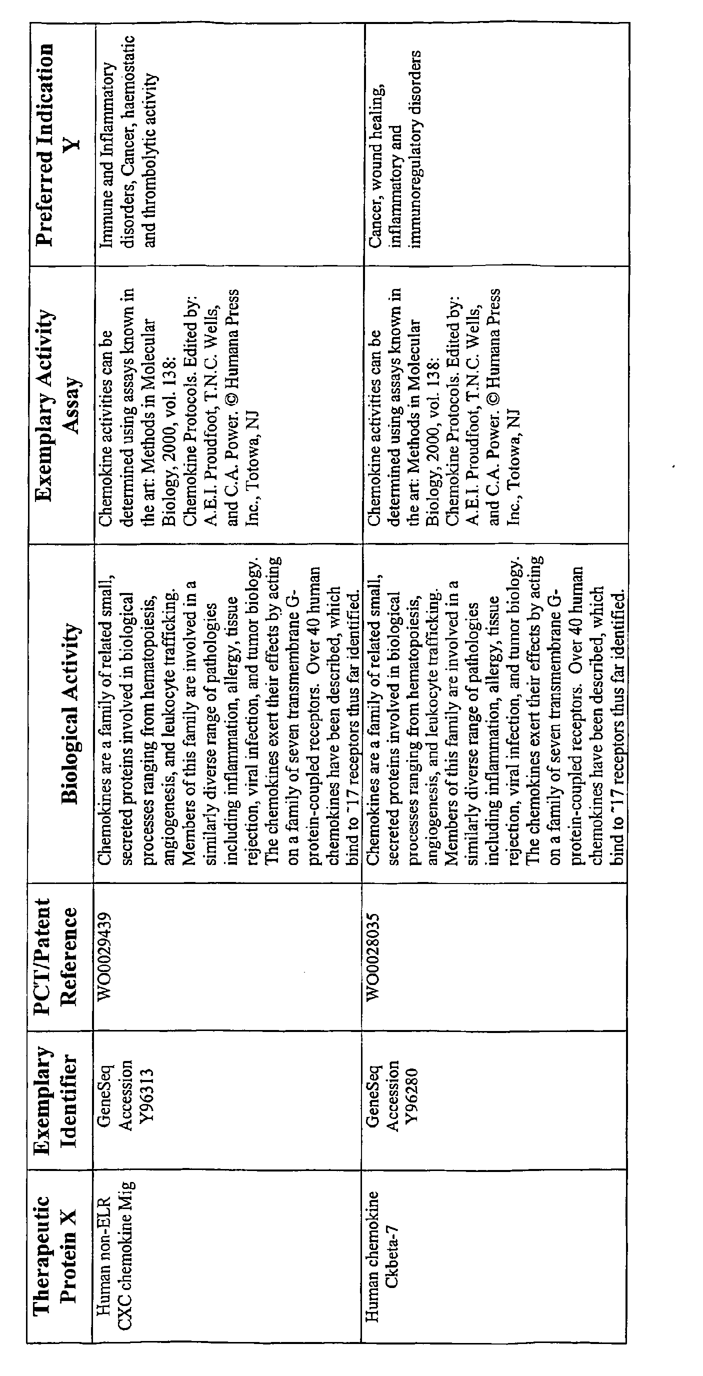

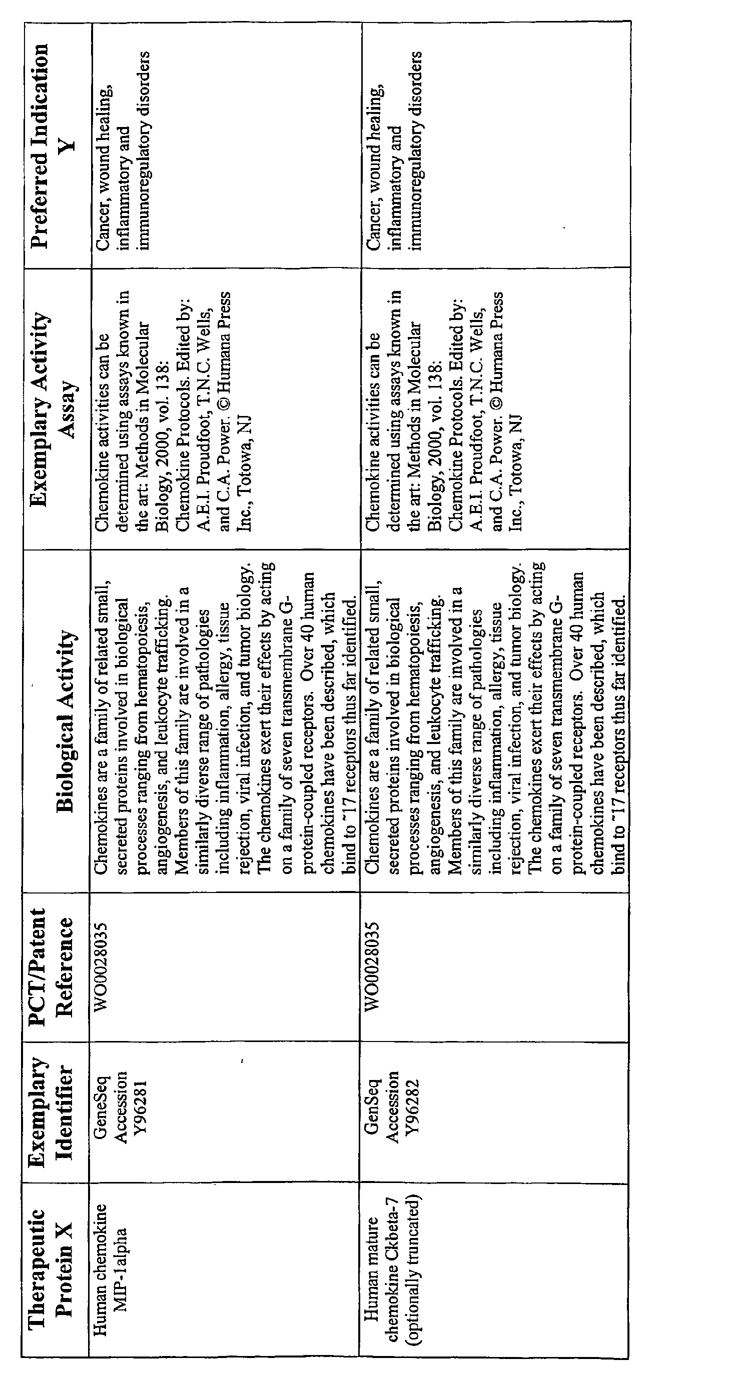

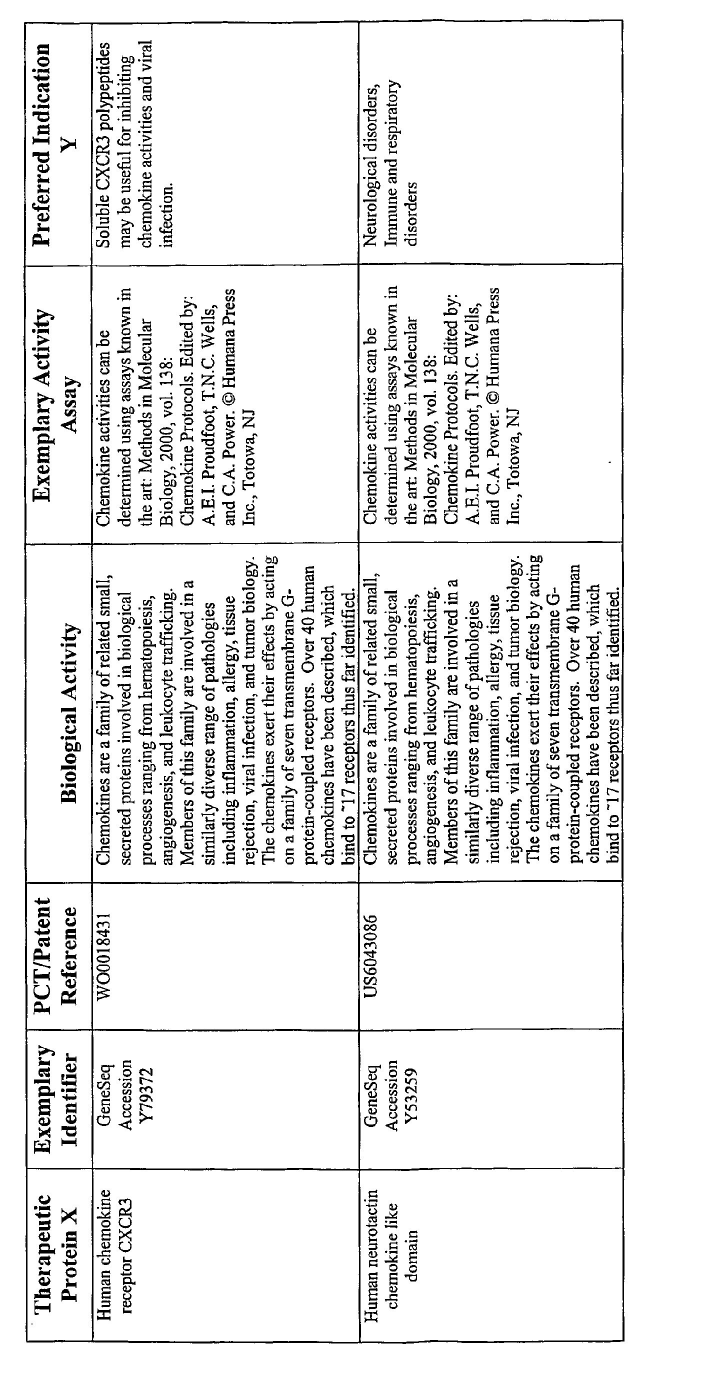

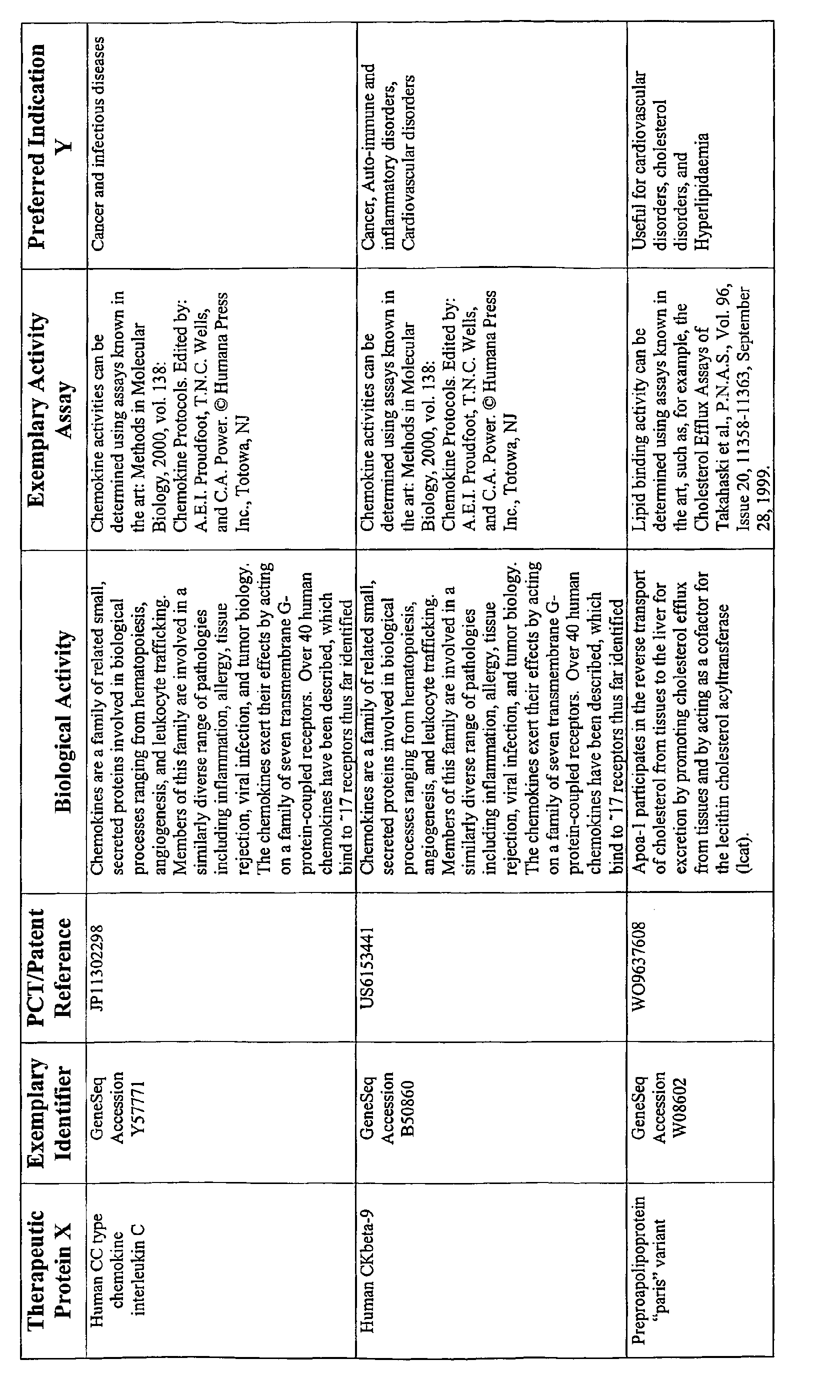

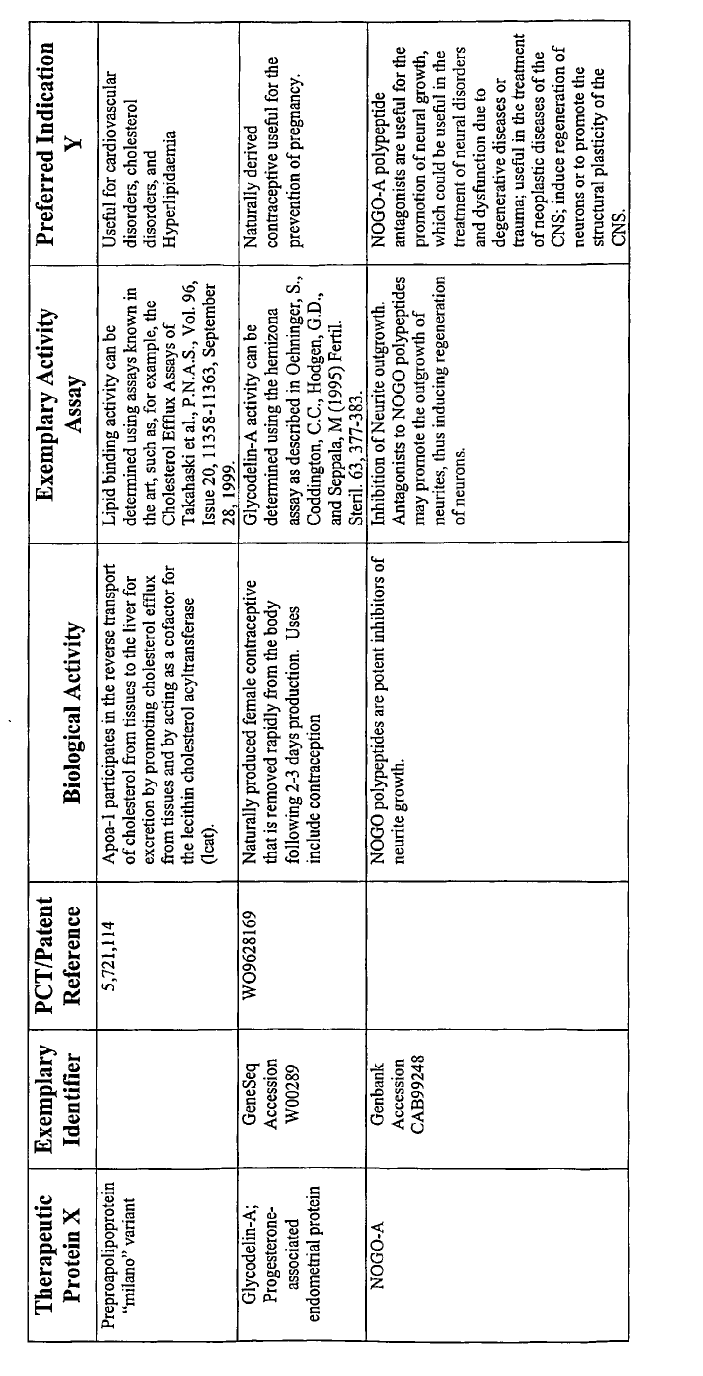

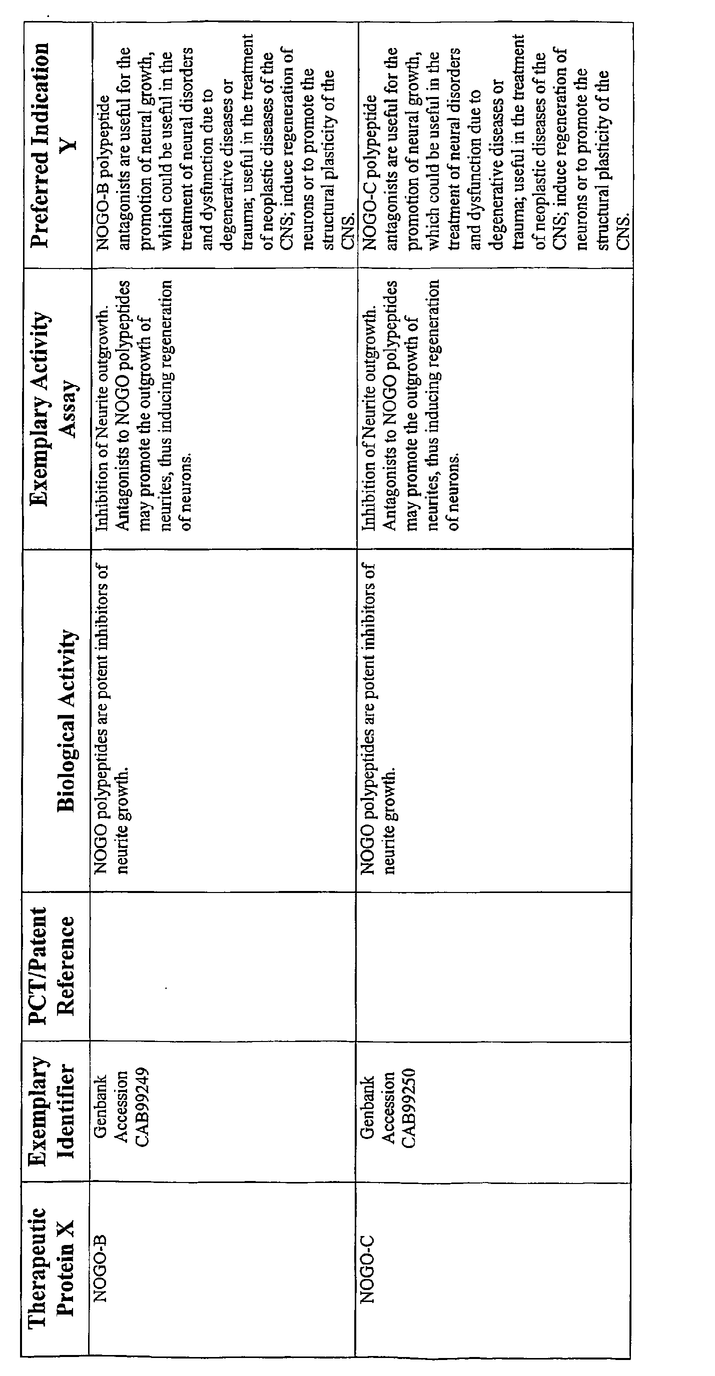

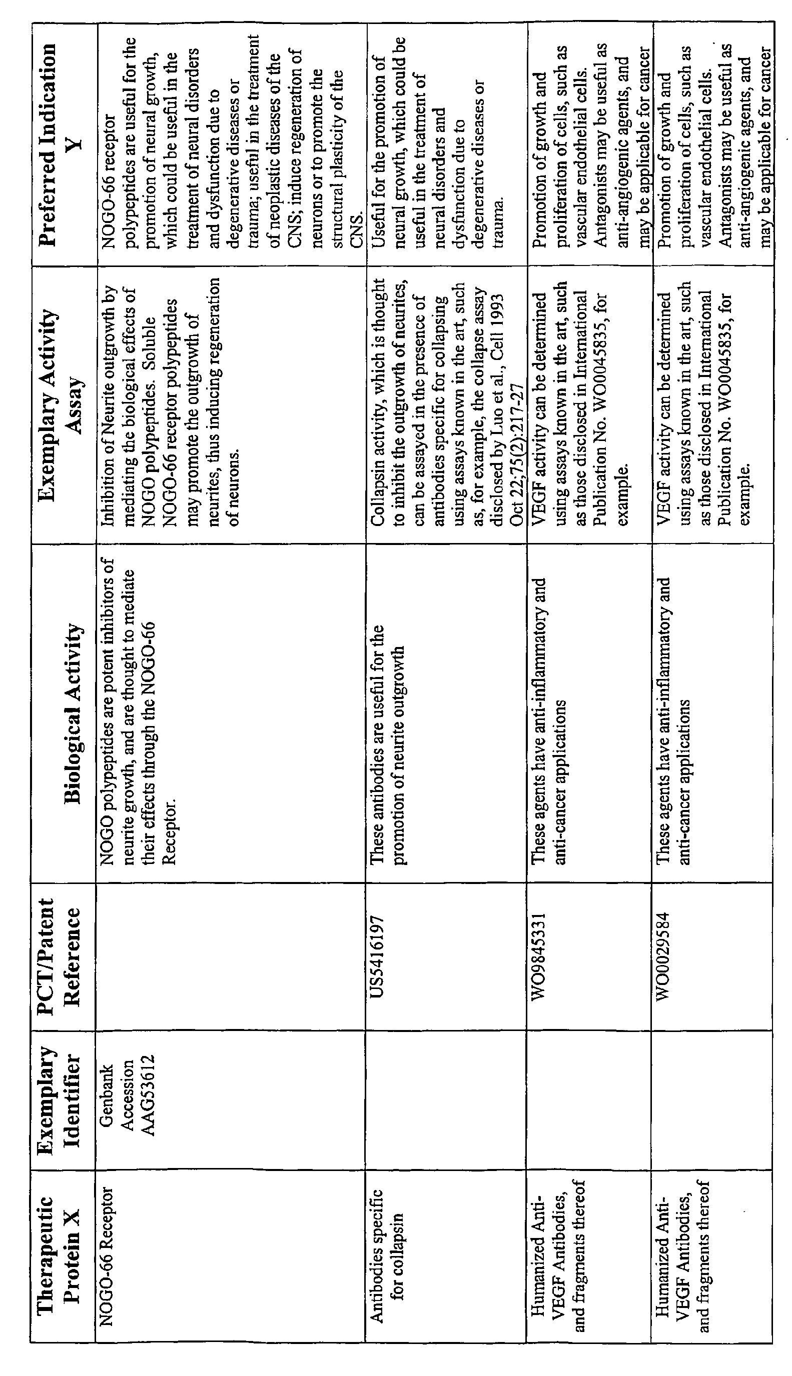

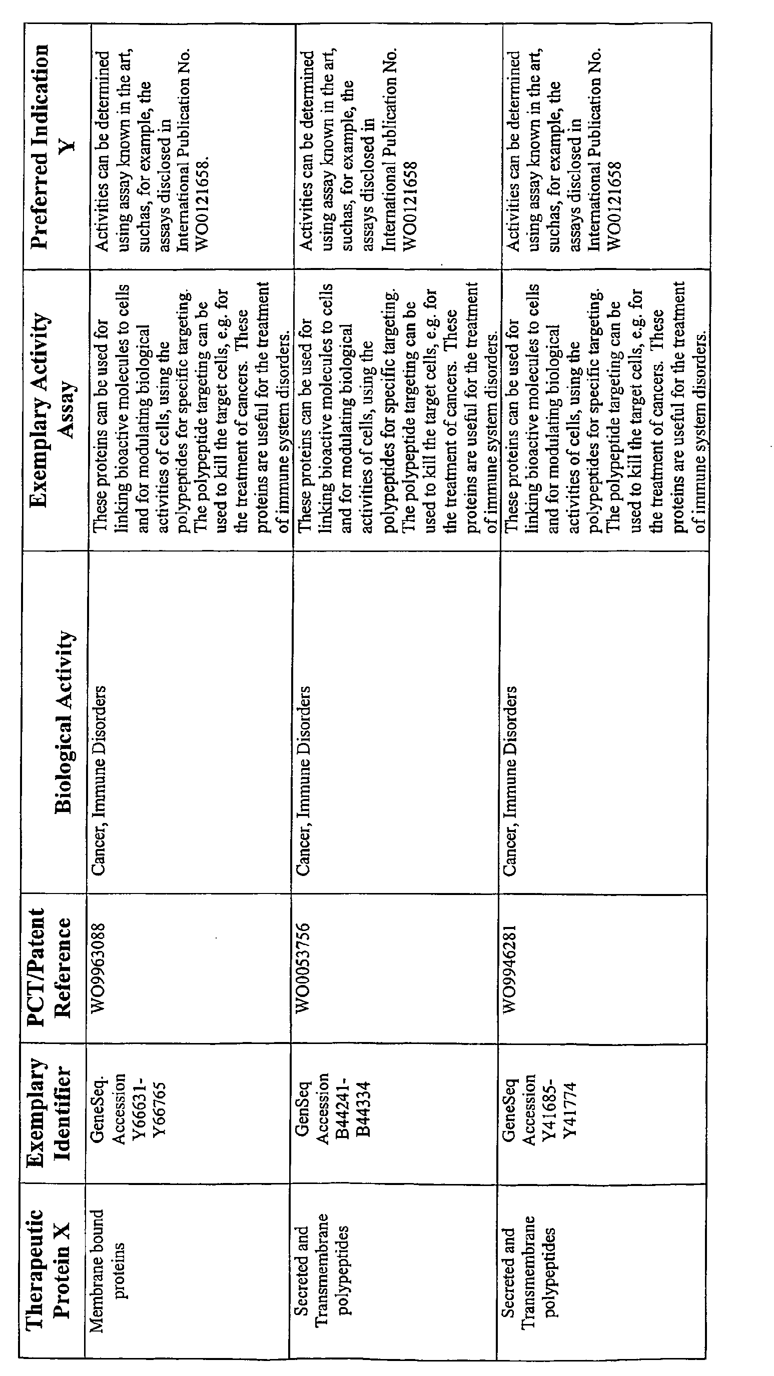

### Delivery of a Drug or Therapeutic Protein to the inside of a Cell and/or across the Blood Brain Barrier (BBB)

Within the scope of the invention, the modified transferrin fusion proteins may be used as a carrier to deliver a molecule or small molecule therapeutic complexed to the ferric ion of transferrin to the inside of a cell or across the blood brain barrier. In these embodiments, the Tf fusion protein will typically be engineered or modified to inhibit, prevent or remove glycosylation to extend the serum half-life of the fusion protein and/or therapeutic protein portion. The addition of a targeting peptide or, for example, a single chain antibody is specifically contemplated to further target the Tf fusion protein to a particular cell type, *e.g*., a cancer cell.

In one embodiment, the iron-containing, anti-anemic drug, ferric-sorbitol-citrate complex is loaded onto a modified Tf fusion protein of the invention. Ferric-sorbitol-citrate (FSC) has been shown to inhibit proliferation of various murine cancer cells *in vitro* and cause tumor regression *in vivo,* while not having any effect on proliferation of non-malignant cells (Poljak-Blazi et al. (June 2000) Cancer Biotherapy and Radiopharmaceuticals (United States), 15/3:285-293).

In another embodiment, the antineoplastic drug adriamycin (Doxorubicin) and/or the chemotherapeutic drug bleomycin, both of which are known to form complexes with ferric ion, is loaded onto a Tf fusion protein of the invention. In other embodiments, a salt of a drug, for instance, a citrate or carbonate salt, may be prepared and complexed with the ferric iron that is then bound to Tf. As tumor cells often display a higher turnover rate for iron; transferrin modified to carry at least one anti-tumor agent, may provide a means of increasing agent exposure or load to the tumor cells. (Demant, E.J., (1983) Eur. J. of Biochem. 137/(1-2):113-118*;* Padbury et al. (1985) J. Biol. Chem. 260/13:7820-7823).

### Pharmaceutical Formulations and Treatment Methods

The modified fusion proteins of the invention may be administered to a patient in need thereof using standard administration protocols. For instance, the modified Tf fusion proteins of the present invention can be provided alone, or in combination, or in sequential combination with other agents that modulate a particular pathological process. As used herein, two agents are said to be administered in combination when the two agents are administered simultaneously or are administered independently in a fashion such that the agents will act at the same or near the same time.

The agents of the present invention can be administered via parenteral, subcutaneous, intravenous, intramuscular, intraperitoneal, transdermal and buccal routes. For example, an agent may be administered locally to a site of injury via microinfusion. Alternatively, or concurrently, administration may be noninvasive by either the oral, inhalation, nasal, or pulmonary route. The dosage administered will be dependent upon the age, health, and weight of the recipient, kind of concurrent treatment, if any, frequency of treatment, and the nature of the effect desired.

The present invention further provides compositions containing one or more fusion proteins of the invention. While individual needs vary, determination of optimal ranges of effective amounts of each component is within the skill of the art. Typical dosages comprise about 1 pg/kg to about 100 mg/kg body weight. The preferred dosages for systemic administration comprise about 100 ng/kg to about 100 mg/kg body weight or about 100-200 mg of protein/dose. The preferred dosages for direct administration to a site via microinfusion comprise about 1 ng/kg to about 1 mg/kg body weight. When administered via direct injection or microinfusion, modified fusion proteins of the invention may be engineered to exhibit reduced or no binding of iron to prevent, in part, localized iron toxicity.

In addition to the pharmacologically active fusion protein, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the invention may be formulated for enteral, parenteral or topical administration. Indeed, all three types of formulations may be used simultaneously to achieve systemic administration of the active ingredient. Suitable formulations for oral administration include hard or soft gelatin capsules, pills, tablets, including coated tablets, elixirs, suspensions, syrups or inhalations and controlled release forms thereof.

In practicing the methods of this invention, the agents of this invention may be used alone or in combination, or in combination with other therapeutic or diagnostic agents. In certain preferred embodiments, the compounds of this invention may be co-administered along with other compounds typically prescribed for these conditions according to generally accepted medical practice. The compounds of this invention can be utilized *in vivo,* ordinarily in mammals, such as humans, sheep, horses, cattle, pigs, dogs, cats, rats and mice, or *in vitro.*

Modified fusion proteins of the present invention may be used in the diagnosis, prognosis, prevention and/or treatment of diseases and/or disorders relating to diseases and disorders of the endocrine system, the nervous system, the immune system, respiratory system, cardiovascular system, reproductive system, digestive system, diseases and/or disorders relating to cell proliferation, and/or diseases or disorders relating to the blood.

In yet other embodiments of the invention, modified Tf fusion proteins may be used in the diagnosis, prognosis, prevention and/or treatment of diseases and/or disorders relating to diseases and disorders known to be associated with or treatable by therapeutic protein moieties as known in the art and exemplified by PCT Patent Publication Nos. WO 01/79258, WO 01/77137, WO 01/79442, WO 01/79443, WO 01/79444 and WO 01/79480. Accordingly, the present invention encompasses a method of treating a disease or disorder listed in the Preferred Indication Y" column of Table 1 comprising administering to a patient in which such treatment, prevention or amelioration is desired a modified transferrin fusion protein of the invention that comprises a therapeutic protein portion corresponding to a therapeutic protein disclosed in the "Therapeutic Protein X" column of Table 1 in an amount effective to treat, prevent or ameliorate the disease or disorder.

In certain embodiments, a transferrin fusion protein of the present invention may be used to diagnose and/or prognose diseases and/or disorders.

Modified transferrin fusion proteins of the invention and polynucleotides encoding transferrin fusion proteins of the invention may be useful in treating, preventing, diagnosing and/or prognosing diseases, disorders, and/or conditions of the immune system. Moreover, fusion proteins of the invention and/or polynucleotides encoding transferrin fusion proteins of the invention can be used as a marker or detector of a particular immune system disease or disorder.

In a preferred embodiment fusion proteins of the invention and/or polynucleotides encoding modified transferrin fusion proteins of the invention could be used as an agent to boost immunoresponsiveness among immunodeficient individuals. In specific embodiments, fusion proteins of the invention and/or polynucleotides encoding transferrin fusion proteins of the invention could be used as an agent to boost immunoresponsiveness among B cell and/or T cell immunodeficient individuals.

The modified transferrin fusion proteins of the invention and/or polynucleotides encoding transferrin fusion proteins of the invention may be useful in treating, preventing, diagnosing, and/or prognosing autoimmune disorders. Many autoimmune disorders result from inappropriate recognition of self as foreign material by immune cells. This inappropriate recognition results in an immune response leading to the destruction of the host tissue. Therefore, the administration of fusion proteins of the invention and/or polynucleotides encoding transferrin fusion proteins of the invention, that can inhibit an immune response, particularly the proliferation, differentiation, or chemotaxis of T-cells, may be an effective therapy in preventing autoimmune disorders.

Modified transferrin fusion proteins of the invention and/or polynucleotides encoding transferrin fusion proteins of the invention may be useful in treating, preventing, prognosing, and/or diagnosing diseases, disorders, and/or conditions of hematopoietic cells. Transferrin fusion proteins of the invention and/or polynucleotides encoding transferrin fusion proteins of the invention could be used to increase differentiation and proliferation of hematopoietic cells, including the pluripotent stem cells, in an effort to treat or prevent those diseases, disorders, and/or conditions associated with a decrease in certain (or many) types hematopoietic cells, including but not limited to, leukopenia, neutropenia, anemia, and thrombocytopenia.

Alternatively, modified fusion proteins of the invention and/or polynucleotides encoding transferrin fusion proteins of the invention could be used to increase differentiation and proliferation of hematopoietic cells, including the pluripotent stem cells, in an effort to treat or prevent those diseases, disorders, and/or conditions associated with an increase in certain (or many) types of hematopoietic cells, including but not limited to, histiocytosis.

Allergic reactions and conditions, such as asthma (particularly allergic asthma) or other respiratory problems, may also be treated, prevented, diagnosed and/or prognosing and using modified fusion proteins of the invention and/or polynucleotides encoding transferrin fusion proteins of the invention. Moreover, these molecules can be used to treat, prevent, prognose, and/or diagnose anaphylaxis, hypersensitivity to an antigenic molecule, or blood group incompatibility.

Additionally, modified fusion proteins of the invention and/or polynucleotides encoding transferrin fusion proteins of the invention, may be used to treat, prevent, diagnose and/or prognose IgE-mediated allergic reactions. Such allergic reactions include, but are not limited to, asthma, rhinitis, and ecizema. In specific embodiments, fusion proteins of the invention and/or polynucleotides encoding transferrin fusion proteins of the invention may be used to modulate IgE concentrations in vitro or in vivo.

Moreover, modified fusion proteins of the invention and/or polynucleotides encoding transferrin fusion proteins of the invention have uses in the diagnosis, prognosis, prevention, and/or treatment of inflammatory conditions. For example, since fusion proteins of the invention and/or polynucleotides encoding transferrin fusion proteins of the invention may inhibit the activation, proliferation, and/or differentiation of cells involved in an inflammatory response, these molecules can be used to prevent and/or treat chronic and acute inflammatory conditions. Such inflammatory conditions include, but are not limited to, for example, inflammation associated with infection (*e.g*., septic shock, sepsis, or systemic inflammatory response syndrome), ischemia-reperfusion injury, endotoxin lethality, complement-mediated hyperacute rejection, nephritis, cytokine or chemokine induced lung injury, inflammatory bowel disease, Crohn's disease, over production of cytokines (*e.g*., TNF or IL-1.), respiratory disorders (*e.g*., asthma and allergy); gastrointestinal disorders (*e.g*., inflammatory bowel disease); cancers (*e.g*., gastric, ovarian, lung, bladder, liver, and breast); CNS disorders (*e.g*., multiple sclerosis; ischemic brain injury and/or stroke, traumatic brain injury, neurodegenerative disorders (*e.g*., Parkinson's disease and Alzheizmer's disease); AIDS-related dementia; and prion disease); cardiovascular disorders (*e.g*., atherosclerosis, myocarditis, cardiovascular disease, and cardiopulmonary bypass complications); as well as many additional diseases, conditions, and disorders that are characterized by inflammation (*e.g*., hepatitis, rheumatoid arthritis, gout, trauma, pancreatitis, sarcoidosis, dermatitis, renal ischemia-reperfusion injury, Grave's disease, systemic lupus erythematosus, diabetes mellitus, and allogenic transplant rejection).

Because inflammation is a fundamental defense mechanism, inflammatory disorders can effect virtually any tissue of the body. Accordingly, modified fusion proteins of the invention and/or polynucleotides encoding transferrin fusion proteins of the invention, have uses in the treatment of tissue-specific inflammatory disorders, including, but not limited to, adrenalitis, alveolitis, angiocholecystitis, appendicitis, balanitis, blepharitis, bronchitis, bursitis, carditis, cellulitis, cervicitis, cholecystitis, chorditis, cochiftis, colitis, conjunctivitis, cystitis, dermatitis, diverticulitis, encephalitis, endocarditis, esophagitis, eustachitis, fibrositis, folliculitis, gastritis, gastroenteritis, gingivitis, glossitis, hepatosplenitis, keratitis, labyrinthitis, laryngitis, lymphangitis, mastitis, media otitis, meningitis, metritis, mucitis, myocarditis, myosititis, myringitis, nephritis, neuritis, orchitis, osteochondritis, otitis, pericarditis, peritendonitis, peritonitis, pharyngitis, phlebitis, poliomyelitis, prostatititis, Pulpitis, retinitis, rhinitis, salpingitis, scleritis, sclerochoroiditis, scrotitis, sinusitis, spondylitis, steatitis, stomatitis, synovitis, syringitis, tendonitis, tonsillitis, urethritis, and vaginitis.

In specific embodiments, modified fusion proteins of the invention and/or polynucleotides encoding transferrin fusion proteins of the invention, are useful to diagnose, prognose, prevent, and/or treat organ transplant rejections and graft-versus-host disease. Organ rejection occurs by host immune cell destruction of the transplanted tissue through an immune response. Similarly, an immune response is also involved in GVHD, but, in this case, the foreign transplanted immune cells destroy the host tissues. Polypeptides, antibodies, or polynucleotides of the invention, and/or agonists or antagonists thereof, that inhibit an immune response, particularly the activation, proliferation, differentiation, orchemotaxis of T-cells, may be an effective therapy in preventing organ rejection or GVHD.

In another specific embodiment, modified transferrin fusion proteins of the invention and/or polynucleotides encoding transferrin fusion proteins of the invention are used as an adjuvant to enhance anti-viral immune responses. Anti-viral immune responses that may be enhanced using the compositions of the invention as an adjuvant, include virus and virus associated diseases or symptoms described herein or otherwise known in the art. In specific embodiments, the compositions of the invention are used as an adjuvant to enhance an immune response to a virus, disease, or symptom selected from the group consisting of AIDS, meningitis, Dengue, EBV, and hepatitis (*e.g*., hepatitis B). In another specific embodiment, the compositions of the invention are used as an adjuvant to enhance an immune response to a virus, disease, or symptom selected from the group consisting of: HIV/AIDS, respiratory syncytial virus, Dengue, rotavirus, Japanese B encephalitis, influenza A and B, parainfluenza, measles, cytomegalovirus, rabies, Junin, Chikungunya, Rift Valley Fever, herpes simplex, and yellow fever.

In another specific embodiment, modified transferrin fusion proteins of the invention and/or polynucleotides encoding transferrin fusion proteins of the invention are used as an adjuvant to enhance anti-bacterial or anti-fungal immune responses. Anti-bacterial or anti-fungal immune responses that may be enhanced using the compositions of the invention as an adjuvant, include bacteria or fungus and bacteria or fungus associated diseases or symptoms described herein or otherwise known in the art. In specific embodiments, the compositions of the invention are used as an adjuvant to enhance an immune response to a bacteria or fungus, disease, or symptom selected from the group consisting of tetanus, Diphtheria, botulism, and meningitis type B.

In another specific embodiment, the compositions of the invention are used as an adjuvant to enhance an immune response to a bacteria or fungus, disease, or symptom selected from the group consisting of Vibrio cholerae, Mycobacterium leprae, Salmonellatyphi, Salmonella paratyphi, Meisseria meningitidis, Streptococcus pneumoniae, Group B streptococcus, Shigella spp., Enterotoxigenic Escherichia coli, Enterohemorrhagic E. coli, and Borrelia burgdorferi.

In another specific embodiment, modified transferrin fusion proteins of the invention and/or polynucleotides encoding transferrin fusion proteins of the invention are used as an adjuvant to enhance anti-parasitic immune responses. Anti-parasitic immune responses that may be enhanced using the compositions of the invention as an adjuvant, include parasite and parasite associated diseases or symptoms described herein or otherwise known in the art. In specific embodiments, the compositions of the invention are used as an adjuvant to enhance an immune response to a parasite. In another specific embodiment, the compositions of the invention are used as an, adjuvant to enhance an immune response to Plasmodium (malaria) or Leishmania.

In another specific embodiment, modified transferrin fusion proteins of the invention and/or polynucleotides encoding transferrin fusion proteins of the invention may also be employed to treat infections diseases including silicosis, sarcoidosis, and idiopathic pulmonary fibrosis; for example, by preventing the recruitment and activation of mononuclear phagocytes.

In another specific embodiment, modified transferrin fusion proteins of the invention and/or polynucleotides encoding transferrin fusion proteins of the invention are used as an antigen for the generation of antibodies to inhibit or enhance immune mediated responses against polypeptides of the invention.

In one embodiment, modified transferrin fusion proteins of the invention and/or polynucleotides encoding transferrin fusion proteins of the invention are administered to an animal (*e.g*., mouse, rat, rabbit, hamster, guinea pig, pigs, micro-pig, chicken, camel, goat, horse, cow, sheep, dog, cat non-human primate, and human, most preferably human) to boost the immune system to produce increased quantities, of one or more antibodies (*e.g*., IgG, IgA, IgM, and IaE), to induce higher affinity antibody production and immunoglobulin class switching (*e.g*., IgG, IgA, IgM, and IaE), and/or to increase an immune response.

In another embodiment, modified transferrin fusion proteins of the invention and/or polynucleotides encoding transferrin fusion proteins of the invention are used in one or more of the applications described herein, as they may apply to veterinary medicine.

In another specific embodiment, modified transferrin fusion proteins of the invention, and/or polynucleotides encoding transferrin fusion proteins of the invention are used as a means of blocking various aspects of immune responses to foreign agents or self. Examples of diseases or conditions in which blocking of certain aspects of immune responses may be desired include autoimmune disorders such as lupus, and arthritis, as well as immunoresponsiveness to skin allergies, inflammation, bowel disease, injury, and diseases/disorders associated with pathogens.

In another specific embodiment, modified transferrin fusion proteins of the invention and/or polynucleotides encoding transferrin fusion proteins of the invention are used as a therapy for preventing the B cell proliferation and Ig secretion associated with autoimmune diseases such as idiopathic thrombocytopenic purpura, systemic lupus erythematosus and multiple sclerosis.

In another specific embodiment, modified transferrin fusion proteins or polynucleotides encoding transferrin fusion proteins of the invention are used as an inhibitor of B and/or T cell migration in endothelial cells. This activity disrupts tissue architecture or cognate responses and is useful, for example in disrupting immune responses, and blocking sepsis.

In another specific embodiment, modified transferrin fusion proteins of the invention, and/or polynucleotides encoding transferrin fusion proteins of the invention are used as a therapy for chronic hypergammaglobulinen evident in such diseases as monoclonal gammopathy of undetermined significance (MGUS), Waldenstrom's disease, related idiopathic monocional gammopathies, and plasmacytomas.

Another specific embodiment, modified transferrin fusion proteins of the invention and/or polynucleotides encoding transferrin fusion proteins of the invention may be employed for instance to inhibit polypeptide chemotaxis and activation of macrophages and their precursors, and of neutrophils, basophils, B lymphocytes and some T-cell subsets, *e.g*., activated and CD8 cytotoxic T cells and natural killer cells, in certain autoimmune and chronic inflammatory and infective diseases. Examples of autoimmune diseases are described herein and include multiple sclerosis, and insulin-dependent diabetes.

In another specific embodiment, modified transferrin fusion proteins of the invention and/or polynucleotides encoding transferrin fusion protein of the invention may also be employed for treating atherosclerosis, for example, by preventing monocyte infiltration in the artery wall.

In another specific embodiment, modified transferrin fusion proteins of the invention and/or-polynucleotides encoding transferrin fusion proteins of the invention may be employed to treat adult respiratory distress syndrome (ARDS).

In another specific embodiment, modified transferrin fusion proteins of the invention and/or polynucleotides encoding transferrin fusion proteins of the invention may be useful for stimulating wound and tissue repair, stimulating angiogenesis, and/or stimulating the repair of vascular or lymphatic diseases or disorders. Additionally, fusion proteins of the invention and/or polynucleotides encoding transferrin fusion proteins of the invention may be used to stimulate the regeneration of mucosal surfaces.

In a specific embodiment, modified transferrin fusion proteins of the invention and/or polynucleotides encoding transferrin fusion proteins of the invention are used to diagnose, prognose, treat, and/or prevent a disorder characterized by primary or acquired immunodeficiency, deficient serum immunoglobulin production, recurrent infections, and/or immune system dysfunction. Moreover, modified fusion proteins of the invention and/or polynucleotides encoding transferrin fusion proteins of the invention may be used to treat or prevent infections of the joints, bones, skin, and/or parotid glands, blood-borne infections (*e*.*g*., sepsis, meningitis, septic arthritis, and/or osteomyelitis), autoimmune diseases (*e.g*., those disclosed herein), inflammatory disorders, and malignancies, and/or any disease or disorder or condition associated with these infections, diseases, disorders and/or malignancies) including, but not limited to, CVID, other primary immune deficiencies, HIV disease, CLL, recurrent bronchitis, sinusitis, otitis media, conjunctivitis, pneumonia, hepatitis, meningitis, herpes zoster (*e.g*., severe herpes zoster), and/or pneumocystis carnii. Other diseases and disorders that may be prevented, diagnosed, prognosed, and/or treated with fusion proteins of the invention and/or polynucleotides encoding transferrin fusion proteins of the invention include, but are not limited to, HIV infection, HTLV-BLV infection, lymphopenia, phagocyte bactericidal dysfunction anemia, thrombocytopenia, and hemoglobinuria.

In a specific embodiment, modified transferrin fusion proteins of the invention and/or polynucleotides encoding transferrin fusion proteins of the invention may be used to diagnose, prognose, prevent, and/or treat cancers or neoplasms including immune cell or immune tissue-related cancers or neoplasms. Examples of cancers or neoplasms that may be prevented, diagnosed, or treated by fusion proteins of the invention and/or polynucleotides encoding transferrin fusion proteins of the invention include, but are not limited to, acutemyelogenous leukemia, chronic myelogenous leukemia, Hodgkin's disease, non-Hodgkin's lymphoma, acute lymphocytic anemia (ALL) Chronic lymphocyte leukemia, plasmacytomas, multiple myetoma, Burkitt's lymphoma, EBV transformed diseases, and/or diseases and disorders described in the section entitled "Hyperproliferative Disorders" elsewhere herein.

In another specific embodiment, modified transferrin fusion proteins of the invention and/or polynucleotides encoding transferrin fusion proteins of the invention are used as a therapy for decreasing cellular proliferation of Large B-cell Lymphomas.

In specific embodiments, the compositions of the invention are used as an agent to boost immunoresponsiveness among B cell immunodeficient individuals, such as, for example, an individual who has undergone a partial or complete splenectomy.

The modified transferrin fusion proteins of the invention and/or polynucleotides encoding transferrin fusion proteins of the invention may be used to modulate homeostatic (the stopping of bleeding) or thrombolytic (clot dissolving) activity. For example, by increasing homeostatic or thrombolytic activity, fusion proteins of the invention and/or polynucleotides encoding transferrin fusion proteins of the invention could be used to treat or prevent blood coagulation diseases, disorders, and/or conditions (*e.g*., afibrinogenemia, factor deficiencies, hemophilia), blood platelet diseases, disorders, and/or conditions (*e.g*. thrombocytopenia), or wounds resulting from trauma, surgery, or other causes. Alliteratively, fusion proteins of the invention and/or polynucleotides encoding transferrin fusion proteins of the invention that can decrease hemostatic or thrombolytic activity could be used to inhibit or dissolve clotting. These molecules could be important in the treatment or prevention of heart attacks (infarction), strokes, or scarring.

In specific embodiments, the modified transferrin fusion proteins of the invention and/or polynucleotides encoding transferrin fusion proteins of the invention may be used to prevent diagnose, prognose, and/or treat thrombosis, arteria thrombosis, venous thrombosis, thromboembolism, pulmonary embolism, atherosclerosis, myocardial infarction, transient ischenuc attack, unstable angina. In specific embodiments, the transferrin fusion proteins of the invention and/or polynucleotides encoding transferrin fusion proteins of the invention maybe used for the prevention of occulsion of saphenous grafts, for reducing the risk of periprocedural thrombosis as might accompany angioplasty procedures, for reducing the risk of stroke in patients with atrial fibrillation including nonrheumatic atria fibrillation, for reducing the risk of embolism associated with mechanical heart valves and/or mitral valves disease. Other uses for the modified transferrin fusion proteins of the invention and/or polynucleotides encoding transferrin fusion proteins of the invention, include, but are not limited to, the prevention of occlusions in extrcorporeal devices (*e.g*., intravascular canals, vascular access shunts in hemodialysis patients, hemodialysis machines, and cardiopulmonary bypass machines).

In another embodiment, modified transferrin fusion proteins of the invention and/or polynucleotides encoding transferrin fusion proteins of the invention, may be used to prevent, diagnose, prognose, and/or treat diseases and disorders of the blood and/or blood forming organs associated with the tissue(s) in which the polypeptide of the invention is expressed.

The modified fusion proteins of the invention and/or polynucleotides encoding transferrin fusion proteins of the invention may be used to modulate hematopoietic activity (the formation of blood cells). For example, the transferrin fusion proteins of the invention and/or polynucleotides encoding transferrin fusion proteins of the invention may be used to increase the quantity of all or subsets of blood cells, such as, for example, erythrocytes, lymphocytes (B or T cells), myeloid cells (*e.g*., basophfis, eosinophtis, neutrophls, mast cells, macrophages) and platelets. The ability to decrease the quantity of blood cells or subsets of blood cells may be useful in the prevention, detection, diagnosis, and/or treatment of anemias and leukopenias described below. Alternatively, the modified transferrin fusion proteins, of the invention and/or polynucleotides encoding transferrin fusion proteins of the invention maybe used to decrease the quantity of all or subsets of blood cells, such as, for example, erythrocytes, lymphocytes (B or T cells), myeloid cells (*e.g*., basophils, eosinoptifis, neutrophils, mast cells, macrophages) and platelets. The ability to decrease the quantity of blood cells or subsets of blood cells may be useful in the prevention, detection, diagnosis, and/or treatment of leukocytoses, such as, for example eosinophilia. The modified fusion proteins of the invention and/or polynucleotides encoding transferrin fusion proteins of the invention may be used to prevent, treat, or diagnose blood dyscrasia.

Anemias are conditions in which the number of red blood cells or amount of hemoglobin (the protein that carries oxygen) in them is below normal. Anemia may be caused by excessive bleeding, decreased red blood cell production, or increased red blood cell destruction (hemolysis). The modified transferrin fusion proteins of the invention and/or polynucleotides encoding transferrin fusion proteins of the invention may be useful in treating, preventing, and/or diagnosing anemias. Anemias that may be treated prevented or diagnosed by the transferrin fusion proteins of the invention and/or polynucleotides encoding transferrin fusion proteins of the invention include iron deficiency anemia, hypochromic anemia, microcytic anemia, chlorosis, hereditary sideroblastic anemia, idiopathic acquired sideroblastic anemia, red cell aplasia, megaloblastie anemia (*e*.*g*., pemiciovis anemia, (vitamin B12 deficiency) and folic acid deficiency anemia), aplastic anemia, hemolytic anemias (*e*.*g*., autoinunune helolytic anemia, microarigiopathic hemolytic anemia, and paroxysmal nocturnal hemoglobinunía). The modified transferrin fusion proteins of the invention and/or polynucleotides encoding transferrin fusion proteins of the invention may be useful in treating, preventing, and/or diagnosing anemias associated with diseases including but not limited to, anemias associated with systemic lupus erythematosus, cancers, lymphomas, chronic renal disease, and enlarged spleens. The transferrin fusion proteins of the and/or polynucleotides encoding transferrin fusion proteins of the invention may be useful in treating, preventing, and/or diagnosing anemia arising from drug treatments such as anemias associated with methyldopa, dapsone, and/or sulfa drugs. Additionally, modified fusion proteins of the invention and/or polynucleotides encoding transferrin fusion proteins of the invention maybe useful in treating, preventing, and/or diagnosing anemias associated with abnormal red blood cell architecture including but not limited to, hereditary spherocytosis, hereditary elliptocytosis, glucose-6-phosphate dehydrogenase deficiency, and sickle cell anemia.

The modified transferrin fusion proteins of the invention and/or polynucleotides encoding transferrin fusion proteins of the invention may be useful in treating, preventing, and/or diagnosing hemoglobin abnormalities, (*e.g*., those associated with sickle cell anemia, hemoglobin C disease, hemoglobin S-C disease, and hemoglobin E disease). Additionally, the transferrin fusion proteins of the invention and/or polynucleotides encoding transferrin fusion proteins of the invention may be useful in diagnosing, preventing, and/or prognosing in treating thalassemias, including, but not limited to, major and minor forms of alpha-thalassemia and beta-thalassemia.

In another embodiment, the modified transferrin fusion proteins of the invention and/or polynucleotides encoding transferrin fusion proteins of the invention may be useful in diagnosing, prognosing, preventing, and/or treating bleeding disorders including, but not limited to, thrombocytopenia (*e.g*., idiopathic thrombocytopenic purpura, and thrombotic thrombocytopenic purpura), Von Willebrand's disease, hereditary platelet disorders (*e.g*., storage pool disease such as Chediak-Higashi and Hermansky-Pudlak syndromes, thromboxane A2 dysfunction, thromboasthenia, and Bernard-Soulier syndrome), hemolyticuremic syndrome, hemophelias such as hemophelia A or Factor V-11 deficiency and Christmas disease or Factor IX deficiency, Hereditary Hemorhhagic Telangiectsia, also known as Rendu-Osler-Webe syndrome, allergic purpura (Henoch Schonlein purpura) and disseminated intravascular coagulation.

In other embodiments, the modified transferrin fusion proteins of the invention and/or polynucleotides encoding transferrin fusion proteins of the invention may be useful as an agent to increase cytokine production.

Hyperproliferative disorders in certain embodiments, fusion proteins of the invention, and/or polynucleotides encoding transferrin fusion proteins of the invention can be used to treat or detect hyperproliferative disorders, including neoplasms. Transferrin fusion proteins of the invention and/or polynucleotides encoding transferrin fusion proteins of the invention may inhibit the proliferation of the disorder through direct or indirect interactions. Alliteratively, fusion proteins of the invention and/or polynucleotides encoding transferrin fusion proteins of the invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alliteratively, decreasing an immune response may also be a method of treating hyperproliferative disorders, such as a chemotherapeutic agent.

Examples of hyperproliferative disorders that can be treated or detected by modified fusion proteins of the invention and/or polynucleotides encoding transferrin fusion proteins of the invention include, but are not limited to neoplasms located in the: colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvis, skin, soft tissue, spleen, thorax, and urogenital tract.

Similarly, other hyperproliferative disorders can also be treated or detected by modified fusion proteins of the invention and/or polynucleotides encoding transferrin fusion proteins of the invention. Examples of such hyperproliferative disorders include, but are not limited to Acute Childhood Lymphoblastic Leukemia; Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphorria, Adult Lymphocytic Leukemia, Adult Non-Hodgkin's Lymphoma, Adult Primary Liver Cancer, Adult Soft Tissue Sarcoma, AIDS-Related Lymphorria, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphorria, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalanic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma. Endometrial Cancer, Ependymoma, Epithelial Cancer, Esophageal Cancer, Ewing's Sarcoma and Related Tumors, Exocrine Pancreatic Cancer, Extraeranial Germ Cell Tumor, Extragonadal Germ Cell Tumor, Extrahepatie Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lympho proliferative Disorders, Macroglobulinemia, Male Breast Cancer, Malignant Mesothelioma, Malignant Thymoma, Medulloblastomia, Melanoma, Mesothelioma, Metastatie Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyrigeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo/Malignant Fibrous Sarcoma,Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid, Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethial Cancer, Uterine Cancer, Uterine Sarcoma, Vaginal Cancer, Visual Pathway and Hypothalarruc Glioma, Vulvar Cancer, Waldenstroin's Macroglobulinemia, Wilm's Tumor, and any other hyperproliferative disease, besides neoplasia, located in an organ system listed above.

In another preferred embodiment, modified transferrin fusion proteins of the invention and/or polynucleotides encoding transferrin fusion proteins of the invention are used to, diagnose, prognose, prevent, and/or treat premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth is consisting of hyperplasia, metaplasia, or most particularly, dysplasia has occurred (for review of such abnormal growth conditions, see Robbins. and Angell, 1976, Basic Pathology, 2d Ed. W. B. Saunders Co., Philadelphia, pp. 68-79).

Hyperplasia is a form of controlled cell proliferation, involving an increase in cell number in a tissue or organ, without, significant alteration in structure or function. Hyperplastic disorders which can be diagnosed, prognosed, prevented, and/or treated with fusion proteins of the invention and/or polynucleotides encoding transferrin fusion proteins of the invention include, but are not limited to, angiofollicular mediastinal lymph node hyperplasia, angiolymphoid hyperplasia with eosinophilia, atypical melanocytic hyperplasia, basal cell hyperplasia, benign giant lymph node hyperplasia, cementum hyperplasia, congenital adrenal hyperplasia, congenital sebaceous hyperplasia, cystic hyperplasia, cystic hyperplasia of the breast, denture hyperplasia, ductal hyperplasia, endometrial hyperplasia, fibromuscular hyperplasia, foca epithelial hyperplasia, gingival hyperplasia, inflammatory fibrous hyperplasia, inflammatory papillary hyperplasia, intravascular papillary endothelial hyperplasia, nodular hyperplasia of prostate, nodular regenerative hyperplasia, pseudoepitheliomatous hyperplasia, senile sebaceous hyperplasia, and verrucous hyperplasia.

In another embodiment, modified transferrin fusion proteins of the invention and/or polynucleotides encoding transferrin fusion proteins of the invention conjugated to a toxin or a radio active isotope, as described herein, may be used to treat cancers and neoplasms, including, but not limited to, those described herein. In a further preferred embodiment, transferrin fusion proteins of the invention and/or polynucleotides encoding transferrin fusion proteins of the invention conjugated to a toxin or a radioactive isotope, as described herein, may be used to treat acute myelogenous leukemia.

Additionally, modified fusion proteins of the invention and/or polynucleotides encoding transferrin fusion proteins of the invention may affect apoptosis, and therefore, would be useful in treating a number of diseases associated with increased cell survival or the inhibition of apoptosis. For example, diseases associated with increased cell survival or the inhibition of apoptosis that could be diagnosed, prognosed, prevented, and/or treated by polynucleotides, polypeptides, and/or agonists or antagonists of the invention, include cancers (such as follicular- lymphomas, carcinomas with p53 mutations, and hormone-dependent tumors, including, but not limited to, colon cancer, cardiac tumors, pancreatic cancer, melanoma, retinoblastoma, glioblastoma, lung cancer, intestinal cancer, testicular cancer, stomach cancer, neuroblastoma, myxoma, inyoma, lymphoma, endothelioma, osteoblastoma, osteoclastoma, osteosarcoma, chondrosarcoma, adenoma, breast cancer, prostrate cancer, Kaposi's sarcoma and ovarian cancer); autoimmune disorders such as, multiple sclerosis, Sjogren's syndrome, Hashimoto's thyroiditis, biliary cirrhosis, Behcet's disease, Crohn's disease, polymyosifis, systemic lupus erythematosus and immune-related glomeruionephritis and rheumatoid arthritis) and viral infections (such as herpes viruses, pox viruses and adenoviruses), inflammation, graft v. host disease, acute graft rejection, and chronic graft rejection.

In preferred embodiments, modified fusion proteins of the invention and/or polynucleotides encoding transferrin fusion proteins of the invention are used to inhibit growth, progression, and/or metastasis of cancers, in particular those listed above.

Additional diseases or conditions associated with increased cell survival that could be diagnosed, prognosed, prevented, and/or treated by modified fusion proteins of the invention and/or polynucleotides encoding transferrin fusion proteins of the invention, include but are not limited to, progression, and/or metastases of malignancies and related disorders such as leukemia (including acute leukemia (*e.g*., acute lymphocytic leukemia, acute myelocytic leukemia (including myeloblastic, promyelocytic, mylomonocytic, monocytic, and erythroleukemia)) and chronic leukemia (*e.g*., chronic myelocytic (granulocytic) leukemia and chronic lymphocytic leukemia)), polycythemia vera, lymphomas (*e.g*., Hodgkin's disease and non-Hodgkin's disease), multiple myeloma, Waldenstrom's macroglobulinemia, heavy chain disease, and solid tumors including, but not limited to, Sarcomas and, carcinomas such as fibrosarcoma, myxosarcoma, fiposarcoma, chondrosarcoma, osteogenic sarcoma, chordoma, anglosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendrogliomia, menangioma, melanoma, neuroblastoma, and retinoblastoma.

Diseases associated with increased apoptosis that could be diagnosed, prognosed, prevented, and/or treated by modified fusion proteins of the invention and/or polynucleotides encoding transferrin fusion proteins of the invention, include AIDS; neurodegenerative disorders (such as Alzheimer's disease, Parkinson's disease, amyotrophic lateral sclerosis, retinitis pigmentosa, cerebral degeneration and brain tumor or prior associated disease); autoimmune disorders (such as, multiple sclerosis, Sjogren's syndrome, Hashimoto's thyroiditis, biliary cirrhosis, Behcet's disease, Crohn's disease, polymyositis, systemiclupus erythematosus and immune-related glomerulonephritis and rheumatoid arthritis) myelodysplastic syndromes (such as a plastic anemia), graft Y host disease, ischemic injury (such as that caused by myocardial infarction, stroke and repercussion injury), liver injury (*e.g*., hepatitis related liver injury, ischemia/Eeperfusion injury, cholestosis (bile duct injury) and liver cancer); toxin-induced liver disease (such as that caused by alcohol), septic shock, cachexia and anorexia.

Another preferred embodiment utilizes polynucleotides encoding modified transferrin fusion proteins of the invention to inhibit aberrant cellular division, by gene therapy using the present invention, and/or protein fusions or fragments thereof.

Thus, the present invention provides a method for treating cell proliferative disorders by inserting into an abnormally proliferating cell a polynucleotide encoding modified transferrin fusion protein of the present invention, wherein said polynucleotide represses said expression.

Another embodiment of the present invention provides a method of treating cell proliferative disorders in individuals comprising administration of one or more active gene copies of the present invention to an abnormally proliferating cell or cells.

The polynucleotides of the present invention may be delivered directly to cell proliferative disorderly disease sites in internal organs, body cavities, and the like by use of imaging devices used to guide an injecting needle directly to the disease site. The polynucleotides of the present invention may also be administered to disease sites at the time of surgical intervention.

By cell proliferative disease is meant any human or animal disease or disorder, affecting any one or any combination of organs, cavities, or body parts, which is characterized by single or multiple local abnormal proliferations of cells, groups of cells, or tissues, whether benign or malignant.

Any amount of the polynucleotides of the present invention may be administered as long as it has a biologically inhibiting effect on the proliferation of the treated cells.

Moreover, it is possible to administer more than one of the polynucleotides of the present invention simultaneously to the same site. By "biologically inhibiting" is meant partial or total growth inhibition as well as decreases in the rate of proliferation or growth of the cells.

Modified transferrin fusion proteins of the invention and/or polynucleotides encoding transferrin fusion proteins of the invention are useful in inhibiting the metastasis of proliferative cells or tissues. Inhibition may occur as a direct result of administering these transferrin fusion proteins and/or polynucleotides, or indirectly, such as activating the expression of proteins known to inhibit metastasis, for example alpha, integrins, (See, *e.g*., Curr Top Mirobiol Immunol 1998; 231:1 41, which is hereby incorporated by reference). Such therapeutic affects of the present invention may be achieved either alone, or in combination with small molecule drugs or adjutants.

In another embodiment, the invention provides a method of delivering compositions containing the transferrin fusion proteins of the invention and/or polynucleotides encoding transferrin fusion proteins of the invention to targeted cells expressing the a polypeptide bound by, that binds to, or associates with a modified transferrin fusion protein of the invention. Transferrin fusion proteins of the invention may be associated with heterologous polypeptides, heterologous nucleic acids, toxins, or prodrugs via hydrophobic, hydrophilic, ionic and/or covalent interactions.

Kidney diseases which can be diagnosed, prognosed, prevented, and/or treated with compositions of the invention include, but are not limited to, acute kidney failure, chronic kidney failure, atheroembolic renal failure, end-stage renal disease, inflammatory diseases of the kidney (*e*.*g*., acute glomerulonephritis, post infectious glomerulonephritis, rapidly progressive glomerulonephritis, nephritic syndrome, membranous glomeruionephritis, familial nephritic syndrome, membrane proliferative glomerulonephritis and mesangial proliferative glomerulonephritis, chronic glomerulonephritis, acute tubulo intestinal nephritis, chronic tubulointerstitial nephritis, acute post-streptococcal glomeruionephritis(PSGN), pyelonephritis, lupus nephritis, chronic nephritis, interstitial nephritis, and post streptococcal glomerulonephritis), blood vessel disorders of the kidneys (*e.g*., kidney infarction, atheroembolic kidney disease, cortical necrosis, malignant nephrosclerosis, renal vein thrombosis, renal under perfusion, renal retinopathy, renal ischemia-reperfusion, renal artery embolism and renal artery stenosis), and kidney disorders resulting form urinary tract disease (*e.g*., pyelonephritis, hydronephrosis, urolithiasis (renal lithiasis, nephrolithiasis), reflux nephropathy, urinary tract infections, urinary retention, and acute or chronic unilateral obstructive uropathy). In addition, compositions of the invention can be used to diagnose, prognose, prevent, and/or treat metabolic and congenital disorders of the kidney (*e*.*g*., uremia, renalamyloidosis, renal osteodystrophy, renal tubular acidosis, renal glycosuria, nephrogenic diabetes insipidus, cystinuria, Fanconi's syndrome, renal fibrocystic osteosis (renal rickets), Hartnup disease, Bartter's syndrome, Liddle's syndrome, polycystic kidney disease, medullary cystic disease, medullary sponge kidney, Alport's syndrome, nail-patella syndrome, congenital nephritic syndrome, CRUSH syndrome, horseshoe kidney, diabetic nephropathy, nephrogenic diabetes insipidus, analgesic nephropathy, kidney stones, and membranous nephropathy), and autoimmune disorders of the kidney (*e.g*., systemic lupuserythematosus (SLE), Good pasture syndrome, IgA nephropathy, and ICFM mesangial proliferative glomerulonephritis).

Compositions of the invention can also be used to diagnose, prognose, prevent, and/or treat sclerotic or lecrotic disorders of the kidney (*e.g*., glomeruloselerosis, diabeticnephropathy, faca Fsegmental glomerulo sclerosis (FSGS), narcotizing glomerulonephritis, and renal papillary necrosis), cancers of the kidney (*e.g*., nephroma, hypemephroma, nephroblastoma, renal cell cancer, transitional cell cancer, renal adenocarcinoma, squamous cell cancer, and Wilm's tumor), and electrolyte imbalances (*e.g*., nephrocalcinosis, pyuria, edema, hydronephritis, proteinuria, hyponatrerrua, hypematremia, hypokalemia, hyperkalemia, hypocalcemia, hypercalcemia. hypophosphatemia, and hyperphosphatemia).

Compositions of the invention may be administered using any method known in the art, including, but not limited to, direct needle injection at the delivery site, intravenous injection, topical administration, catheter infusion, biolistic injectors, particle accelerators, gel foam sponge depots, other commercially available depot materials, osmotic pumps, oral or suppositorial solid pharmaceutical formulations, decanting or topical applications during surgery, aerosol delivery. Such methods are known in the art. Compositions of the invention may be administered as part of a Therapeutic, described in more detail below.

Modified transferrin fusion proteins of the invention and/or polynucleotides encoding transferrin fusion proteins of the invention, may be used to treat, prevent, diagnose, and/or prognose cardiovascular disorders, including, but not limited to, peripheral artery disease, such as limb ischemia.

Cardiovascular disorders, includes, but is not limited to, cardiovascular abnormalities, such as arterio arterial fistula, arterioyenous fistula, cerebral arteriovertous malformations, congenital heart defects, pulmonary atresia, and Scimitar Syndrome.

Congenital heart defects include, but are not limited to, aortic coarctation, cortriatriatum, coronary vessel anomalies, crisscross heart, dextrocardia, patent ductus arteriosus, Ebstein's anomaly, Eisenmenger complex, hypoplastic left heart syndrome, levocardia, tetralogy of fallot, transposition of great vessels, double outlet right ventricle, tricuspidatresia, persistent truncus arteriosus, and heart septal defects, such as aortopulmonary septald defect, endocardial cushion defects, Lutembacher's Syndrome, trilogy of Fallot, ventricular heart septal defects.

Cardiovascular disorders also include, but are not limited to, heart disease, such asamhythmias, carcinoid heart disease, high cardiac output, low cardiac output, cardiactamponade, endocarditis (including bacteria), heart aneurysm, cardiac arrest, congestive heart failure, congestive cardiomyopathy, paroxysmal dyspnea, cardiac edema, heart hypertrophy, congestive cardiomyopathy left ventricular hypertrophy, right ventricularhypertrophy, post-infarction heart rupture, ventricular septal ruoture, heart valve diseases myocardial diseases, myocardial ischemia, pericardial effusion, pericarditis (including constrictive and tuberculous), pricumopericardium, post pericardiotomy syndrome, pulmonary heart disease, rheumatic heart disease, ventricular dysfunction, hyperemia, cardiovascular pregnancy complications, Scimitar Syndrome, cardiovascular syphilis, and cardiovascular tuberculosis.

Arrhythmias include, but are not limited to, sinus arrhythmia, atrial fibrillation, atrial flutter, bradycardia, extrasystole, Adams-Stokes Syndrome, bundle-branch block, sinoatrial block, long QT syndrome, parasystole, Lown-Ganong-Levine Syndrome, Mahaim-type pre-excitation syndrome, Wolff-Parkinson-White syndrome, sick sinus syndrome, itachycardias, and ventricular fibrillation. Tachycardias include paroxysmal tachycardia, suprayentriculai tachycardia, accelerated idioventricular rhythm, atrioventricular nodal reentry tachyeardia, ectopic atrial tachycardia, ectopic junctional tachycardia, sinoattial nodalreentry tachycardia, sinus tachycardia, Torsades de Pointes, and ventricular tachycardia.

Heart valve diseases include, but are not limited to, aortic valve insufficiency aorticvalve stenosis, hear murmurs, aortic valve prolapse, neutral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

Myocardial diseases include, but are not limited to, alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Myocardial schemias include, but are not limited to, coronary disease, such as angina pectoris, coronary aneurysm, coronary arteriosclerosis, coronary thrombosis, coronary vasospasm, myocardial infarction, and myocardial stunning.

Cardiovascular diseases also include vascular diseases such as aneurysms, angiodysplasia, angiomatosis, bacillary arigiomiatosis, Hippel-Lindau Disease, Klippel Trenaunay Weber Syndrome, Sturge Weber Syndrome, angioneurotic edema, aorfic diseases, Takayasu's Arthritis, aortitis, Leriche's Syndrome, arterial occlusive diseases, arthritis, enarteritis, polyarteritis nodosa, cerebrovascular disorders, diabetic angiopathies, diabetic retinopathy, embolisms, thrombosis, erythromelalgia, hemorrhoids, hepatic veno-occlusive disease, hypertension, hypotension, ischemia, peripheral vascular diseases, phlebitis, pulmonary veno-occlusive disease, Raynaud's disease, CREST syndrome, retinal vein occlusion, Scimitar syndrome, superior vena cava syndrome, telangiectasia, ataciatelangiectasia, hereditary hemorrhagic telangiectasia, varicocele, varicose veins, varicoseulcer, vasculitis, and venous insufficiency.

Cerebrovascular disorders include, but are not limited to, cardio artery diseases, Respiratory Disorders Transferrin fusion proteins of the invention and/or polynucleotides encoding transferrin fusion proteins of the invention may be used to treat, prevent, diagnose, and/or prognose diseases and/or disorders of the respiratory system.

Diseases and disorders of the respiratory system include, but are not limited to, nasalvestibulitis, nonallergic rhinitis (*e.g*., acute rhinitis, chronic rhinitis, atrophic rhinitis, vasomotor rhinitis), nasal polyps, and sinusitis, juvenile angiofibromas, cancer of the nose and juvenile papillomas, vocal cord polyps, nodules (singer's nodules), contact ulcers, vocal cord paralysis, laryngoceles, pharynefitis (*e.g*., viral and bacterial), tonsillitis, tonsillar cellulitis, parapharyrigeal abscess, laryngitis, laryngoceles, and throat cancers (*e.g*., cancer of the nasopharynx, tonsil cancer, larynx cancer), lung cancer (*e.g*., squamous cell carcinoma, small cell (oat cell) carcinoma, large cell carcinoma, and adenocarcinoma), allergic disorders (eosinophilie pneumonia, hypersensitivity pneumonitis (*e.g*., extrinsicallergic alveolitis, allergic interstitial pneumonitis, organic dust pneumoconiosis, allergic bronchopulmoniary aspergillosis, asthma, Wegener's granulomatosis (granulomatousvasculifis), Goodpasture's syndrome)), pneumonia (*e*.*g*., bacterial pneumonia (*e.g*., Streptococcus pneumoniae (pneumoncoccal pneumonia), Staphylococcus aureus (staphylococeal pneumonia), Gram negative bacteria pneumonia (caused by, *e.g*., Klebuella and Pseudomas spp.), Mycoplasma pneumoniae pneumonia, Hemophilus influenza pneumonia, Legionella pneumophila (Legionnaires' disease), and Chlamydapsittaci (Psittacosis)), and viral pneumonia (*e.g*., influenza, chickenpox (varicella).

Additional diseases and disorders of the respiratory system include, but are not limited to bronchiolitis, polio (poliomyelitis), croup, respiratory syncytial viral infection, mumps, erythema infectiosum (fifth disease), roseola infantum, progressive rubellapanencephalitis, German measles, and subacute selerosing panencephalitis), fungal pneumonia (*e.g*., Histoplasmosis, Coccidioidomycosis, Blastomycosis, fungal infections in people with severely suppressed immune systems (*e.g*., cryptocoecosis, caused by Cryptococcus neoformans; aspergillosis, caused by Aspergillus spp.) candidiasis, caused by Candida; and mucormycosis)), Pneumocystl's carinu (pneumocystis pneumonia), atypicalpneumonias (*e.g*., Mycoplasma and Chlamyda spp.), opportunistic infection pneumonia, nosocomial pneumonia, chemical pneumonitis, and aspiration pneumonia, pleural disorders(*e.g*., pleurisy, pleural effusion, and pneumothorax (*e.g*., simple spontaneous pneumothorax, complicated spontaneous pneumothorax, tension pneumothorax)),obstructive airway diseases (*e.g*., asthma,. chronic obstructive pulmonary disease (COPID),emphysema, chronic or acute bronchitis), occupational lung diseases (*e*.*g*., silicosis, blacklung (coal workers' pneumoconiosis , asbestosis, berylliosis, occupational asthma, byssinosis, and benign pritumoconioses), Infiltrative Lung Disease (*e.g*., pulmonary fibrosis (*e.g*., fibrosincralveolifi, usual interstitial pneumonia), idiopathic pulmonary fibrosis, desquamative interstitial pneumonia, lymphoid interstitial pnetimonia, histiocytosis (*e.g*., Letterer-Siwe disease, Hand-Schuller-Christian disease, eoslnophific granuloma), idiopathic pulmonary hemosiderosis, sarcoidosis and pulmonary, alveolar proteinosis), Acute respiratory distress syndrome (also called, *e*.*g*., adult respiratory distress syndrome), edema, pulmonary embolism, bronchitis (*e.g*., viral, bacterial), bronchiectasis, atelectasis, lung abscess (caused by, *e.g*., Staphylococcus aureus or Legionella pneumophila), and cystic fibrosis.

Cancers which may be treated with modified fusion proteins of the invention and/or polynucleotides encoding transferrin fusion proteins of the invention include, but are not limited to solid tumors, including prostate, lung, breast, ovarian, stomach, pancreas, larynx, esophagus, lesteg, liver, parotid, biliary tract, colon, rectuffi, cervix, uterus, 1endometrium, kidney, bladder, thyroid cancer; primary tumors and metastases; melanomas; glioblastoma; Kaposi's sarcoma; leiomyosarcoma; non- small cell lung cancer; colorectal cancer; advanced malignancies; and blood born tumors such as leukemia. For example, fusion proteins of the invention and/or polynucleotides encoding transferrin fusion proteins of the invention may be delivered topically, in order to treat cancers such as skin cancer, head and neck tumors, breast tumors, and Kaposi's sarcoma.

Modified transferrin fusion proteins of the invention and/or polynucleotides encoding transferrin fusion proteins of the invention may be useful, in treating other disorders, besides cancers, which involve angiogenesis. These disorders include, but are not limited to: benigri tumors, for example hemanglomas, acoustic neuromas, neurofibromas, trachomas, and pyogenicgranulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygiaab normal blood vessel growth) of the eye; rheumatoid arthritis; psoriasis; delayed wound healing; endometriosis; vasculogenesis; grantilations; hypertrophic scars (keloids); nonunion fractures; scleroderma; trachoma; vascular adhesions; myocardial angiogenesis; coronary collaterals; cerebral collaterals; arteriovenotis malformations; ischemic limb angiogenesis; Osler-Webber Syndrome; plaque neovascularization; telangiectasia; hemophiliac joints; angiofíbroima; fibromuscular dysplasia; wound granulation; Crohn's disease; and atherosclerosis.

Thus, within one aspect of the present invention methods are provided for treating neovascular diseases of the eye.

Additionally, disorders which can be treated with modified fusion proteins of the invention and/or polynucleotides encoding transferrin fusion proteins of the invention include, but are not limited to, hemangioma, arthritis, psoriasis, angiofibroma, atherosclerotic plaques, delayed wound healing, granulations hemophilic joints hypertrophic scars, nonunion fractures, Osler-Weber syndrome, pyogenic granuloma, scleroderma, trachoma; and vascular adhesions.

Moreover, disorders and/or states, which can be treated, prevented, diagnosed, and/or prognosed with the modified transferrin fusion proteins of the invention and/or polynucleotides encoding transferrin fusion proteins of the invention include, but are not limited to, solid tumors, blood born tumors such as leukemia, tumor metastasis, Kaposi's sarcoma, benign tumors, for example hemangiomas, acoustic neuromas, neurofibromas, trachomas, and pyogenic granulomas, rheumatoid arthritis, psoriasis, ocularangiogenic diseases, for example, diabetic retinopathy, retinopathy of prematurity, macular degeneration, corneal graft rejection, neovascular glaucoma, retrolental fibroplasia, rubeosis, refinoblastoma, and uvietis, delayed wound healing, endometriosis, vascluogenesis, granulations, hypertrophic scars (keloidsj, nonunion fractures, scleroderma, trachoma, vascular adhesions, myocardial angiogenesis, coronary collaterals, cerebral collaterals, arteriovenous malformations, ischemic limb angiogenesis, Osler-Webber Syndrome, plaque neovascularization, telangiectasia, hemophiliac joints, angiofibroma fibromuscular dysplasia, wound granulation, Crohn's disease, atherosclerosis, birth control agent by preventing vascularization required for embryo, implantation controlling menstruation, diseases that have angiogenesis as a pathologic consequence such as cat scratch disease (Rochele nunalia quintosa), ulcers (Helicobacterpylori), Bartonellosis and baculary angiomatosis.

In one aspect of the birth control method, an amount of the compound sufficient to block embryo implantation is administered before or after intercourse and fertilization have occurred, thus providing an effective method of birth control, possibly a "morning after" method. Modified transferrin fusion proteins of the invention and/or polynucleotides encoding transferrin fusion proteins of the invention may also be used in controlling menstruation or administered as either a peritoneal lavage fluid or for peritoneal implantation in the treatment of endometriosis.

Modified transferrin fusion proteins of the invention and/or polynucleotides encoding transferrin fusion proteins of the invention may be utilized in a wide variety of surgical procedures.

Diseases associated with increased cell survival or the inhibition of apoptosis that could be treated, prevented, diagnosed, and/or prognosed using modified fusion proteins of the invention and/or polynucleotides encoding transferrin fusion proteins of the invention, include cancers (such as follicular lymphomas, carcinomas with mutations, and hormone-dependent tumors, including, but not limited to colon cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune disorders (such as, multiple sclerosis, Sjogren's syndrome, Hashimoto's thyroiditis, biliary cirrhosis, Behcet's disease, Crohn's disease, polymyositis, systemic lupus thematosus and immune-related ryglomerulonephritis and rheumatoid arthritis) and viral infections (such as herpes viruses, pox viruses and adenoviruses), inflammation, graft v. host disease, acute graft rejection, and chronic graft rejection.

In preferred embodiments, modified fusion proteins of the invention and/or polynucleotides encoding transferrin fusion proteins of the invention are used to inhibit growth, progression, and/or metasis of cancers, in particular those listed above.

Additional diseases or conditions associated with increased cell survival that could be treated or detected by modified fusion proteins of the invention and/or polynucleotides encoding, transferrin fusion proteins of the invention include, but are not limited to, progression, and/or metastases of malignances and related disorders such as leukemia (including acute leukemia (*e.g*., acute lymphocytic leukemia, acute myelocytic leukemia (including myeloblastic, promyelocytic, myelomonocytic, monocytic, and erythroleukemia)) and chronic leukemia (*e.g*., chronic myelocytie (granulocytic) leukemia and chroniclymphocytic leukemia)), polycytemia vera, lymphomas (*e.g*., Hodgkin's disease and non-Hodgkin's disease), multiple myeloma, Waldenstrom's macroglobulinemia, heavy chain disease, and solid tumors including, but not limited to, Sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, civarian cancer, prostate cancer, squamous cell carcinoma, basa cell carcinoma, adenocarcinoma, sweat gland carcinoma, sebaceous aland carcinoma, papillary carcinoma, papillary adenocarcinomas, cystadenocarcinoma, medullary carcinoma, bronchogeniccarcinoma, renal cell carcinoma, hepatoma, bile duct carcinoma, choriocarcinoma, seminoma, embryonal carcinoma, Wilm's tumor, cervical cancer, testicular tumor, Jung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma neuroblastoma, and retinoblastoma.

Diseases associated with increased apoptosis that could be treated, prevented, diagnosed, and/or prognosed using modified fusion proteins of the invention and/or polynucleotides encoding transferrin fusion proteins of the invention, include, but are not limited to, AIDS; neurodegenerative disorders (such as Alzthmer's disease, Parkinson's disease, Amyotrophic lateral sclerosis, Retinitis pigmentosa, Cerebellar degeneration and brain tumor or prior associated disease); autoimmune disorders (such as, multiple sclerosis, Sjogren's syndrome, Hashimoto's thyroiditis, biliary cimhosis, Behcet's disease, Crohn's' disease, polymyositis, systemic lupus erythematosus and immune-related glomerulonephritis and rheumatoid arthritis) Myelodysplastic syndromes (such as aplasiic anemia), graft v. host disease, ischenuc injury (such as that caused, by myocardial. infarction, stroke and reperfusion injury), liver injury (*e.g*., hepatitis related liver injury, ischemia/reperfusion injury, cholestosis (bile duct injury) and liver cancer); toxin-induced liver disease (such as that caused by alcohol), septic shock, cachexia and anorexia.

In addition, modified fusion proteins of the invention and/or polynucleotides encoding transferrin fusion proteins of the invention could be, used to treat or prevent the onset of diabetes mellitus. In patients with newly diagnosed Types 1 and 11 diabetes, where some islet cell function remains, fusion proteins of the invention and/or polynucleotides encoding transferrin fusion proteins of the invention, could be used to maintain the islet function so as to alleviate, delay or prevent permanent manifestation of the disease. Also, fusion proteins of the invention and/or polynucleotides encoding transferrin fusion proteins of the invention could be used as an auxiliary in islet cell transplantation to improve or promote islet cell function.

The modified transferrin fusion proteins of the invention and/or polynucleotides encoding transferrin fusion proteins of the invention may be used for the diagnosis and/or treatment of diseases, disorders, damage or injury of the brain and/or nervous system. Nervous system disorders that can be treated with the compositions of the invention (*e.g*., fusion proteins of the invention and/or polynucleotides encoding transferrin fusion proteins of the invention), limited to nervous systems include, but are not limited injuries, and diseases or disorders which result in either a disconnection of axons, a diminution or degeneration of neurons, ordemyelination. Nervous system lesions which may be treated in a patient (including human and non-human mammalian patients) according to the methods of the invention, include but are not limited to, the following lesions of either the central (including spinal cord, brain) or peripheral nervous systems: (1) ischemic lesions, in which a lack of oxygen in a portion of the nervous system results in neuronal injury or death, including cerebral infarction orischemia, or spinal cord infarction or ischemia; (2) traumatic lesions, including lesions caused by physical injury or associated with surgery, for example, lesions which sever a portion of the nervous system, or compression injuries; (3) malignant lesions, in which a portion of the nervous system is destroyed or injured by malignant tissue which is either a nervous system associated malignancy or a malignancy derived from nervous system tissue; (4) infectious lesions in which a portion of the nervous system is destroyed or injured as a result of infection, for example, by an abscess or associated with infection by human immunodeficiency virus, herpes zoster, or herpes simplex virus or with Lyme disease, tuberculosis, or syphilis; (5) degenerative lesions, in which a portion of the nervous system is destroyed or injured as a result of a degenerative process including but not limited to, degeneration associated with Parkinson's disease, Alzheimer's disease, Huntington's chorea, or amyotrophic lateral sclerosis (ALS); (6) lesions associated with nutritional diseases or disorders, in which a portion of the nervous system is destroyed or injured by a nutritional disorder or disorder of metabolism including, but not limited to vitamin B 12 deficiency, folic acid deficiency, Wernicke disease, tobacco-alcohol amblyopic, Marchiafava-Blanami disease (primary degeneration of the corpus callosum), and alcoholic cerebral degeneration; (7) neurological lesions associated with systemic diseases including, but not limited to diabetes (diabetic neuropathy, Bell's palsy), systemic lupuserythematosus, carcinoma, or sarcoidoisis; (8) lesions caused by toxic substances including alcohol, lead, or particular, neurotoxins; and (9) demyelinated lesions in which a portion of the nervous system is destroyed or injured by a demyelinating disease including, but not limited to, multiple sclerosis, human immunodeficiency virus-associated myelopathy, transverse myelopathy or various etiologies, progressive multifocal leukoencephalopathy, and central pontine myelinolysis.

In one embodiment, the modified transferrin fusion proteins of the invention and/or polynucleotides encoding transferrin fusion proteins of the invention are used to protect neural cells from the damaging effects of hypoxia. In a further preferred embodiment, the modified transferrin fusion proteins of the invention and/or polynucleotides encoding transferrin fusion proteins of the invention are used to protect neural cells from the damaging effects of cerebral hypoxia.

In specific embodiments, motor neuron disorders that may be treated according to the invention include, but are not limited to, disorders such as infarction, infection, exposure to toxin, trauma, surgical damage, degenerative disease or malignancy that may affect motor neurons as well as other components of the nervous system, as well as disorders that selectively affect neurons such as amyotrophic lateral sclerosis, and including, but not limited to, progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motor sensory Neuropathy (Charcot-Marie-Tooth Disease).

Further, modified fusion proteins of the invention and/or polynucleotides encoding transferrin fusion proteins of the invention may play a role in neuronal survival; synapse formation; conductance; neural differentiation, etc. Thus, compositions of the invention (including fusion proteins of the invention and/or polynucleotides encoding transferrin fusion proteins of the invention) may be used to diagnose and/or treat or prevent diseases or disorders associated with these roles, including, but not limited to, learning and/or cognition disorders. The compositions of the invention may also be useful in the treatment or prevention of neurodegenerative disease states and/or behavioral disorders. Such neurodegenerative disease states and/or behavioral disorders include, but are not limited to, Alzheimer's Disease, Parkinson's Disease, Huntington's Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception.

Examples of neurologic diseases which can be treated or detected with modified fusion proteins of the invention and/or polynucleotides encoding transferrin fusion proteins of the invention include, brain diseases, such as metabolic brain diseases which includes phenylketonuria such as maternal phenylketonuria, pyruvate carboxylase deficiency, pyruyate dehydrogenase complex deficiency, Wernicke's Encephalopathy, brain edema, brain neoplasms such as cerebellar neoplasms which include infratentorial neoplasms, cerebral ventricle neoplasms such as chorod plexus neoplasms, hypothalanic necoplasms, supratentorial neoplasms, canavan disease, cerebellar diseases such as cerebellar ataxia which include spinocerebellar degeneration such as ataxia telangiectasia, cerebellar dyssynergia, Friederich's Ataxia, Machado-Joseph Disease, olivopontocerebellar atrophy, cerebellar neoplasms such as infratentorial neoplasms, diffuse cerebral sclerosis such asencephalitis periaxialis, globoid cell leukodystrophy, metachromatic leukodystrophy and subacute sclerosing panencephalitis.

Additional neurologic diseases which can be treated or detected with modified fusion proteins of the invention and/or polynucleotides encoding transferrin fusion proteins of the invention include cerebrovascular disorders (such as carotid artery diseases which include carotid artery thrombosis, carotid stenosis and Moyamoya Disease), cerebral amyloid angiopathy, cerebral aneurysm, cerebral anoxia, cerebral arteriosclerosis, cerebral arterioyenous malformations, cerebral artery diseases, cerebral embolism and thrombosis such as carotid artery thrombosis, sinus thrombosis and Wallenberg's Syndrome, cerebral hemorrhage such as epidermal hematoma, subdural hematoma and subarachnoid hemorrhage, cerebral infarction, cerebral ischemia such as transient cerebral ischemia, Subclavian Steal Syndrome and vertebrobasilar insufficiency, vascular dementia such as multi-infarct dementia, periventricular leukomalacia, vascular headache such as cluster headache and migraine.

Additional neurologic diseases which can be treated or detected with modified fusion proteins of the invention and/or polynucleotides encoding transferrin fusion proteins of the invention include dementia such as AIDS Dementia Complex, presenile dementia such as Alzheimer's Disease and Creutzféldt-Jakob Syndrome, senile dementia such as Alzheimer's Disease and progressive supranuclear palsy, vascular dementia such as multi-infarct dementia, encephalitis which include encephalitis periaxialis, viral encephalitis such as epidemicencephalitis, Japanese Encephalitis, St. Louis Encephalitis, tick-borne encephalitis and West Nile Fever, acute disseminated encephalomyelitis, meningoencephalitis such as uveomeningoencephalitic syndrome, Postencephalitic Parkinson Disease and subacute sclerosing panencephalitis, encephalomalacia such as periventricular lieukomalacia, epilepsy such as generalized epilepsy, which includes infantile spasms, absence epilepsy, myoclonic epilepsy which includes MERRF Syndrome, tonic-clonic epilepsy, partial epilepsy such as complex partial epilepsy, frontal lobe epilepsy and temporal lobe epilepsy, post-traumatic epilepsy, status epilepticus such as Epilepsia Partialis Continua, and Hallervorden-Spatz Syndrome.

Additional neurologic diseases which can be treated or detected with modified fusion proteins of the invention and/or polynucleotides encoding transferrin fusion proteins of the invention include hydrocephalus such as Dandy-Walker Syndrome and normal pressure hydrocephalus, hypothalamic diseases such as hypothalamic neoplasms, cerebral malaria, narcolepsy which includes cataplexy, bulbar poliomyelitis, cerebripseudo tumor, Rett Syndrome, Reye's Syndrome, thalamic diseases, cerebral toxoplasmosis, intracranialtuberculoma and Zellweger Syndrome, central nervous system infections such as AIDS, Dementia Complex, Brain Abscess, subdural empyema, encephalomyelitis such as Equine Encephalomyelitis, Venezuelan Equine Encephalomyelitis, Necrotizing Hemorrhabaic Encephalomyelitis, Visna, and cerebral malaria.

Additional neurologic diseases which can be treated or detected with modified fusion proteins of the invention and/or polynucleotides encoding transferrin fusion proteins of the invention include meningitis such as araclinoiditis, aseptic meningitis such as viral meningitis which includes lymphocytic chronic meningitis, Bacterial meningitis which includes Haemophilus Meningitis, Listeria Meningitis, Meningococcal Meningitis such as Waterhouse-Fridericlisen Syndrome, Pneumococcal Meningitis and meningeal tuberculosis, fungal meningitis such as Cryptococcal Meningitis, subdural effusion, meniapencephalitis such as uvemenineroencephalitic syndrome, myelitis such as transverse myelitis, neurosyphilis such as tabes dorsalis, poliomyelitis which includes. bulbar poliomyelitis and post poliomyelitis syndrome, prion diseases (such as Creutzfeldt-Jakob Syndrome, Bovine Spongiform Encephalopathy, Gerstinann-Straussler Syndrome, Kuru, Scrapie), and cerebral toxoplasmosis.

Additional neurologic diseases which can be treated or detected with modified fusion proteins of the invention and/or polynucleotides encoding transferrin fusion proteins of the invention include central nervous system neoplasms such as brain neoplasms that include cerebellarneoplasms such as infratentorial neoplasms, cerebral ventricle neoplasms such as choroidplexus neoplasms, hypothalamic neoplasms and supratentorial neoplasms, meningealneoplasms, spinal cord neoplasms which include epidural neoplasms, demyelinating diseases such as Canavan Diseases, diffuse cerebral sculleries which include sadrenoleukodystrophy, encephalifis periaxialis, globoid cell leukodystrophy, diffuse cerebral sclerosis such as metachromatic leukodystrophy, allergic encephalomyelitis, necrotizina hemorrhagic encephalomyelitis, progressive multifocal leukoencephalopathy, in multiple sclerosis, central pontine iriyelinolysis, transverse myelitis, neuroinyelitis optica, Scrapie, Swayback, Chronic Fatigue Syndrome, Visna, High Pressure Nervous Syndrome, Meningism, spinal cord diseases such as arriyotonia congenita, amyotrophic lateral-sclerosis, spinal muscular atrophy such as Werdnig-Hoffmann Disease, spinal cord compression, spinal cord neoplasms such as epidural neoplasms, syringomyelia., Tabes Dorsalis, Stiff-Man Syndrome, mental retardation such as Angelman Syndrome, Cri-du-Chat Syndrome, De Lange's Syndrome, Down Syndrome, Gangliosidoses such as gangliosidoses G(MI), Sandhoff Disease, Tay-Sachs Disease, Hartnup Disease, homocystinuria, Laurence-Moon- Bied Syndrome, Lesch-Nylian Syndrome, Maple Syrup Urine Disease, mucolipidosis such as fucosidosis, neuronal ceroid-fipofuscinosis, oculocerebrorenal syndrome, phenylketonuria such as maternal phenylketonuria, Prader-Willi Syndrome, Rett Syndrome, Rubinstein-Taybi Syndrome, Tuberous Sclerosis, WAGR Syndrome, nervous system abnormalities such as holoprosencephaly, neural tube defects such as anencephaly which includes hydrangencephaly, Arnold-Chairi Deformity, encephalocele, meningocele, meningomyelocele, spinal dysraphism such as Spina bifidacystica and spina bifida occulta.

Endocrine system and/or hormone imbalance and/or diseases encompass disorders of uterine motility including, but not limited to complications with pregnancy and labor (*e.g*., pre-term labor, post-term pregnancy, spontaneous abortion, and slow or stopped labor); and disorders and/or diseases of the menstrual cycle, (*e.g*., dysmenorrhea and endometriosis).

Endocrine system and/or hormone imbalance disorders and/or diseases include disorders and/or diseases of the pancreas, such as, for example, diabetes mellitus, diabetes insipidus, congenital pancreatic agenesis, pheochromocytoma islet cell tumor syndrome; disorders and/or diseases of the adrenal glands such as, for example, Addison's Disease, corticosteroid deficiency, virilizing disease, hirsutism, Cushing's Syndrome, hyperaldosterlonism, pheochromocytoma; disorders and/or diseases of the pituitary gland, such as, for example, hyperpituitarism, hypopituitarism, pituitary dwarfism, pituitaryadenoma, panhypopituitarism, acromegaly, gigantism; disorders and/or diseases of the thyroid, including but not limited to, hyperthyroidism, hypothyroidism, P1urnrner's disease, Graves' disease (toxic diffuse goiter), toxic nodular goiter, thyroiditis (Hashimoto's thyroiditis, subacute granulomatous thyroiditis, and silent lymphocytic thyroiditis), PendreWs syndrome, myxedema, cretinism, thyrotoxicosis, thyroid hormone coupling defect, thymic aplasia, Hurthle cell tumors of the thyroid, thyroid cancer, thyroid carcinoma, Medullary thyroid carcinoma; disorders and/or diseases of the parathyroid, such as, for example, hyperparathyroidism, hypoparathyroidism; disorders and/or diseases of the hypothalamus.

In addition, endocrine system and/or hormone imbalance disorders and/or diseases may also include disorders and/or diseases of the testes or ovaries, including cancer. Other disorders and/or diseases of the testes or ovaries further include, for example, ovarian cancer, polycystic ovary syndrome, Klinefelter's syndrome, vanishing testes syndrome (bilateral anorchia), congenital absence of Leydig's cells, cryptorchidism, Noonan's syndrome, myotonic dystrophy, capillary haemangioma of the testis (benign), neoplasias of the testis and neotestis.

Moreover, endocrine system and/or hormone imbalance disorders and/or diseases may also include disorders and/or diseases such as, for example, polyglandular deficiency syndromes, pheochromocytoma, neuroblastoma, multiple Endocrine neoplasia, and disorders and/or cancers of endocrine tissues.

The modified transferrin fusion proteins of the invention and/or polynucleotides encoding transferrin fusion proteins of the invention may be used for the diagnosis, treatment, or prevention of diseases and/or disorders of the reproductive system. Reproductive system disorders that can be treated by the compositions of the invention, include, but are not limited to, reproductive system injuries, infections, neoplastic disorders, congenital defects, and diseases or disorders will result in infertility, complications with pregnancy, labor, or parturition, and postpartum difficulties.

Reproductive system disorders and/or diseases include diseases and/or disorders, of the testes, including testicular atrophy, testicular feminization, cryptorchism (unilateral and. bilateral), anorchia, ectopic testis, epididymitis and orchitis (typically resulting from infections such as, for example, gonorrhea, mumps, tuberculosis, and syphilis), testiculartorsiori, vasitis nodosa, germ cell tumors (*e.g*., seminomas, embryonal cell carcinomas, teratocarcinomas, choriocarcinomas, yolk sac tumors, and teratomas), stromal tumors (e. g., Leydig cell tumors), hydrocele, hematocele, varicocele, spermatocele, inguinal hemia, and disorders of sperm production (e. g. immotile cilia syndrome, spermia, asthenozoospermia, azoospermia, oligospermia, and teratozoospermia).

Reproductive system disorders also include disorders of the prostate gland, such as acute non-bacterial rostatitis, chronic non-bacterial prostatitis, acute bacterial prostatitis, chronic bacterial prostatitis, postatodystonia, prostatosis, granulomatotis prostatitis, malacoplakia, benign prostatic hypertrophy or hyperplasia, and prostate neoplastic disorders, including adenocarcinomas, transitional cell carcinomas, ductal carcinomas, and squamous cell carcinomas.

Additionally, the compositions of the invention may be useful in the diagnosis, treatment, and/or prevention of disorders or diseases of the penis and urethra, including inflammatory disorders, such as balanoposthitis, balanitis xerotica obliterans, phimosis, paraphmosis, syphilis, herpes simplex virus, gonorrhea, non-gonococcal urethritis, clilamydia, ruycoplasma, trichomonas, HIV, AIDS, Reiter's syndrome, condylomaacuminatum, condyloma latum, and pearly penile papules, urethral abnormalities, such as hypospadias, epispadias, and phimosis, premalignant lesions, including Erythroplasia of Queyrat, Bowen's disease, Bowenoid paplosis, criant condyloma of Buscke-Lowenstein, and varrucous carcinoma; penile cancers, including squamous cell carcinomas, carcinoma in situ, verrucous carcinoma, and disseminated penile carcinoma; urethral neoplastic disorders, including penile urethial carcinoma, bulbomembranotis urethial carcinoma, and prostaticurethral carcinoma; and erectile disorders, such as priapism, Peyronie's disease, erectile dysfunction, and impotence.

Moreover, diseases and/or disorders of the vas deferens include vasculititis and CBAVD (congenital bilateral absence of the vas deferens); additionally, the transferrin fusion proteins of the invention and/or polynucleotides encoding transferrin fusion proteins of the invention may be used in the diagnosis, treatment, and/or prevention of diseases and/or disorders of the seminal vesicles, including hydatid disease, congenital chioride diarrhea, and polycystic kidney disease.

Other disorders and/or diseases of the male reproductive system include, for example, Klinefelters syndrome, Young's syndrome, premature ejaculation, diabetes mellitus, cystic fibrosis, Kartagener's syndrome, high fever, multiple sclerosis, and gynecomastia.

Further, the polynucleotides, modified fusion proteins of the invention and/or polynucleotides encoding transferrin fusion proteins of the invention may be used in the diagnosis treatment and/or prevention of diseases and/or disorders of the vagina and vulva, including bacterial vaginosis, candida vaginitis, herpes simplex virus, chancroid, granuloma inguinale, lymphogranuloma venereum, scabies, human papillomavirus, vaginal trauma, vulvartrauma, adenosis, chlamydia vaginitis, gonorrhea, trichomonas vaginitis, condylomaacuminatum, syphilis, molluscum contagiosum, atrophic vaginitis, Paaet's disease, lichensclerosus, lichen planus, vulvodynia, toxic shock syndrome, vaginismus, vulvovaginitis, vulvar vestibulitis, and neoplastic disorders, such as squamous cell hyperplasia, clear cellcarcinoma, basal cell carcinoma, melanomas, cancer of Bartholin's gland, and vulvarintraepaelial neoplasia.

Disorders and/or diseases of the uterus include dysmenorrhea, retroverted uterus, endometriosis, fibroids, adenomyosis, anovulatory bleeding, amenorrhea, Cushiner's syndrome, hydatidiform moles, Asherman's syndrome, premature menopause, precocious puberty, uterine polyps, dysfunctional uterine bleeding (*e.g*., due to aberrant hormonal signals), and neoplastic disorders, such as adenocarcinomas, keiomyosarcomas, and sarcomas. Additionally, the transferrin fusion proteins of the invention and/or polynucleotides encoding transferrin fusion proteins of the invention may be useful as a marker or detector of, as well as, in the diagnosis, treatment, and/or prevention of congenital uterine abnormalities, such as bicomuate uterus, septate uterus, simple unicomuate uterus, unicomuate uterus with a noncavitary rudimentary horn, unicorriuate uterus with a non-communicating cavitary rudimentary horn, unicomuate uterus with a communicating cavitary horn, arcuate uterus, uterine didelfus, and T-shaped uterus.

Ovarian diseases and/or disorders include an ovulation, polycystic ovary syndrome (Stein-Leventhal syndrome), ovarian cysts, ovarian hypofunction, ovarian insensitivity to gonadotropins, ovarian over production of androgens, right ovarian vein syndrome, in amenorrhea, hirutism, and ovarian cancer (including, but not limited to, primary and secondary cancerous growth, Sertoli-Leydig tumors, endometriod carcinoma of the ovary, ovarian papillary serous adenocarcinoma, ovarian mucinous adenocarcinoma, and Ovarian Krukenberg tumors).

Cervical diseases and/or disorders include cervicitis, chronic cervicitis, mucopurulent cervicitis, and cervical. dysplasia, cervical polyps, Nabothian cysts, cervical erosion, cervical incompetence, and cervical neoplasms (including, for example, cervical carcinoma, squamous metaplasia, squamous cell carcinoma, adenosquamous cell neoplasia, and columnar cell neoplasia).

Modified transferrin fusion proteins of the invention and/or polynucleotides encoding transferrin fusion proteins of the invention can be used to treat or detect infectious agents. For example, by increasing the immune response, particularly increasing the proliferation and differentiation of B and/or T cells, infectious diseases may be treated. The immune response may be increased by either enhancing an existing immune response, or by fusion proteins of the invention and/or initiating a new immune response. Alternatively, polynucleotides encoding transferrin fusion proteins of the invention may also directly inhibit infectious agent, without necessarily eliciting an immune response.

Viruses are one example of an infectious agent that can cause disease or symptoms that can be treated or detected by transferrin fusion proteins of the invention and/or polynucleotides encoding transferrin fusion proteins of the invention. Examples of viruses, include, but are not limited to the following DNA and RNA viruses and viral families: Arbovirus, Adenoviridae, Arenaviridae, Arterivirus, Bimaviridae, Bunyaviridae, Caliciviridae, Circoviridae, Coronaviridae, Dengue, EBV, HIV, Flaviviridae, Hepadnaviridae Hepatitis, Herpesviridae (such as, Cytomegalovirus, Herpes Simplex, Berpes Zoster), Mononegavirus (*e.g*., Paramyxoviridae, Morbillivirus, Rhabdoviridae), Orthomyxoviridae (*e.g*., Influenza A, Influenza B, and parainfluenza), Papiloma virus, Papovaviridae, Parvoviridae, Picomaviridae, Poxviridae (such as Smallpox or Vaccinia), Reoviridae (e. g., Rotavirus), Retroviridae (HTLV-I, HTLV-11, -Lentivirus), and Togaviridae (*e.g*., Rubivirus).

Similarly, bacterial and fungal agents that can cause disease or symptoms that can be treated or detected by transferrin fusion proteins of the invention and/or polynucleotides encoding transferrin fusion proteins of the invention include, but not limited to, the following Gram-negative and Gram-positive bacteria, bacterial families, and fungi: Actinomyces (e. g., Norcardia), Acinetobacter, Cryptococcus neoformans, Aspergillus, Bacillaceae (e. g., Bacillus anthrasis), Bacteroides (*e.g*., Bacteroides fragilis), Blastomycosis, Bordetella, Borrelia (*e.g*., Borrelia burgdorferi), Brucella, Candidia, Campylobacter, Chiamydia, Clostridiuffi (*e.g*., Clostridium botulinum, Clostridium dificile, Clostridium perfringens, Clostridiumtetani), Coccidioides, Corynebacterium (*e.g*., Corynebacterium- diptheriae), Cryptococcus, Dermatocycoses, E. coli (e. g., Enterotoxigenic E. coli and Enterohemorrhagic E. coli), Enterobacter (*e.g*. Enterobacter aerogenes), Enterobacteriaceac (Klebsiella, Salmonella (*e.g*., Salmonella typhi, Salmonella enteritidis, Salmonella typhi), Serratia, Yersinia, Shigella), Erysipelothrix, Haemophilus (*e.g*., Haemophilus influenza type B), Helicobacter, Legionella (e. g., Legionella pneumophila), Leptospira, Listeria (*e.g*., Listeria monocytogenes), Mycoplasma, Mycobacterium (*e.g*., Mycobacterium, leprae and Mycobacterium tuberculosis), Vibrio (*e.g*., Vibrio cholerae), Neisseriaceae (*e.g*., Neisseriagonorrhea, Neisseria meningitidis), Pasteurellacea,, Proteus, Pseudomonas (*e.g*., Pseudomionas aeruginosa), Rickettsiaceae, Spirochetes (*e.g*., Treponema. spp., Leptospiraspp., Borrielia spp.), Shigella spp., Staphylococcus (*e.g*., Staphylococcttaureus), Meningiococeus, Pneumococeus and Streptococeus (*e.g*., Streptococeus pneumoniae and Groups A, B, and C Streptococci), and Ureaplasmas.

Moreover, parasitic agents causing disease or that can be treated, prevented, and/or diagnosed by fusion proteins of the invention and/or polynucleotides encoding transferrin fusion proteins of the invention include, but not limited to, the following families or class: Amebiasis, Babesiosis, Coccidiosis, Cryptosporidiosis, Dientamoebiasis, Dourine, Ectoparasitic, Giardias, Helminthiasis, Leishmaniasis, Schistisoma, Theileriasis, Toxoplasmosis, Trypanosomiasis, and Trichomonas and Sporozoans (*e.g*., Plasmodiumvirax, Plasmodium falciparium, Plasmodium malariae and Plasmodium ovale).

Modified transferrin fusion proteins of the invention and/or polynucleotides encoding transferrin fusion proteins of the invention can be used to differentiate, proliferate, and attract cells, pleading to the regeneration of tissues. (See, Science 276:59-87 (1997)). The regeneration of tissues could be used to repair, replace, or protect tissue damaged by congenital defects, trauma (wounds, bums, incisions, or ulcers), age, disease (*e.g*. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (*e.g*., pancreas, liver, intestine, kidney, skin, endothelium), muscle (smooth, skeletal or cardiac), vasculature (including vascular and lymphatics), nervous, hematopoietic, and skeletal (bone, cartilage, tendon, and ligament) tissue. Preferably, regeneration occurs without or decreased scarrina. Regeneration also may include angiogenesis.

Modified transferrin fusion proteins of the invention and/or polynucleotides encoding transferrin fusion proteins of the invention, may be used to treat, prevent, diagnose, and/or prognose gastrointestinal disorders, including inflammatory diseases and/or conditions, infections, cancers (*e.g*., intestinal neoplasms (carcinoid tumor of the small intestine, non-Hodgkin's lymphoma of the small intestine, small bowilymphoma), and ulcers, such as peptic ulcers.

Gastrointestinal disorders include dysphagia, odynophagia, inflammation of the esophagus, peptic esophagitis, gastric reflux, submucosal fibrosis and structuring, Mallory-Weiss lesions, leioinyomas, lipomas, epidennal cancers, adeoncarcinomas, gastric retention disorders, gastroenteritis, gastric atrophy, gastric/stomach cancers, polyps of the stomach, autoimmune disorders such as pemicious anemia, pyloric stenosis, gastritis (bacterial, viral, eosinophilic, stress-induced, chronic erosive, atrophic, plasma cell, and Menetrier's), and peritoneal diseases (*e.g*., chylo perioneum, hemoperitoneum, mesenteric cyst, mesentericlymphadenitis, mesentenic vascular occlusion, panniculiti, neoplasins, peritonitis, prieumoperitoneum, bubphrenic abscess.

Gastrointestinal disorders also include disorders associated with the small intestine, such as malabsorption syndrome's, distension, irritable bowel syndrome, sugar idolerance, celiac disease, duodenal ulcers, duodenitis, tropical sprue, Whipple's disease, intestinal lymphangiectasia, Crohn's disease, appendicitis, obstructions of the ilum, Meckel's diverticulum, multiple diverticula, failure of complete rotation of the small and large intestine, lymphoma, and bacterial and parasitic diseases (such as Traveler's diarrhea, typhoid and paratyphoid, cholera, infection by Roundworms (Ascariasis Itimbricoides), Hookworms (Anclostoma duodenale), Threadworms (Enterobius vermicularis), Tapeworms jaenia saginata, Echinococcus granulosus, Diphyllobothrium spp. and T. SOHUM).

Liver diseases and/or disorders include intrahepatic cholestasis (alagille syndrome, biliary liver cirrhosis), fatty, liver (alcoholic fatty liver, reye syndrome), hepatic veiri, thrombosis, hepatolentricular degeneration, hepatomegaly, hepatopulmonary syndrome, hepatorenal, syndrome, portal hypertension (esophageal and gastric varices), liver abscess (amebie liver abscess), liver cirrhosis (alcoholic, biliary and experimental), alcoholic liver diseases (fatty liver, hepatitis, cirrhosis), parasitic (hepatic echinocoecosis, fascioliasis, amebic liver abscess), jaundice (hemolytic, hepatocellular, and cholestatic), cholestasis, portal hypertension, liver, enlargement, ascites, hepatitis (alcoholic hepatitis, aniffial hepatitis, chronic hepatitis (autoimmune, hepatitis B, hepatitis C, hepatitis D, drug induced), toxic hepatitis, viral human hepatitis (hepatitis A, hepatitis B, hepatitis C, hepatitis D, hepatitis E), Wilson's disease, granulomatous hepatitis, secondary biliary cirrhosis, hepaticencephalopathy, portal hypertension, varices, hepatic encepbalopathy, primary biliary heinarigiomas, bilecirrhosis, primary sclerosing cholangitis, hepatocellular adenoma, stones, liver failure (hepatic encephalopathy, acute liver failure), and liver neoplasins (ancriomyolipoma, calcified liver metastases, cystic liver metastases, epithelial tumors, fibro lamellar hepatocarcinoma, focal nodular hyperplasia, hepafic adenoma, hepatobiliarycystadenoma, hepatoblastorria, hepatocellular carcinoma, hepatoma, liver cancer, liver hemanaioendothelioma, mesenchymal hamartoma, mesenchymal tumors of liver, nodular regenerative hyperplasia, benign liver tumors (Hepatic cysts, Simple cysts, Polycystic liver disease, Hepatobiliary cystadenoma, Chofedochal cysts, Mesenchymal tumors, Mesenchymal hamartoma, Infantile hemarigioendothelioma, Hemangioma, Peliosis hepatis, Lipomas, Inflammatory pseudo tumor, Miscellaneous Epithelial tumors, Bile ductepitheflum (Bile duct hamartoma, Bile duct adenoma), Hepatocyte (Adenoma, Focal nodular hyperplasia, Nodular regenerative hyperplasia), malignant liver tumors (hepatocellular, hepatoblastoma, hepatocellular carcinoma, cholangiocellular, cholangiocarcinoma, cystadenocarcinoma, tumors of blood vessels, anaiosarcoma, Karposi's sarcoma, hemangioendothelioma, other tumors, embryonal sarcoma, fibrosarcoma, Ieiorriyosarcoma, rhabdomyosarcoma, carcinosarcoma, teratoma, carcinoid, squamous carcinoma, primarylymphorria)), peliosis hepatis, erythrohepatic porphyria, hepatic porphyria (acute interirtittentporphyria, porphyria cutanea tarda), Zelli Neger syndrome).

Pancreatic diseases and/or disorders include acute pancreatitis, chronic pancreatitis (acute necrotizing pancreatitis, alcoholic pancreatitis), neoplasins (adenocarcinoma of the pancreas, cystadenocarcinoma, insulinoma, gastrinoma, and glucacronoma, cysticcitmeoplasms, islet-cell tumors, pancreoblastoma), and other pancreatic diseases (*e.g*., cysticfibrosis, cyst (pancreatic pseudocyst, pancreatic fistula, insufficiency)).

Gallbladder diseases include gallstones (cholelithiasis and choledocholithiasis), postcholeeystectomy syndrome, diverticulosis of the gallbladder, acute cholecystitis, chronic cholecystitis, bile duct tumors, and mucocele.

Diseases and/or disorders of the large intestine include antibiotic-associated colitis, diverticulitis, ulcerative colitis, acquired megacolon, abscesses, fungal and bacterial infections, anorectal disorders (*e.g*., fissures, hemorrhoids), colonic diseases (colitis, colonic neoplastris, colon cancer, adenomatous colon polyps (*e.g*., villous adenoma), coloncarcinoma, colorectal cancer, colonic diverticulitis, colonic diverticulosis, megacolon, Hirschsprung disease, toxic inegacolon, sigmoid diseases proctocolitis, sigmoinneoplasmsj, constipation, Crohn's disease, diarrhea (infantile diarrhea, dysentery), duodenal diseases (duodenal neoplasins, duodenal obstruction, duodenal ulcer, duodenitis), enteritis (enterocolitis), HIV enteropathy, leal diseases (leal neoplasins, ileitis), immunoproliferative small intestinal disease, inflammatory bowel disease (ulcerative colitis, Crohn's disease), intestinal atresia, parasitic diseases (anisakiasis, balantidiasis, blastocystis infections, cryptosporidiosis, dientamoebiasis, amebic dysentery, giardiasis), intestinal fistula (rectal fistula), intestinal neoplasms (cecal neoplasms, colonic neolasms, duodenalpneoplasms, leal neoplasms, intestinal polyps, jejunal neoplasins, rectal neoplasms), intestinal obstruction (afferent loop syndrome, duodenal obstruction, impacted feces, intestinal pseudo obstruction cecal volvulus, intussusception), intestinal perforation, intestinal polyps (colonic polyps, gardner syndrome, peutz-jeghers syndrome), jejunal diseases Oejunal neoplasms), mal absorption syndromes (blind loop syndrome, celiac disease, lactose intolerance, short bowl syndrome, tropical sprue, whipple's disease), mesenteric vascular occlusion, pneumatosis cystoides intestinalis, protein losing enteropathies (intestinal lymphagiectasis), rectal diseases (anus diseases, fecal incontinence, hemorrhoids, proctitis, rectal fistula, rectal prolapse, rectocele), peptic ulcer (duodenalulcer, peptic esophagitis, hemorrhage, perforation, stomach ulcer, Zollinger-Ellison syndrome), postgastrectomy syndromes (dumping syndrome), stomach diseases (*e.g*., achlorhydria, duodenogastric reflux (bile reflux), gastric antral vascular ectasia, gastricfistula, gastric outlet obstruction, gastritis (atrophic or hypertrophic), gastroparesis, stomach dilatation, stomach diverticulum, stomach neoplasms (gastric cancer, gastric polyps, gastric adenocarcinoma, hyperplastic gastric polyp), stomach rupture, stomach ulcer, stomach volvulus), tuberculosis, visceroptosis, vomiting (*e.g*., hematemesis, hyperemesisgravidarum, postoperative nausea-and vomiting) and hemorrhagic colitis.

Further diseases and/or disorders of the gastrointestinal system include biliary tract diseases, such as, gastroschisis, fistula (*e.g*., biliary fistula, esophageal fistula, gastricfistula, intestinal fistula, pancreatic fistula), neoplasms (*e.g*., biliary tract neoplasins, esophageal neoplasms, such as adenocarcinoma of the esophagus, esophageal squamous cell carcinoma, gastrointestinal neoplasms, pancreatic neoplasins, such as adenocarcinoma of the pancreas, mucinous cystic neoplasm of the pancreas, pancreatic eystic neoplasms, pancreatoblastoma, and peritoneal neoplasms), esophageal disease (*e.g*., bullous diseases, candidiasis, glycoaenie acanthosis, ulceration, barrett esophagus varices, atresia, cyst, diverticulum. (*e.g*., Zenker's diverticulum), fistula (*e.g*., tracheoesophageal fistula), motility disorders (*e.g*., CREST syndrome, deglutition disorders, achalasia, spasm, gastroesophageal reflux), neoplasms, perforation (*e.g*., Boerhaave syndrome, Mallory-Weiss syndrome), stenosis, esophagitis, diaphragmatic hernia (*e.g*., hiatal hernia); gastrointestinal diseases, such as, gastroenteritis (*e.g*., cholera morbus, norwalk virus infection), hemorrhage (*e.g*., hematemesis, melena, peptic ulcer hemorrhage), stomach neoplasms (gastric cancer, gastric polyps, gastric adenocarcinoma, stomach cancer)), hernia (*e.g*., congenital diaphragmatic hernia, femoral hernia, inguinal hernia, obturator hernia, umbilical hernia, ventral hernia), and intestinal diseases (*e.g*., cecal diseases (appendicitis, cecal neoplasms)).

Modified transferrin fusion proteins of the invention and/or polynucleotides encoding transferrin fusion proteins of the invention may have chemotaxis activity. A chemotaxic molecule attracts or mobilizes cells (*e.g*., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

Modified transferrin fusion proteins of the invention and/or polynucleotides encoding transferrin fusion proteins of the invention may increase chemotaxic activity of particular cells. These chemotactic molecules can then be used to treat inflammation, infection, hyperproliferative disorders, or any immune system disorder by increasing the number of cells targeted to a particular location in the body.

### Transgenic Animals

The production of transgenic non-human animals that contain a modified transferrin fusion construct with increased serum half-life increased serum stability or increased bioavailability of the instant invention is contemplated in one embodiment of the present invention. In some embodiments, lactoferrin may be used as the Tf portion of the fusion protein so that the fusion protein is produced and secreted in milk.

The successful production of transgenic, non-human animals has been described in a number of patents and publications, such as, for example U.S. Patent 6,291,740 (issued September 18, 2001); U.S. Patent 6,281,408 (issued August 28, 2001); and U.S. Patent 6,271,436 (issued August 7, 2001).

The ability to alter the genetic make-up of animals, such as domesticated mammals including cows, pigs, goats, horses, cattle, and sheep, allows a number of commercial applications. These applications include the production of animals which express large quantities of exogenous proteins in an easily harvested form (*e.g*., expression into the milk or blood), the production of animals with increased weight gain, feed efficiency, carcass composition, milk production or content, disease resistance and resistance to infection by specific microorganisms and the production of animals having enhanced growth rates or reproductive performance. Animals which contain exogenous DNA sequences in their genome are referred to as transgenic animals.

The most widely used method for the production of transgenic animals is the microinjection of DNA into the pronuclei of fertilized embryos (Wall et al., J. Cell. Biochem. 49:113 [1992]). Other methods for the production of transgenic animals include the infection of embryos with retroviruses or with retroviral vectors. Infection of both pre-and post-implantation mouse embryos with either wild-type or recombinant retroviruses has been reported (Janenich, Proc. Natl. Acad. Sci. USA 73:1260 [1976]; Janenich et al., Cell 24:519 [1951]; Stuhlmann et al., Proc, Natl. Acad. Sci. USA 81:7151 [1984]; Jahner et al., Proc. Natl. Acad Sci. USA 82:6927 [1985]; Van der Putten et al., Proc. Natl. Acad Sci. USA 82:6148-6152 [1985]; Stewart et al., EMBO J. 6:383-388 [1987]).

An alternative means for infecting embryos with retroviruses is the injection of virus or virus-producing cells into the blastocoele of mouse embryos (Jahner, D. et al., Nature 298:623 [1982]). The introduction of transgenes into the germline of mice has been reported using intrauterine retroviral infection of the midgestation mouse embryo (Jahner *et al*., supra [1982]). Infection of bovine and ovine embryos with retroviruses or retroviral vectors to create transgenic animals has been reported. These protocols involve the microinjection ofretroviral particles or growth arrested (*i.e*., mitomycin C-treated) cells which shed retroviral particles into the perivitelline space of fertilized eggs or early embryos (PCT International Application WO 90/08832 [1990]; and Haskell and Bowen, Mol. Reprod. Dev., 40:386 [1995]. PCT International Application WO 90/08832 describes the injection of wild-type feline leukemia virus B into the perivitelline space of sheep embryos at the 2 to 8 cell stage. Fetuses derived from injected embryos were shown to contain multiple sites of integration.

U.S. Patent 6,291,740 (issued September 18, 2001) describes the production of transgenic animals by the introduction of exogenous DNA into pre-maturation oocytes and mature, unfertilized oocytes (*i.e*., pre-fertilization oocytes) using retroviral vectors which transduce dividing cells (*e.g*., vectors derived from murine leukemia virus [MLV]). This patent also describes methods and compositions for cytomegalovirus promoter-driven, as well as mouse mammary tumor LTR expression of various recombinant proteins.

U.S. Patent 6,281,408 (issued August 28, 2001) describes methods for producing transgenic animals using embryonic stem cells. Briefly, the embryonic stem cells are used in a mixed cell co-culture with a morula to generate transgenic animals. Foreign genetic material is introduced into the embryonic stem cells prior to co-culturing by, for example, electroporation, microinjection or retroviral delivery. ES cells transfected in this manner are selected for integrations of the gene via a selection marker such as neomycin.

U.S. Patent 6,271,436 (issued August 7, 2001) describes the production of transgenic animals using methods including isolation of primordial germ cells, culturing these cells to produce primordial germ cell-derived cell lines, transforming both the primordial germ cells and the cultured cell lines, and using these transformed cells and cell lines to generate transgenic animals. The efficiency at which transgenic animals are generated is greatly increased, thereby allowing the use of homologous recombination in producing transgenic non-rodent animal species.

### Gene Therapy

The use of modified transferrin fusion constructs for gene therapy wherein a modified transferrin protein or transferrin domain is joined to a therapeutic protein or peptide is contemplated in one embodiment of this invention. The modified transferrin fusion constructs with increased serum half-life or serum stability of the instant invention are ideally suited to gene therapy treatments.

The successful use of gene therapy to express a soluble fusion protein has been described. Briefly, gene therapy via injection of an adenovirus vector containing a gene encoding a soluble fusion protein consisting of cytotoxic lymphocyte antigen 4 (CTLA4) and the Fc portion of human immunoglubulin Gl was recently shown in Ijima et al. (June 10, 2001) Human Gene Therapy (United States) 12/9:1063-77. In this application of gene therapy, a murine model of type II collagen-induced arthritis was successfully treated via intraarticular injection of the vector.

Gene therapy is also described in a number of U.S. patents including U.S. Pat. 6,225,290 (issued May 1, 2001); U.S. Pat. 6,187,305 ( issued February 13, 2001); and U.S. Pat. 6,140,111 (issued October 31,2000).

U.S. Patent 6,225,290 provides methods and constructs whereby intestinal epithelial cells of a mammalian subject are genetically altered to operatively incorporate a gene which expresses a protein which has a desired therapeutic effect. Intestinal cell transformation is accomplished by administration of a formulation composed primarily of naked DNA, and the DNA may be administered orally. Oral or other intragastrointestinal routes of administration provide a simple method of administration, while the use of naked nucleic acid avoids the complications associated with use of viral vectors to accomplish gene therapy. The expressed protein is secreted directly into the gastrointestinal tract and/or blood stream to obtain therapeutic blood levels of the protein thereby treating the patient in need of the protein. The transformed intestinal epithelial cells provide short or long term therapeutic cures for diseases associated with a deficiency in a particular protein or which are amenable to treatment by overexpression of a protein.

U.S. Pat. 6,187,305 provides methods of gene or DNA targeting in cells of vertebrate, particularly mammalian, origin. Briefly, DNA is introduced into primary or secondary cells of vertebrate origin through homologous recombination or targeting of the DNA, which is introduced into genomic DNA of the primary or secondary cells at a preselected site.

U.S. Pat. 6,140,111 (issued October 31, 2000) describes retroviral gene therapy vectors. The disclosed retroviral vectors include an insertion site for genes of interest and are capable of expressing high levels of the protein derived from the genes of interest in a wide variety of transfected cell types. Also disclosed are retroviral vectors lacking a selectable marker, thus rendering them suitable for human gene therapy in the treatment of a variety of disease states without the co-expression of a marker product, such as an antibiotic. These retroviral vectors are especially suited for use in certain packaging cell lines. The ability of retroviral vectors to insert into the genome of mammalian cells have made them particularly promising candidates for use in the genetic therapy of genetic diseases in humans and animals. Genetic therapy typically involves (1) adding new genetic material to patient cells *in vivo,* or (2) removing patient cells from the body, adding new genetic material to the cells and reintroducing them into the body, *i.e*., *in vitro* gene therapy. Discussions of how to perform gene therapy in a variety of cells using retroviral vectors can be found, for example, in U.S. Pat. Nos. d,868,116, issued Sep. 19,1989, and 4,980,286, issued Dec. 25, 1990 (epithelial cells), WO89/07136 published Aug. 10, 1989 (hepatocyte cells), EP 378,576 published Jul. 25, 1990 (fibroblast cells), and WO89/05345 published Jun. 15, 1989 and WO/90/06997, published Jun. 28, 990 (endothelial cells).

Without further description, it is believed that a person of ordinary skill in the art can, using the preceding description and the following illustrative examples, make and utilize the present invention and practice the claimed methods. For example, a skilled artisan would readily be able to determine the biological activity, both *in vitro* and *in vivo,* for the fusion protein constructs of the present invention as compared with the comparable activity of the therapeutic moiety in its unfused state. Similarly, a person skilled in the art could readily determine the serum half life and serum stability of constructs according to the present invention. The following working examples therefore, specifically point out the preferred embodiments of the present invention, and are not to be construed as limiting in any way the remainder of the disclosure.

### EXAMPLES

### Example 1

A fusion protein between modified Tf and an antifusogenic HIV-1 peptide (T-20) comprising the sequence is made by fusing one or more copies of the nucleotide sequence encoding the peptide to the nucleotide sequence of TF to produce a fusion protein with a peptide fused to the N- or C-terminus of Tf.

In one embodiment, the Tf portion of the fusion protein is engineered to not allow glycosylation when produced in yeast. As discussed above, human transferrin has two N-linked glycosylation sites at about N413 and about N611. The N-linked glycosylation site comprises the sequence N-X-S/T. In one embodiment, N (Asn) is changed to Q (Gln); other changes are contemplated such as Asn to Ala or Ser or any other amino acid.

Specifically, the N413 and N611 codons are converted to GAT and GAC by oligonucleotide directed mutagenesis using the dut- and ung-method. See Kunkel et al. (1985) Proc. Natl. Acad. Sci. 82:488-492). The mutagenic oligonucleotides 5'-GCAGAAAACTACGATAAGAGCGATAAT-3' (SEQ ID NO: 9) and 5'-CTATTTGGAAGCGACGTAACTGACTGC-3' (SEQ ID NO: 10) are synthesized and used to mutagenize the N413 and N611 codons according to the methods of Funk et al. (U.S. Patent 5,986,067).

Receptor binding and/or iron or carbonate binding is then disrupted by mutating the following iron and/or carbonate ion binding residues:

| **Iron binding** | |
|---|---|
| N domain | C domain |
| Asp 63 (Asp 82 of SEQ ID NO: 2) | Asp 392 (Asp 411 of SEQ ID NO: 2) |
| Tyr 95 (Tyr 114 of SEQ ID NO: 2) | Tyr 426 (Tyr 445 of SEQ ID NO: 2) |
| Tyr 188 (Tyr 207 of SEQ ID NO: 2) | Tyr 514 or 517(Tyr 533 or Tyr 536 SEQ ID NO:2) |
| His 249 (His 268 of SEQ ID NO: 2) | His 585 (His 604 of SEQ ID NO: 2) |

| **Carbonate ion binding** | |
|---|---|
| N domain | C domain |
| Thr 120 (Thr 139 of SEQ ID NO: 2) | Thr 452 (Thr 471 of SEQ ID NO: 2) |
| Arg 124 (Arg 143 of SEQ ID NO: 2) | Arg 456 (Arg 475 of SEQ ID NO: 2) |
| Ala 126 (Ala 145 of SEQ ID NO: 2) | Ala 458 (Ala 477 of SEQ ID NO: 2) |
| Gly 127 (Gly 146 of SEQ ID NO: 2) | Gly 459 (Gly 478 of SEQ ID NO: 2) |

The production of mutants deficient in iron binding may be accomplished by numerous techniques. See U.S. Patent 5,986,067. A D63S substitution may prepared using the method of Nelson, R. M. and Long, G. L. (1989) Analyt. Biochem. 180:147-151. Briefly, a HpaII/BamHI fragment from the 5' end of the hTF/2N coding sequence is subcloned into pUC18 and then used as a template for a two step PCR-based mutagenesis procedure. The fragment is then released from the double stranded form of the sequencing vector by digestion with XbaI and BamHI and then ligated to a BamHI/HindIII fragment from the original human Tf construct to produce a full length D63S-coding sequence, the fidelity of this splicing is confirmed by restriction digestion analysis.

For expression in *Pichia* the system from RCT/Invitrogen can be used. Three vectors are available for multicopy expression, pPIC9K, pPIC3.5K and pAO815. For this example the pPIC9K vector, which allows secretion into the growth medium, is used.

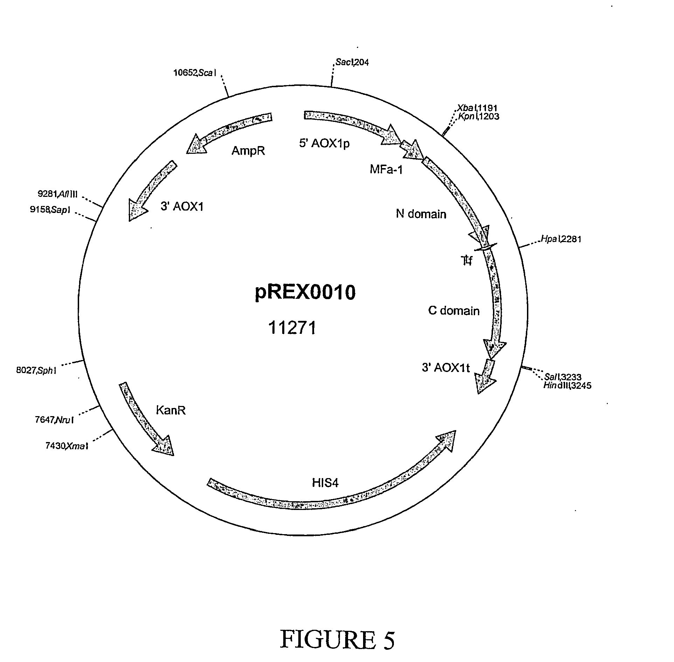

The modified transferrin sequence was cloned into the pPIC9K vector by altering the ends of the transferrin cDNA by overlapping PCR mutagenesis, this yielded the vector pREX0010. A number of restriction sites within the vector and coding sequence were removed or added to aid later cloning steps (Figure 5).

The sequence for the HIV anti-fusogenic peptide DP-178 is also known as T-20. This peptide lends itself to fusion at the N- or C- termini of Transferrin, as the peptide may need freedom of movement to fulfill its function. DP-178 sequence: YTSLIHSLIEESQNQQEKNEQELLELDKWASLWNWF (SEQ ID NO: 4)

When back translated in to DNA (using codons optimized for yeast) the following sequence was obtained (SEQ ID NOS: 13 and 14):

To inset the above sequence the vector pREX0010 with the modified transferrin cDNA, was digested with the restriction enzymes *Xba* I/*Kpn* I for insertion at the 5' end and *Sal* I*lHind* III for insertion at the 3' end.

For the 5' insertion two overlapping oligos that form an *Xba* I overhang at the 5' end and a *Kpn* I overhang at the 3' end of the DP-178 sequence given above were synthesized. These oligos were then annealed together (see below) and ligated into the *Xba* I/ *Kpn* I digested pREX0010 vector.

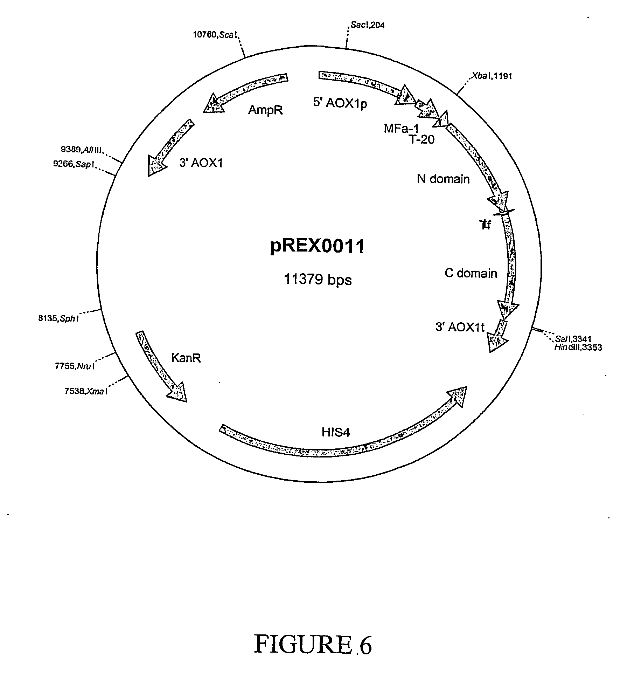

Insertion of the annealed oligos resulted in loss of the *Kpn* I site upon insertion. This resulted in the vector pREX0011 (Figure 6).

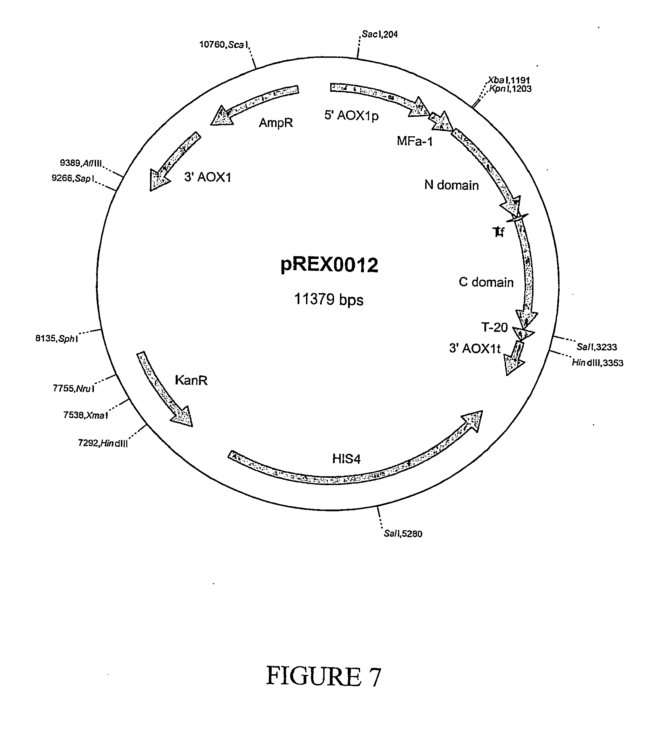

For insertion at the C-terminus a similar approach was taken by the addition of a *Sal*I site at the 5' end and a *Hind*III at the 3' end (Figure 7).

Transformation, selection and expression are then performed as described in the Invitrogen *Pichia* Expression kit protocol booklet.

### Example 2

INGAP fusions are prepared using a reverse translated human INGAP amino acid sequence. The protein sequence is as follows: sp|Q92778|PBCG_HUMAN Human INGAP

Reverse translated in to DNA (codons optimized for yeast) gave the following (SEQ ID NO: 18 and 19).

The most likely point for cleavage of the leader sequence is at the KK at the end of the underlined sequence above.

One methodology which may be used to generate constructs for the expression of INGAP fused to the N- or C-terminus of transferrin is to synthesize a series of overlapping oligos designed from the sequence given above (minus the underlined leader sequence). Annealing of these primers generates the INGAP cDNA. With different oligos designed for the 5' and 3' ends the annealed cDNA can be ligated into pREX0010 at the 5' or 3' end of Transferrin.

The bracketed sequence is the peptide used to induce INGAP activity. Hence the sequence could be paired down to some point between the whole and this minimal sequence.

### N-terminal fusion.

For the N-terminus these would have an overhang which forms an *Xba*I site at the 5' end and an overhang compatible with a *Kpn*I site at the 3' end but which results in the destruction of the *Kpn*I site.

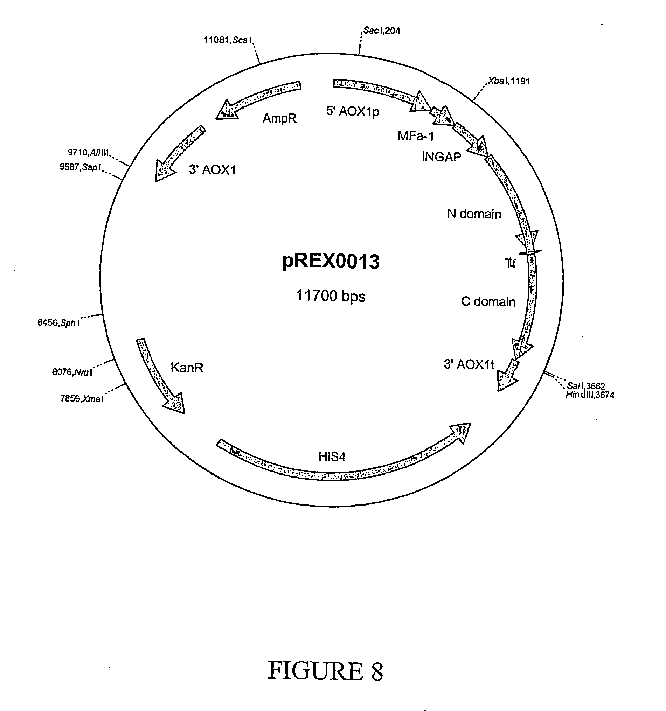

Digestion of pREX0010 with *Xba*I and *Kpn*I and ligation of the above sequence yields the vector pREX0013 (Figure 8).

### C-terminal fusion.

For the C-terminus the 5' end would form a *Sal*I site and at the 3' end a stop codon plus a *Hin*dIII site.

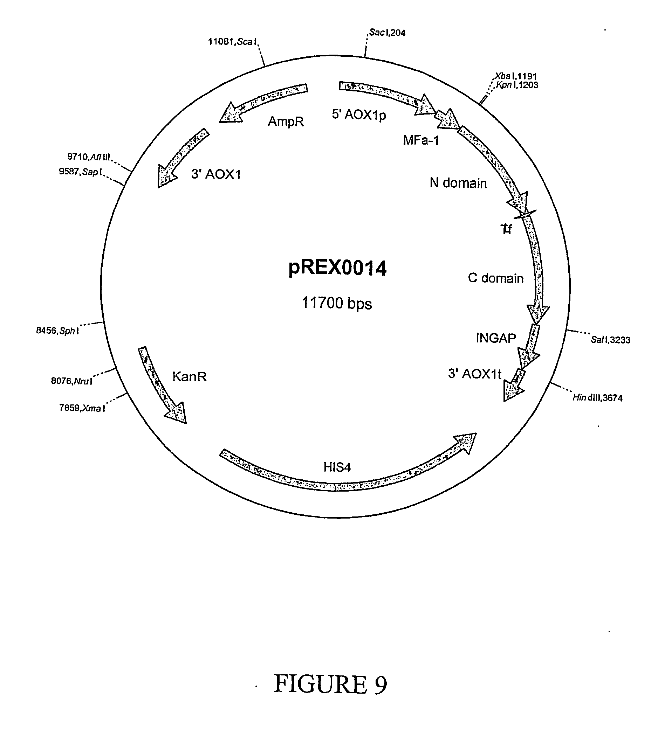

Digestion of pREX0010 with *Sal*I and *Hin*dIII and ligation of the above sequence yields the vector pREX0014 (Figure 9).

Transformation, selection and expression are then performed as described in the Invitrogen *Pichia* Expression kit protocol booklet.

### Example 3

The peptide given below has been shown to mimic EPO activity by causing dimerisation of the EPO receptor. The peptide, which is cyclic, has no homology to EPO. For activity the peptide has to act in concert with another peptide, *i.e.* as a dimer, such that two copies of the receptor are brought in close enough proximity to form an active complex. As with many peptides the peptide dimer suffers from short half life and would benefit from the longevity that fusion to transferrin would give. In this example two peptides are engineered into the transferrin scaffold. SEQ ID NOS: 24 and 25.

As detailed by Ali *et al,* a peptide can be successfully engineered into Transferrin between His289 and Gly290. The duplication inherent to the transferrin molecule, with the two domains mirroring each other, means that it is possible to engineer a peptide into the duplicate region of the C domain, between Glu625 and Thr626. SEQ ID NOS: 26 and 27.

For each insertion two overlapping mutagenic primers are synthesized (see below). Using pREX0010 as a template reactions were performed with each mutagenic primer and an external primer from the 5' or 3' of the Tf cDNA. The products from these two reactions were then mixed and a further reaction performed with the external primers to join the two products together. The His289-Gly290 insert PCR product was digested with *Xba*I and *Hpa*I for ligation into *Xba*I/*Hpa*I digested pREX0010. The resulting vector was then digested with *Hpa*I and *Sal*I for ligation of *Hpa*I*lSal*I digested the Glu625-Thr626 insert PCR product.

His289-Gly290 insert (SEQ ID NO: 28).

Glu625-Thr626 insert (SEQ ID NO: 29).

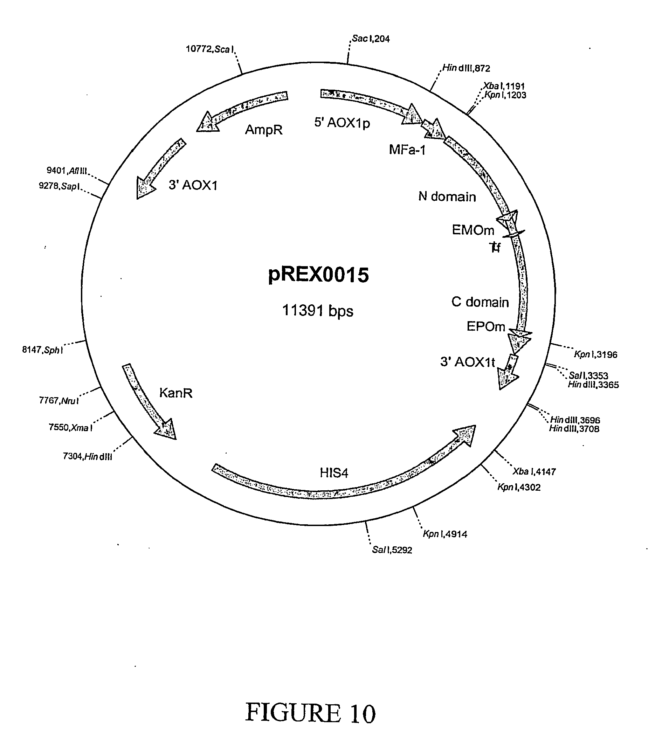

These gave the plasmid pREX0015 (Figure 10). Transformation, selection and expression are then performed as described in the Invitrogen *Pichia* Expression kit protocol booklet.

Alternative points for insertion of the EPO mimetic peptide(s), or any other peptide(s) are the two glycosylation sites on the C domain of Transferrin at N413 and N611. The advantage of this would be that insertion is achieved and glycosylation prevented, by disruption of the N-X-S/T sequence, in one and the same event.

### Example 4

Fusion proteins between Tf and fusogenic inhibitor peptides against RSV are made by fusing the peptide sequences to the N- or C- terminal ends of Tf or by the insertion of the sequences into a loop of Tf, wherein the Tf is modified to not bind iron and/or is modified to prevent glycosylation. The RSV peptide may include: T786: VYPSDEYDASISQVNEEINQALAYIRKADELLENV (SEQ ID NO: 5) and/or T1584: AVSKVLHLEGEVNKIKSALLSTNKAWSLSNGVSVLTSKVLDLKNYIDKQL (SEQ ID NO: 6).

The T786 peptide has a RK dipeptide which could act as a cleavage site for the yeast protease Kex2p. This would result in a truncated peptide. Accordingly, this peptide may be modified from RK to RE. Another version of the T786 peptide, T112 (VFPSDEFDASISQVNEKINQSLAFIRESDELLHNV, SEQ ID NO: 7), which is more potent than T786 has solubility problems in its unfused form. Accordingly, a version of T112 modified for the RK to RE is also made to produce a version of the peptide fused to Tf.

To produce the genetic constructs, the peptide sequences are backtranslated in DNA using codon bias for human, yeast or any other organism as appropriate.

### Example 5

Various cytokines can be fused to the N-, C- or N- and C- termini of Tf. These fusions can also be constructed using different parts or domains of modified transferrin such as the N domain or C domain. The proteins can be fused directly or using a linker peptide of various lengths. It is also possible to fuse all or part of the active cytokine within the scaffold of transferrin.

The cDNA for the cytokine of interest, such as EPO, can be isolated by a variety of means such as RT-PCR from mRNA, from cDNA libraries, by synthetically constructing the cDNA from overlapping oligonucleotides, by PCR or by other means known to the art, all using standard methods. The nucleotide sequences for all of these proteins are known and available, for instance, in U.S. Patents 4,703,008, 4,810,643 and 5,908,763 as well as in public databases such as GenBank. The cDNA can be tailored at the 5' and 3' ends to generate restriction sites, such that oligonucleotide linkers can be used, for cloning of the cDNA into a vector containing the cDNA for Transferrin. This can be at the N- or C-terminus, with or without the use of a spacer sequence, or by inserting the cDNA of the cytokine within the cDNA of Transferrin. The cytokine, e.g. EPO, and Tf cDNA are cloned into a vector from which the complete expression cassette is then excised and inserted into an expression vector to allow the expression of the fusion protein in yeast (or any other appropriate expression system). The fusion protein secreted from the yeast can then be collected and purified from the media and tested for its biological activity.

For expression in mammalian cell lines, a similar procedure is adopted except that the expression cassette used employs a mammalian promoter, leader sequence and terminator. This expression cassette is then excised and inserted into a plasmid suitable for the transfection of mammalian cell lines.

### Example 6

Various interferons can be fused to the N-, C- or N- and C- termini of modified transferrin. These fusions can be constructed using different parts or domains of transferrin such as the N domain or C domain. The proteins can be fused directly or using a linker peptide of various lengths. It is also possible to fuse all or part of the interferon within the scaffold of transferrin.

A specific example of an interferon that can be fused to Tf is interferon-β. The cDNA for the interferon of interest such as IFN β can be isolated by a variety of means such as RT-PCR from mRNA or cDNA, from cDNA libraries, by synthetically constructing the cDNA from overlapping oligonucleotides, by PCR or by other means known to the art, all using standard methods. The nucleotide sequences for interferons, such as IFNα, IFNβ, and IFNγ are known and available, for instance, in U.S. Patents 5,326,859 and 4,588,585, in EP 32 134, as well as in public databases such as GenBank. The cDNA can be tailored at the 5' and 3' ends to generate restriction sites, such that oligonucleotide linkers can be used to clone the cDNA into a vector containing the cDNA for modified transferrin. This can be at the N-, C- or N- and C-termini of the transferrin sequence, with or without the use of a spacer sequence. The IFN β (or other interferon) cDNA is cloned into a vector from which the complete expression cassette is then excised and inserted into an expression vector to allow the expression of the fusion protein in yeast. The fusion protein secreted from the yeast can then be collected and purified from the media and tested for its biological activity.

For expression in mammalian cell lines a similar procedure is adopted except that the expression cassette used employs a mammalian promoter, leader sequence and terminator. This expression cassette is then excised and inserted into a plasmid suitable for the transfection of mammalian cell lines. IFNs fused to transferrin have much longer half-life, thus, the therapeutic dosages of the fused proteins are much less than the IFNs. Therefore, the fused interferons are more efficacious with much less toxicity.

### Example 7

Various single chain antibodies (SCA) were originally invented to simplify antibody selection and production. However, they prove to be of limited or no therapeutic values due to their small size and short *in vivo* half-life. Addition of transferrin to SCA significantly increases the *in vivo* half-life of SCA.

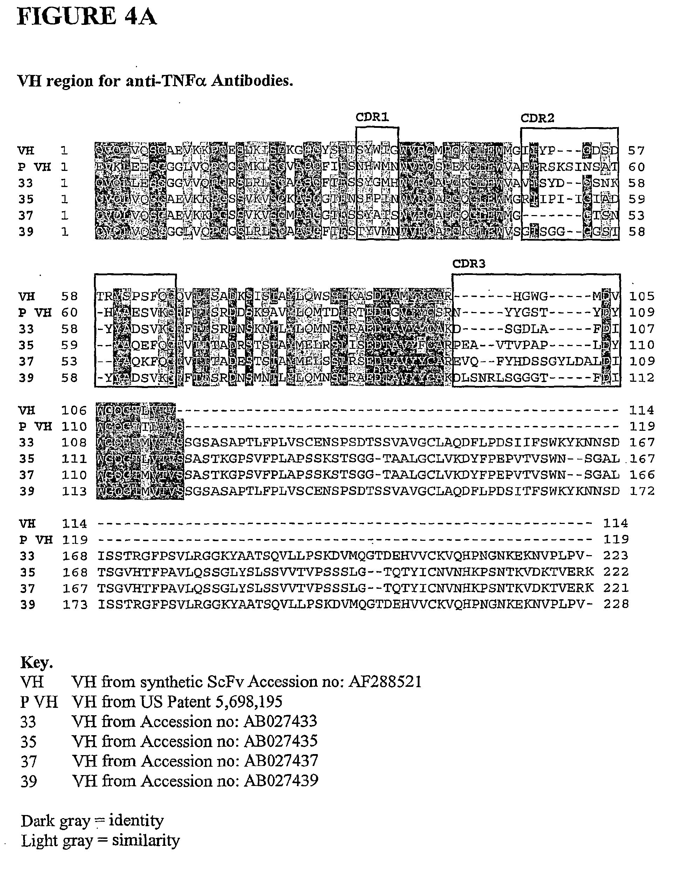

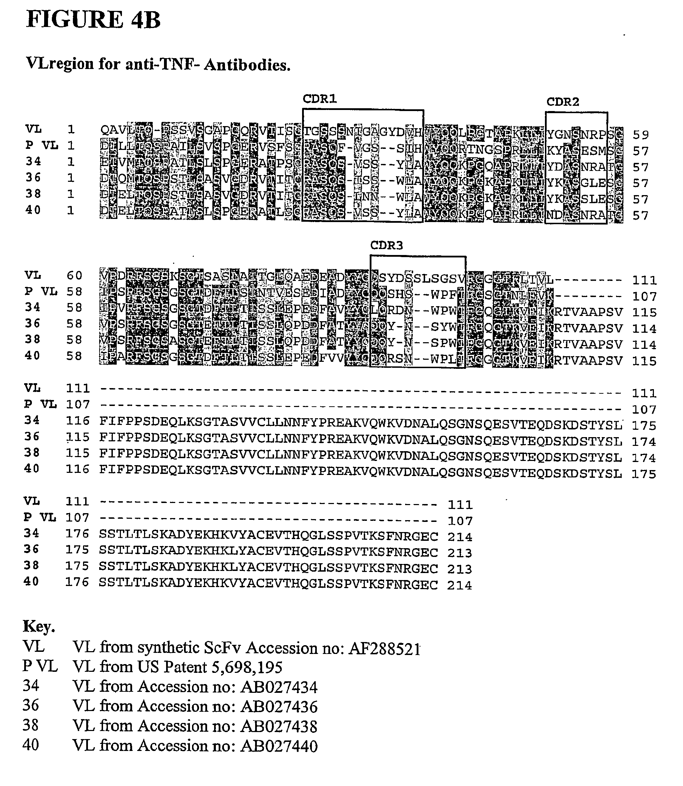

SCA can be fused to the N-, C- or N- and C- termini of modified transferrin. These fusions could also be carried out using different parts or domains of transferrin such as the N domain or C domain. The proteins could be fused directly or using a linker peptide of various length. It is also possible to fuse all or part of the active SCA within the scaffold of transferrin. In such instances the fusion protein is made by inserting the cDNA of the SCA within the cDNA of transferrin for production of the protein in cells. A specific example of a SCA that can be fused to Transferrin is anti-TNF (tumor necrosis factor). Anti-TNF has been used to treat various inflammatory and autoimmune diseases. TNF-SCA could be fused to the N- or C- terminus of modified transferrin in such manner that the coding N-terminus of TNF-SCA is directly attached to the C-terminal amino acid of Transferrin or the C-terminal amino acid of TNF-SCA is directly attached to the N-terminal amino acid of Transferrin. Alternatively, a peptide linker could be inserted to provide more separation between Transferrin and TNF-SCA and allow more spatial mobility to the two fused proteins. Several examples of TNF-SCA are shown in Figure 4A-4B.

Single chain antibodies are produced by several methods including but not limited to: selection from phage libraries, cloning of the variable region of a specific antibody by cloning the cDNA of the antibody and using the flanking constant regions as the primer to clone the variable region, or by synthesizing an oligonucleotide corresponding to the variable region of any specific antibody. The cDNA can be tailored at the 5' and 3' ends to generate restriction sites, such that oligonucleotide linkers can be used, for cloning of the cDNA into a vector containing the cDNA for transferrin. This can be at the N- or C-terminus or N- and C- termini with or without the use of a spacer sequence. The SCA molecule cDNA is cloned into a vector from which the complete expression cassette is then excised and inserted into an expression vector to allow the expression of the fusion protein in yeast. The fusion protein secreted from the yeast can then be collected and purified from the media and tested for its activity. For expression in mammalian cell lines a similar procedure is adopted except that the expression cassette used employs a mammalian promoter, leader sequence and terminator. This expression cassette is then excised and inserted into a plasmid suitable for the transfection of mammalian cell lines. The antibody produced in this manner can be purified from media and tested for its binding to its antigen using standard immunochemical methods.

### Example 8

CDRs are the variable regions of antibodies that interact with antigens. These usually consist of relatively short stretches of peptides. Antibodies normally have three CDRs in their heavy chains and three in their light chains. One or more CDRs of an antibody which can interact with the antigen can be fused to modified transferrin to confer antigen binding activity to Transferrin molecule. The CDRs can be fused to the N-, C-, N-and C- termini or engineered into the interior scaffold of transferrin. Examples of the CDRs sequences from anti-TNF antibodies are shown in the TNF-SCA Figure 4A-4B. cDNAs corresponding to one or more CDRs can be fused with modified transferrin to confer TNF binding activity to transferrin.

### Example 9

Transferrin fusion technology can also be used to improve the therapeutic properties of peptides that are discovered in various systems such as phage display libraries and peptide libraries. Many of these peptides have biological activities without any homology to natural proteins or peptides. These peptides, due to their short *in vivo* half-lives, are good candidates for fusion to modified transferrin. Because of their small size they can be fused in variety of regions of transferrin molecule. In addition to the N-and C- termini, they can be inserted in various regions within Transferrin including but not limited to the cystine loops. In this manner the three-dimensional structure of the peptide within transferrin stays relatively rigid. More than one copy of each peptide and more than one peptide can be fused to modified transferrin. Moreover, the peptide sequence may be used to replace portion of transferrin to confer therapeutic activity to transferrin. Since most of these peptides are short, their cDNA can be synthesized with appropriate restriction sites for insertion into the modified transferrin cDNA. The cDNA could then be inserted in a vector containing the transferrin cDNA in such a manner that the peptide is expressed as part of transferrin or fused to transferrin molecule. Alternatively, PCR primers could be synthesized that contain the peptide of interest and appropriate section of Transferrin. Using these primers amplification of transferrin cDNA results in the fusion of the peptide to the chosen site on Transferrin. Examples of such peptides are the EPO mimetic peptides: GGTYSCHFGPLTWVCKPQGG (SEQ ID NO: 11); DREGCRRGWVGQCKAWFN (SEQ ID NO: 12); and QRVEILEGRTECVLSNLRGRTRY (SEQ ID NO: 30), which have no homology with the natural EPO but have similar biological activities in that they activate the EPO receptor acting as agonists. These peptides also need to have specific conformation for their optimal activity. EPO mimetic peptides can be inserted (or it can replace) in one or more cystine loops of Transferrin. In this manner Transferrin can acquire EPO activity. Other peptides that can be fused to Transferrin are peptides with binding activity similar to antibodies. These peptides can bind to proteins with relatively high affinity and provide the same biological function as antibodies except their in vivo half-life is very short. Fusion of these peptides to Transferrin could confer much longer half-life for these peptides without destroying their binding activities. These peptides could be fused to N- or C- terminus or both or within the Transferrin molecule. The peptides can also replace part of transferrin. In addition more than one copy of a peptide or several different peptides could be attached to a single transferrin molecule. An example of such molecule is a peptide that can bind TNF. Attachment of this peptide to Transferrin gives Transferrin the ability to bind TNF and act similar to anti-TNF antibodies. In this manner antibody like molecules with much easier and economical manufacturing protocol could be made.

### Example 10

Targeted Tf fusion proteins have a combination of two or more proteins or peptides fused to modified transferrin to serve as a bifunctional molecule. In this case modified transferrin is fused to one protein or peptide to have a new biological activity and to another protein or peptide to targeting. An example of such protein is a transferrin that contains an inhibitory protein such as endostatin and a targeting peptide such as SCA or binding peptide which can recognize tumours. In this manner the inhibitory molecule is targeted to the tumour where it is needed. The cDNA for the protein of interest can be isolated from cDNA library or can be made synthetically using several overlapping oligonucleotide primers using standard molecular biology methods. The appropriate nucleotides can be engineered in the cDNA to form convenient restriction sites and also allow the attachment of the protein cDNA to transferrin cDNA similar to the method described for other fusions. Also a targeting protein or peptide cDNA such as single chain antibody or peptides, such as nuclear localization signals, that can direct proteins inside the cells can be fused to the other end or within transferrin. The protein of interest and the targeting peptide is cloned into a vector, which allows the fusion with transferrin cDNA. In this manner both proteins/peptides are fused to modified transferrin. The fused cDNA is then excised and is inserted into an expression vector to allow the expression of the fusion protein in yeast.

All the above procedures can be performed using standard methods in molecular biology. The fusion protein secreted from yeast can be collected and purified from the media and tested for its biological activity and its targeting activity using appropriate biochemical and biological tests. These proteins could also be made in other systems such as mammalian tissue culture using appropriate vector and transfection protocol.

### Example 11

The cDNA for the enzyme of interest can be isolated by a variety of means such as RT-PCR from mRNA, from cDNA libraries, by synthetically constructing the cDNA from overlapping oligonucleotides, by PCR or by other means known to the art, all using standard methods. The cDNA can be tailored at the 5' and 3' ends to generate restriction sites, such that oligonucleotide linkers can be used, for cloning of the cDNA into a vector containing the cDNA for modified transferrin. This can be at the N or C-terminus with or without the use of a spacer sequence. The enzyme cDNA is cloned into a vector such from which the complete expression cassette is then excised and inserted an expression vector to allow the expression of the fusion protein in yeast. The fusion protein secreted from the yeast can then be collected and purified from the media and tested for its biological activity. For expression in mammalian cell lines a similar procedure is adopted except that the expression cassette used employs a mammalian promoter, leader sequence and terminator. This expression cassette is then excised and inserted into a plasmid suitable for the transfection of mammalian cell lines.

### Example 12

Using phage display, peptides are isolated specific for a specific cell marker on the surface of, for example, a tumor cell. The peptide is then fused to the N-, C- or N- and C-termini of modified transferrin to target the fusion to that specific cell type. The transferrin fusion protein is then loaded with a metal ion which resembles iron in its transferrin binding properties, but which is cytotoxic, for example gallium or radioactive ions. By this mechanism the gallium or the radioactive ion is targeted to the cell type.

Although the present invention has been described in detail with reference to examples above, it is understood that various modifications can be made without departing from the scope of the claims. Accordingly, the invention is limited only by the following claims.

## Claims

1. A purified fusion protein comprising a transferrin (Tf) protein exhibiting reduced glycosylation fused to at least one therapeutic protein or peptide, wherein the serum half-life or serum stability of the therapeutic protein or peptide is increased over the serum half-life or serum stability of the therapeutic protein or peptide in an unfused state and wherein said Tf protein comprises at least one mutation that reduces or prevents glycosylation.

2. A fusion protein of claim 1, wherein the Tf protein contains one or more amino acid substitutions, insertions or deletions compared to a wild type sequence which result in reduced affinity for a Tf receptor (TfR).

3. The fusion protein of claim 2, wherein the Tf protein does not bind a TfR.

4. The fusion protein of claim 1, wherein the Tf protein has reduced iron binding.

5. The fusion protein of claim 4, where the Tf protein does not bind iron.

6. The fusion protein of claim 1, wherein the Tf protein is lacto transferrin (lactoferrin).

7. The fusion protein of any one of the preceding claims, wherein the glycosylation site is selected from the group consisting of an amino acid residue corresponding to amino acids N413, N611, or an amino acid adjacent to or in an N-X-S/T glycosylation site.

8. The fusion protein of claim 2 or 3, wherein the Tf comprises at least one amino acid substitution, deletion or addition at an amino acid residue corresponding to an amino acid selected from the group consisting of Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 and Gly 459.

9. The fusion protein of claim 1, wherein the therapeutic protein or peptide is fused to the C-terminal end of Tf.

10. The fusion protein of claim 1, wherein the therapeutic protein or peptide is fused to the N-terminal end of Tf.

11. The fusion protein of claim 1, wherein the therapeutic protein or peptide is inserted into at least one loop of the Tf.

12. The fusion protein of claim 1, wherein said Tf protein comprises a portion of the N domain of a Tf protein, a bridging peptide and a portion of the C domain of a Tf protein.

13. The fusion protein of claim 12, wherein the bridging peptide links the therapeutic protein or peptide to Tf.

14. The fusion protein of claim 12, wherein said therapeutic protein, peptide or polypeptide is inserted between an N and a C domain of Tf protein.

15. The fusion protein of claim 1, wherein the Tf protein has a hinge region and at least one amino acid substitution, deletion or addition in the Tf hinge region.

16. The fusion protein of claim 15, wherein said hinge region in selected from the group consisting of about residue 94 to about residue 96, about residue 245 to about residue 247, about residue 316 to about residue 318, about residue 425 to about residue 427, about residue 581 to about residue 582 and about residue 652 to about residue 658.

17. The fusion protein of claim 1, wherein said Tf protein has at least one amino acid substitution, deletion or addition at a position selected from the group consisting of Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 and Gly 459.

18. The fusion protein of claim 9, wherein the therapeutic protein or peptide replaces at least one loop.

19. A nucleic acid molecule encoding a fusion protein of claim 1.

20. A vector comprising a nucleic acid molecule of claim 19.

21. A host cell comprising a vector of claim 20.

22. A host cell comprising a nucleic acid molecule of claim 19.

23. A method of expressing a Tf fusion protein comprising culturing a host cell of claim 21 under conditions which express the encoded fusion protein.

24. A method of expressing a Tf fusion protein comprising culturing a host cell of claim 22 under conditions which express the encoded fusion protein.

25. The method of claim 23 or 24, wherein the Tf fusion protein is expressed in the presence of an agent that prevents glycosylation.

26. A host cell of claim 21 or 22, wherein the cell is prokaryotic or eukaryotic.

27. A host cell of claim 26, wherein the cell is a yeast cell.

28. A non-human transgenic animal comprising a nucleic acid molecule of claim 19.

29. A method of producing a Tf fusion protein comprising isolating a fusion protein from a transgenic animal of claim 28.

30. A method of claim 29, wherein the Tf fusion protein comprises lactoferrin.

31. A method of claim 30, wherein the fusion protein is isolated from a biological fluid from the transgenic animal.

32. A method of claim 30, wherein the fluid is serum or milk.

33. A method of increasing the serum half life or serum stability of a therapeutic protein or peptide, comprising expressing said therapeutic protein or peptide as a fusion protein with a transferrin (Tf) protein, wherein:
said Tf protein comprises at least one mutation that reduces or
prevents glycosylation;
such that the transferrin (Tf) protein exhibits reduced glycosylation.

34. A fusion protein of any one of claims 1-18, wherein the therapeutic protein or peptide is selected from the group consisting of hormones, matrix proteins, immunosuppressants, bronchodilators, cardiovascular agents, enzymes, CNS agents, neurotransmitters, growth hormones, growth factors, antiviral peptides, fusogenic inhibitor peptides, cytokines, lymphokines, monokines, interleukins, colony stimulating factors, differentiation factors, angiogenic factors, receptor ligands, cancer-associated proteins, antineoplastics, viral peptides, antibiotic peptides, blood proteins, antagonist proteins, transcription factors, anti-angiogenic factors, antagonist proteins or peptides, receptor antagonists, antibodies, single chain antibodies, and cell adhesion molecules.

35. A fusion protein of claim 34, wherein the therapeutic protein is an antibody or fragment thereof.

36. The fusion protein of claim 35, wherein the antibody or fragment thereof comprises at least one CDR.

37. The fusion protein of claim 35, wherein the antibody or fragment thereof is a single chain antibody.

38. The fusion protein of claim 34, wherein the therapeutic protein or peptide is a cytokine.

39. The fusion protein of claim 38, wherein the cytokine is selected from the group consisting of IL-2, IL-3, IL-4, IL-5, IL-6, IL-7, IL-10, IL-12, IL-13, IL-14, IL-15, IL-16, IL-17, IL-18, granulocyte-macrophage colony stimulating factor, granulocyte colony stimulating factor, platelet activating factor, erythropoeietin, TNFα, lymphotoxin-α, lymphotoxin-β, leukoregulin, macrophage migration inhibitory factor, neuroleukin, leptin, IFNα, IFNβ, IFNγ, IFNo, thrombospondin 1, THBS2, THBS3, THBS4 and chemokine.

40. The fusion protein of claim 39, wherein the cytokine is IFNβ.

41. The fusion protein of claim 34, wherein the therapeutic protein or peptide is a hormone.

42. The fusion protein of claim 41, wherein the hormone is selected from the group consisting of proinsulin, insulin, growth hormone 1, growth hormone 2, growth hormone release factor, insulin-like growth factor I, insulin-like growth factor II, insulin-like growth factor binding protein 1 (IGFBP-1), IGFBP-2, IGFBP-3, IGFBP-4, IGFBP-5, IGFBP-6, IGFBP-7, chorionic gonadotropin β chain, chorionic gonadotropin α chain, luteinizing hormone β, follicle-stimulating hormone β, thyroid-stimulating hormone β, prolactin, pro-opiomelanocortin, corticotrophin (ACTH), β-lipotropin, α-melanocyte stimulating hormone (α-MSH), γ-lipotropin, β-MSH, β-endorphin and corticotrophin-like intermediate lobe peptide (CLIP).

43. The fusion protein of claim 34, wherein the therapeutic protein or peptide is a growth factor.

44. The fusion protein of claim 43, wherein the growth factor is selected from the group consisting of platelet-derived growth factor-α (PDGF-α), PDGF-β, steroid hormones, epidermal growth factor (EGF), fibroblast growth factors such as fibroblast growth factor 1 (FGF1), FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF22, FGF23, angiogenin, brain derived neurotrophic factor, ciliary neurotrophic growth factor, transforming growth factor-α (TGF-α), TGF- β, nerve growth factor-α (NGF-α), NGF-β, tissue inhibitor of metalloproteinase 1 (TIMP1), TIMP2, TIMP3, TIMP4 and macrophage stimulating 1.

45. The fusion protein of claim 34, wherein the therapeutic protein or peptide is a matrix protein.

46. The fusion protein of claim 45, wherein the matrix protein is selected from the group consisting of collagen I, collagen II, collagen III, collagen IV, collagen V, collagen VI, collagen VII, collagen VIII, collagen IX, collagen X, collagen XI, and collagen XII, LAMA2, LAMA3, LAMA4, LAMB1, LAMB3, LAMC1, nidogen, α-tectorin, β-tectorin and fibronectin.

47. The fusion protein of claim 34, wherein the therapeutic protein or peptide is a blood protein.

48. The fusion protein of claim 47, wherein the blood protein is selected from the group consisting of a native serum protein, a blood clotting factor, antithrombin 3, alpha-1 antitrypsin, urokinase-type plasminogen activator, an immunoglobulin, von Willebrand factor, fibronectin, fibrinogen, thrombin and haemoglobin.

49. The fusion protein of claim 34, wherein the therapeutic protein or peptide is an enzyme.

50. The fusion protein of claim 49, wherein the enzyme is selected from the group consisting of a coagulation factor, a matrix metalloproteinase, adenosine deaminase, a mitogen activated protein kinase, a kinase and a phosphatase.

51. The fusion protein of claim 34, wherein the therapeutic protein or peptide is a transcription factor.

52. The fusion protein of claim 51, wherein the transcription factor is selected from the group consisting of Sp1, Sp2, Sp3, Sp4, NFYB, Hap2, GATA-1, GATA-2, GATA-3, GATA-4, GATA-5, GATA-6, FOG2, Eryfl, TRPS1, NF-E2, NF-E3, NF-E4, TFCP2, Oct-1, HOXB2, HOX2H, hairless homolog, MADH1, MADH2, MADH3, MADH4, MADH5, MADH6, MADH7, MADH9, STAT1, STAT2, STAT3, STAT4, STAT5 and STAT6.

53. The fusion protein of claim 34, wherein the therapeutic protein or peptide is a cancer-associated protein.

54. The fusion protein of claim 53, wherein the cancer-associated protein is selected from the group consisting of p16, p53, p63, p73, BRCA1, BRCA2, CTBP interacting protein, DMBT1, HRAS, NCYM, FGR, myb, raf1, erB2, VAV, c-fos, c-fes, c-jun, MAS1, pim-1, TIF1, c-fms, EGFR, erbA, c-src tyrosine kinase, c-abl, N-ras, K-ras, jun-B, c-myc, RB1, DCC, APC, NF1, NF2, and bcl-2.

55. The fusion protein of claim 34, wherein the therapeutic protein or peptide is a fusogenic inhibitor peptide.

56. The fusion protein of claim 55, wherein the fusogenic inhibitor peptide is selected from the group consisting of HIV T-20, HIV T-1249, RSV T786, RSV T1584 and RSV T112.

57. The fusion protein of claim 56, which is expressed in the presence of tunicamycin.

58. The fusion protein of claims 1-18, wherein the Tf protein is lacto transferrin.

59. The fusion protein of claim 4 or 5, wherein the Tf comprises at least one amino acid substitution, deletion or addition at an amino acid residue corresponding to an amino acid selected from the group consisting of Asp 63, Gly 65, Asp 392, Tyr 95, Tyr 426, Tyr 188, Tyr 514, Tyr 517, His 249, His 585, Lys 206, His 207 and Arg 632.

60. A fusion protein according to any one of claims 1 to 18 for use in a method of medical treatment.

61. The fusion protein of any one of the preceding claims wherein the mutation is in an N-linked glycosylation site comprising the sequence N-X-S/T.

62. The fusion protein of claim 61, wherein the sequence N-X-S/T sequence begins at an amino acid corresponding to N413 or N611 of SEQ ID NO: 3.

63. The fusion protein of claim 61, wherein the Tf protein lacks N-linked glycosylation.

64. The fusion protein of claim 1, wherein the Tf protein is unglycosylated.

65. A purified fusion protein comprising a Tf protein comprising mutation in an N-linked N-X-S/T glycosylation site fused to at least one therapeutic protein or peptide, wherein the transferrin protein exhibits reduced glycosylation as compared to a fully glycosylated Tf protein and wherein the serum half-life of the therapeutic protein or peptide is increased over the serum half-life of the therapeutic protein or peptide in an unfused state.

66. The fusion protein of claim 1, wherein said Tf protein is modified at S32 to reduce O glycosylation.

67. The fusion protein of claim 1 or 65, wherein the transferrin protein is human transferrin.

68. The fusion protein of claim 67, wherein the human transferrin comprises SEQ ID NO: 3.

69. The fusion protein of claims 1, 15 or 65, wherein the transferrin protein is a transferrin splice variant.

70. The fusion protein of claims 1 or 65, wherein said fusion protein increases *in vitro* stability of the at least one therapeutic protein or peptide.

71. The fusion protein of claims 1 or 65, wherein said fusion protein increases bioavailability of the at least one therapeutic protein or peptide.

72. The fusion protein of claim 1, wherein said fusion protein binds iron.

73. The fusion protein of claim 1, wherein said fusion protein is capable of crossing a blood brain barrier.

74. The fusion protein of claim 1 or 65, wherein the Tf protein is connected to the at least one therapeutic protein or peptide by a linker peptide.

75. The fusion protein of claim 74 , wherein said linker is less than 50 amino acid residues.

76. The fusion protein of claim 74, wherein said linker comprises a polyglycine stretch.

77. The fusion protein of claim 1 or 65, wherein said Tf fusion protein exhibits reduced carbonate binding.

78. The fusion protein of claim 1 or 65, wherein said fusion protein contains one or more amino acid substitutions, insertions or deletions compared to a wild type sequence and is not capable of crossing a blood brain barrier.

79. A composition comprising a fusion protein of any one of claims 1-18 and 34-78.

80. Use of a transferrin protein exhibiting reduced glycosylation to increase the serum half-life or serum stability of a therapeutic protein, wherein said transferrin protein is fused to said therapeutic protein to form a fusion protein according to any of claims 1 to 18 or 34 to 78.

## Patentansprüche

1. Gereinigtes Fusionsprotein, umfassend ein Transferrin (Tf)-Protein, das reduzierte Glykosylierung aufweist, das an wenigstens ein therapeutisches Protein oder Peptid fusioniert ist, wobei die Serumhaibwertszeit oder die Serumstabilität des therapeutischen Proteins oder Peptids gegenüber der Serumhalbwertszeit oder Serumstabilität des therapeutischen Proteins oder Peptids in einem nichtfusionierten Zustand erhöht ist und wobei das Tf-Protein wenigstens eine Mutation umfasst, die eine Glykosylierung verringert oder verhindert.

2. Fusionsprotein nach Anspruch 1, wobei das Tf-Protein eine oder mehrere Aminosäure-Substitutionen, -Insertionen oder -Deletionen, verglichen mit einer Wildtyp-Sequenz, enthält, welche in einer reduzierten Affinität für einen Tf-Rezeptor (TfR) resultiert/resultieren.

3. Fusionsprotein nach Anspruch 2, wobei das Tf-Protein nicht an einen TfR bindet.

4. Fusionsprotein nach Anspruch 1, wobei das Tf-Protein eine verringerte Eisenbindung hat.

5. Fusionsprotein nach Anspruch 4, wobei das Tf-Protein kein Eisen bindet.

6. Fusionsprotein nach Anspruch 1, wobei das Tf-Protein Lactotransferrin (Lactoferrin) ist.

7. Fusionsprotein nach einem der vorangehenden Ansprüche, wobei die Glykosylierungsstelle aus der Gruppe, bestehend aus einem Aminosäurerest, der Aminosäuren N413, N611 und einer Aminosäure, benachbart zu oder in einer N-X-S/T-Glykosylierungsstelle, entspricht, ausgewählt ist.

8. Fusionsprotein nach Anspruch 2 oder 3, wobei das Tf wenigstens eine Aminosäure-Substitution, -Deletion oder -Addition an einem Aminosäurerest umfasst, der einer Aminosäure entspricht, die ausgewählt ist aus der Gruppe, bestehend aus Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 und Gly 459.

9. Fusionsprotein nach Anspruch 1, wobei das therapeutische Protein oder Peptid an das C-terminale Ende von Tf fusioniert ist.

10. Fusionsprotein nach Anspruch 1, wobei das therapeutische Protein oder Peptid an das N-terminale Ende von Tf fusioniert ist.

11. Fusionsprotein nach Anspruch 1, wobei das therapeutische Protein oder Peptid in wenigstens eine Schleife des Tf inseriert ist.

12. Fusionsprotein nach Anspruch 1, wobei das Tf-Protein einen Teil der N-Domäne eines Tf-Proteins, ein Brückenpeptid und einen Teil der C-Domäne eines Tf-Proteins umfasst.

13. Fusionsprotein nach Anspruch 12, wobei das Brückenpeptid das therapeutische Protein oder Peptid mit Tf verknüpft.

14. Fusionsprotein nach Anspruch 12, wobei das therapeutische Protein, Peptid oder Polypeptid zwischen eine N- und eine C-Domäne von Tf-Protein inseriert ist.

15. Fusionsprotein nach Anspruch 1, wobei das Tf-Protein eine Hinge-Region und wenigstens eine Aminosäure-Substitution, -Deletion oder -Addition in der Tf-Hinge-Region hat.

16. Fusionsprotein nach Anspruch 15, wobei die Hinge-Region aus der Gruppe, bestehend aus etwa Rest 94 bis etwa Rest 96, etwa Rest 245 bis etwa Rest 247, etwa Rest 316 bis etwa Rest 318, etwa Rest 425 bis etwa Rest 427, etwa Rest 581 bis etwa Rest 582 und etwa Rest 652 bis etwa Pest 658, ausgewählt ist.

17. Fusionsprotein nach Anspruch 1, wobei das Tf-Protein wenigstens eine Aminosäure-Substitution, -Deletion oder -Addition an einer Position hat, die aus der Gruppe, bestehend aus Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 und Gly 459, ausgewählt ist.

18. Fusionsprotein nach Anspruch 9, wobei das therapeutische Protein oder Peptid wenigstens eine Schleife ersetzt.

19. Nucleinsäuremolekül, das für ein Fusionsprotein nach Anspruch 1 codiert.

20. Vektor, der ein Nucleinsäuremolekül nach Anspruch 19 umfasst.

21. Wirtszelle, die einen Vektor nach Anspruch 20 umfasst.

22. Wirtszelle, die ein Nucleinsäuremolekül nach Anspruch 19 umfasst.

23. Verfahren zum Exprimieren eines Tf-Fusionsproteins, das Kultivierten einer Wirtszelle nach Anspruch 21 unter Bedingungen, welche das codierte Fusionsprotein exprimieren, umfasst.

24. Verfahren zum Exprimieren eines Tf-Fusionsproteins, das Kultivieren einer Wirtszelle nach Anspruch 22 unter Bedingungen, die das codierte Fusionsprotein exprimieren, umfasst.

25. Verfahren nach Anspruch 23 oder 24, wobei das Tf-Fusionsprotein in Gegenwart eines Mittels, das eine Glykosylierung verhindert, exprimiert wird.

26. Wirtszelle nach Anspruch 21 oder 22, wobei die Zelle prokaryotisch oder eukaryctisch ist.

27. Wirtszelle nach Anspruch 26, wobei die Zelle eine Hefezelle ist.

28. Nichtmenschliches transgenes Tier, das ein Nucleinsäuremolekül nach Anspruch 19 umfasst.

29. Verfahren zur Herstellung eines Tf-Fusionsproteins, umfassend Isolieren eines Fusionsproteins aus einem transgenen Tier nach Anspruch 28.

30. Verfahren nach Anspruch 29, wobei das Tf-Fusionsprotein Lactoferrin umfasst.

31. Verfahren nach Anspruch 30, wobei das Fusionsprotein aus einer biologischen Flüssigkeit aus dem transgenen Tier isoliert wird.

32. Verfahren nach Anspruch 30, wobei die Flüssigkeit Serum oder Milch ist.

33. Verfahren zur Erhöhung der Serumhalbwertszeit oder Serumstabilität eines therapeutischen Proteins oder Peptids, das Exprimieren des therapeutischen Proteins oder Peptids als Fusionsprotein mit einem Transferrin (Tf)-Protein umfasst, wobei:
das Tf-Protein wenigstens eine Mutation umfasst, die eine Glykosylierung verringert oder verhindert,
derart, dass das Transferrin (Tf)-Protein verringerte Glykosylierung aufweist.

34. Fusionsprotein nach einem der Ansprüche 1 bis 18, wobei das therapeutische Protein oder Peptid aus der Gruppe, bestehend aus Hormonen, Matrixproteinen, Immunsuppressiva, Bronchodilatatoren, kardiovaskulären Mitteln, Enzymen, ZNS-Mitteln, Neurotransmittern, Wachstumshormonen, Wachstumsfaktoren, anti-viralen Peptiden, fusogenen Inhibitorpeptiden, Cytokinen, Lymphokinen, Monokinen, Interleukinen, das Koloniewachstum stimulierenden Faktoren, Differenzierungsfaktoren, angiogenen Faktoren, Rezeptorliganden, mit Krebs assoziierten Proteinen, anti-neoplastischen Mitteln, viralen Peptiden, antibiotischen Peptiden, Blutproteinen, Antagonistproteinen, Transkriptionsfaktoren, anti-angiogenen Faktoren, Antagonistproteinen oder -peptiden, Rezeptorantagonisten, Antikörpern, Einkettenantikörpern und Zelladhäsionsmolekülen, ausgewählt ist.

35. Fusionsprotein nach Anspruch 34, wobei das therapeutische Protein ein Antikörper oder Fragment davon ist.

36. Fusionsprotein nach Anspruch 35, wobei der Antikörper oder das Fragment davon wenigstens einen CDR umfasst.

37. Fusionsprotein nach Anspruch 35, wobei der Antikörper oder das Fragment davon ein Einkettenantikörper ist.

38. Fusionsprotein nach Anspruch 34, wobei das therapeutische Protein oder Peptid ein Cytokin ist.

39. Fusionsprotein nach Anspruch 38, wobei das Cytokin aus der Gruppe, bestehend aus IL-2, IL-3, IL-4, IL-5, IL-6, IL-7, IL-10, IL-12, IL-13, IL-14, IL-15, IL-16, IL-17, IL-18, Granulozyten-Makrophagen-Kolonie-stimulierendem Faktor, Granulozyten-Kolonie-stimulierendem Faktor, Plättchen-Aktivierungsfaktor, Erythropoeietin, TNFα, Lymphotoxin-α, Lymphotoxin-β, Leukoregulin, Makrophagenmigrations-Inhibitor-Faktor, Neuroleukin, Leptin, IFNα, IFNβ, IFNγ, IFNo, Thrombospondin 1, THBS2, THBS3, THBS4 und Chemokin, ausgewählt ist.

40. Fusionsprotein nach Anspruch 39, wobei das Cytokin IENβ ist.

41. Fusionsprotein nach Anspruch 34, wobei das therapeutische Protein oder Peptid ein Hormon ist.

42. Fusionsprotein nach Anspruch 41, wobei das Hormon aus der Gruppe, bestehend aus Proinsulin, Insulin, Wachstumshormon 1, Wachstumshormon 2, Wachstumshormon-Freisetzungsfaktor, insulinartigem Wachstumsfaktor I, insulinartigem Wachstumsfaktor II, insulinartigem Wachstumsfaktor-Bindungsprotein 1 (IGFBP-1), IGFBP-2, IGFBP-3, IGFBP-4, IGFBP-5, IGFBP-6, IGFBP-7, Choriongonadotropin-β-Kette, Choriongonatropin-α-Kette, Luteinisierungshormon β, Follikel-stimulierendem Hormon β, Schilddrüse-stimulierendem Hormon β, Prolactin, Proopiomelanokortin, Corcicotrophin (ACTH), β-Lipotropin, α-Melanozyten-stimulierendem Hormon (α-MSH), γ-Lipotropin, β-MSH, β-Endorphin und Corticotrophin-artigem Zwischenlappenpeptid (CLIP), ausgewählt ist.

43. Fusionsprotein nach Anspruch 34, wobei das therapeutische Protein oder Peptid ein Wachstumsfaktor ist.

44. Fusionsprotein nach Anspruch 43, wobei der Wachstumsfaktor aus der Gruppe, bestehend aus Platelet-derived growth factor-α (PDGF-α), PDGF-β, Steroidhormonen, epidermalem Wachstumsfaktor (EGF), Fibroblastenwachstumsfaktoren, zum Beispiel Fibroblastenwachstumsfaktor 1 (FGF1), FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF22, FGF23, Angiogenin, aus dem Gehirn stammendem neurotrophem Faktor, neurotrophem Ziliarwachstumsfaktor, transformierendem Wachstumsfaktor-α, (TGF-α), TGF-β, Nervenwachstumsfaktor-α (NGF-α), NGF-β, Gewebeinhibitor von Metalloproteinase 1 (TIMP1), TIMP2, TIMP3, TIMP4 und Makrophagenstimulierendem Faktor 1, ausgewählt ist.

45. Fusionsprotein nach Anspruch 34, wobei das therapeutische Protein oder Peptid ein Matrixprotein ist.

46. Fusionsprotein nach Anspruch 45, wobei das Matrixprotein aus der Gruppe, bestehend aus Kollagen I, Kollagen II, Kollagen III, Kollagen IV, Kollagen V, Kollagen VI, Kollagen VII, Kollagen VIII, Kollagen IX, Kollagen X, Kollagen XI und Kollagen XII, LAMA2, LAMA3, LAMA4, LAMB1, LAMB3, LAMC1, Nidogen, α-Tectorin, β-Tectorin und Fibronectin, ausgewählt ist.

47. Fusionsprotein nach Anspruch 34, wobei das therapeutische Protein oder Peptid ein Blutprotein ist.

48. Fusionsprotein nach Anspruch 47, wobei das Blutprotein aus der Gruppe, bestehend aus einem nativen Serumprotein, einem Blutgerinnungsfaktor, Antithrombin 3, Alpha-1-antitrypsin, Plasminogenaktivator des Urokinase-Typs, einem Immunglobulin, von-Willebrand-Faktor, Fibronectin, Fibrinogen, Thrombin und Hämoglobin, ausgewählt ist.

49. Fusionsprotein nach Anspruch 34, wobei das therapeutische Protein oder Peptid ein Enzym ist.

50. Fusionsprotein nach Anspruch 49, wobei das Enzym aus der Gruppe, bestehend aus einem Gerinnungsfaktor, einer Matrixmetalloproteinase, Adenosindeaminase, einer Mitogenaktivierten Proteinkinase, einer Kinase und einer Phosphatase, ausgewählt ist.

51. Fusionsprotein nach Anspruch 34, wobei das therapeutische Protein oder Peptid ein Transkriptionsfaktor ist.

52. Fusionsprotein nach Anspruch 51, wobei der Transkriptionsfaktor aus der Gruppe, bestehend aus Sp1, Sp2, Sp3, Sp4, NFYB, Hap2, GATA-1, GATA-2, GATA-3, GATA-4, GATA-5, GATA-6, FOG2, Eryfl, TRPS1, NF-E2, NF-E3, NF-E4, TFCP2, Oct-1, HOXB2, HOX2H, Hairless Homolog, MADH1, MADH2, MADH3, MADH4, MADH5, MADH6, MADH7, MADH9, STAT1, STAT2, STAT3, STAT4, STAT5 und STAT6, ausgewählt ist.

53. Fusionsprotein nach Anspruch 34, wobei das therapeutische Protein oder Peptid ein mit Krebs assoziiertes Protein ist.

54. Fusionsprotein nach Anspruch 53, wobei das mit Krebs assoziierte Protein aus der Gruppe, bestehend aus p16, p53, p63, p73, BRCA1, BRCA2, mit CTBP interagierendem Protein, DMBT1, HRAS, NCYM, FGR, myb, raf1, erB2, VAV, c-fos, c-fes, c-jun, MAS1, pim-1, TIF1, c-fms, EGFR, erbA, c-src-Tyrosinkinase, c-abl, N-ras, K-ras, jun-B, c-myc, RB1, DCC, APC, NF1, NF2 und bcl-2, ausgewählt ist.

55. Fusionsprotein nach Anspruch 34, wobei das therapeutische Protein oder Peptid ein fusogenes Inhibitorpeptid ist.

56. Fusionsprotein nach Anspruch 55, wobei das fusogene Inhibitorpeptid aus der Gruppe, bestehend aus HIV T-20, HIV T-1249, RSV T786, RSV T1584 und RSV T112, ausgewählt ist.

57. Fusionsprotein nach Anspruch 56, welches in Gegenwart von Tunicamycin exprimiert wird.

58. Fusionsprotein nach den Ansprüchen 1 bis 18, wobei das Tf-Protein Lactotransferrin ist.

59. Fusionsprotein nach Anspruch 4 oder 5, wobei das Tf wenigstens eine Aminosäure-Substitution, -Deletion oder -Addition an einem Aminosäurerest umfasst, welche einer Aminosäure entspricht, die aus der Gruppe, bestehend aus Asp 63, Gly 65, Asp 392, Tyr 95, Tyr 426, Tyr 188, Tyr 514, Tyr 517, His 249, His 585, Lys 206, His 207 und Arg 632, ausgewählt ist.

60. Fusionsprotein nach einem der Ansprüche 1 bis 18 zur Verwendung in einem Verfahren zur medizinischen Behandlung.

61. Fusionsprotein nach einem der vorangehenden Ansprüche, wobei die Mutation eine N-verknüpfte Glykosylierungsstelle ist, die die Sequenz N-X-S/T umfasst.

62. Fusionsprotein nach Anspruch 61, wobei die Sequenz N-X-S/T an einer Aminosäure beginnt, die N413 oder N611 von SEQ ID NO: 3 entspricht.

63. Fusionsprotein nach Anspruch 61, wobei dem Tf-Protein eine N-verknüpfte Glykosylierung fehlt.

64. Fusionsprotein nach Anspruch 1, wobei das Tf-Protein unglykosyliert ist.

65. Gereinigtes Fusionsprotein, umfassend ein Tf-Protein, umfassend eine Mutation in einer N-verknüpften N-X-S/T-Glykosylierungssteile, fusioniert an wenigstens ein therapeutisches Protein oder Peptid, wobei das Transferrin-Protein eine verringerte Glykosylierung im Vergleich zu einem vollständig glykosylierten Tf-Protein aufweist und wobei die Serumhalbwertszeit des therapeutischen Proteins oder Peptids gegenüber der Serumhalbwärtszeit des therapeutischen Proteins oder Peptids in einem unfusionierten Zustand erhöht ist.

66. Fusionsprotein nach Anspruch 1, wobei das Tf-Protein an S32 modifiziert ist, um eine O-Glykosylierung zu verringern.

67. Fusionsprotein nach Anspruch 1 oder 65, wobei das Transferrin-Protein humanes Transferrin ist.

68. Fusionsprotein nach Anspruch 67, wobei das humane Transferrin SEQ ID NO: 3 umfasst.

69. Fusionsprotein nach den Ansprüchen 1, 15 oder 65, wobei das Transferrin-Protein eine Transferrin-Spleißvariante ist.

70. Fusionsprotein nach Anspruch 1 oder 65, wobei das Fusionsprotein die in *vitro*-Stabilität des wenigstens einen therapeutischen Proteins oder Peptids erhöht.

71. Fusionsprotein nach Anspruch 1 oder 65, wobei das Fusionsprotein die Bioverfügbarkeit des wenigstens einen therapeutischen Proteins oder Peptids erhöht.

72. Fusionsprotein nach Anspruch 1, wobei das Fusionsprotein Eisen bindet.

73. Fusionsprotein nach Anspruch 1, wobei das Fusionsprotein fähig ist, die Blut-Hirn-Schranke zu durchqueren.

74. Fusionsprotein nach Anspruch 1 oder 65, wobei das Tf-Protein an wenigstens ein therapeutisches Protein oder Peptid durch ein Linkerpeptid gebunden ist.

75. Fusionsprotein nach Anspruch 74, wobei der Linker weniger als 50 Aminosäurereste ist.

76. Fusionsprotein nach Anspruch 74, wobei der Linker eine Polyglycin-Verlängerung umfasst.

77. Fusionsprotein nach Anspruch 1 oder 65, wobei das Tf-Fusionsprotein eine reduzierte Carbonatbindung aufweist.

78. Fusionsprotein nach Anspruch 1 oder 65, wobei das Fusionsprotein eine oder mehrere Aminosäure-Substitutionen, -Insertionen oder -Deletionen im Vergleich zu einer Wildtyp-Sequenz enthält und nicht fähig ist, eine Blut-Hirn-Schranke zu durchqueren.

79. Zusammensetzung, die ein Fusionsprotein nach einem der Ansprüche 1 bis 18 und 34 bis 78 umfasst.

80. Verwendung eines Transferrin-Proteins, das eine verringerte Glykosylierung aufweist, um die Serumhalbwertszeit oder die Serumstabilität eines therapeutischen Proteins zu erhöhen, wobei das Transferrin-Protein unter Bildung eines Fusionsproteins nach einem der Ansprüche 1 bis 18 oder 34 bis 78 an das therapeutische Protein fusioniert ist.

## Revendications

1. Protéine de fusion purifiée comprenant une protéine transferrine (Tf) présentant une glycosylation réduite fusionnée à au moins une protéine thérapeutique ou un peptide thérapeutique, dans laquelle la demi-vie sérique ou stabilité dans le sérum de la protéine thérapeutique ou du peptide thérapeutique est accrue par rapport à la demi-vie sérique ou la stabilité dans le sérum de la protéine thérapeutique ou du peptide thérapeutique à l'état non fusionné, et dans laquelle ladite protéine Tf comprend au moins une mutation qui réduit ou empêche la glycosylation.

2. Protéine de fusion suivant la revendication 1, dans laquelle la protéine Tf contient une ou plusieurs substitutions, insertions ou délétions d'aminoacides, comparativement à une séquence de type sauvage, ayant pour résultat une affinité réduite pour un récepteur de Tf (TfR).

3. Protéine de fusion suivant la revendication 2, dans laquelle la protéine Tf ne se lie pas un TfR.

4. Protéine de fusion suivant la revendication 1, dans laquelle la protéine Tf présente une liaison réduite au fer.

5. Protéine de fusion suivant la revendication 4, dans laquelle la protéine Tf ne se lie pas au fer.

6. Protéine de fusion suivant la revendication 1, dans laquelle la protéine Tf est la lactotranferrine (lactoferrine).

7. Protéine de fusion suivant l'une quelconque des revendications précédentes, dans laquelle le site de glycosylation est choisi dans le groupe consistant en un résidu d'aminoacide correspondant aux aminoacides N413 à N611, ou un aminoacide adjacent à ou dans un site de glycosylation N-X-S/T.

8. Protéine de fusion suivant la revendication 2 ou 3, dans laquelle la Tf comprend au moins une substitution, délétion ou addition d'aminoacide à un résidu d'aminoacide correspondant à un aminoacide choisi dans le groupe consistant en Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 et Gly 459.

9. Protéine de fusion suivant la revendication 1, dans la quelle la protéine thérapeutique ou le peptide thérapeutique est fusionné à l'extrémité C-terminale de Tf.

10. Protéine de fusion suivant la revendication 1, dans laquelle la protéine thérapeutique ou le peptide thérapeutique est fusionné à l'extrémité N-terminale de Tf.

11. Protéine de fusion suivant la revendication 1, dans laquelle la protéine thérapeutique ou le peptide thérapeutique est inséré dans au moins une boucle de la Tf.

12. Protéine de fusion suivant la revendication 1, dans laquelle ladite protéine Tf comprend une portion du domaine N d'une protéine Tf, un peptide de pontage et une portion du domaine C d'une protéine Tf.

13. Protéine de fusion suivant la revendication 12, dans laquelle le peptide de pontage lie la protéine thérapeutique ou le peptide thérapeutique à Tf.

14. Protéine de fusion suivant la revendication 12, dans laquelle ladite protéine thérapeutique, ledit peptide thérapeutique ou ledit polypeptide thérapeutique est inséré entre un domaine N et un domaine C de la protéine Tf.

15. Protéine de fusion suivant la revendication 1, dans laquelle la protéine Tf possède une région de charnière et au moins une substitution, délétion ou addition d'aminoacide dans la région de charnière de Tf.

16. Protéine de fusion suivant la revendication 15, dans laquelle ladite région de charnière est choisie dans le groupe consistant en les régions d'approximativement le résidu 94 à approximativement le résidu 96, d'approximativement le résidu 245 à approximativement le résidu 247, d'approximativement le résidu 316 à approximativement le résidu 318, d'approximativement le résidu 425 à approximativement le résidu 427, d'approximativement le résidu 581 à approximativement le résidu 582 et d'approximativement le résidu 652 à approximativement le résidu 658.

17. Protéine de fusion suivant la revendication 1, dans laquelle ladite protéine Tf possède au moins une substitution, délétion ou addition d'aminoacide à une position choisie dans le groupe consistant en Asp 63, Gly 65, Tyr 95, Tyr 188, Lys 206, His 207, His 249, Asp 392, Tyr 426, Tyr 514, Tyr 517, His 585, Thr 120, Arg 124, Ala 126, Gly 127, Thr 452, Arg 456, Ala 458 et Gly 459.

18. Protéine de fusion suivant la revendication 9, dans laquelle la protéine thérapeutique ou le peptide thérapeutique remplace au moins une boucle.

19. Molécule d'acide nucléique codant pour une protéine de fusion de la revendication 1.

20. Vecteur comprenant une molécule d'acide nucléique de la revendication 19.

21. Cellule hôte comprenant un vecteur de la revendication 20.

22. Cellule hôte comprenant une molécule d'acide nucléique de la revendication 19.

23. Procédé d'expression d'une protéine de fusion de Tf, comprenant la culture d'une cellule hôte de la revendication 21 dans des conditions permettant l'expression de la protéine de fusion codée.

24. Procédé d'expression d'une protéine de fusion de Tf, comprenant la culture d'une cellule hôte de la revendication 22 dans des conditions permettant l'expression de la protéine de fusion codée.

25. Procédé suivant la revendication 23 ou 24, dans lequel la protéine de fusion de Tf est exprimée en présence d'un agent qui empêche la glycosylation.

26. Cellule hôte suivant la revendication 21 ou 22, ladite cellule étant procaryotique ou eucaryotique.

27. Cellule hôte suivant la revendication 26, ladite cellule étant une cellule de levure.

28. Animal transgénique non humain, comprenant une molécule d'acide nucléique de la revendication 19.

29. Procédé pour la production d'une protéine de fusion de Tf, comprenant l'isolement d'une protéine de fusion à partir d'un animal transgénique de la revendication 28.

30. Procédé suivant la revendication 29, dans lequel la protéine de fusion de Tf comprend la lactoferrine.

31. Procédé suivant la revendication 30, dans lequel la protéine de fusion est isolée d'un fluide biologique provenant de l'animal transgénique.

32. Procédé suivant la revendication 30, dans lequel le fluide est le sérum ou le lait.

33. Procédé pour augmenter la demi-vie sérique ou la stabilité dans le sérum d'une protéine thérapeutique ou d'un peptide thérapeutique, comprenant l'expression de ladite protéine thérapeutique ou dudit peptide thérapeutique sous forme d'une protéine de fusion avec une protéine transferrine (Tf), dans lequel :
ladite protéine Tf comprend au moins une mutation qui réduit ou empêche la glycosylation ;
de telle sorte que la protéine transferrine (Tf) présente une glycosylation réduite.

34. Protéine de fusion suivant l'une quelconque des revendications 1 à 18, dans laquelle la protéine thérapeutique ou le peptide thérapeutique est choisi dans le groupe consistant en des hormones, des protéines de matrice, des immunosuppresseurs, des bronchodilatateurs, des agents cardiovasculaires, des enzymes, des agents agissant sur le SNC, des neurotransmetteurs, des hormones de croissance, des facteurs de croissance, des peptides antiviraux, des peptides inhibiteurs fusogéniques, des cytokines, des lymphokines, des monokines, des interleukines, des facteurs de stimulation de colonie, des facteurs de différentiation, des facteurs angiogéniques, des ligands de récepteurs, des protéines associées au cancer, des agents antinéoplasiques, des peptides viraux, des peptides antibiotiques, des protéines sanguines, des protéines antagonistes, des facteurs de transcription, des facteurs anti-angiogéniques, des protéines ou peptides antagonistes, des antagonistes de récepteurs, des anticorps, des anticorps monocaténaires et des molécules d'adhésion cellulaire.

35. Protéine de fusion suivant la revendication 34, dans laquelle la protéine thérapeutique est un anticorps ou un de ses fragments.

36. Protéine de fusion suivant la revendication 35, dans laquelle l'anticorps ou son fragment comprend au moins une CDR.

37. Protéine de fusion suivant la revendication 35, dans laquelle l'anticorps ou son fragment est un anticorps monocaténaire.

38. Protéine de fusion suivant la revendication 34, dans laquelle la protéine thérapeutique ou le peptide thérapeutique est une cytokine.

39. Protéine de fusion suivant la revendication 38, dans laquelle la cytokine est choisie dans le groupe consistant en la IL-2, la IL-3, la IL-4, la IL-5, la IL-6, la IL-7, la IL-10, la IL-12, la IL-13, la IL-14, la IL-15, la IL-16, la IL-17, la IL-18, le facteur de stimulation des colonies de granulocytes-macrophages, le facteur de stimulation des colonies de granulocytes, le facteur d'activation plaquettaire, l'érythropoïétine, le TNFα, la lymphotoxine α, la lymphotoxine β, la leukoréguline, le facteur inhibiteur de migration des macrophages, la neuroleukine, une leptine, le IFNα, le IFNβ, le IFNγ, le IFNo, la thrombospondine 1, THBS2, THBS3, THBS4 et une chimiokine.

40. Protéine de fusion suivant la revendication 39, dans laquelle la cytokine est le IFNβ.

41. Protéine de fusion suivant la revendication 34, dans laquelle la protéine thérapeutique ou le peptide thérapeutique est une hormone.

42. Protéine de fusion suivant la revendication 41, dans laquelle l'hormone est choisie dans le groupe consistant en la pro-insuline, l'insuline, l'hormone de croissance 1, l'hormone de croissance 2, le facteur de libération d'hormone de croissance, le facteur de croissance 1 analogue à l'insuline, le facteur de croissance 2 analogue à l'insuline, la protéine de liaison 1 au facteur de croissance analogue à l'insuline (IGFBP-1), la IGFBP-2, la IGFBP-3, la IGFBP-4, la IGFBP-5, la IGBP-6, la IGFBP-7, la chaîne β de la gonadotrophine chorionique, la chaîne α de la gonadotrophine chorionique, l'hormone lutéinisante β, la folliculostimuline β, la thyréostimuline β, la prolactine, la pro-opiomélanocortine, la corticotrophine (ACTH), la β-lipotrophine, l'hormone de stimulation des mélanocytes α (α-MSH), la γ-lipotrophine, la β-MSH, la β-endorphine et le peptide du lobe intermédiaire analogue à la corticotrophine (CLIP).

43. Protéine de fusion suivant la revendication 34, dans laquelle la protéine thérapeutique ou le peptide thérapeutique est un facteur de croissance.

44. Protéine de fusion suivant la revendication 43, dans laquelle le facteur de croissance est choisi dans le groupe consistant en le facteur de croissance α dérivé des plaquettes (PDGF-α), le PDGF-β, des hormones stéroïdiennes, le facteur de croissance épidermique (EGF), des facteurs de croissance des fibroblastes, tels que le facteur de croissance des fibroblastes 1 (FGF1), le FGF2, le FGF3, le FGF4, le FGF5, le FGF6, le FGF7, le FGF8, le FGF9, le FGF10, le FGF11, le FGF12, le FGF13, le FGF14, le FGF16, le FGF17, le FGF18, le FGF19, le FGF20, le FGF21, le FGF22, le FGF23, l'angiogénine, le facteur neurotrope dérivé du cerveau, le facteur de croissance neurotrope ciliaire, le facteur de croissance transformant α (TGF-α), le TGF-α, le facteur de croissance des nerfs α (NGF-α) le NGF-β, l'inhibiteur tissulaire de métalloprotéinase 1 (TIMP1), TIMP2, TIMP3, TIMP4 et le facteur de stimulation des macrophages 1.

45. Protéine de fusion suivant la revendication 34, dans laquelle la protéine thérapeutique ou le peptide thérapeutique est une protéine de matrice.

46. Protéine de fusion suivant la revendication 45, dans laquelle la protéine de matrice est choisie dans le groupe consistant en le collagène I, le collagène II, le collagène III, le collagène IV, le collagène V, le collagène VI, le collagène VII, le collagène VIII, le collagène IX, le collagène X, le collagène XI et le collagène XII, LAMA2, LAMA3, LAMA4, LAMB1, LAMB3, LAMC1, le nidogène, l'α-tectorine, la β-tectorine et la fibronectine.

47. Protéine de fusion suivant la revendication 34, dans laquelle la protéine thérapeutique ou le peptide thérapeutique est une protéine sanguine.

48. Protéine de fusion suivant la revendication 47, dans laquelle la protéine sanguine est choisie dans le groupe consistant en une protéine sérique native, un facteur de coagulation sanguine, l'antithrombine 3, l'antitrypsine alpha-1, l'activateur du plasminogène du type urokinase, une immunoglobuline, le facteur de von Willebrand, la fibronectine, le fibrinogène, la thrombine et l'hémoglobine.

49. Protéine de fusion suivant la revendication 34, dans laquelle la protéine thérapeutique ou le peptide thérapeutique est une enzyme.

50. Protéine de fusion suivant la revendication 49, dans laquelle l'enzyme est choisie dans le groupe consistant en un facteur de coagulation, une métalloprotéinase de matrice, l'adénosine-désaminase, une protéine-kinase activée par un agent mitogène, une kinase et une phosphatase.

51. Protéine de fusion suivant la revendication 34, dans laquelle la protéine thérapeutique ou le peptide thérapeutique est un facteur de transcription.

52. Protéine de fusion suivant la revendication 51, dans laquelle le facteur de transcription est choisi dans le groupe consistant en Sp1, Sp2, Sp3, Sp4, NFYB, Hap2, GATA-1, GATA-2, GATA-3, GATA-4, GATA-5, GATA-6, FOG2, Eryfl, TRPS1, NF-E2, NF-E3, NF-E4, TFCP2, Oct-1, HOXB2, HOX2H, un homologue du facteur de hairless, MADH1, MADH2, MADH3, MADH4, MADH5, MADH6, MADH7, MADH9, STAT1, STAT2, STAT3, STAT4, STAT5 et STAT6.

53. Protéine de fusion suivant la revendication 34, dans laquelle la protéine thérapeutique ou le peptide thérapeutique est une protéine associée au cancer.

54. Protéine de fusion suivant la revendication 53, dans laquelle la protéine associée au cancer est choisie dans le groupe consistant en p16, p53, p63, p73, BRCA1, BRCA2, la protéine interagissant avec CTBP, DMBT1, HRAS, NCYM, FGR, myb, raf1, erBé, VAV, c-fos, c-fes, c-jun, MAS1, pim-1, TIF1, c-fms, EGFR, erbA, la tyrosine-kinase c-src, c-abl, N-ras, K-ras, jun-B, c-myc, RB1, DCC, APC, NF1, NF2 et bcl-2.

55. Protéine de fusion suivant la revendication 34, dans laquelle la protéine thérapeutique ou le peptide thérapeutique est un peptide inhibiteur fusogénique.

56. Protéine de fusion suivant la revendication 55, dans laquelle le peptide inhibiteur fusogénique est choisi dans le groupe consistant en T-20 du HIV, T-1249 du HIV, T786 du RSV, T1584 du RSV et T112 du RSV.

57. Protéine de fusion suivant la revendication 56, qui est exprimée en présence de tunicamycine.

58. Protéine de fusion suivant les revendications 1 à 18, dans laquelle la protéine Tf est la lactotransferrine.

59. Protéine de fusion suivant la revendication 4 ou 5, dans laquelle la Tf comprend au moins une substitution, délétion ou addition d'aminoacide à un résidu d'aminoacide correspondant à un aminoacide choisi dans le groupe consistant en Asp 63, Gly 65, Asp 392, Tyr 95, Tyr 426, Tyr 118, Tyr 514, Tyr 517, His 249, His 585, Lys 206, His 207 et Arg 632.

60. Protéine de fusion suivant les revendications 1 à 18, destiné à être utilisée dans une méthode de traitement médical.

61. Protéine de fusion suivant l'une quelconque de revendications précédentes, dans laquelle la mutation est située dans un site de glycosylation lié par N comprenant la séquence N-X-S/T.

62. Protéine de fusion suivant la revendication 61, dans laquelle la séquence N-X-S/T débute à un aminoacide correspondant à N413 ou N611 de la SEQ ID N° 3.

63. Protéine de fusion suivant la revendication 61, dans laquelle la protéine Tf est dépourvue de glycosylation liée par N.

64. Protéine de fusion suivant la revendication 1, dans laquelle la protéine Tf est non glycosylée.

65. Protéine de fusion purifiée comprenant une protéine Tf comprenant une mutation dans un site de glycosylation N-X-S/T lié par N fusionnée à au moins une protéine thérapeutique ou un peptide thérapeutique, dans laquelle la protéine transferrine présente une glycosylation réduite comparativement à une protéine Tf totalement glycosylée, et dans laquelle la demi-vie sérique de la protéine thérapeutique ou du peptide thérapeutique est augmentée par rapport à la demi-vie sérique de la protéine thérapeutique ou du peptide thérapeutique à l'état non fusionné.

66. Protéine de fusion suivant la revendication 1, dans laquelle ladite protéine Tf est modifiée à S32 pour réduire la O-glycosylation.

67. Protéine de fusion suivant la revendication 1 ou 65, dans laquelle la protéine transferrine est la transferrine humaine.

68. Protéine de fusion suivant la revendication 67, dans laquelle la transferrine humaine comprend la SEQ ID N° 3.

69. Protéine de fusion suivant la revendication 1, 15 ou 65, dans laquelle la protéine transferrine est un variant d'épissage de transferrine.

70. Protéine de fusion suivant la revendication 1 ou 65, ladite protéine de fusion augmentant la stabilité in vitro de ladite au moins une protéine thérapeutique ou dudit au moins un peptide thérapeutique.

71. Protéine de fusion suivant la revendication 1 ou 65, ladite protéine de fusion augmentant la biodisponibilité de ladite au moins une protéine thérapeutique ou dudit au moins un peptide thérapeutique.

72. Protéine de fusion suivant la revendication 1, ladite protéine de fusion se liant au fer.

73. Protéine de fusion suivant la revendication 1, ladite protéine de fusion étant capable de traverser une barrière hémato-encéphalique.

74. Protéine de fusion suivant la revendication 1 ou 65, dans laquelle la protéine Tf est connectée à ladite au moins une protéine thérapeutique ou audit au moins un peptide thérapeutique par un peptide de liaison.

75. Protéine de fusion suivant la revendication 74, dans laquelle ledit peptide de liaison comprend moins de 50 résidus d'aminoacides.

76. Protéine de fusion suivant la revendication 74, dans laquelle ledit peptide de liaison comprend un segment polyglycine.

77. Protéine de fusion suivant la revendication 1 ou 65, dans laquelle ladite protéine de fusion de Tf présente une liaison de carbonate réduite.

78. Protéine de fusion suivant la revendication 1 ou 65, ladite protéine de fusion contenant une ou plusieurs substitutions, insertions ou délétions d'aminoacides, comparativement à une séquence de type sauvage, et n'est pas capable de traverser une barrière hémato-encéphalique.

79. Composition comprenant une protéine de fusion de l'une quelconque des revendications 1 à 18 et 34 à 78.

80. Utilisation d'une protéine transferrine présentant une glycosylation réduite pour augmenter la demi-vie sérique ou stabilité dans le sérum d'une protéine thérapeutique, dans laquelle ladite protéine transferrine est fusionnée à ladite protéine thérapeutique pour former une protéine de fusion suivant l'une quelconque des revendications 1 à 18 ou 34 à 78.
